(19) Europäisches Patentamt
European Patent Office
Office européen des brevets

(11) EP 1 424 392 B1

(12) **EUROPEAN PATENT SPECIFICATION**

(45) Date of publication and mention of the grant of the patent:
**24.10.2012 Bulletin 2012/43**

(21) Application number: **02762956.7**

(22) Date of filing: **02.09.2002**

(51) Int Cl.:
***C12N 15/12*** (2006.01) ***C07K 14/47*** (2006.01)
***A61K 38/17*** (2006.01) ***A61K 39/395*** (2006.01)
***A61P 25/00*** (2006.01) ***A61P 43/00*** (2006.01)
***C07K 16/18*** (2006.01) ***C12P 21/02*** (2006.01)
***G01N 33/50*** (2006.01) ***G01N 33/15*** (2006.01)

(86) International application number:
**PCT/JP2002/008872**

(87) International publication number:
**WO 2003/020932 (13.03.2003 Gazette 2003/11)**

(54) **RF-peptide ligands of G-protein coupled receptor**

RF-peptide als Ligande für G-Protein-gekoppelte Rezeptor

Peptides-RF comme ligands pour récepteurs couplés aux protéines G

(84) Designated Contracting States:
**AT BE BG CH CY CZ DE DK EE ES FI FR GB GR IE IT LI LU MC NL PT SE SK TR**

(30) Priority:
**03.09.2001 JP 2001265829**
**26.09.2001 JP 2001294586**
**20.12.2001 JP 2001388011**
**28.12.2001 JP 2001401948**
**24.01.2002 JP 2002015976**
**27.03.2002 JP 2002088383**

(43) Date of publication of application:
**02.06.2004 Bulletin 2004/23**

(73) Proprietor: **Takeda Pharmaceutical Company Limited**
**Osaka (JP)**

(72) Inventors:
- **HINUMA, Shuji**
**Tsukuba-shi, Ibaraki 305-0821 (JP)**
- **FUKUSUMI, Shoji**
**Tsukuba-shi, Ibaraki 305-0044 (JP)**
- **KOMATSU, Hidetoshi**
**Nihari-gun, Ibaraki 300-4117 (JP)**
- **FUJII, Ryo**
**Tsukuba-shi, Ibaraki 305-0821 (JP)**
- **KITADA, Chieko**
**Sakai-shi, Osaka 590-0073 (JP)**
- **HOSOYA, Masaki**
**Tsuchiura-shi, Ibaraki 300-0007 (JP)**
- **HABATA, Yugo**
**Tsukuba-shi, Ibaraki 305-0044 (JP)**
- **HARADA, Masataka**
**Tsukuba-shi, Ibaraki 305-0046 (JP)**
- **NOGUCHI, Yuko**
**Tsukuba-shi, Ibaraki 305-0051 (JP)**

(74) Representative: **Böhm, Brigitte et al**
**Weickmann & Weickmann**
**Patentanwälte**
**Postfach 86 08 20**
**81635 München (DE)**

(56) References cited:
**WO-A-01/16316** **WO-A1-00/29441**
**WO-A1-01/16313** **WO-A1-01/66134**
**WO-A2-02/20762**

- **DATABASE EMBL EBI; Mus musculus 0 day neonate thymus cDNA 24 August 2001 (2001-08-24), ARAKAWA T ET AL.: XP002296258 Database accession no. BB634210**
- **DATABASE EPO PROTEINS [Online] 15 January 2004 'Sequence 5686 from Patent EP1104808.' Retrieved from EBI, accession no. EPOP: AX974883 Database accession no. AX974883**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 424 392 B1

## Description

### FIELD OF THE INVENTION

The present invention relates to novel peptides, DNA thereof and uses thereof.

### BACKGROUND ART

In recent years, human genes come to be acceleratedly elucidated by rapid progress of gene analysis techniques and accumulation of sequence information by random sequencing of cDNA derived from human genomic DNA or various human tissues. The presence of many genes estimated to encode proteins of unknown functions is thereby revealed.
WOO1/16313 describes AQ27 that is an orphan G protein-conjugated receptor protein and its DNA.
WO00/29441 describes OT7T022 that is an orphan G protein-conjugated receptor protein and its DNA, and describes RFRP (RF-related peptide) as a ligand binding to OT7T022.
WO01/66134 describes that the RFRP has a regulatory action on prolactin secretion.
The present invention provides novel peptides, DNA thereof and uses thereof.

### DISCLOSURE OF THE INVENTION

In order to solve the foregoing problem, the inventors made extensive studies and as a result, successfully obtained 9 kinds of DNA encoding novel peptides containing a secretory signal, from human entire brains etc. and found that such DNA encodes a secretory peptide. Further, the inventors successively obtained 9 kinds of DNA encoding mouse- and rat-derived AQ27 receptors. Based on these findings, the inventors have made extensive studies and come to accomplish the present invention. The invention is defined by the claims.

### BRIEF DESCRIPTION OF THE DRAWINGS

Fig. 1 shows the DNA sequence of secretory protein A.
Fig. 2 shows the amino acid sequence of secretory protein A.
Fig. 3 shows the DNA sequence of secretory protein B.
Fig. 4 shows the amino acid sequence of secretory protein B.
Fig. 5 shows the DNA sequence of secretory protein C.
Fig. 6 shows the amino acid sequence of secretory protein C.
Fig. 7 shows the DNA sequence of secretory protein D.
Fig. 8 shows the amino acid sequence of secretory protein D.
Fig. 9 shows the DNA sequence of secretory protein E.
Fig. 10 shows the amino acid sequence of secretory protein E.
Fig. 11 shows the DNA sequence of secretory protein F.
Fig. 12 shows the amino acid sequence of secretory protein F.
Fig. 13 shows the DNA sequence of secretory protein G.
Fig. 14 shows the amino acid sequence of secretory protein G.
Fig. 15 shows comparison between the amino acid sequence of secretory protein F and that of secretory protein G.
Fig. 16 shows the activity (in terms of luciferase activity) of polypeptide F: Gly-Gly-Phe-Ser-Phe-Arg-Phe-$NH_2$ (positions 127 to 133 in SEQ ID NO: 23) and polypeptide F: Glu-His-Ala-Gly-Cys-Arg-Phe-Arg-Phe-$NH_2$ (positions 80 to 88 in SEQ ID NO: 23) in increasing the amount of the expressed reporter gene in HEK 293 cells not expressing human AQ27 receptor and human OT7T022 receptor. The ordinate shows luciferase activity (cps). The abscissa shows polypeptide concentration (μM). FSK indicates forskolin. EHAGCRFRF-$NH_2$ indicates a polypeptide Glu-His-Ala-Gly-Cys-Arg-Phe-Arg-Phe-$NH_2$ (positions 80 to 88 in SEQ ID NO: 23), and GGFSFRF indicates a polypeptide Gly-Gly-Fhe-Ser-Phe-Arg-Phe-$NH_2$ (positions 127 to 133 in SEQ ID NO: 23). The "base" indicates the case where the polypeptide was not added.
Fig. 17 shows the activity (in terms of luciferase activity) of polypeptide F: Gly-Gly-Phe-Ser-Phe-Arg-Phe-$NH_2$ (positions 127 to 133 in SEQ ID NO: 23) and polypeptide F: Glu-His-Ala-Gly-Cys-Arg-Phe-Arg-Phe-$NH_2$ (positions 80 to 88 in SEQ ID NO: 23) in increasing of the amount of the expressed reporter gene in HEK 293 cells transiently expressing human AQ27 receptor. The ordinate shows luciferase activity (cps). The abscissa shows polypeptide concentration (μM). FSK indicates forskolin. ERAGCRFRF-$NH_2$ indicates a polypeptide Glu-Ms-Ala-Gly-Cys-Arg-Phe-Arg-Phe-$NH_2$ (positions 80 to 88 in SEQ ID NO: 23), and GGFSFRF shows polypeptide Gly-Gly-Phe-Ser-Phe-

Arg-Phe-$NH_2$ (positions 127 to 133 in SEQ ID NO: 23). The "base" indicates the case where the polypeptide was not added.

Fig. 18 shows the activity (in terms of luciferase activity) of polypeptide F: Gly-Gly-Phe-Ser-Phe-Arg-Phe-$NH_2$ (positions 127 to 133 in SEQ ID NO: 23) and polypeptide F: Glu-His-Ala-Gly-Cys-Arg-Phe-Arg-Phe-$NH_2$ (positions 80 to 88 in SEQ ID NO: 23) in increasing the amount of the expressed reporter gene in HEK 293 cells transiently expressing human OT7T022 receptor. The ordinate shows luciferase activity (cps). The abscissa shows polypeptide concentration (μM). FSK indicates forskolin. EHAGCRFRF-$NH_2$ indicates a polypeptide Glu-His-Ala-Gly-Cys-Arg-Phe-Arg-Phe-$NH_2$ (positions 80 to 88 in SEQ ID NO: 23), and GGFSFRF indicates a polypeptide Gly-Gly-Phe-Ser-Phe-Arg-Phe-$NH_2$ (positions 127 to 133 in SEQ ID NO: 23). The "base" indicates the case where the polypeptide was not added.

Fig. 19 shows the DNA sequence of secretory protein H.

Fig. 20 shows the amino acid sequence of secretory protein H.

Fig. 21 shows the activity (in terms of luciferase activity) of polypeptide F: Arg-Lys-Lys-Gly-Gly-Phe-Ser-Phe-Arg-Phe-$NH_2$ (positions 124 to 133 in SEQ ID NO: 23) in increasing the amount of the expressed reporter gene in HEK 293 cells not expressing human AQ27 receptor and human OT7T022 receptor. The ordinate shows luciferase activity (cps). The abscissa shows polypeptide concentration (μM). FSK indicates forskolin. RKKGGFSFRF-$NH_2$ indicates a polypeptide Arg-Lys-Lys-Gly-Gly-Phe-Ser-Phe-Arg-Phe-$NH_2$ (positions 124 to 133 in SEQ ID NO: 23). The "base" indicates the case where the polypeptide was not added.

Fig. 22 shows the activity (in terms of luciferase activity) of polypeptide F: Arg-Lys-Lys-Gly-Gly-Phe-Ser-Phe-Arg-Phe-$NH_2$ (positions 124 to 133 in SEQ ID NO: 23) in increasing the amount of the expressed reporter gene in HEK 293 cells expressing human AQ27 receptor. The ordinate shows luciferase activity (cps). The abscissa shows polypeptide concentration (μM). FSK indicates forskolin. RKKGGFSFRF-$NH_2$ shows polypeptide Arg-Lys-Lys-Gly-Gly-Phe-Ser-Phe-Arg-Phe-$NH_2$ (positions 124 to 133 in SEQ ID NO: 23). The "base" indicates the case where the polypeptide was not added.

Fig. 23 shows the activity (in terms of luciferase activity) of polypeptide F: Arg-Lys-Lys-Gly-Gly-Phe-Ser-Phe-Arg-Phe-$NH_2$ (positions 124 to 133 in SEQ ID NO: 23) in increasing the amount of the expressed reporter gene in HEK 293 cells expressing human OT7T022 receptor. The ordinate shows luciferase activity (cps). The abscissa shows polypeptide concentration (μM). FSK indicates forskolin. RKKGGFSFRF-$NH_2$ indicates a polypeptide Arg-Lys-Lys-Gly-Gly-Phe-Ser-Phe-Arg-Phe-$NH_2$ (positions 124 to 133 in SEQ ID NO: 23). The "base" indicates the case where the polypeptide was not added.

Fig. 24 shows a cAMP production-inhibiting activity in AQ27-expressing CHO cells. Average activity (n = 2) is shown. The ordinate shows cAMP concentration (pmol). The abscissa shows polypeptide concentration in terms of log M. FSK- shows the case where forskolin was not added, while FSKs+ shows the case where forskolin was added. RKKGGFSFRF-$NH_2$ indicates a polypeptide F:
Arg-Lys-Lys-Gly-Gly-Phe-Ser-Phe-Arg-Phe-NH2 (positions 124 to 133 in SEQ ID NO: 23).

Fig. 25 shows a cAMP production-inhibiting activity in mock CHO cells. Average activity (n = 2) is shown. The ordinate shows cAMP concentration (pmol). The abscissa shows polypeptide concentration in terms of log M. FSK- shows the case where forskolin was not added, while FSK+ shows the case where forskolin was added. RKKGGFSFRF-$NH_2$ indicates a polypeptide F:
Arg-Lys-Lys-Gly-Gly-Phe-Ser-Phe-Arg-Phe-$NH_2$ (positions 124 to 133 in SEQ ID NO: 23).

Fig. 26 shows the promoting activity of GGFSFRF-$NH_2$ on release of intracellular calcium ions in AQ27-expressing CHO cells. Fluorescence on the ordinate shows fluorescence intensity (cps). The abscissa shows time (sec). GGFSFRF-$NH_2$ indicates a polypeptide F: Gly-Gly-Phe-Ser-Phe-Arg-Phe-$NH_2$ (positions 127 to 133 in SEQ ID NO: 23). ○, □ and ▲ show that the concentrations of GGFSRKF-$NH_2$ added are 0 μM, 1 μM and 10 μM. respectively.

Fig. 27 shows the promoting activity of RKKGGFSFRF-$NH_2$ on release of intracellular calcium ions in AQ27-expressing CHO cells. Fluorescence on the ordinate shows fluorescence intensity (cps). The abscissa shows time (sec). RKKGGFSFRF-$NH_2$ indicates a polypeptide F:
Arg-Lys-Lys-Gly-Gly-Phe-Ser-Phe-Arg-Phe-$NH_2$ (positions 124 to 133 in SEQ ID NO: 23). ○, □ and ▲ show that the concentrations of GGFSFRF-$NH_2$ added were 0 μM, 1 μM and 10 μM, respectively.

Fig. 28 shows the activity (in terms of luciferase activity) of polypeptides F:
Thr-Ser-Gly-Pro-C.eu-Gly-Asn-Leu-Ata-Glu-Glu-L,eu-Asn-Gly-Tyr-Ser-Arg-Lys-Lys-Gly-Gly-Phe-Ser-Phe-Arg-Phe-$NH_2$ and
Pyr-Asp-Glu-Gly-Ser-Glu-Ala-Thr-Gly-Phe-Leu-Pro-Ala-Ala-Gly-Glu-Lys-Thr-Ser-Gly-P ro-Leu-Gly-Arn-L.eu-Ala-Glu-Glu-L,eu-Asn-Gly-Tyr-Ser-Arg-Lys-Lys-Gly-Gly-Phe-Ser-P he Arg-Phe-$NH_2$ (positions 108 to 133 and positions 91 to 133 in SEQ ID NO: 23) in increasing the amount of the expressed reporter gene in HEK 293 cells not expressing human AQ27 receptor. The ordinate shows luciferase activity (cps). The abscissa shows polypeptide concentration (μM) in terms of log M. FSK- shows the case where forskolin was not added, while FSK+ shows the case where forskolin was added.

T1-F26-$NH_2$ indicates polypeptide F:
Thr-Ser-Gly-Pro-Leu-Gly-Asn-Leu-Ala-Glu-Glu-Leu-Asn-Gly-Tyr-Ser-Arg-Lys-Lys-Gly-Gly-Phe-Ser-Phe Arg-Phe-$NH_2$ (positions 108 to 133 in SEQ ID NO: 23), and
Pyrl-F43-$NH_2$ indicates polypeptide F:
Pyr-Asp-Glu-Gly-Ser-Glu-Ala-Thr-Gly-Phe-Leu-Pro-Ala-Ala-Gly-Glu-Lys-Thr-Ser-Gly-P ro-Leu-Gly-Asn-Leu-Ala-Glu-Glu-Leu-Asn-Gly-Tyr-Ser-Arg-Lys-Lys-Gly-Gly-Phe-Ser-P he Arg-Phe-$NH_2$ (positions 91 to 133 in SEQ ID NO: 23). Pyr indicates cyclic pyroglutaminated glutamine.

Fig. 29 shows the activity (in terms of luciferase activity) of polypeptides F:
Thr-Ser-Gly-Pro-Leu-Gly-Asn-Leu-Ala-Glu-Glu-Leu-Asn-Gly-Tyr-Ser-Arg-Lys-Lys-Gly-Gly-Phe-Ser-Phe-Arg-Phe-$NH_2$ and
Pyr-Asp-Glu-Gly-Ser-Glu-Ala-Thr-Gly-Phe-Leu-Pro-Ala-Ala-Gly-Glu-Lys-Thr-Ser-Gly-P ro-Leu-Gly-Asn-Leu-Ala-Glu-Glu-Leu-Asn-Gly-Tyr-Ser-Arg-Lys-Lys-Gly-Gly-Phe-Ser-P he Arg-Phe-$NH_2$ (positions 108 to 133 and positions 91 to 133 in SEQ ID NO: 23) in increasing the amount of the expressed reporter gene in HEK 293 cells expressing human AQ27 receptor. The ordinate shows luciferase activity (cps). The abscissa shows polypeptide concentration in terms of log M. FSK- shows the case where forskolin was not added, while FSK+ shows the case where forskolin was added. T1-F26-$NH_2$ indicates polypeptide F:
Thr-Ser-Gly-Pro-Leu-Gly-Asn-Leu-Ala-Glu-Glu-Leu-Asn-Gly-Tyr-Ser-Arg-Lys-Lys-Gly-Gly-Phe-Ser-Phe Arg-Phe-$NH_2$ (positions 108 to 133 in SEQ ID NO: 23), and
Pyr1-F43-$NH_2$ indicates polypeptide F:
Pyr-Asp-Glu-Gly-Ser-Glu-Ala-Thr-Gly-Phe-Leu-Pro-Ala-Ala-Gly-Glu-Lys-Thr-Ser-Gly-P ro-Leu-Gly-Asn-Leu-Ala-Glu-Glu-Leu-Asn-Gly-Tyr-Ser-Arg-Lys-Lys-Gly-Gly-Phe-Ser-P he Arg-Phe-$NH_2$ (positions 91 to 133 in SEQ ID NO: 23). Pyr indicates cyclic pyroglutaminated glutamine.

Fig. 30 shows the activity of polypeptide Pyrl-F43-NH= in mobilizing intracellular calcium ions in AQ27-expressing CHO cells. Fluorescence on the ordinate shows fluorescence intensity (cps). The abscissa shows time (sec). □ shows the case in the absence of the peptide, and ■ △, ▲, ○, ● and ◊ show that the concentrations of the peptide added are 5, 6, 7, 8, 9 and 10 respectively in terms of log M.

Fig. 31 shows the result of examination of the influence of polypeptide Pyr1-F43-$NH_2$ on change in the concentration of intracellular calcium ions in mock CHO cells. Fluorescence on the ordinate shows fluorescence intensity (cps). The abscissa shows time (sec). □ shows the case in the absence of the peptide, and ■ △, ▲, ○, ● and ◊ show that the concentrations of the peptide added are 5, 6, 7, 8, 9 and 10 respectively in terms of log M.

Fig. 32 shows the activity of polypeptide Pyrl-F43-$NH_2$ in inhibiting production of cAMP in AQ27-expressing CHO cells. Average activity (n = 3) is shown. Inhibition on the ordinate indicates the degree (%) of inhibition of cAMP production. Conc. on the abscissa shows the concentration of the added peptide in terms of log M.

Fig. 33 shows the result of examination of the influence of polypeptide Pyrl-F43-$NH_2$ on cAMP production-inhibiting activity in mock CHO. Average activity (n = 3) is shown. Inhibition on the ordinate indicates the degree (%) of inhibition of cAMP production. Conc. on the abscissa indicates the concentration of the added peptide in terms of log M.

Fig. 34 shows the activity (in terms of luciferase activity) of polypeptide F:
Ala-Ser-Gln-Pro-Gln-Ala-Leu-Leu-Val-Ile-Ala-Arg-Gly-Leu-Gln-Thr-Ser-Gly-Arg-Glu-H is-Ala-Gly-Cys-Arg-Phe-$NH_2$ (positions 61 to 86 in SEQ ID NO: 23) in increasing the amount of the expressed reporter gene in HEK 293 cells not expressing human AQ27 receptor. The ordinate shows luciferase activity (cps). The abscissa shows polypeptide concentration in terms of log M. FSK- shows the case where forskolin was not added, while FSK+ shows the case where forskolin was added. A1-F28-$NH_2$ indicates polypeptide F:
Ala-Ser-Gln-Pro-Gln-Ala-Leu-Leu-Val-Ile-Ala-Arg-Gly-Leu-Gln-Thr-Ser-Gly-Arg-Glu-H is-Ala-Gly-Cys-Arg-Phe-$NH_2$ (positions 61 to 86 in SEQ ID NO: 23).

Fig. 35 shows the activity (in terms of luciferase activity) of polypeptide F:
Ala-Ser-Gln-Pro-Gln-Ala-Leu-Leu-Val-Ile-Ala-Arg-Gly-Leu-Gln-Thr-Ser-Gly-Arg-Glu-H is-Ala-Gly-Cys-Arg-Phe-$NH_2$ (positions 61 to 86 in SEQ ID NO: 23) in increasing the amount of the expressed reporter gene in HEK 293 cells expressing human AQ27 receptor. Fluorescence on the ordinate shows luciferase activity (cps), and the abscissa shows polypeptide concentration in terms of log M. FSK- shows the case where forskolin was not added, while FSK+ shows the case where forskolin was added. A1-F28-$NH_2$ indicates polypeptide F:
Ala-Ser-Gln-Pro-Gln-Ala-Leu-Leu-Val-De-Ala-Arg-Gly-Leu-Gln-Thr-Ser-Gly-Arg-Glu-H is-Ala-Gly-Cys-Arg-Phe-$NH_2$ (positions 61 to 86 in SEQ m NO:23).

Fig. 36 shows the activity of polypeptide A1-F28-$NH_2$ in mobilizing intracellular calcium ions in AQ27-expressing CHO cells. Fluorescence on the ordinate shows fluorescence intensity (cps). The abscissa shows time (sec). * shows the case in the absence of the peptide, and ■, ●, ○, ▲, ♦ and ◊ show that the concentrations of the peptide added are 5, 6, 7, 8, 9 and 10 respectively in terms of log M.

Fig. 37 shows the result of examination of the influence of polypeptide A1-F28-$NH_2$ on change in the concentration of intracellular calcium ions in mock CHO cells. Fluorescence on the ordinate shows fluorescence intensity (cps).

The abscissa shows time (sec). * shows the case in the absence of the peptide, and ■, ●, ○, ▲, ♦ and ◊ show that the concentrations of the peptide added are 5, 6, 7, 8, 9 and 10 respectively in terms of log M.

Fig. 38 shows the inhibitory activity of polypeptide Al-F28-$NH_2$ on production of cAMP in CHO cells expressing AQ27. Average activity (n = 2) is shown. Inhibition on the ordinate shows the degree (%) of inhibition of cAMP production. Conc. on the abscissa shows the concentration of the added peptide in terms of log M.

Fig. 39 shows the result of examination of the influence of polypeptide A1-F28-$NH_2$ on cAMP production-inhibiting activity in mock CHO cells. Average activity (n = 2) is shown. Inhibition on the ordinate shows the degree (%) of inhibition of cAMP production. Conc. on the abscissa shows the concentration of the added peptide in terms of log M.

Fig. 40 shows the DNA sequence of secretory protein I.

Fig. 41 shows the amino acid sequence of secretory protein I.

Fig. 42 shows the nucleotide sequence of rat AQ27 receptor.

Fig. 43 shows the amino acid sequence of rat AQ27 receptor.

Fig. 44 shows the nucleotide sequence of mouse AQ27 receptor.

Fig. 45-shows the amino acid sequence of mouse AQ27 receptor.

Fig. 46 shows homology in amino acid sequence among human AQ27 receptor (A), rat AQ27 receptor (B) and mouse AQ27 receptor (C).

Fig. 47 shows the distribution of AQ27 receptor mRNA in human tissues.

Fig. 48 shows the distribution of AQ27 receptor mRNA in rat tissues. In the Fig., "i" indicates infant.

Fig. 49 shows the distribution of secretory protein G mRNA in rat tissues.

Fig. 50 shows the distribution of secretory protein F mRNA in human tissues.

Fig. 51 shows the distribution of AQ27 mRNA in a rat brain by the in situ hybridization method. As signal relative density, +++ indicates the highest density, ++ indicates moderate density, and + indicates low density.

Fig. 52 shows the result of detection of specific stimulating activity when a supernatant of CHO cells into which secretory protein F gene had been introduced or a supernatant of CHO cells into which secretory protein F gene had not been introduced was brought into contact with cells (AQ27/HEK) expressing AQ27 receptor or cells (pAKKO/HEK) not expressing AQ27 receptor. The ordinate shows the amount of luciferase expressed (cps). The abscissa shows the concentration or dilution of a substance reacted with AQ27 receptor. In "Basal", no substance was added. FSK shows the case where forskolin was added. Peptide (1) shows the case where peptide F: Glu-His-Ala-Gly-Cys-Arg-Phe-Arg-Phe-$NH_2$ (positions 80 to 88 in SEQ ID NO: 23) was added, and peptide (2) shows the case where polypeptide F:

Gly-Gly-Phe-Ser-Phe-Arg-Phe-$NH_2$ (positions 127 to 133 in SEQ ID NO: 23) was added. mock SUP. shows the case where a supernatant of CHO cells into which secretory protein F gene had not been introduced was added. Peptide F SUP. shows the case where a supernatant of CHO cells into which secretory protein F gene had been introduced was added.

Fig. 53 shows a curve of reaction of known anti-RFRP-1 antibody 1F3-1 with secretory protein F. $B/B_0$ (%) on the ordinate shows the ratio of the binding amount in the presence of the sample to the binding amount in the absence of the sample, and Peptide conc. on the abscissa shows the concentration of secretory protein F in terms of log M.

Fig. 54 shows the specific stimulating activity of a fraction bound in affinity chromatography using known anti-RFRP-1 antibody IF3-1 on cells (AQ27) expressing AQ27 receptor or cells (pAKKO) not expressing AQ27 receptor. Luciferase activity on the abscissa shows luciferase activity (cps). The abscissa shows bound fraction number in affinity chromatography.

Fig. 55 shows the specific stimulating activity of each fraction upon separation of fraction 20 (fr. 20) in Fig. 54, on cells expressing AQ27 receptor. Luciferase activity on the abscissa [sic] shows luciferase activity (cps). The abscissa shows fraction numbers. 3 and 4 indicate activity peaks.

Fig. 56 shows the specific stimulating activity of each fraction upon separation of fraction 20-13 (fr. 20-23) in Fig. 54, on cells expressing AQ27 receptor. Luciferase activity on the abscissa [sic] shows luciferase activity (cps). The abscissa shows fraction numbers. 1 and 2 indicate activity peaks.

Fig. 57 shows the experimental and theoretical molecular weights and amino acid sequences of peptide F contained in activity peaks I to 4 in Figs. 55 and 56.

Fig. 58 shows the cAMP production-inhibiting activity ($EC_{50}$) of the amino acid sequences of peptide F different in length and various kinds of peptide F on a CHO cell strain expressing AQ27.

Fig. 59 shows the degree of inhibition, by peptide F shown in Fig. 58, of cAMP production in a CHO cell strain expressing AQ27. Inhibition (%) on the ordinate indicates the degree (%) of inhibition of cAMP production. Concentration (log M) on the abscissa indicates the concentration of added peptide F in terms of log M.

## BEST MODE FOR CARRYING OUT THE INVENTION

(1) A secretory protein having the same or substantially the same amino acid sequence as the amino acid sequence represented by SEQ ID NO: 1 (hereinafter, the above secretory protein is sometimes referred to as secretory protein A of the present application),
(2) A secretory protein having the same or substantially the same amino acid sequence as an amino acid sequence in positions 22 to 112 of the amino acid sequence represented by SEQ ID NO: 3 (hereinafter, the above secretary protein is sometimes referred to as secretory protein B of the present application),
(3) A secretory protein having the same or substantially the same amino acid sequence as an amino acid sequence in positions 15 to 234 of the amino acid sequence represented by SEQ ID NO: 4 (hereinafter, the above secretory protein is sometimes referred to as secretory protein C of the present application),
(4) A secretory protein having the same or substantially the same amino acid sequence as an amino acid sequence in positions 25 to 166 of the amino acid sequence represented by SEQ ID NO: 5 (hereinafter, the above secretory protein is sometimes referred to as secretory protein D of the present application),
(5) A secretory protein having the same or substantially the same amino acid sequence as an amino acid sequence in positions 19 to 241 of the amino acid sequence represented by SEQ ID NO: 6 (hereinafter, the above secretory protein is sometimes referred to as secretory protein E of the present application),
(6) A secretory protein having the same or substantially the same amino acid sequence as an amino acid sequence in positions 19 to 136 of the amino acid sequence represented by SEQ ID NO: 23 (hereinafter, the above secretory protein is sometimes referred to as secretory protein F of the present application),
(7) A secretory protein having the same or substantially the same amino acid sequence as an amino acid sequence in positions 18 to 124 of the amino acid sequence represented by SEQ ID NO: 27 (hereinafter, the above secretory protein is sometimes referred to as secretory protein G of the present application),
(8) A secretory protein having the same or substantially the same amino acid sequence as an amino acid sequence in positions 18 to 124 of the amino acid sequence represented by SEQ ID NO: 38 (hereinafter, the above secretory protein is sometimes referred to as secretory protein H of the present application), or
(9) A secretory protein having the same or substantially the same amino acid sequence as an amino acid sequence in positions 18 to 134 of the amino acid sequence represented by SEQ ID NO: 42 (hereinafter, the above secretory protein is sometimes referred to as secretory peptide I of the present application) may be any peptide derived from any cells of human and other warm-blooded animals (e.g., guinea pig, rat, mouse, chicken, rabbit, swine, sheep, bovine, monkey, etc.) such as retina cells, liver cells, splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophils, basophil, eosinophils, monocytes), megakaryocyte, synovial cells, chondrocytes, bone cells, osteoblasts, osteoclasts, mammary gland cells, hepatocytes or interstitial cells, the corresponding precursor cells, stem cells, cancer cells, etc., or any tissues where such cells are present, such as brain or any of brain regions (e.g., retina, olfactory bulb, amygdaloid nucleus, basal ganglia, hippocampus, thalamus, hypothalamus, cerebral cortex, medulla oblongata, cerebellum), spinal cord, pituitary, stomach, pancreas, kidney, liver, gonad, thyroid, gall-bladder, bone marrow, adrenal gland, skin, muscle, lung, digestive tract (e.g., large intestine and small intestine), blood vessels, heart, thymus, spleen, submandibular gland, peripheral blood, prostate, testis, ovary, placenta, uterus, borne, joint, skeletal muscle, etc., or blood cells or cultured cells thereof (for example, MEL, M1, CTLL-2, HT-2, WEHI-3, HL-60, JOSK-1. K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); the secretory protein may also be a synthetic peptide.

In the present specification, amino acid sequence X refers to one amino acid sequence selected from the following 9 amino acid sequences:

(A) the amino acid sequence represented by SEQ ID NO: 1,
(B) an amino acid sequence in positions 22 to 112 of the amino acid sequence represented by SEQ ID NO: 3,

(C) an amino acid sequence in positions 15 to 234 of the amino acid sequence represented by SEQ ID NO: 4,
(D) an amino acid sequence in positions 25 to 166 of the amino acid sequence represented by SEQ ID NO: 5,
(E) an amino acid sequence in positions 19 to 241 of the amino acid sequence represented by SEQ ID NO: 6,
(F) an amino acid sequence in positions 19 to 136 of the amino acid sequence represented by SEQ ID NO: 23,
(G) an amino acid sequence in positions 18 to 124 of the amino acid sequence represented by SEQ ID NO: 27,
(H) an amino acid sequence positions in 18 to 124 of the amino acid sequence represented by SEQ ID NO: 38, and
(I) an amino acid sequence in positions 18 to 134 of the amino acid sequence represented by SEQ ID NO: 42.

Substantially the same amino acid sequence as amino acid sequence X includes nucleotide sequences having at least about 50% homology, preferably at least about 60% homology, more preferably at least about 70% homology, still more preferably at least about 80% homology, further still more preferably at least about 90% homology and most preferably at least about 95% homology, to amino acid sequence X. Specifically, the amino acid sequence having at least 60% homology to the amino acid sequence in positions 19 to 136 of the amino acid sequence represented by SEQ ID NO: 23 includes an amino acid sequence in positions 18 to 124 of the amino acid sequence represented by SEQ ID NO: 27, an amino acid sequence in positions 18 to 124 of the amino acid sequence represented by SEQ ID NO: 38, and an amino acid sequence in positions 18 to 134 of the amino acid sequence represented by SEQ ID NO: 42.

For example, the peptide having substantially the same amino acid sequence as amino acid sequence X is preferably a peptide having substantially the same amino acid sequence as amino acid sequence X and a substantially equivalent activity to that of the peptide having amino acid sequence X.

The substantially equivalent activity has, for example, the activity of the peptide of the present application (e.g., a receptor-binding activity, a cell-stimulating activity that stimulates receptor-expressing cells (e.g., the activity that promotes arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, GTPγS binding activity, etc.), enzyme activity, transcription activity, binding activity to a binding protein, etc.).

The terms "substantially equivalent" mean that the activity is inherently (e.g. physicochemically or pharmacologically) equivalent.

Specific examples of the same or substantially the same amino acid sequence as amino acid sequence X include:

(i) amino acid sequence X,
(ii) an amino acid sequence wherein in amino acid sequence X, 1 to 30 (e.g. 1 to 10, preferably 1 to 5, more preferably 1 to 3, still more preferably 1) amino acids were deleted,
(iii) an amino acid sequence wherein in amino acid sequence X, 1 to 30 (e.g. 1 to 10, preferably 1 to 5, more preferably 1 to 3, still more preferably 1) amino acids were added,
(iv) an amino acid sequence wherein in amino acid sequence X, 1 to 30 (e.g. 1 to 10, preferably 1 to 5, more preferably 1 to 3, still more preferably 1) amino acids were inserted,
(v) an amino acid sequence wherein in amino acid sequence X, 1 to 30 (e.g. 1 to 10, preferably 1 to **5,** more preferably 1 to 3, still more preferably 1) amino acids were replaced by other amino acids, and
(vi) an amino acid sequence wherein the above-mentioned (ii) to (v), were combined.

Specific examples of the peptide of the present application include:

(1) secretory protein A consisting of the amino acid sequence represented by SEQ ID NO: 1,
(2) secretory protein B consisting of an amino acid sequence in positions 22 to 112 of the amino acid sequence represented by SEQ ID NO: 3,
(3) secretory protein C consisting of an amino acid sequence in positions 15 to 234 of the amino acid sequence represented by SEQ ID NO: 4,
(4) secretory protein D consisting of an amino acid sequence in positions 25 to 166 of the amino acid sequence represented by SEQ ID NO: 5,
(5) secretory protein E consisting of an amino acid sequence in positions 19 to 241 of the amino acid sequence represented by SEQ ID NO: 6,
(6) secretory protein F consisting of an amino acid sequence in positions 19 to 136 of the amino acid sequence represented by SEQ ID NO: 23,
(7) secretory protein G consisting of an amino acid sequence in positions 18 to 124 of the amino acid sequence represented by SEQ ID NO: 27,
(8) secretory protein H consisting of an amino acid sequence in positions 18 to 124 of the amino acid sequence represented by SEQ ID NO: 38, and
(9) secretory protein I consisting of an amino acid sequence in positions 18 to 134 of the amino acid sequence represented by SEQ ID NO: 42.

In the present specification, the secretory protein A, secretory protein B, secretory protein C, secretory protein D, secretory protein E, secretory protein F, secretory protein G, secretory protein H and secretory protein I are sometimes collectively referred to as the peptide of the present application.

(1) A peptide having the same or substantially the same amino acid sequence as an amino acid sequence in positions 22 to 37 or positions 40 to 55 of the amino acid sequence represented by SEQ ID NO: 3 (hereinafter, the above peptide is sometimes referred to as peptide B of the present application),

(2) A peptide having the same or substantially the same amino acid sequence as an amino acid sequence in positions 15 to 21, an amino acid sequence in positions 26 to 58, an amino acid sequence in positions 60 to 88, an amino acid sequence in positions 90 to 107, an amino acid sequence in positions 110 to 187 or an amino acid sequence in positions 190 to 234 of the amino acid sequence represented by SEQ ID NO: 4 (hereinafter, the above peptide is sometimes referred to as peptide C of the present application),
(3) A peptide having the same or substantially the same amino acid sequence as an amino acid sequence in positions 25 to 85, an amino acid sequence in positions 86 to 114 or an amino acid sequence in positions 115 to 166 of the amino acid sequence represented by SEQ ID NO: 5 (hereinafter, the above peptide is sometimes referred to as peptide D of the present application),
(4) A peptide having the same or substantially the same amino acid sequence as an amino acid sequence in positions 19 to 58, an amino acid sequence in positions 63 to 202 or an amino acid sequence in positions 205 to 241 of the amino acid sequence represented by SEQ ID NO: 6 (hereinafter, the above peptide is sometimes referred to as peptide E of the present application),
(5) A peptide having the same or substantially the same amino acid sequence as an amino acid sequence in positions 26 to 88, an amino acid sequence in positions 80 to 88, an amino acid sequence in positions 91 to 133 or an amino acid sequence in positions 127 to 133 of the amino acid sequence represented by SEQ ID NO: 23 (hereinafter, the above peptide is sometimes referred to as peptide F of the present application).
(6) A peptide having the same or substantially the same amino acid sequence as the amino acid sequence in positions 115 to 122 or an amino acid sequence in positions 116 to 122 of the amino acid sequence represented by SEQ ID NO: 27 (hereinafter, the above peptide is sometimes referred to as peptide G of the present application),
(7) A peptide having the same or substantially the same amino acid sequence as the amino acid sequence in positions 115 to 122 or an amino acid sequence in positions 116 to 122 of the amino acid sequence represented by SEQ ID NO: 38 (hereinafter, the above peptide is sometimes referred to as peptide H of the present application), or
(8) A peptide having the same or substantially the same amino acid sequence as an amino acid sequence in positions 124 to 131 or positions 125 to 131 in the amino acid sequence represented by SEQ ID NO: 42 (hereinafter, the above peptide is sometimes referred to as peptide I of the present application) may be any peptide derived from any cells of human and other warm-blooded animals (e.g., guinea pig, rat, mouse, chicken, rabbit, swine, sheep, bovine, monkey, etc.) such as retina cells, liver cells, splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophils, basophils, eosinophils, monocytes), megakaryocyte, synovial cells, chondrocytes, bone cells, osteoblasts, osteoclasts, mammary gland cells, hepatocytes or interstitial cells, the corresponding precursor cells, stem cells, cancer cells, etc., or any tissues where such cells are present, such as brain or any of brain regions (e.g., retina, olfactory bulb, amygdaloid nucleus, basal ganglia, hippocampus, thalamus, hypothalamus, cerebral cortex, medulla oblongata, cerebellum), spinal cord, pituitary, stomach, pancreas, kidney, liver, gonad, thyroid, gall-bladder, bone marrow, adrenal gland, skin, muscle, lung, digestive tract (e.g., large intestine and small intestine), blood vessels, heart, thymus, spleen, submandibular gland, peripheral blood, prostate, testis, ovary, placenta, uterus, bone, joint, skeletal muscle, etc., or blood cells or cultured cells thereof (for example, MEL, M1, Cell-2. HT-2, WEHI-3, H-60, JOSK-1, K562, ML-1, MOLT-3, MOULT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); the peptide may also be a synthetic peptide.

In the present specification, amino acid sequence Y refers to one amino acid sequence selected from the following 25 amino acid sequences:

(1)

[1] an amino acid sequence in positions 22 to 37 of the amino acid sequence represented by SEQ ID NO: 3,
[2] an amino acid sequence in positions 40 to 55 of the amino acid sequence represented by SEQ ID NO: 3,

(2)

[1] an amino acid sequence in positions 15 to 21 of the amino acid sequence represented by SEQ ID NO: 4,
[2] an amino acid sequence in positions 26 to 58 of the amino acid sequence represented by SEQ ID NO: 4,
[3] an amino acid sequence in positions 60 to 88 of the amino acid sequence represented by SEQ ID NO: 4,
[4] an amino acid sequence in positions 90 to 107 of the amino acid sequence represented by SEQ ID NO: 4,
[5] an amino acid sequence in positions 110 to 187 of the amino acid sequence represented by SEQ ID NO: 4,
[6] an amino acid sequence in positions 190 to 234 of the amino acid sequence represented by SEQ ID NO: 4,

(3)

[1] an amino acid sequence in positions 25 to 85 of the amino acid sequence represented by SEQ ID NO: 5,
[2] an amino acid sequence in positions 86 to 114 of the amino acid sequence represented by SEQ ID NO: 5,
[3] an amino acid sequence in positions 115 to 166 of the amino acid sequence represented by SEQ ID NO: 5,

(4)

[1] an amino acid sequence in positions 19 to 58 of the amino acid sequence represented by SEQ ID NO: 6,
[2] an amino acid sequence in positions 63 to 202 of the amino acid sequence represented by SEQ ID NO: 6,
[3] an amino acid sequence in positions 205 to 241 of the amino acid sequence represented by SEQ ID NO: 6,

(5)

[1] an amino acid sequence in positions 26 to 88 of the amino acid sequence represented by SEQ ID NO: 23,
[2] an amino acid sequence in positions 80 to 88 of the amino acid sequence represented by SEQ ID NO: 23;
[3] an amino acid sequence in positions 91 to 133 of the amino acid sequence represented by SEQ ID NO: 23,
[4] an amino acid sequence in positions 127 to 133 of the amino acid sequence represented by SEQ ID NO: 23,

(6)

[1] an amino acid sequence in positions 115 to 122 of the amino acid sequence represented by SEQ ID NO: 27,
[2] an amino acid sequence in positions 116 to 122 of the amino acid sequence represented by SEQ ID NO: 27,

(7)

[1] an amino acid sequence in positions 115 to 122 of the amino acid sequence represented by SEQ ID NO: 38,
[2] an amino acid sequence in positions 116 to 122 of the amino acid sequence represented by SEQ ID NO: 38,

(8)

[1] an amino acid sequence in positions 124 to 134 of the amino acid sequence represented by SEQ ID NO: 42, and
[2] an amino acid sequence in positions 124 to 134 of the amino acid sequence represented by SEQ ID NO: 42.

Substantially the same amino acid sequence as amino acid sequence Y includes amino acid sequences having at least about 70% homology, preferably at least about 80% homology, more preferably at least about 90% homology, most preferably at least about 95% homology, to amino acid sequence Y.

For example, the peptide having substantially the same amino acid sequence as amino acid sequence.Y is preferably a peptide has substantially the same amino acid sequence as amino acid sequence Y and a substantially equivalent activity to that of the peptide having amino acid sequence Y.

The substantially equivalent activity having, for example, the activity of the peptide of the present application (e.g., a receptor-binding activity, a cell-stimulating activity that stimulates receptor-expressing cells (e.g., the activity that promotes arachidonic acid release, acetylcholine release, intracellular $Ca^{2+}$ release, intracellular cAMP production, intracellular cGMP production, inositol phosphate production, change in cell membrane potential, phosphorylation.of intracellular proteins, activation of c-fos, pH reduction, . GTPγS binding activity, etc.), enzyme activity, transcription activity, binding activity to a binding protein, etc.).

The terms "substantially equivalent" mean that the activity is inherently (e.g. physicochemically or pharmacologically) equivalent.

Specific examples of the same or substantially the same amino acid sequence as amino acid sequence Y include:

(i) amino acid sequence Y,
(ii) an amino acid sequence wherein in amino acid sequence Y, 1 to 5 (preferably 1 to 3, more preferably 1 to 2, still more preferably 1) amino acids were deleted,
(iii) an amino acid sequence wherein in amino acid sequence Y, 1 to 5 (preferably 1 to 3, more preferably 1 to 2, still more preferably 1) amino acids were added,
(iv) an amino acid sequence wherein in amino acid sequence Y, 1 to 5 (preferably I to 3, more preferably 1 to 2, still more preferably 1) amino acids were inserted,

(v) an amino acid sequence wherein in amino acid sequence Y, 1 to 10 (preferably 1 to 5, more preferably 1 to 3, still more preferably 1) amino acids were replaced by other amino acids, and
(vi) an amino acid sequence wherein the above-mentioned (ii) to (v) were combined.

Specific examples of the peptide of the present application include:

(1) peptide B consisting of an amino acid sequence in positions 22 to 37 or an amino acid sequence in positions 40 to 55 of the amino acid sequence represented by SEQ ID NO: 3,
(2) peptide C consisting of an amino acid sequence in positions 15 to 21, an amino acid sequence in positions 26 to 58, an amino acid sequence in positions 60 to 88, an amino acid sequence in positions 90 to 107, an amino acid sequence in positions 110 to 187 or an amino acid sequence in positions 190 to 234 of the amino acid sequence represented by SEQ ID NO: 4,
(3) peptide D consisting of an amino acid sequence in positions 25 to 85, an amino acid sequence in positions 86 to 114 or an amino acid sequence in positions 115 to 166 of the amino acid sequence represented by SEQ ID NO: 5,
(4) peptide E consisting of an amino acid sequence in positions 19 to 58, an amino acid sequence in positions 63 to 202 or an amino acid sequence in positions 205 to 241 of the amino acid sequence represented by SEQ ID NO: 6,
(5) peptide F consisting of an amino acid sequence in positions 19 to 88, an amino acid sequence in positions 20 to 88, an amino acid sequence in positions 21 to 88, an amino acid sequence in positions 22 to 88, an amino acid sequence in positions 23 to 88, an amino acid sequence in positions 24 to 88, an amino acid sequence in positions 25 to 88, an amino acid sequence in positions 26 to 88, an amino acid sequence in positions 27 to 88, an amino acid sequence in positions 28 to 88, an amino acid sequence in positions 29 to 88, an amino acid sequence in positions 30 to 88, an amino acid sequence in positions 31 to 88, an amino acid sequence in positions 32 to 88, an amino acid sequence in positions 33 to 88, an amino acid sequence in positions 34 to 88, an amino acid sequence in positions 35 to 88, an amino acid sequence in positions 36 to 88, an amino acid sequence in positions 37 to 88, an amino acid sequence in positions 38 to 88, an amino acid sequence in positions 39 to 88, an amino acid sequence in positions 40 to 88, an amino acid sequence in positions 41 to 88, an amino acid sequence in positions 42 to 88, an amino acid sequence in positions 43 to 88, an amino acid sequence in positions 44 to 88, an amino acid sequence in positions 45 to 88, an amino acid sequence in positions 46 to 88, an amino acid sequence in positions 47 to 88, an amino acid sequence in positions 48 to 88, an amino acid sequence in positions 49 to 88, an amino acid sequence in positions 50 to 88, an amino acid sequence in positions 51 to 88, an amino acid sequence in positions 52 to 88, an amino acid sequence in positions 53 to 88, an amino acid sequence in positions 54 to 88, an amino acid sequence in positions 55 to 88, an amino acid sequence in positions 56 to 88, an amino acid sequence in positions 57 to 88, an amino acid sequence in positions 58 to 88, an amino acid sequence in positions 59 to 88, an amino acid sequence in positions 60 to 88, an amino acid sequence in positions 61 to 88, an amino acid sequence in positions 62 to 88, an amino acid sequence in positions 63 to 88, an amino acid sequence in positions 64 to 88, an amino acid sequence in positions 65 to 88, an amino acid sequence in positions 66 to 88, an amino acid sequence in positions 67 to 88, an amino acid sequence in positions 68 to 88, an amino acid sequence in positions 69 to 88, an amino acid sequence in positions 70 to 88, an amino acid sequence in positions 71 to 88, an amino acid sequence in positions 72 to 88, an amino acid sequence in positions 73 to 88, an amino acid sequence in positions 74 to 88, an amino acid sequence in positions 75 to 88, an amino acid sequence in positions 76 to 88, an amino acid sequence in positions 77 to 88, an amino acid sequence in positions 78 to 88, an amino acid sequence in positions 79 to 88 or an amino acid sequence in positions 80 to 88 of the amino acid sequence represented by SEQ ID NO: 23, or peptide F consisting of an amino acid sequence in positions 19 to 133, an amino acid sequence in positions 20 to 133, an amino acid sequence in positions 21 to 133, an amino acid sequence in positions 22 to 133, an amino acid sequence in positions 23 to 133, an amino acid sequence in positions 24 to 133, an amino acid sequence in positions 25 to 133, an amino acid sequence in positions 26 to 133, an amino acid sequence in positions 27 to 133, an amino acid sequence in positions 28 to 133, an amino acid sequence in positions 29 to 133, an amino acid sequence in positions 30 to 133, an amino acid sequence in positions 31 to 133, an amino acid sequence in positions 32 to 133, an amino acid sequence in positions 33 to 133, an amino acid sequence in positions 34 to 133, an amino acid sequence in positions 35 to 133, an amino acid sequence in positions 36 to 133, an amino acid sequence in positions 37 to 133, an amino acid sequence in positions 38 to 133, an amino acid sequence in positions 39 to 133, an amino acid sequence in positions 40 to 133, an amino acid sequence in positions 41 to 133, an amino acid sequence in positions 42 to 133, an amino acid sequence in positions 43 to 133, an amino acid sequence in positions 44 to 133, an amino acid sequence in positions 45 to 133, an amino acid sequence in positions 46 to 133, an amino acid sequence in positions 47 to 133, an amino acid sequence in positions 48 to 133, an amino acid sequence in positions 49 to 133, an amino acid sequence in positions 50 to 133, an amino acid sequence in positions 51 to 133, an amino acid sequence in positions 52 to 133, an amino acid sequence in positions 53 to 133, an amino acid sequence in positions 54 to 133, an amino acid sequence in positions 55 to 133, an amino acid sequence in positions 56 to 133, an amino acid sequence in positions 57 to 133, an amino acid sequence in positions 58 to 133, an amino acid sequence in positions 59 to 133, an amino acid sequence in positions 60 to 133, an amino acid sequence in positions 61 to 133, an amino acid sequence in positions 62 to 133, an amino acid sequence in positions 63 to 133, an amino acid sequence in positions 64 to 133, an amino acid sequence in positions 65 to 133, an amino acid sequence in positions 66 to 133, an amino acid sequence in positions 67 to 133, an amino acid sequence in positions 68 to 133, an amino acid sequence in positions 69 to 133, an amino acid sequence in positions 70 to 133, an amino acid sequence in positions 71 to 133, an amino acid sequence in positions 72 to 133, an amino acid sequence in positions 73 to 133, an amino acid sequence in positions 74 to 133, an amino acid sequence in positions 75 to 133, an amino acid sequence in positions 76 to 133, an amino acid sequence in positions 77 to 133, an amino acid sequence in positions 78 to 133, an amino acid sequence in positions 79 to 133, an amino acid sequence in positions 80 to 133, an amino acid sequence in positions 81 to 133, an amino acid sequence in positions 82 to 133, an amino acid sequence in positions 83 to 133, an amino acid sequence in positions 84 to 133, an amino acid sequence in positions 85 to 133, an amino acid sequence in positions 86 to 133, an amino acid sequence in positions 87 to 133, an amino acid sequence in positions 88 to 133, an amino acid sequence in positions 89 to 133, an amino acid sequence in positions 90 to 133, an amino acid sequence in positions 91 to 133, an amino acid sequence in positions 92 to 133, an amino acid sequence in positions 93 to 133, an amino acid sequence in positions 94 to 133, an amino acid sequence in positions 95 to 133, an amino acid sequence in positions 96 to 133, an amino acid sequence in positions 97 to 133, an amino acid sequence in positions 98 to 133, an amino acid sequence in positions 99 to 133, an amino acid sequence in positions 100 to 133, an amino acid sequence in positions 101 to 133, an amino acid sequence in positions 102 to 133, an amino acid sequence in positions 103 to 133, an amino acid sequence in positions 104 to 133, an amino acid sequence in positions 105 to 133, an amino acid sequence in positions 106 to 133, an amino acid sequence in positions 107 to 133, an amino acid sequence in positions 108 to 133, an amino acid sequence in positions 109 to 133, an amino acid sequence in positions 110 to 133, an amino acid sequence in positions 111 to 133, an amino acid sequence in positions 112 to 133, an amino acid sequence in positions 113 to 133, an amino acid sequence in positions 114 to 133, an amino acid sequence in positions 115 to 133, an amino acid sequence in positions 116 to 133, an amino acid sequence in positions 117 to 133, an amino acid sequence in positions 118 to 133, an amino acid sequence in positions 119 to 133, an amino acid sequence in positions 120 to 133, an amino acid sequence in positions 121 to 133, an amino acid sequence in positions 122 to 133, an amino acid sequence in positions 123 to 133, an amino acid sequence in positions 124 to 133, an amino acid sequence in positions 125 to 133, an amino acid sequence in positions 126 to 133 or an amino acid sequence in positions 127 to 133 of the amino acid sequence represented by SEQ ID NO: 23 (particularly preferably peptide F consisting of an amino acid sequence in positions 26 to 88, an amino acid sequence in positions 80 to 88, an amino acid sequence in positions 90 to 133, an amino acid sequence in positions 91 to 133, an amino acid sequence in positions 93 to 133, an amino acid sequence in positions 104 to 133, an amino acid sequence in positions 108 to 133, an amino acid sequence in positions 109 to 133, an amino acid sequence in positions 111 to 133, an amino acid sequence in positions 115 to 133, an amino acid sequence in positions 119 to 133, an amino acid sequence in positions 124 to 133, an amino acid sequence in positions 126 to 133 or an amino acid sequence in positions 127 to 133, more preferably an amino acid sequence in positions 90 to 133, an amino acid sequence in positions 91 to 133, an amino acid sequence in positions 93 to 133, an amino acid sequence in positions 104 to 133, an amino acid sequence in positions 108 to 133, an amino acid sequence in positions 109 to 133, an amino acid sequence in positions 111 to 133, an amino acid sequence in positions 115 to 133, an amino acid sequence in positions 119 to 133, an amino acid sequence in positions 124 to 133, an amino acid sequence in positions 126 to 133 or an amino acid sequence in positions 127 to 133 of the amino acid sequence represented by SEQ ID NO: 23, and in peptide F consisting of an amino acid sequence in positions 91 to 133, a glutamic residue at the N-terminus may be pyroglutaminated, and in peptide F consisting of an amino acid sequence in positions 90 to 133, a glutamine residue at the N-terminus may be replaced by Tyr (see Figs. 57 and 58.)),

(6) peptide G consisting of an amino acid sequence in positions 18 to 122, an amino acid sequence in positions 19 to 122, an amino acid sequence in positions 20 to 122, an amino acid sequence in positions 21 to 122, an amino acid sequence in positions 22 to 122, an amino acid sequence in positions 23 to 122, an amino acid sequence in positions 24 to 122, an amino acid sequence in positions 25 to 122, an amino acid sequence in positions 26 to 122, an amino acid sequence in positions 27 to 122, an amino acid sequence in positions 28 to 122, an amino acid sequence in positions 29 to 122, an amino acid sequence in positions 30 to 122, an amino acid sequence in positions 31 to 122, an amino acid sequence in positions 32 to 122, an amino acid sequence in positions 33 to 122, an amino acid sequence in positions 34 to 122, an amino acid sequence in positions 35 to 122, an amino acid sequence in positions 36 to 122, an amino acid sequence in positions 37 to 122, an amino acid sequence in positions 38 to 122, an amino acid sequence in positions 39 to 122, an amino acid sequence in positions 40 to 122, an amino acid sequence in positions 41 to 122, an amino acid sequence in positions 42 to 122, an amino acid sequence in positions 43 to 122, an amino acid sequence in positions 44 to 122, an amino acid sequence in positions 45 to 122, an amino acid sequence in positions 46 to 122, an amino acid sequence in positions 47 to 122, an amino acid sequence in positions 48 to 122, an amino acid sequence in positions 49 to 122, an amino acid sequence in positions 50 to 122, an amino acid sequence in positions 51 to 122, an amino acid sequence in positions 52 to 122, an amino acid sequence in positions 53 to 122, an amino acid sequence in positions 54 to 122, an amino acid sequence in positions 55 to 122, an amino acid sequence in positions 56 to 122, an amino acid sequence in positions 57 to 122, an amino acid sequence in positions 58 to 122, an amino acid sequence in positions 59 to 122, an amino acid sequence in positions 60 to 122, an amino acid sequence in positions 61 to 122, an amino acid sequence in positions 62 to 122, an amino acid sequence in positions 63 to 122, an amino acid sequence in positions 64 to 122, an amino acid sequence in positions 65 to 122, an amino acid sequence in positions 66 to 122, an amino acid sequence in positions 67 to 122, an amino acid sequence in positions 68 to 122, an amino acid sequence in positions 69 to 122, an amino acid sequence in positions 70 to 122, an amino acid sequence in positions 71 to 122, an amino acid sequence in positions 72 to 122, an amino acid sequence in positions 73 to 122, an amino acid sequence in positions 74 to 122, an amino acid sequence in positions 75 to 122, an amino acid sequence in positions 76 to 122, an amino acid sequence in positions 77 to 122, an amino acid sequence in positions 78 to 122, an amino acid sequence in positions 79 to 122, an amino acid sequence in positions 80 to 122, an amino acid sequence in positions 81 to 122, an amino acid sequence in positions 82 to 122, an amino acid sequence in positions 83 to 122, an amino acid sequence in positions 84 to 122, an amino acid sequence in positions 85 to 122, an amino acid sequence in positions 86 to 122, an amino acid sequence in positions 87 to 122, an amino acid sequence in positions 88 to 122, an amino acid sequence in positions 89 to 122, an amino acid sequence in positions 90 to 122, an amino acid sequence in positions 91 to 122, an amino acid sequence in positions 92 to 122, an amino acid sequence in positions 93 to 122, an amino acid sequence in positions 94 to 122, an amino acid sequence in positions 95 to 122, an amino acid sequence in positions 96 to 122, an amino acid sequence in positions 97 to 122, an amino acid sequence in positions 98 to 122, an amino acid sequence in positions 99 to 122, an amino acid sequence in positions 100 to 122, an amino acid sequence in positions 101 to 122, an amino acid sequence in positions 102 to 122, an amino acid sequence in positions 103 to 122, an amino acid sequence in positions 104 to 122, an amino acid sequence in positions 105 to 122, an amino acid sequence in positions 106 to 122, an amino acid sequence in positions 107 to 122, an amino acid sequence in positions 108 to 122, an amino acid sequence in positions 109 to 122, an amino acid sequence in positions 110 to 122, an amino acid sequence in positions 111 to 122, an amino acid sequence in positions 112 to 122, an amino acid sequence in positions 113 to 122, an amino acid sequence in positions 114 to 122, an amino acid sequence in positions 115 to 122 or an amino acid sequence in positions 116 to 122 of the amino acid sequence represented by SEQ ID NO: 27 (particularly preferably peptide G comprising an amino acid sequence in positions 115 to 122 or an amino acid sequence in positions 116 to 122 of the amino acid sequence represented by SEQ ID NO: 27),

(7) peptide H consisting of an amino acid sequence in positions 18 to 122, an amino acid sequence in positions 19 to 122, an amino acid sequence in positions 20 to 122, an amino acid sequence in positions 21 to 122, an amino acid sequence in positions 22 to 122, an amino acid sequence in positions 23 to 122, an amino acid sequence in positions 24 to 122, an amino acid sequence in positions 25 to 122, an amino acid sequence in positions 26 to 122, an amino acid sequence in positions 27 to 122, an amino acid sequence in positions 28 to 122, an amino acid sequence in positions 29 to 122, an amino acid sequence in positions 30 to 122, an amino acid sequence in positions 31 to 122, an amino acid sequence in positions 32 to 122, an amino acid sequence in positions 33 to 122, an amino acid sequence in positions 34 to 122, an amino acid sequence in positions 35 to 122, an amino acid sequence in positions 36 to 122, an amino acid sequence in positions 37 to 122, an amino acid sequence in positions 38 to 122, an amino acid sequence in positions 39 to 122, an amino acid sequence in positions 40 to 122, an amino acid sequence in positions 41 to 122, an amino acid sequence in positions 42 to 122, an amino acid sequence in positions 43 to 122, an amino acid sequence in positions 44 to 122, an amino acid sequence in positions 45 to 122, an amino acid sequence in positions 46 to 122, an amino acid sequence in positions 47 to 122, an amino acid sequence in positions 48 to 122, an amino acid sequence in positions 49 to 122, an amino acid sequence in positions 50 to 122, an amino acid sequence in positions 51 to 122, an amino acid sequence in positions 52 to 122, an amino acid sequence in positions 53 to 122, an amino acid sequence in positions 54 to 122, an amino acid sequence in positions 55 to 122, an amino acid sequence in positions 56 to 122, an amino acid sequence in positions 57 to 122, an amino acid sequence in positions 58 to 122, an amino acid sequence in positions 59 to 122, an amino acid sequence in positions 60 to 122, an amino acid sequence in positions 61 to 122, an amino acid sequence in positions 62 to 122, an amino acid sequence in positions 63 to 122, an amino acid sequence in positions 64 to 122, an amino acid sequence in positions 65 to 122, an amino acid sequence in positions 66 to 122, an amino acid sequence in positions 67 to 122, an amino acid sequence in positions 68 to 122, an amino acid sequence in positions 69 to 122, an amino acid sequence in positions 70 to 122, an amino acid sequence in positions 71 to 122, an amino acid sequence in positions 72 to 122, an amino acid sequence in positions 73 to 122, an amino acid sequence in positions 74 to 122, an amino acid sequence in positions 75 to 122, an amino acid sequence in positions 76 to 122, an amino acid sequence in positions 77 to 122, an amino acid sequence in positions 78 to 122, an amino acid sequence in positions 79 to 122, an amino acid sequence in positions 80 to 122, an amino acid sequence in positions 81 to 122, an amino acid sequence in positions 82 to 122, an amino acid sequence in positions 83 to 122, an amino acid sequence in positions 84 to 122, an amino acid sequence in positions 85 to 122, an amino acid sequence in positions 86 to 122, an amino acid sequence in positions 87 to 122, an amino acid sequence in positions 88 to 122, an amino acid sequence in positions 89 to 122, an amino acid sequence in positions 90 to 122, an amino acid sequence in positions 91 to 122, an amino acid sequence in positions 92 to 122, an amino acid sequence in positions 93 to 122, an amino acid sequence in positions 94 to 122, an amino acid sequence in positions 95 to 122, an amino acid sequence in positions 96 to 122, an amino acid sequence in positions 97 to 122, an amino acid sequence in positions 98 to 122, an amino acid sequence in positions 99 to 122, an amino acid sequence in positions 100 to 122, an amino acid sequence in positions 101 to 122, an amino acid sequence in positions 102 to 122, an amino acid sequence in positions 103 to 122, an amino acid sequence in positions 104 to 122, an amino acid sequence in positions 105 to 122, an amino acid sequence in positions 106 to 122, an amino acid sequence in positions 107 to 122, an amino acid sequence in positions 108 to 122, an amino acid sequence in positions 109 to 122, an amino acid sequence in positions 110 to 122, an amino acid sequence in positions 111 to 122, an amino acid sequence in positions 112 to 122, an amino acid sequence in positions 113 to 122, an amino acid sequence in positions 114 to 122, an amino acid sequence in positions 115 to 122 or an amino acid sequence in positions 116 to 122 of the amino acid sequence represented by SEQ ID NO: 38 (particularly preferably peptide H consisting of an amino acid sequence in positions 115 to 122 or an amino acid sequence in positions 116 to 122 of the amino acid sequence represented by SEQ ID NO: 38), and

(8) peptide I consisting of an amino acid sequence in positions 18 to 131, an amino acid sequence in positions 19 to 131, an amino acid sequence in positions 20 to 131, an amino acid sequence in positions 21 to 131, an amino acid sequence in positions 22 to 131, an amino acid sequence in positions 23 to 131, an amino acid sequence in positions 24 to 131, an amino acid sequence in positions 25 to 131, an amino acid sequence in positions 26 to 131, an amino acid sequence in positions 27 to 131, an amino acid sequence in positions 28 to 131, an amino acid sequence in positions 29 to 131, an amino acid sequence in positions 30 to 131, an amino acid sequence in positions 31 to 131, an amino acid sequence in positions 32 to 131, an amino acid sequence in positions 33 to 131, an amino acid sequence in positions 34 to 131, an amino acid sequence in positions 35 to 131, an amino acid sequence in positions 36 to 131, an amino acid sequence in positions 37 to 131, an amino acid sequence in positions 38 to 131, an amino acid sequence in positions 39 to 131, an amino acid sequence in positions 40 to 131, an amino acid sequence in positions 41 to 131, an amino acid sequence in positions 42 to 131, an amino acid sequence in positions 43 to 131, an amino acid sequence in positions 44 to 131, an amino acid sequence in positions 45 to 131, an amino acid sequence in positions 46 to 131, an amino acid sequence in positions 47 to 131, an amino acid sequence in positions 48 to 131, an amino acid sequence in positions 49 to 131, an amino acid sequence in positions 50 to 131, an amino acid sequence in positions 51 to 131, an amino acid sequence in positions 52 to 131, an amino acid sequence in positions 53 to 131, an amino acid sequence in positions 54 to 131, an amino acid sequence in positions 55 to 131, an amino acid sequence in positions 56 to 131, an amino acid sequence in positions 57 to 131, an amino acid sequence in positions 58 to 131, an amino acid sequence in positions 59 to 131, an amino acid sequence in positions 60 to 131, an amino acid sequence in positions 61 to 131, an amino acid sequence in positions 62 to 131, an amino acid sequence in positions 63 to 131, an amino acid sequence in positions 64 to 131, an amino acid sequence in positions 65 to 131, an amino acid sequence in positions 66 to 131, an amino acid sequence in positions 67 to 131, an amino acid sequence in positions 68 to 131, an amino acid sequence in positions 69 to 131, an amino acid sequence in positions 70 to 131, an amino acid sequence in positions 71 to 131, an amino acid sequence in positions 72 to 131, an amino acid sequence in positions 73 to 131, an amino acid sequence in positions 74 to 131, an amino acid sequence in positions 75 to 131, an amino acid sequence in positions 76 to 131, an amino acid sequence in positions 77 to 131, an amino acid sequence in positions 78 to 131, an amino acid sequence in positions 79 to 131, an amino acid sequence in positions 80 to 131, an amino acid sequence in positions 81 to 131, an amino acid sequence in positions 82 to 131, an amino acid sequence in positions 83 to 131, an amino acid sequence in positions 84 to 131, an amino acid sequence in positions 85 to 131, an amino acid sequence in positions 86 to 131, an amino acid sequence in positions 87 to 131, an amino acid sequence in positions 88 to 131, an amino acid sequence in positions 89 to 131, an amino acid sequence in positions 90 to 131, an amino acid sequence in positions 91 to 131, an amino acid sequence in positions 92 to 131, an amino acid sequence in positions 93 to 131, an amino acid sequence in positions 94 to 131, an amino acid sequence in positions 95 to 131, an amino acid sequence in positions 96 to 131, an amino acid sequence in positions 97 to 131, an amino acid sequence in positions 98 to 131, an amino acid sequence in positions 99 to 131, an amino acid sequence in positions 100 to 131, an amino acid sequence in positions 101 to 131, an amino acid sequence in positions 102 to 131, an amino acid sequence in positions 103 to 131, an amino acid sequence in positions 104 to 131, an amino acid sequence in positions 105 to 131, an amino acid sequence in positions 106 to 131, an amino acid sequence in positions 107 to 131, an amino acid sequence in positions 108 to 131, an amino acid sequence in positions 109 to 131, an amino acid sequence in positions 110 to 131, an amino acid sequence in positions 111 to 131, an amino acid sequence in positions 112 to 131, an amino acid sequence in positions 113 to 131, an amino acid sequence in positions 114 to 131, an amino acid sequence in positions 115 to 131, an amino acid sequence in positions 116 to 131, an amino acid sequence in positions 117 to 131, an amino acid sequence in positions 118 to 131, an amino acid sequence in positions 119 to 131, an amino acid sequence in positions 120 to 131, an amino acid sequence in positions 121 to 131, an amino acid sequence in positions 122 to 131, an amino acid sequence in positions 123 to 131, an amino acid sequence in positions 124 to 131 or an amino acid sequence in positions 125 to 131 of the amino acid sequence represented by SEQ ID NO: 42 (particularly preferably peptide I consisting of an amino acid sequence in positions 124 to 131 or an amino acid sequence in positions 125 to 131 of the amino acid sequence represented by SEQ ID NO: 42).

As these peptides, those having an amide at the C-terminus are preferably used.

In the present specification, the above peptide B, peptide C, peptide D, peptide E, peptide F, peptide G, peptide H and peptide I are sometimes collectively referred to as the peptide of the present application.

The partial peptide of the present application may be any partial peptide of the peptide of the present application, but is usually a peptide consisting of 5 or more amino acids, preferably 10 or more amino acids, more preferably the one having the same activity as that of the peptide of the present application.

The precursor protein of the secretory protein of the present application or of the peptide of the present application is a protein containing the secretory protein of the present application or the peptide of the present application and capable of being cleaved with a suitable peptidase etc. to produce the secretory protein of the present application or the peptide of the present application.

Specifically,

(1) The precursor protein of secretory protein A includes precursor protein A having the same or substantially the same amino acid sequence as the amino acid sequence represented by SEQ ID NO: 2, etc.;
(2) The precursor protein of secretory protein B or peptide B includes precursor protein B consisting of the amino acid sequence represented by SEQ ID NO: 3, etc.;
(3) The precursor protein of secretory protein C or peptide C includes precursor protein C consisting of the amino acid sequence represented by SEQ ID NO: 4, etc.;
(4) The precursor protein of secretory protein D or peptide D includes precursor protein D consisting of the amino acid sequence represented by SEQ ID NO: 5, etc.;
(5) The precursor protein of secretory protein E or peptide E includes precursor protein E consisting of the amino acid sequence represented by SEQ ID NO: 6, etc.;
(6) The precursor protein of secretory protein F or peptide F includes precursor protein F consisting of the amino acid sequence represented by SEQ ID NO: 23, etc.;
(7) The precursor protein of secretory protein G or peptide G includes precursor protein G consisting of the amino acid sequence represented by SEQ ID NO: 27, etc.;
(8) The precursor protein of secretory protein H or peptide H includes precursor protein H consisting of the amino acid sequence represented by SEQ ID NO: 38, etc.; and
(9) The precursor protein of secretory protein I or peptide I includes precursor protein I consisting of the amino acid sequence represented by SEQ ID NO: 42, etc.

In the present specification, amino acid sequence Z refers to the amino acid sequence represented by one sequence identification number selected from SEQ ID NO: 2 to SEQ ID NO: 6, SEQ ID NO: 23, SEQ ID NO: 27, SEQ ID NO: 38 and SEQ ID NO: 42.

A protein having the same or substantially the same amino acid sequence as amino acid sequence Z may be any protein derived from any cells of human and other warm-blooded animals (e.g., guinea pig, rat, mouse, chicken, rabbit, swine, sheep, bovine, monkey, etc.) such as retina cells, liver cells, splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophils, basophils, eosinophils, monocytes), megakaryocyte, synovial cells, chondrocytes, bone cells, osteoblasts, osteoclasts, mammary gland cells, hepatocytes or interstitial cells, the corresponding precursor cells, stem cells, cancer cells, etc., or any tissues where such cells are present, such as brain or any of brain regions (e.g., retina, olfactory bulb, amygdaloid nucleus, basal ganglia, hippocampus, thalamus, hypothalamus, cerebral cortex, medulla oblongata, cerebellum), spinal cord, pituitary, stomach, pancreas, kidney, liver, gonad, thyroid, gall-bladder, bone marrow, adrenal gland, skin, muscle, lung, digestive tract (e.g., large intestine and small intestine), blood vessels, heart, thymus, spleen, submandibular gland, peripheral blood, prostate, testis, ovary, placenta, uterus, bone, joint, skeletal muscle, etc., or blood cells or cultured cells thereof (for example, MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); the protein may also be a synthetic protein.

Substantially the same amino acid sequence as amino acid sequence Z include amino acid sequences having at least about 50% homology, preferably at least about 60% homology, more preferably at least about 70% homology, still more preferably at least about 80% homology, further more preferably at least about 90% homology, most preferably at least about 95% homology, to amino acid sequence Z. Specifically, the amino acid sequence having at least about 60% homology to the amino acid sequence represented by SEQ ID NO: 23 includes the amino acid sequence represented by SEQ ID NO: 27, the amino acid sequence represented by SEQ ID NO: 38, and the amino acid sequence represented by SEQ ID NO: 42.

Particularly, substantially the same amino acid sequence as amino acid sequence Z includes:

(i) amino acid sequence Z,
(ii) an amino acid sequence wherein in amino acid sequence Z, 1 to 30 (e.g. 1 to 15, preferably I to 10, more preferably I to 5, still more preferably 1 to 3, further more preferably 1) amino acids were deleted,
(iii) an amino acid sequence wherein in amino acid sequence Z,1 to 30 (e.g. 1 to 15, preferably 1 to 10, more preferably 1 to 5, still more preferably 1 to 3, further more preferably 1) amino acids were added,
(iv) an amino acid sequence wherein in amino acid sequence Z, 1 to 30 (e.g. 1 to 15, preferably 1 to 10, more preferably 1 to 5, still more preferably 1 to 3, further still more preferably 1) amino acids were inserted,
(v) an amino acid sequence wherein in amino acid sequence Z, 1 to 30 (e.g. 1 to 15, preferably 1 to 10, more preferably 1 to 5, still more preferably 1 to 3, further more preferably 1) amino acids were replaced by other amino acids, and
(vi) an amino acid sequence wherein the above-mentioned (ii) to (v) were combined.

Examples of the precursor protein of the application include:

(1) precursor protein A consisting of the amino acid sequence represented by SEQ ID NO: 2,
(2) precursor protein B consisting of the amino acid sequence represented by SEQ ID NO: 3,
(3) precursor protein C consisting of the amino acid sequence represented by SEQ ID NO: 4,
(4) precursor protein D consisting of the amino acid sequence represented by SEQ ID NO: 5,
(5) precursor protein E consisting of the amino acid sequence represented by SEQ ID NO: 6,
(6) precursor protein F consisting of the amino acid sequence represented by SEQ ID NO: 23,
(7) precursor protein G consisting of the amino acid sequence represented by SEQ ID NO: 27,
(8) precursor protein H consisting of the amino acid sequence represented by SEQ ID NO: 38, and
(9) precursor protein I consisting of the amino acid sequence represented by SEQ ID NO: 42.

Secretory protein A consisting of the amino acid sequence represented by SEQ ID NO: 1 is a protein comprising precursor protein A consisting of that amino acid sequence represented by SEQ ID NO: 2 from which a secretory signal sequence was deleted.

The amino acid sequence in positions 1 to 21 of the amino acid sequence represented by SEQ ID NO: 3 indicates a secretory signal sequence.

An amino acid sequence in positions 1 to 14 of the amino acid sequence represented by SEQ ID NO: 4 indicates a secretory signal sequence.

An amino acid sequence in positions 1 to 24 of the amino acid sequence represented by SEQ ID NO: 5 indicates a secretory signal sequence.

An amino acid sequence in positions 1 to 18 of the amino acid sequence represented by SEQ ID NO: 6 indicates a secretory signal sequence.

An amino acid sequence in positions 1 to 18 of the amino acid sequence represented by SEQ ID NO: 23 indicates a secretory signal sequence.

An amino acid sequence in positions 1 to 17 of the amino acid sequence represented by SEQ ID NO: 27 indicates a secretory signal sequence.

An amino acid sequence in positions 1 to 17 of the amino acid sequence represented by SEQ ID NO: 38 indicates a secretory signal sequence.

An amino acid sequence in positions 1 to 17 of the amino acid sequence represented by SEQ ID NO: 42 indicates a secretory signal sequence.

The precursor protein of the present application may have the same activity as that of the secretory protein of the present application or the peptide of the present

The secretory protein of the present application or the peptide of the present application, its partial peptide or its precursor protein (hereinafter referred to sometimes collectively as the peptide of the present application) is represented in accordance with the conventional way of describing peptides, that is, the N-terminus (amino terminus) at the left hand and the C-terminus (carboxyl terminus) at the right hand.

In the peptides of the application including the peptide consisting of the amino acid sequence represented by SEQ ID NO: 1, the C-terminus is usually in the form of a carboxyl group (-COOH) or a carboxylate (-COO) but may be in the form of an amide ($-CONH_2$) or an ester (-COOR).

In the ester group, R includes, for example, a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl such as a phenyl-$C_{1-2}$alkyl group, e.g., benzyl, phenethyl, etc.; an α-naphthyl-$C_{1-2}$ alkyl group such as α-naphthylmethyl, etc.; and the like. In addition, pivaloyloxymethyl or the like which is used widely as an ester for oral administration may also be used.

Where the peptide of the present application has a carboxyl group (or a carboxylate) at a position other than the C-terminus, it may be amidated or esterified and such an amide or ester is also included within the peptide of the present application. As the ester group herein, the same esters as those described with respect to the above C-terminal are used.

Furthetmore, examples of the peptide of the present application include variants of the above polypeptides, wherein the amino group at the N-terminus (e.g., methionine residue) of the polypeptide is protected with a protecting group (e.g., a $C_{1-6}$ acyl group such as a $C_{1-6}$ alkanoyl group e.g., formyl group, acetyl group, etc.); those wherein the N-terminal region is cleaved in vivo and the glutamyl group thus formed is pyroglucaminated; those wherein a substituent (e.g., -OH,-SH, amino group, imidazole group, indole group, guanidino group, etc.) on the side chain of an amino acid in the molecule is protected with a suitable protecting group (e.g., a $C_{1-6}$ acyl group such as a $C_{1-6}$ alkanoyl group , e.g., formyl group, acetyl group, etc.), or conjugated proteins such as glycoproteins having sugar chains.

The peptide of the present application or its salt may be used in the form of salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like. Hereinafter, these salts are also referred to as the peptide of the present application.

The peptide of the present application may be produced by a publicly known method used to purify a peptide from human or other warm-blooded animal cells or tissues described above, or may also be manufactured by culturing a transformant containing DNA encoding the peptide later described. Furthermore, the peptide of the present application may also be manufactured by a modification of the peptide synthesis method, which will be described below.

Where the peptides of the present application are produced from human or mammalian tissues or cells, human or mammalian tissues or cells are homogenized and then extracted with an acid or the like, and the extract is isolated and purified by a combination of chromatography techniques such as reverse phase chromatography, ion exchange chromatography, and the like.

To synthesize the peptide of the present application or its amides, commercially available resins that are used for polypeptide synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl) phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective polypeptide according to various condensation methods publicly known in the art. At the end of the reaction, the polypeptide is excised from the resin and at the same time, the protecting groups are removed. Then, intramolecular disulfide bond-forming reaction is performed in a highly diluted solution to obtain the objective peptide of the present application or amides thereof.

For condensation of the protected amino acids described above, a variety of activation reagents for polypeptide synthesis may be used, but carbodiimides are particularly preferably employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents that are known to be usable for peptide condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to peptide bond-forming reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse effect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower ($C_{1-6}$) alkanoyl group such as acetyl group, an aroyl group such as benzoyl group, and a group derived from carbonic acid, such as benzyloxycarbonyl group and ethoxycarbonyl group. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Burn, Boc, Trt, Fmoc, etc.

Examples of the activated carboxyl groups in the starting amino acids include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)], etc. As the activated amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine, piperazine, etc.; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group used as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups, activation of functional groups involved in the reaction, etc. may be appropriately selected from publicly known groups and publicly known means.

In another method of obtaining the amides of the peptide of the present application, for example, the α-carboxyl group of the carboxy terminal amino acid is first protected by amidation; the peptide (polypeptide) chain is then extended from the amino group side to a desired length. Thereafter, a peptide in which only the protecting group of the N-terminal α-amino group has been eliminated from the peptide and a peptide in which only the protecting group of the C-terminal carboxyl group has been eliminated are manufactured. The two peptides are condensed in a mixture of the solvents described above. The details of the condensation reaction are the same as described above. After the protected peptide obtained by the condensation is purified, all the protecting groups are eliminated by the method described above to give the desired crude peptide. This crude peptide is purified by various known purification means. Lyophilization of the major fraction gives the amide of the desired peptide of the present application.

To prepare the esterified peptide, for example, the α-carboxyl group of the carboxy terminal amino acid is condensed with a desired alcohol to prepare the amino acid ester, which is followed by procedure similar to the preparation of the amidated peptide of the present application described above to give the desired esterified peptide.

A partial peptide of the peptide of the present application can be manufactured by publicly known methods for peptide synthesis. For the methods for peptide synthesis, for example, either solid phase synthesis or liquid phase synthesis may be used. That is, the partial peptide or amino acids that can construct the partial peptide of the peptide of the present application are condensed with the remaining part. Where the product has protecting groups, these protecting groups are removed to give the desired peptide. Publicly known methods for condensation and elimination of the protecting groups are described in (1) to (5) below.

(1) M. Bodanszky & M.A: Ondetti: Peptide Synthesis, Interscience Publishers, New York (1966)
(2) Schroeder & Luebke: The Peptide, Academic Press, New York (1965)
(3) Nobuo Izumiya, et al.: Peptide Gosei-no-Kiso to Jikken (Basics and experiments of peptide synthesis), published by Mamzen Co. (1975)
(4) Haruaki Yajima & Shunpei Sakakibara: Seikagaku Jikken Koza (Biochemical Experiment) 1, Tanpakushitsu no Kagaku (Chemistry of Proteins) IV, 205 (1977)
(5) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the peptide of the present application or its partial peptide. When the partial peptide of the peptide of the present application obtained by the above methods is in a free form, the peptide can be converted into an appropriate salt by a publicly known method; when the peptide is obtained in a salt form, it can be converted into a free form or a different salt form by a publicly known method.

The polynucleotide encoding the peptide of the present application may be any polynucleotide (DNA or RNA, preferably DNA) so long as it comprises the nucleotide sequence encoding the peptide of the present application described above. Such a polynucleotide is DNA encoding the peptide of the present application or RNA such as mRNA, which may be a single or double strand. The double strand may be double-stranded DNA, double-stranded RNA, or a DNA/RNA hybrid. The single strand may be a sense chain (that is, a coding chain) or an antisense chain (that is, a non-coding chain).

Using the polynucleotide encoding the peptide of the present application, the mRNA of the peptide of the present application can be quantified according to a method described in, for example, Shin PCR to Sonooyo (New PCR and Its Application), extra issue, Jikken Igaku (Experimental Medicine), 15(7), (1997) or its modified method.

The DNA encoding the peptide of the present application may be any DNA so long as it comprises the nucleotide sequence encoding the peptide of the presen application described above. Such a DNA may also be any of genomic DNA, genomic cDNA library, cDNA derived from the cells or tissues described above, cDNA library derived from the cells or tissues described above, and synthetic DNA.

The vector to be used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

In the present specification, nucleotide sequence P refers to one nucleotide sequence selected from the following 9 nucleotide sequences:

(1) the nucleotide sequence represented by SEQ ID NO: 7,
(2) a nucleotide sequence in positions 64 to 336 of the nucleotide sequence represented by SEQ ID NO: 9,
(3) a nucleotide sequence in positions 43 to 702 of the nucleotide sequence represented by SEQ ID NO: 10,
(4) a nucleotide sequence in positions 73 to 498 of the nucleotide sequence represented by SEQ ID NO: 11,
(5) a nucleotide sequence in positions 55 to 724 of the nucleotide sequence represented by SEQ ID NO: 12,
(6) a nucleotide sequence in positions 55 to 408 of the nucleotide sequence represented by SEQ ID NO: 24,
(7) a nucleotide sequence in positions 52 to 372 of the nucleotide sequence represented by SEQ ID NO: 28,
(8) a nucleotide sequence in positions 52 to 372 of the nucleotide sequence represented by SEQ ID NO: 39, and
(9) a nucleotide sequence in positions 52 to 402 of the nucleotide sequence represented by SEQ ID NO: 43.

The DNA encoding the secretory protein of the present application may be any DNA insofar as it comprises, for example, a nucleotide sequence hybridizing with nucleotide sequence P under high stringent conditions, and encodes a peptides having an activity substantially equivalent to that of the peptide of the present application.

The nucleotide sequence capable of hybridizing with nucleotide sequence P under high stringent conditions may, for example, be a nucleotide sequence having at least about 70% homology, preferably at least about 80% homology, more preferably at least about 90% homology, still more preferably at least about 95% homology, to nucleotide sequence P.

The hybridization can be carried out by publicly known methods or by a modification thereof, for example, according to the method described in Molecular Cloning, 2nd Ed., J. Sambrook et al., Cold Spring Harbor Lab. Press, (1989). A commercially available library may also be used according to the instructions of the attached manufacturer's protocol. The hybridization can be carried out preferably under high stringent conditions.

The high stringent conditions used herein are, for example, those in a sodium concentration of about 19 mM to about 40 mM, preferably about 19 mM to about 20 mM at a temperature of about 50°C to about 70°C, preferably about 60°C to about 65°C. The high stringent conditions are most preferably those in a sodium concentration of about 19 mM at a temperature of about 65°C.

Specifically,

(1) As DNA encoding secretory protein A consisting of the amino acid sequence represented by SEQ ID NO: 1, DNA consisting of the nucleotide sequence represented by SEQ ID NO: 7, etc., is used;
(2) As DNA encoding secretory protein B consisting of an amino acid sequence in positions 22 to 112 of the amino acid sequence represented by SEQ ID NO: 3, DNA consisting of a nucleotide sequence in positions 64 to 336 of the nucleotide sequence represented by SEQ ID NO: 9, etc., is used;
(3) As DNA encoding secretory protein C consisting of an amino acid sequence in positions 15 to 234 of the amino acid sequence represented by SEQ ID NO: 4, DNA consisting of a nucleotide sequence in positions 43 to 702 of the nucleotide sequence represented by SEQ ID NO: 10, etc., is used;
(4) As DNA encoding secretory protein D consisting of an amino acid sequence in positions 25 to 166 of the amino acid sequence represented by SEQ ID NO: 5, DNA consisting of a nucleotide sequence in positions 73 to 498 of the nucleotide sequence represented by SEQ ID NO: 11, etc., is used;
(5) As DNA encoding secretory protein E consisting of an amino acid sequence in positions 19 to 241 of the amino acid sequence represented by SEQ ID NO: 6, DNA consisting of a nucleotide sequence in positions 55 to 723 of the nucleotide sequence represented by SEQ ID NO: 12, etc., is used;
(6) As DNA encoding secretory protein F consisting of an amino acid sequence in positions 19 to 136 of the amino acid sequence represented by SEQ ID NO: 23, DNA consisting of a nucleotide sequence in positions 55 to 408 of the nucleotide sequence represented by SEQ ID NO: 24, etc., is used;
(7) As DNA encoding secretory protein G consisting of an amino acid sequence in positions 18 to 124 of the amino acid sequence represented by SEQ ID NO: 27, DNA consisting of a nucleotide sequence in positions 52 to 372 of the nucleotide sequence represented by SEQ ID NO: 28, etc., is used;
(8) As DNA encoding secretory protein H consisting of an amino acid sequence in positions 18 to 124 of the amino acid sequence represented by SEQ ID NO: 38, DNA consisting of a nucleotide sequence in positions 52 to 372 of the nucleotide sequence represented by SEQ ID NO: 39, etc., is used; and
(9) As DNA encoding secretory protein H consisting of an amino acid sequence in positions 18 to 134 of the amino acid sequence represented by SEQ ID NO: 42, DNA

consisting of a nucleotide sequence in positions 52 to 402 of the nucleotide sequence represented by SEQ ID NO: 43, etc., is used.

In the present specification, nucleotide sequence Q refers to one nucleotide sequence selected from the following 24 nucleotide sequences:

(1)

[1] a nucleotide sequence in positions 64 to 111 of the nucleotide sequence represented by SEQ ID NO: 9,
[2] a nucleotide sequence in positions 118 to 165 of the nucleotide sequence represented by SEQ ID NO: 9,

(2)

[1] a nucleotide sequence in positions 43 to 63 of the nucleotide sequence represented by SEQ ID NO: 10,
[2] a nucleotide sequence in positions 76 to 174 of the nucleotide sequence represented by SEQ ID NO: 10,
[3] a nucleotide sequence in positions 178 to 264 of the nucleotide sequence represented by SEQ ID NO: 10,
[4] a nucleotide sequence in positions 268 to 321 of the nucleotide sequence represented by SEQ ID NO: 10,
[5] a nucleotide sequence in positions 328 to 561 of the nucleotide sequence represented by SEQ ID NO: 10,
[6] a nucleotide sequence in positions 568 to 702 of the nucleotide sequence represented by SEQ ID NO: 10,

(3)

[1] a nucleotide sequence in positions 73 to 255 of the nucleotide sequence represented by SEQ ID NO: 11,
[2] a nucleotide sequence in positions 256 to 342 of the nucleotide sequence represented by SEQ ID NO: 11,
[3] a nucleotide sequence in positions 343 to 498 of the nucleotide sequence represented by SEQ ID NO: 11,

(4)

[1] a nucleotide sequence in positions 55 to 174 of the nucleotide sequence represented by SEQ ID NO: 12,
[2] a nucleotide sequence in positions 187 to 606 of the nucleotide sequence represented by SEQ ID NO: 12,
[3] a nucleotide sequence in positions 613 to 723 of the nucleotide sequence represented by SEQ ID NO: 12,

(5)

[1] a nucleotide sequence in positions 76 to 264 of the nucleotide sequence represented by SEQ ID NO: 24,
[2] a nucleotide sequence in positions 238 to 264 of the nucleotide sequence represented by SEQ ID NO: 24,
[3] a nucleotide sequence in positions 271 to 399 of the nucleotide sequence represented by SEQ ID NO: 24,
[4] a nucleotide sequence in positions 379 to 399 of the nucleotide sequence represented by SEQ ID NO: 24,

(6)

[1] a nucleotide sequence in positions 343 to 366 of the nucleotide sequence represented by SEQ ID NO: 28,
[2] a nucleotide sequence in positions 346 to 366 of the nucleotide sequence represented by SEQ ID NO: 28,

(7)

[1] a nucleotide sequence in positions 343 to 366 of the nucleotide sequence represented by SEQ ID NO: 39,
[2] a nucleotide sequence in positions 346 to 366 of the nucleotide sequence represented by SEQ ID NO: 39,

(8)

[1] a nucleotide sequence in positions 370 to 393 of the nucleotide sequence represented by SEQ ID NO: 43, and
[2] a nucleotide sequence in positions 373 to 393 of the nucleotide sequence represented by SEQ ID NO: 43.

The DNA encoding the peptide of the present application may be any DNA insofar as it comprises, for example, a nucleotide sequence hybridizing with nucleotide sequence Q under high stringent conditions, and encodes a peptide having an activity substantially equivalent to that of the peptide of the present application.

The nucleotide sequence capable of hybridizing with nucleotide sequence Q under high stringent conditions may, for example, be a nucleotide sequence having at least about 70% homology, preferably at least about 80% homology, more preferably at least about 90% homology, further more preferably at least about 95 % homology, to nucleotide sequence Q.

The hybridization method and high stringent conditions used are the same as described above.

Specifically,

(1)

(i) As DNA encoding peptide B consisting of an amino acid sequence in positions 22 to 37 of the amino acid sequence represented by SEQ ID NO: 3, DNA consisting of a nucleotide sequence in positions 64 to 111 of the nucleotide sequence represented by SEQ ID NO: 9, etc., is used;
(ii) As DNA encoding peptide B consisting of an amino acid sequence in positions 40 to 55 of the amino acid sequence represented by SEQ ID NO: 3, DNA consisting of a nucleotide sequence in positions 118 to 165 of the nucleotide sequence represented by SEQ ID NO: 9, etc., is used;

(2)

(i) As DNA encoding peptide C consisting of an amino acid sequence in positions 15 to 21 of the amino acid sequence represented by SEQ ID NO: 4, DNA consisting of a nucleotide sequence in positions 43 to 63 of the nucleotide sequence represented by SEQ ID NO: 10, etc., is used;
(ii) As DNA encoding peptide C consisting of an amino acid sequence in positions 26 to 58 of the amino acid sequence represented by SEQ ID NO: 4, DNA consisting of a nucleotide sequence in positions 76 to 174 of the nucleotide sequence represented by SEQ ID NO: 10, etc., is used;
(iii) As DNA encoding peptide C consisting of an amino acid sequence in positions 60 to 88 of the amino acid sequence represented by SEQ ID NO: 4, DNA consisting of a nucleotide sequence in positions 178 to 264 of the nucleotide sequence represented by SEQ ID NO: 10, etc., is used;
(iv) As DNA encoding peptide C consisting of an amino acid sequence in positions 90 to 107 of the amino acid sequence represented by SEQ ID NO: 4, DNA consisting of a nucleotide sequence in positions 268 to 321 of the nucleotide sequence represented by SEQ ID NO: 10, etc., is used;
(v) As DNA encoding peptide C consisting of an amino acid sequence in positions 110 to 187 of the amino acid sequence represented by SEQ ID NO: 4, DNA consisting of a nucleotide sequence in positions 328 to 561 of the nucleotide sequence represented by SEQ ID NO: 10, etc., is used;

(vi) As DNA encoding peptide C consisting of an amino acid sequence in positions 190 to 234 of the amino acid sequence represented by SEQ ID NO: 4, DNA consisting of a nucleotide sequence in positions 568 to 702 of the nucleotide sequence represented by SEQ ID NO: 10, etc., is used;

(3)

(i) As DNA encoding peptide D consisting of an amino acid sequence in positions 25 to 85 of the amino acid sequence represented by SEQ ID NO: 5, DNA consisting of a nucleotide sequence in positions 73 to 255 of the nucleotide sequence represented by SEQ ID NO: 11, etc., is used;
(ii) As DNA encoding peptide D consisting of an amino acid sequence in positions 86 to 114 of the amino acid sequence represented by SEQ ID NO: 5, DNA consisting of a nucleotide sequence in positions 256 to 342 of the nucleotide sequence represented by. SEQ ID NO: 11, etc., is used;
(iii) As DNA encoding peptide D consisting of an amino acid sequence in positions 115 to 166 of the amino acid sequence represented by SEQ ID NO: 5, DNA consisting of a nucleotide sequence in positions 343 to 498 of the nucleotide sequence represented by SEQ ID NO: 11, etc., is used;

(4)

(i) As DNA encoding peptide E consisting of an amino acid sequence in positions 19 to 58 of the amino acid sequence represented by SEQ ID NO: 6, DNA consisting of a nucleotide sequence in positions 55 to 174 of the nucleotide sequence represented by SEQ ID NO: 12, etc., is used;
(ii) As DNA encoding peptide E consisting of an amino acid sequence in positions 63 to 202 of the amino acid sequence represented by SEQ ID NO: 6, DNA consisting of a nucleotide sequence in positions 187 to 606 of the nucleotide sequence represented by SEQ ID NO: 12, etc., is used;
(iii) As DNA encoding peptide E consisting of an amino acid sequence in positions 205 to 241 of the amino acid sequence represented by SEQ ID NO: 6, DNA consisting of a nucleotide sequence in positions 613 to 723 of the nucleotide sequence represented by SEQ ID NO: 12, etc., is used;

(5)

(i) As DNA encoding peptide F consisting of an amino acid sequence in positions 26 to 88 of the amino acid sequence represented by SEQ ID NO: 23, DNA consisting of a nucleotide sequence in positions 76 to 264 of the nucleotide sequence represented by SEQ ID NO: 24, etc., is used;
(ii) As DNA encoding peptide F consisting of an amino acid sequence in positions 80 to 88 of the amino acid sequence represented by SEQ ID NO: 23, DNA consisting of a nucleotide sequence in positions 238 to 264 of the nucleotide sequence represented by SEQ ID NO: 24, etc., is used;
(iii) As DNA encoding peptide F consisting of an amino acid sequence in positions 91 to 133 of the amino acid sequence represented by SEQ ID NO: 23, DNA consisting of a nucleotide sequence in positions 271 to 399 of the nucleotide sequence represented by SEQ ID NO: 24, etc., is used;
(iv) As DNA encoding peptide F consisting of an amino acid sequence in positions 127 to 133 of the amino acid sequence represented by SEQ ID NO: 23, DNA consisting of a nucleotide sequence in positions 379 to 399 of the nucleotide sequence represented by SEQ ID NO: 24, etc., is used;

(6)

(i) As DNA encoding peptide G consisting of an amino acid sequence in positions 115 to 122 of the amino acid sequence represented by SEQ ID NO: 27, DNA consisting of a nucleotide sequence in positions 343 to 366 of the nucleotide sequence represented by SEQ ID NO: 28, etc., is used;
(ii) As DNA encoding peptide G consisting of an amino acid sequence in positions 116 to 122 of the amino acid sequence represented by SEQ ID NO: 27, DNA consisting of a nucleotide sequence in positions 346 to 366 of the nucleotide sequence represented by SEQ ID NO: 28, etc., is used;

(7)

(i) As DNA encoding peptide H consisting of an amino acid sequence in positions 115 to 122 of the amino acid sequence represented by SEQ ID NO: 38, DNA consisting of g a nucleotide sequence in positions 343 to 366 of the nucleotide sequence represented by SEQ ID NO: 39, etc., is used;
(ii) As DNA encoding peptide H consisting of an amino acid sequence in positions 116 to 122 of the amino acid sequence represented by SEQ ID NO: 38, DNA consisting of a nucleotide sequence in positions 346 to 366 of the nucleotide sequence represented by SEQ ID NO: 39, etc., is used;

(8)

(i) As DNA encoding peptide I consisting of an amino acid sequence in positions 124 to 131 of the amino acid sequence represented by SEQ ID NO: 43, DNA consisting of a nucleotide sequence in positions 370 to 393 of the nucleotide sequence represented by SEQ ID NO: 43, etc., is used; and
(ii) As DNA encoding peptide I consisting of an amino acid sequence in positions 125 to 131 of the amino acid sequence represented by SEQ ID NO: 43, DNA consisting of a nucleotide sequence in positions 373 to 393 of the nucleotide sequence represented by SEQ ID NO: 43, etc., is used;

The DNA encoding the partial peptide of the present application may be any DNA so long as it comprises the nucleotide sequence encoding the partial peptide of the present application described above. Such a DNA may also be any of genomic DNA, genomic cDNA library, cDNA derived from the cells or tissues described above, cDNA library derived from the cells or tissues described above, and synthetic DNA.
The DNA encoding the partial peptide of the present application may, for example, DNA having a partial nucleotide sequence of DNA having nucleotide sequence P or Q or having a nucleotide sequence hybridizing with nucleotide sequence P or Q under high stringent conditions, and DNA having a partial nucleotide sequence of DNA encoding a peptide having an activity substantially equivalent to that of the peptide of the present application is used.
The nucleotide sequence capable of hybridizing with nucleotide sequence P or Q has the same meaning as defined above.
The hybridization method and high stringent conditions used are the same as described above.
The DNA encoding the partial peptide of the present application may be any DNA so long as it comprises the nucleotide sequence encoding the partial peptide of the present application described above. Such a DNA may also be any of genomic DNA, genomic cDNA library, cDNA derived from the cells or tissues described above, cDNA library derived from the cells or tissues described above, and synthetic DNA.
In the present specification, nucleotide sequence R refers to a nucleotide sequence represented by one sequence identification number selected from SEQ ID NO: 8 to SEQ ID NO: 12, SEQ ID NO: 24, SEQ ID NO: 28, SEQ ID NO: 39 and SEQ ID NO: 43.
The DNA encoding the precursor protein of the present application may, for example, DNA having the nucleotide sequence of DNA comprising nucleotide sequence R or comprising a nucleotide sequence hybridizing with nucleotide sequence R under high stringent conditions, and DNA comprising the nucleotide sequence of DNA encoding a precursor protein having an activity substantially equivalent to that of the precursor protein of the present application is used.
The nucleotide sequence capable of hybridizing with nucleotide sequence R has the same meaning as defined above.
The hybridization method and high stringent conditions used are the same as described above.
More specifically,

(1) As DNA encoding precursor protein A consisting of the amino acid sequence represented by SEQ ID NO: 2, DNA consisting of the nucleotide sequence represented by SEQ ID NO: 8, etc., is used;
(2) As DNA encoding precursor protein B consisting of the amino acid sequence represented by SEQ ID NO: 3, DNA consisting of the nucleotide sequence represented by SEQ ID NO: 9, etc., is used;
(3) As DNA encoding precursor protein C consisting of the amino acid sequence represented by SEQ ID NO: 4, DNA consisting of the nucleotide sequence represented by SEQ ID NO: 10, etc., is used;
(4) As DNA encoding precursor protein D consisting of the amino acid sequence represented by SEQ ID NO: 5, DNA consisting of the nucleotide sequence represented by SEQ ID NO: 11, etc., is used;
(5) As DNA encoding precursor protein E consisting of the amino acid sequence represented by SEQ ID NO: 6, DNA consisting of the nucleotide sequence represented by SEQ ID NO: 12, etc., is used;
(6) As DNA encoding precursor protein F consisting of the amino acid sequence represented by SEQ ID NO: 23, DNA consisting of the nucleotide sequence represented by SEQ ID NO: 24, etc., is used;
(7) As DNA encoding precursor protein G consisting of the amino acid sequence represented by SEQ ID NO: 27, DNA consisting of the nucleotide sequence represented by SEQ ID NO: 28, etc., is used;
(8) As DNA encoding precursor protein H consisting of the amino acid sequence represented by SEQ ID NO: 38, DNA consisting of the nucleotide sequence represented by SEQ ID NO: 39, etc., is used; and
(9) As DNA encoding precursor protein I consisting of the amino acid sequence represented by SEQ ID NO: 42, DNA consisting of the nucleotide sequence represented by SEQ ID NO: 43, etc., is used.

The polynucleotide comprising a part of the nucleotide sequence of DNA encoding the peptide of the present application or its partial peptide or a part of a nucleotide sequence complementary to the DNA is intended to encompass not only the DNA encoding the partial peptide of the present application but also the RNA therefor.

According to the present application, an antisense polynucleotide (nucleic acid) which can inhibit replication or expression of the peptide gene of the present application can be designed and synthesized on the basis of the cloned or determined nucleotide sequence of DNA encoding the peptide of the present application. The polynucleotide (nucleic acid) can hybridize with the RNA for the peptide gene of the present application, and can inhibit the synthesis or functions of the RNA, or can control and regulate the expression of the peptide gene of the present application via interaction with the peptide-related RNA of the present application. The polynucleotide complementary to the selected sequence of the peptide-related RNA of the present application, and the polynucleotide capable of specifically hybridizing with the peptide-related RNA of the present application, is useful for controlling and regulating the expression of the peptide gene of the present application in vivo and in vitro, and is useful for therapy and diagnosis of diseases. The term "correspondence" refers to homology or complementarity to a specific sequence of a nucleotide, a nucleotide sequence or nucleic acid including a gene. The term "correspondence" between the nucleotide, nucleotide sequence or nucleic acid and the peptide (protein) usually means that amino acids of the peptide (protein) are instructed to be derived from the sequence of the nucleotide (nucleic acid) or its complementary sequence. Regions in the peptide gene of the present application, such as 5'-terminal hairpin loop, 5'-terminal 6-base pair repeat, 5'-terminal non-translational region, peptide translation initiation codon, protein-coding region, ORF translation termination codon, 3'-terminal non-translation region, 3'-terminal parin dorome region, and 3'-terminal hairpin loop can be selected as a desirable region, but any regions in the peptide gene of the present application can be selected as an object.

The relationship between objective nucleic acid and a polynucleotide complementary to at least a part of a subject region, the relationship between objective nucleic acid and a polynucleotide capable of hybridizing with the subject can be said to be "antisense". The antisense polynucleotide includes a polynucleotide comprising 2-deoxy-D-ribose, a polynucleotide comprising D-ribose, other types of polynucleotide i.e. N-glycosides of a purine or pyrimidine base, other polymers having a non-nucleotide skeleton (for example, commercial protein nucleic acid and synthetic sequence-specific nucleic acid polymer), and other polymers comprising a special bond, provided that the polymers comprise a nucleotide having an arrangement permitting base pairing or base adhesion found in DNA or RNA. These can be double-stranded DNA, single-stranded DNA, double-stranded RNA, single-stranded RNA, or a DNA/RNA hybrid, and may be an unmodified polynucleotide (or an unmodified oligonucleotide), those having known modifications, for example those having a label known in the art, those having a cap, those methylated and those with one or more natural nucleotides substituted by analogues, those having modified intramolecular nucleotides, for example those having uncharged bonds (for example, methylphosphonate, phosphotriester, phosphoramidate, carbamate etc.), those having charged bonds or sulfur-comprising bonds (for example, phosphorothioate, phosphorodithioate etc.), for example those having side chain groups such as proteins (nuclease, nuclease inhibitor, toxin, antibody, signal peptide, poly-L-lysin etc.) and saccharides (for example, monosaccharide etc.), intercalating compounds (for example, acridine, psoralen etc.), those comprising chelate compounds (for example, metal, radioactive metal, boron, oxidizable metal etc.), or those comprising an alkylating agent, those having modified bonds (for example, $\alpha$-anomer type nucleic acid etc.). The nucleotide", "nucleotide" and "nucleic acid" may include not only those comprising purine and pyrimidine bases but also those containing other modified heterocyclic bases. Such modifications may be those containing methylated purine and pyrimidine, acylated purine and pyrimidine, or other heterocyclic rings. In the modified nucleotides and modified nucleotides, their sugar moieties may be modified; for example, one or more hydroxyl groups may be replaced by halogens or aliphatic groups, or converted into functional groups such as ether, amine etc.

The antisense polynucleotide (nucleic acid) of the present application RNA, DNA, or modified nucleic acid (RNA, DNA). Examples of the modified nucleic acid include, but are not limited to, sulfur derivatives and thiophosphate derivatives of nucleic acid, and degradation-resistant derivatives of polynucleoside amides and oligonucleoside amides. The antisense nucleic acid of the present application can be designed preferably according to the following plan; that is, the antisense nucleic acid in cells is made more stable, the permeation of the antisense nucleic acid into cells is further improved, the affinity for a target sense chain is further increased, and the toxicity, if any, of the antisense nucleic acid is made lower.

Such modifications are well known in the art and disclosed in, for example, J. Kawakami et al., Pharm Tech Japan, Vol. 8, p. 247, 1992; Vol. 8, p. 395, 1992; S. T. Crooke et al. ed., Antisense Research and Applications, CRC Press, 1993, etc.

The antisense nucleic acid of the present application may comprise modified sugars, bases and bonds, and can be provided in a special form such as liposomes and microspheres, in a form applicable to gene therapy or in an added form. The antisense nucleic acid used in the added form includes hydrophobic ones, for example polycations such as polylysin working to neutralize the charge of a phosphate skeleton, or lipids (for example, phospholipids, cholesterols etc.) enhancing interaction with cell membranes or increasing incorporation of nucleic acid. Preferable lipids to be added include cholesterols and their derivatives (for example, cholesteryl chloroformate, cholic acid etc.). Such lipids can adhere to the 3'- or 5'-terminus of nucleic acid, and can adhere thereto via a base, sugar, intramolecular nucleotide linkage. Other groups include, but are not limited to, capping groups arranged specifically at the 3'- or 5'-terminus of nucleic acid and preventing decomposition with a nuclease such as exonuclease, RNase etc. Such capping groups include, but are not limited to, protective groups for hydroxyl group known in the art, for example glycols such as polyethylene glycol and tetraethylene glycol.

The inhibitory activity of the antisense nucleic acid can be examined by using the transformant of the present application, the in vivo or in vitro gene expression system of the present application or the in vivo or in vitro translation system of the present application. The nucleic acid can be applied to cells by various methods known per se.

The DNA encoding the peptide of the present application may be labeled by a method known per se, and specific examples thereof include those labeled with an isotope, those labeled with fluorescence (for example, fluorescence label with fluorescein), those biotinated or those labeled with an enzyme.

For cloning of the DNA that completely encodes the peptide of the present application, the DNA may be amplified by publicly known PCR using synthetic DNA primers having a part of the nucleotide sequence of the peptide of the present application, or the DNA inserted into an appropriate vector can be selected by hybridization with a labeled DNA fragment or synthetic DNA that encodes a part or entire region of the peptide of the present application. The hybridization can be carried out, for example, according to the method described in Molecular Cloning, 2nd (J. Sambrook et al., Cold Spring Harbor Lab. Press, 1989). The hybridization may also be performed using commercially available library in accordance with the protocol described in the attached instructions.

Conversion of the nucleotide sequence of DNA can be effected by publicly known methods such as the ODA-LA PCR method, the Gapped duplex method, the Kunkel method, etc., or their modified methods, using a publicly known kit available as Mutan™-super Express Km (manufactured by Takara Shuzo Co., Ltd.), Mutan™-K (manufactured by Takara Shuzo Co., Ltd.), etc.

The cloned DNA encoding the peptide can be used as it is, depending upon purpose or, if desired, after digestion with a restriction enzyme or after addition of a linker thereto. The DNA may have ATG as a translation initiation codon at the 5' end thereof and TAA, TGA or TAG as a translation germination codon at the 3' end thereof. These translation initiation and termination codons may also be added by using an appropriate synthetic DNA adapter.

The expression vector of the peptide of the present application can be produced, for example, by (a) excising the objective DNA fragment from the DNA encoding the peptide of the present application, followed by (b) ligating the DNA fragment with an appropriate expression vector downstream a promoter in the vector.

Examples of the vector include plasmids derived from E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present application may be any promoter if it matches well with a host to be used for gene expression. In the case of using animal cells as the host, examples of the promoter include SRα promoter, SV40 promoter, HIV/LTR promoter, CMV promoter, HSV-TK promoter, etc.

Among them, CMV (cytomegalovirus) promoter, SRα promoter, etc. are preferably used. Where the host is bacteria of the genus Escherichia, preferred examples of the promoter include trp promoter, lac promoter, recA promoter, λPL promoter, 1pp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin promoter, P10 promoter, etc.

In addition to the foregoing examples, the expression vector may further optionally comprise an enhancer, a splicing signal, a poly A addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Ampr), neomycin resistant gene (hereinafter sometimes abbreviated as Neor, G418 resistance), etc. In particular, when dhfr gene is used as the selection marker together with dhfr gene-deficient Chinese hamster's cells, selection can also be made on thymidine free media.

If necessary, a signal sequence that matches with a host is added to the N-terminus of the peptide of the present application. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector comprising the DNA that encodes the peptide of the present application thus constructed, transformants can be produced.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 [Proc. Natl: Acad. Sci. U.S.A., 60, 160 (1968)), JM103 (Nucleic Acids Research, 9, 309 (1981)], JA221 [Journal of Molecular Biology, 120, 517 (1978)), HB101 [Journal of Molecular Biology, 41, 459 (1969)], C600 (Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R', NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG 1 cell derived. from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell, which can be used, are Sf9 cell (ATCC CRL1711), Sf21 cell (both cells are described in Vaughn, J. L. et al., In Vivo, 13, 213-217 (1977)), etc.

As the insect, for example, a larva of Bombyx mori, etc. can be used [Maeda et al., Nature, 315,592 (1985)].

Examples of animal cells include monkey cell COS-7, Vero, Chinese hamster cell CHO (hereinafter referred to as CHO cell), dhfr gene deficient Chinese hamster cell CHO (hereinafter simply referred to as CHO ($dhfr^-$) cell), mouse L cell, mouse AtT-20, mouse myeloma cell, rat GH 3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55 (1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995), published by Shujunsha, or Virology, 52, 456 (1973).

Thus, the transformant transformed with the expression vector comprising the DNA encoding the polypeptide can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, yeast extract, soybean cake, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultivated generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)) or in SD medium comprising 0.5% Casamino acids (Bitter, G. A. et al., Proc. Natl. Acad. Sci. U.S.A., 81,5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as 10% immobilized bovine serum is added. Preferably, pH of the medium is adjusted to about 6.2 to 6.4. Normally, the transformant is cultivated at about 27°C for about 3 to 5 days and, if necessary, the culture can be aerated or agitated.

Where animal cells are employed as the host, the transformant is cultivated in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199,519 (1967)], 199 medium [Proceeding of the Society for the Biological Medicine, 73, 1 (1950)], etc. Preferably, pH of the medium is adjusted to about 6 to about 8. The transformant is usually cultivated at about 30 to 40°C for about 15 to 60 hours and, if necessary, the culture can be aerated or agitated.

As described above, the peptide of the present application can be produced in the transformant, intracellularly, on the cell membrane or extracellularly.

The peptide of the present application can be separated and purified from the culture described above by the following procedures.

When the peptide of the present application is extracted from the culture or cells, the following procedures are appropriately used, namely, after cultivation the transformants or cells are collected by a publicly known method and suspended in an appropriate buffer. The transformants or cells are then disrupted by publicly known methods such as ultrasonication, a treatment with lysozyme and/or freeze-thaw cycling, etc., followed by centrifugation, filtration, etc. to obtain the crude extract of the peptide. A protein modifier such as urea and guanidine hydrochloride and a surfactant such as Triton X-100™ may be contained in the buffer. When the peptide is secreted in the culture broth, after completion of the cultivation the supernatant can be separated from the transformants or cells by a publicly known method to collect the supernatant.

The peptide of the present application contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charges such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the polypeptide thus obtained is in a free form, it can be converted into the salt by publicly known methods or modifications thereof. On the other hand, when the polypeptide is obtained in the form of a salt, it can be converted into the free form or in the form of a different salt by publicly known methods or modifications thereof.

The peptide of the present application produced by the recombinant can be treated, prior to or after the purification, with an appropriate protein-modifying enzyme so that the polypeptide can be appropriately modified to partially remove a polypeptide. For the protein-modifying enzyme, there is used trypsin, chymotrypsin, arginyl endopeptidase, protein kinase, glycosidase, or the like.

The antibodies to the peptide of the present application (hereinafter sometimes merely referred to as the antibody of the present application) may be either polyclonal antibodies or monoclonal antibodies, so long as they are capable of recognizing the peptide of the present application.

The antibodies to the peptide of the present application may be manufactured by publicly know methods for manufacturing antibodies or antisera, using as antigens the peptide of the present application.

**[Preparation of monoclonal antibody]**

**(a) Preparation of monoclonal antibody-producing cells**

The peptide of the present application is administered to warm-blooded animals either solely or together with carriers or diluents to the site where the antibody production is possible by the administration. In order to potentiate the antibody productivity upon the administration, complete Freund's adjuvants or incomplete Freund's adjuvants may be administered. The administration is usually carried out once every 2 to 6 weeks and 2 to 10 times in total. Examples of the applicable warm-blooded animals are monkeys, rabbits, dogs, guinea pigs, mice, rats, sheep, goats and chicken, with the use of mice and rats being preferred.

In the preparation of monoclonal antibody-producing cells, a warm-blooded animal, e.g., mice, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled peptide, which will be described later, with the antiserum followed by assaying the binding activity of the labeling agent bound to the antibody. The fusion may be carried out, for example, by the known method by Koehler and Milstein (Nature, 256, 495, 1975). Examples of the fusion promoter are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by culturing at about 20 to 40°C, preferably at about 30 to 37°C for about 1 to 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the peptide as an antigen directly or together with a carrier, adding an anti-immunoglobulin antibody (where mouse cells are used for the cell fusion, anti-mouse immunoglobulin antibody is used) labeled with a radioactive substance or an enzyme or protein A and detecting the monoclonal antibody bound to the solid phase, a method which comprises adding the culture supernatant of hybridoma to a solid phase adsorbed with an anti-immunoglobulin antibody or protein A, adding the peptide labeled with a radioactive substance or an enzyme and detecting the monoclonal antibody bound to the solid phase, and the like.

The monoclonal antibody can be selected according to publicly known methods or their modifications. In general, the selection can be effected in a medium for animal cells supplemented with HAT (hypoxanthine, aminopterin and thymidine). Any selection and growth medium can be employed as far as the hybridoma can grow there. For example, RPMI 1640 medium containing about 1 to 20%, preferably about 10 to 20% fetal bovine serum, GIT medium (Wako Pure Chemical Industries, Ltd.) containing about 1 to 10% fetal bovine serum, a serum free medium for cultivation of a hybridoma (SFM-101, Nissui Seiyaku Co., Ltd.) and the like can be used for the selection and growth medium. The cultivation is carried out generally at about 20 to 40°C, preferably at about 37°C, for 5 days to 3 weeks, preferably 1 to 2 weeks, normally in 5% $CO_2$. The antibody titer of the culture supernatant of a hybridoma can be determined as in the assay for the antibody titer in antisera described above.

**(b) Purification, of monoclonal antibody**

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, protein A or Protein G and dissociating the binding to obtain the antibody].

**[Preparation of polyclonal antibody]**

The polyclonal antibody of the present application can be manufactured by publicly know methods or modifications thereof. For example, a warm-blooded animal is immunized with an immunogen (polypeptide antigen) per se, or a complex of immunogen and a carrier protein is formed and a warm-blooded animal is immunized with the complex in a manner similar to the method described above for the manufacture of monoclonal antibodies. The product containing the antibody to the peptide of the present application is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin, hemocyanin, etc. is coupled to hapten in a cacrier-to-hapten weight ratio of approximately 0.1 to 20, preferably about 1 to about 5.

A variety of condensation agents can be used for the coupling of carrier to hapten. Glutaraldehyde, carbodiimide, maleimide activated ester and activated ester reagents comprising thiol group or dithiopyridyl group, and the like are used for the coupling.

The condensation product is administered to warm-blooded animals either solely or together with carriers or diluents to the site that can produce the antibody. In order to potentiate the antibody productivity upon the administration, complete Freund's adjuvant or incomplete Freund's adjuvant may be administered. The administration is usually made once every about 2 to 6 weeks and approximately 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as used for the determination of serum antibody titer described above. The separation and purification of the polyclonal antibody can be carried out, following the method for the separation and purification of immunoglobulins performed as in the separation and purification of monoclonal antibodies described hereinabove.

The antisense DNA having a complementary or substantial complementary nucleotide sequence to the DNA encoding the peptide of the present application (hereinafter sometimes referred to as the antisense DNA of the present application) can be any antisense DNA, so long as it has a nucleotide sequence complementary or substantially complementary to that of the DNA of the present application and capable of suppressing expression of the DNA.

The nucleotide sequence substantially complementary to the DNA of the present application may, for example, be a nucleotide sequence having at least about 70% homology, preferably at least about 80% homology, more preferably at least about 90% homology and most preferably at least about 95 % homology, to the full-length nucleotide sequence or partial nucleotide sequence of the nucleotide sequence complementary to the DNA of the present application (i.e., complementary strand to the DNA of the present application). Particularly in the entire nucleotide sequence of the complementary strand to the DNA of the present application, an antisense DNA having at least about 70% homology, preferably at least about 80% homology, more preferably at least about 90% homology and most preferably at least about 95% homology, to the complementary strand of the nucleotide sequence which encodes the N-terminal site of the peptide of the present application (e.g., the nucleotide sequence around the initiation codon). These antisense DNAs can be synthesized using publicly known DNA synthesizers, etc.

Hereinafter, [1] the peptide of the present application, [2] the DNA of the present application [3] the antibody of the present application and [4] the antisense DNA are explained in terms of their use.

### (1) Therapeutic/prophylactic agent for various diseases with which the peptide of the present application is associated

The peptide of the present application and the DNA of the present application can be used as pharmaceutical preparations for the treatment/prevention of diseases caused by abnormalities and deficiency in the peptide of the present application or the DNA of the presen application, for example diseases related to deficiency in the functions of the peptide of the present application.

For example, secretory protein F of the present application, peptide F of the present application or its partial peptide, precursor protein F of the present application, or its amide or ester, or a salt thereof (hereinafter referred to sometimes as peptide F of the present application), [1] secretory protein G of the present application, peptide G of the present application or its partial peptide, precursor protein G of the present application, or its amide or ester, or a salt thereof (hereinafter referred to sometimes as peptide G of the present application), [2] secretory protein H of the present application, peptide H of the present application or its partial peptide, precursor protein H of the present application, or its amide or ester, or a salt thereof (hereinafter referred to sometimes as peptide H of the present application), and [3] secretory protein I of the present application, peptide I of the present application or its partial peptide, precursor protein I of the present application, or its amide or ester, or a salt thereof (hereinafter referred to sometimes as peptide I of the present application) can bind to AQ27 receptor, and therefore, the DNA encoding F, G, H or I of the present application is useful as a safe and low toxic pharmaceutical preparation, for example, a hormone secretion regulator (for example, a promoter for secretion of adrenal cortical hormone such as corticosterone), an eating regulator, a sleep regulator, an arousal regulator, a pain regulator, a stress response regulator, a spontaneous behavior regulator or an emotional behavior regulator.

F, G, H or I of the present application and the DNA encoding the same are useful as prophylactic/therapeutic agents for diseases in the central nerves (for example, Alzheimer's disease/dementia/difficulty in eating (anorexia)/ epilepsy, etc.), diseases in the hormone system (for example, faint labor, flaccid bleeding, before and after placenta expulsion, insufficiency in uterine restoration, Caesarean operation, artificial abortion, milk statis etc.), diseases in liver/ gall/pancreas/internal secretion (for example, diabetes/difficulty in eating, etc.), inflammatory diseases (allergy/asthma/ rheumatism etc.) and circulatory organ diseases (for example, high blood pressure/cardiac hypertrophy/angina/arteriosclerosis, etc.).

The peptide F, G, H or I of the present application can bind to OT7T022 receptor, and therefore, the peptide F, G, H or I of the present application and the DNA coding the same are useful as safe and low toxic pharmaceutical preparations, for example, a prolactin secretion regulator. That is, when the peptide F, G, H or I of the present application has an activity of promoting secretion of prolactin, the peptide F, G, H or I of the present application and the DNA encoding the same are useful as prolactin secretion promoters, for example a prophylactic/therapeutic agent for diseases related to deficiency in secretion of prolactin, such as depression of ovarian functions, deficiency in growth of seminal vesicle, osteoporosis, menopausal disorders, deficiency in secretion of milk, hypothyroidism, renal insufficiency, etc. Further, when the peptide F, G, H or I of the present application has a promoting action on secretion of prolactin, the peptide F, G, H or I of the present application and the DNA encoding the same have a promoting action on sexual desire (pheromone-like action) based on the promoting action on secretion of prolactin, and is thus useful as an agent for promoting sexual desire.

When the peptide F, G, H or I of the present application has an inhibitory action on secretion of prolactin, the peptide F, G, H or I of the present application and the DNA encoding the same is useful as a prolactin secretion inhibitor, for example a prophylactic/therapeutic agent for various diseases related to excessive secretion of prolactin, for example, diseases related to prolactin secretion, such as hyperprolactinemia, pituitary gland tumor, interbrain tumor, abnormal menstruation, stress, autoimmune disease, prolactinoma, sterility, impotence, amenorrhea, galactorrhea, acromegaly, Chiari-Frommel syndrome, Argonz-del Castilo syndrome, Forbes-Albright syndrome, breast cancer lymphoma, Sheehan's syndrome or abnormality in spermatogenesis. Further, when the peptide F, G, H or I of the present application has an inhibitory action on secretion of prolactin, the peptide F, G, H or I of the present application and the DNA encoding the same is also useful as a contraceptive agent based on the inhibitory action on secretion of prolactin.

In addition, the peptide F, G, H or I of the present application is also useful as a diagnostic drug for examining an ability to secrete prolactin or as an animal drug such as a milk secretion promoting agent for mammals such as bovine, goat, swine etc., and can be applied to production of a useful substances by producing the useful substance in the mammals and secreting it in milk.

Since the peptide F, G, H or I of the present application has a regulatory action on the functions of placenta, the peptide F, G, H or I of the present application and the DNA encoding the same are useful as prophylactic/therapeutic agents for choriocarcinoma, hydatidiform mole, invasive mole, abortion, insufficiency in fetal growth, abnormality in sugar metabolism, abnormality in lipid metabolism or delivery induction.

The regulatory activity of prolactin secretion can be measured by a method described in Neuroendocrinology, 62, 198-206 (1995) or Neuroscience Letters, 203, 164-170 (1996), or by a modification thereof.

Further, the peptide F, G, H or I of the present application and the DNA encoding the same can be used as therapeutic/prophylactic agents for diseases such as high blood pressure, autoimmune disease, cardiac insufficiency, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, bone fracture, breast cancer, bulimia, [bulimia], burn healing, uterine cervix cancer, chronic lymphatic leukemia, chronic myeloid leukemia, chronic pancreatitis, hepatocirrhosis, large-intestine cancer (colon/rectum cancer), Crohn's disease, dementia, diabetic complications, diabetic nephritis, diabetic neuropathy, diabetic retinitis, gastritis, helicobacter pylori infection, renal insufficiency, type A hepatitis, type B hepatitis, type C hepatitis, hepatitis, herpes simplex virus infection, varicella zoster virus infection, Hodgkin's disease, AIDS infection, human papilloma virus infection, hypercalcemia, hypercholesterolemia, hyperglyceridemia, hyperlipemia, infections, influenza infection, insulin-dependent diabetes (type I), invasive Staphylococcus infection, malignant melanoma, cancer metastasis, multiple myeloma, allergic rhinitis, nephritis, non-Hodgkin lymphoma, insulin-independent diabetes (type II), non-small cell lung cancer, organ transplant operation, bone arthritis, bone softening, bone decrease, osteoporosis, ovary cancer, bone Behcet's disease, digestive ulcer, peripheral blood vessel disease, prostate cancer, reflux esophagitis, renal insufficiency, rheumatoid arthritis, schizophrenia, septicemia, septicemic shock, severe systemic fungal infection, small cell lung cancer, spinal damage, stomach cancer, systemic lupus erythematosus, transient cerebral ischemia attack, tuberculosis, valvular heart disease, vascular/multiple infarct dementia, wound healing, insomnia, arthritis, insufficiency in secretion of pituitary hormone, frequent urination, uremia, or nerve degeneration disease.

Further, the peptide F, G, H or I of the present application and the DNA encoding the same can be used as pharmaceutical preparations such as therapeutic/prophylactic agents for a disease macular edema cystoid.

Further, the peptide F, G, H or I of the present application and the DNA encoding the same are useful for example as (1) a therapeutic agent for tumors such as acromegaly, TSH-producing tumor, non-secretory (non-functional) pituitary tumor, ectopic ACTH (adrenocorticotropine)-producing tumor, marrow-like thyroid cancer, VIP-producing tumor, glucagon-producing tumor, gastrin-producing tumor, insulinoma, carcinoid etc., (2) a therapeutic agent for insulin dependent or independent diabetes, or various diseases related to such diabetes, that is, diabetic complications (for example, diabetic retinitis, diabetic nephritis, diabetic neuropathy, down syndrome, erectile low blood pressure, etc.), (3) a therapeutic agent for obesity, bulimia etc. by ameliorating hyperinsulinemia or suppressing appetite, (4) a therapeutic agent for acute pancreatitis, chronic pancreatitis, pancreas/intestine physter, bleeding ulcer, digestive ulcer, gastritis, gastric hyperacidity, reflux esophagitis, etc., (5) an agent for ameliorating various symptoms caused by infection with helicobacter pylori bacteria (for example, an agent for inhibiting promotion of gastrin secretion, etc.), (6) an agent for suppressing amylase secretion caused by endoscopic cholangio-pancreatography, and for prognosis of surgical operation of pancreas, (7) a therapeutic agent for diarrhea attributable to a reduction of the absorbing ability of small intestine, acceleration of secretion, or abnormality in mobility of digestive tract (for example, short bowel syndrome, etc.), diarrhea attributable to chemicals in chemotherapy of cancer, diarrhea attributable to congenital shrinkage of small intestine, diarrhea attributable to neuroendocrine tumor such as VIP-producing tumor, diarrhea attributable to AIDS, diarrhea attributable to graft versus host reaction accompanying a marrow transplant, diarrhea attributable to diabetes, diarrhea attributable to blockage of abdominal nerve plexuses, diarrhea attributable to systematic sclerosis, diarrhea attributable to eosinophilia, etc., (8) a therapeutic agent for damping syndrome, hypersensitive colitis, Crohn's disease, inflammatory intestinal diseases, etc., (9) a therapeutic agent for tumors or cancers (for example, thyroid cancer, large intestine cancer, breast cancer, prostate cancer, small cell lung cancer, non-small cell lung cancer, pancreas cancer, stomach cancer, cholangioma, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (for example, basophil leukemia/ chronic lymphatic leukemia, chronic myeloid leukemia, Hodgkin's disease, non-Hodgkin lymphoma, etc.); the therapeutic agent can be used alone or in combination with other anticancer drugs (for example, tamoxifen, LHRH agonist, LHRH antagonist, interferon-$\alpha$,$\beta$ and $\gamma$, interleukin-2, etc.), (10) a prophylactic/therapeutic agent for hypertrophic myocardial disease, arteriosclerosis, valvular heart disease, myocardial infarction (particularly, myocardial infarction after percutaneous transluminal coronary angioplasty), revascularization, (11) a therapeutic agent for esophagus vein cancer bleeding, hepatocirrhosis, peripheral blood vessel disease, (12) a therapeutic agent for diseases accompanying systemic or topical inflammations (for example, multiple arteritis, rheumatoid arthritis, psoriasis, sunburn, eczema, allergies (for example, asthma, atopic dermatitis, allergic rhinitis, etc.)), based on the secretion-regulating action of phsyoloigcally active substance acting on the immune system (for example, substance P, tachykinin, cytokine, etc.), (13) a therapeutic agent for dementia (for example, Alzheimer's disease, Alzheimer type senile dementia, vascular/multiple dementia, etc.), schizophrenia, epilepsy, depression, general anxiety, difficult sleep, multiple sclerosis, etc., because of its influence on production and secretion of nerve-regulating factor, (14) a therapeutic agent for eye diseases (for example, glaucoma, etc.), (15) a prophylactic/therapeutic agent for acute bacterial meningitis, acute viral encephalitis, adult respiratory distress syndrome, bacterial pneumonia, severe systemic fungal infection, tuberculosis, spinal damage, bone fracture, renal insufficiency, pneumonia, alcoholic hepatitis, type A hepatitis, type B hepatitis, type C hepatitis, AIDS infection, human papilloma virus infection, influenza infection, cancer metastasis, multiple myeloma, bone softening, osteoporosis, bone Behcet's disease, nephritis, renal insufficiency, septicemia, septicemic shock, hypercalcemia, hypercholesterolemia, hyperglyceridemia, hyperlipemia, systemic lupus erythematosus, transient cerebral ischemia attack, alcoholic hepatitis, etc. (16) a therapeutic agent for organ transplant operation, burn, wound, alopecia, etc., and (17) an analgesic for suppressing and relieving chronic or acute pains (for example, acute pain after operation, inflammatory acute pain, toothache, bone diseases (for example, acute pains accompanying arthritis, rheumatism, osteoporosis. etc.)).

When a patient has a reduced level of, or deficient of the peptide of the present application in his or her body, the peptide of the present application can provide its role sufficiently or properly for the patient, (a) by administering the DNA of the present application to the patient to express the peptide of the present application in vivo, (b) by inserting the DNA of the present application into a cell, expressing the peptide of the present application and then transplanting the cell to the patient, (c) by administering the peptide of the present application to the patient, or the like.

Where the DNA of the present application is used as the prophylactic/therapeutic agent described above, the DNA itself is administered directly to human or other warm-blooded animal; alternatively, the DNA is inserted into an appropriate vector such as retrovirus vector, adenovirus vector, adenovirus-associated virus vector, etc. and then administered to human or other warm-blooded animal in a conventional manner. The DNA of the present application may also be administered as an intact DNA, or prepared into pharmaceutical preparations together with physiologically acceptable carriers such as adjuvants to assist its uptake, which are administered by gene gun or through a catheter such as a hydrogel catheter.

Where the peptide of the present application is used as the aforesaid therapeutic/prophylactic agents, the polypeptide is advantageously used on a purified level of at least 90%, preferably at least 95%, more preferably at least 98% and most preferably at least 99%.

The peptide of the present application can be used orally, for example, in the form of tablets which may be sugar coated if necessary and desired, capsules, elixirs, microcapsules etc., or parenterally in the form of injectable preparations such as a sterile solution and a suspension in water or with other pharmaceutically acceptable liquid. These preparations can be manufactured by mixing the peptide of the present application with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin, alginic acid, etc., a lubricant such as magnesium stearate, a sweetening agent such as sucrose, lactose or saccharin, and a flavoring agent such as peppermint, akamono oil or cherry. When the unit dosage is in the form of capsules, the material of such type may further comprise liquid carriers such as oils and fats.. A sterile composition for injection may be formulated according to a conventional manner used to make pharmaceutical compositions, e.g., by dissolving or suspending the active ingredient in a vehicle such as water for injection with a naturally occurring vegetable oil such as sesame oil, coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil, soybean oil, etc., which may also be used in combination with a dissolution aid such as benzyl benzoate, benzyl alcohol, etc. The peptide of the present application may further be formulated with a buffer .(e.g., phosphate buffer, sodium acetate buffer, etc.), a soothing agent (e.g., benzalkonium .chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

The vector in which the DNA of the present application is inserted may also be prepared into pharmaceutical preparations in a manner similar to the procedures above, and such preparations are generally used parenterally.

Since the thus obtained pharmaceutical preparation is safe and low toxic, and can be administered to, for example, human or warm-blooded animals (e.g., rat, mouse, guinea pig, rabbit, chicken, sheep, swine, bovine, horse, cat, dog, monkey, etc.).

The dose of the peptide of the present application may vary depending on target disease, subject to be administered, route for administration, etc. When the peptide of the present application is orally administered, the peptide of the present application is administered to adult (as 60 kg) generally in a daily dose of approximately 0.1 mg to 100 mg, preferably approximately 1.0 mg to 50 mg, more preferably approximately 1.0 to 20 mg. When the peptide of the present application is parenterally administered, a single dose of the peptide of the present application may vary depending on subject to be administered, target disease, etc. When the peptide of the present application is administered to adult (as 60 kg body weight), it is convenient to administer the peptide by injection to the effected area, generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

(2) Screening of a candidate drug for diseases

The peptide of the present application can be used in the method of screening a compound or its salt that promotes or inhibits the functions of the peptide of the present application.

The DNA of the present application can be used in the method of screening a compound or its salt that promotes or inhibits the expression of the peptide of the present application.

Hereinafter, the screening method using the peptide of the present application or the DNA of the present application is specifically described.

(2-1) Method of screening an agonist or antagonist for the receptor to the peptide of the present application

In the screening, the peptide of the present application is used, or an expression system for the recombinant peptide of the present application is constructed and a receptor biding assay system using the expression system is used, whereby compounds altering the binding property between the peptide of the present application and the receptor (compounds promoting or suppressing the activity of the peptide of the present application) (for example, peptides, proteins, non-peptide compounds, synthetic compounds, fermentation products, etc.) or salts thereof can be screened. Such compounds include compounds (receptor agonists) which have cell-stimulating activities via the receptor to the peptide of the present application (e.g., the activity that promotes arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, GTPγS binding activity, etc.) or compounds (receptor antagonists) which do not have such activities. The phrase "alternating the binding property between the peptide and the ligand encompasses both inhibition and promotion of binding between the two.

That is, the present application disclosure a method of screening a compound or its salt that promotes or inhibits the activity of the peptide of the present application specifically a method of screening compounds (compounds promoting or inhibiting the activity of the peptide of the present application) altering the binding property between the peptide of the present application and the receptor, which comprises comparing the case (i) where the peptide of the present application is brought into contact with the receptor or its partial peptide (hereinafter referred to collectively as the receptor) and the case (ii) where the peptide of the present application and a test compound are brought into contact with the receptor.

The screening method of the present application comprises measuring and comparing, for example, the amount of the ligand bound to the receptor, cell stimulating activity or the like between the case (i) where the peptide of the present application is brought into contact with the receptor and the case (ii) the peptide of the present application and a test compound are brought into contact with the receptor.

Specifically, the screening method of the present application includes:

[1] a method of screening the compounds (the compounds that promote or inhibit the activities of the peptide of the present application) or salts thereof that alter the binding property between the peptide of the present application and the receptor, which comprises measuring and comparing the binding amount of a labeled form of the peptide of the present application to the receptor, when the labeled peptide of the present application is brought into contact with the receptor, and when the labeled peptide of the present application and a test compound are brought into contact with the receptor;

[2] a method of screening the compounds (the compounds that promote or inhibit the activities of the peptide of the present application) or salts thereof that alter the binding property between the peptide of the present application and the receptor; which comprises measuring and comparing the binding amount of a labeled form of the peptide of the present application to a cell comprising the receptor or a membrane fraction of the cell, when the labeled peptide of the present application is brought into contact with the cell comprising the reseptor or membrane fraction, and when the labeled peptide of the present application and a test compound are brought into contact with the cell or membrane fraction;

[3] a method of screening the compounds (the compounds that promote or inhibit. the activities of the peptide of the present application) or salts thereof that alter the binding property between the peptide of the present application and the receptor, which comprises measuring and comparing the binding amount of a labeled form of the peptide of the present application to the receptor, when the labeled peptide of the present application is brought into contact with the receptor expressed on a cell membrane by culturing the transformant comprising the DNA encoding the receptor, and when the labeled peptide of the present application and a test compound are brought into contact with the receptor expressed on a cell membrane by culturing the transformant comprising the DNA encoding the receptor;

[4] a method of screening the compounds (the compounds that promote or inhibit the activities of the peptide of the present application) or salts thereof that alter the binding property between the peptide of the present application and the receptor, which comprises measuring and comparing a cell stimulating activity mediated by the receptor (e.g., the activity that promotes or suppresses arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, GTP$\gamma$S binding activity etc), when a compound that activates the receptor (e.g., the peptide of the present application) is brought into contact with a cell comprising the receptor, and when a compound that activates the receptor and a test compound are brought into contact with a cell comprising the receptor; and,

[5] a method of screening the compounds (the compounds that promote or inhibit the activities of the peptide of the present application) or salts thereof that alter the binding property between the peptide of the present application and the receptor, which comprises measuring and comparing a cell stimulating activity mediated by the receptor (e.g., the activity that promotes or suppresses arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, GTP$\gamma$S binding actiyity, etc.), when a compound that activates the receptor (e.g., the peptide of the present application) is brought into contact with the receptor expressed on a cell membrane by culturing the transformant comprising the DNA encoding the receptor, and when a compound that activates the receptor and a test compound are brought into contact with the receptor expressed on a cell membrane by culturing the transformant comprising the DNA encoding the receptor.

The screening methods of the present application are specifically explained below.

First, the receptor used in the screening method of the present application may be any receptor insofar as it recognizes the peptide of the present application ligand, but the receptor is preferably the one in membrane fractions from organs in humans or warm-blooded animals. However, acquisition of human-derived organs is extremely difficult, and thus the receptor used in screening is preferably the one expressed in a large amount by a transformant. The receptor can be produced according to a method known in the art.

When cells comprising the receptor or membrane fractions of these cells are employed in the screening methods of the present application they may be prepared following the procedures later described

Where cells comprising the receptor are employed, the cells may be fixed using glutaraldehyde, formalin, etc. The fixation can be made by publicly known methods.

The cells comprising the receptor refer to host cells wherein the receptor is expressed, and such host cells include the above-described Escherichia coli, Bacillus subtilis, yeast, insect cells, animal cells, etc. The host cells that have expressed the receptor may be acquired in a similar manner to the aforesaid method of producing transformants transformed by the expression vector comprising the peptide of the present application.

The cell membrane fraction is a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor expressed and membrane components such as cell-derived phospholipids and membrane proteins.

The amount of the receptor in the cells comprising the receptor or in the membrane fraction is preferably $10^3$

to $10^8$ molecules per cell, more preferably $10^5$ to $10^7$ molecules per cell. As the amount of expression increases, the ligand binding activity per unit of the membrane fraction (specific activity) increases so that not only the highly sensitive screening system can be constructed but also large quantities of samples can be assayed with the same lot.

To perform the methods [1] through [3] for screening the compound (the compound that promotes or inhibits the activity of the peptide of the present application) that alters the binding property between the peptide of the present application and the receptor, an appropriate receptor fraction and a labeled form of the peptide of the present application are employed. For the receptor fraction, the receptor fractions of naturally occurring tissues or cells or recombinant receptor fractions having an activity equivalent thereto, or the like are desirable. Herein, the equivalent activity refers to an equivalent ligand binding activity, etc. As the labeled ligands, there may be used the labeled form of the ligand, the labeled form of a ligand analogue, etc. The ligands labeled with [$^3$H], [$^{125}$I], [$^{14}$C], [$^{35}$S] or the like can be employed. Among them, the ligand labeled with [$^{125}$I] is preferable.

Specifically, the screening of the compound that alters the binding property between the peptide of the present application and the receptor can be performed by the following procedures. First, a receptor preparation is prepared by suspending cells comprising the receptor or their membrane fractions in a buffer appropriate for screening. Any buffer can be used so long as it does not inhibit ligand-receptor binding, such buffers including a phosphate buffer or a Tris-HCl buffer having pH of 4 to 10 (preferably pH of 6 to 8), etc. For the purpose of reducing non-specific binding, a surfactant such as CHAPS, Tween-80™ (manufactured by Kao-Atlas Inc.), digitonin or deoxycholate may be added to the buffer. Further for the purpose of suppressing the degradation of the receptor or the peptide of the present application by a protease, a protease inhibitor such as PMSF, leupeptin, E-64 (manufactured by Peptide Institute, Inc.) and pepstatin may also be added. A given amount (5,000 cpm to 500,000 cpm) of a labeled form of the peptide of the present application is added to 0.01 ml to 10 ml of the receptor solution, and at the same time, $10^{-10}$ to $10^{-7}$ M of a test compound is co-present. To determine the amount of non-specific binding (NSB), a reaction tube containing a large excess of the peptide of the present application in an unlabeled form is also provided. The reaction is carried out at 0° C to 50°C, preferably about 4°C to 37°C for 20 minutes to 24 hours, preferably 30 minutes to 3 hours. After completion of the reaction, the reaction mixture is filtrated through glass fiber filter paper, etc. and washed with an appropriate volume of the same buffer. The residual radioactivity in the glass fiber filter paper is then measured by means of a liquid scintillation counter or a $\gamma$-counter. When the nonspecific binding (NSB) is subtracted from the count ($B_0$) when any antagonizing compound is absent and the thus obtained count ($B_0$ - NSB) is made 100%, a test compound having the specific binding (B - NSB) of, e.g., 50% or less can be selected as a candidate material capable of competitive inhibition.

To perform the method (4) or (5) given above for screening the compound (the compound that promotes or inhibits the activity of the peptide of the present application) that alters the binding property between the peptide of the present application and the receptor, the cell-stimulating activities mediated by the receptor (e.g., the activity that promotes or suppresses arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, GTPyS binding activity, etc.) may be determined by a publicly known method, or using an assay kit commercially available. Specifically, the cells comprising the receptor are first cultured on a multi-well plate, etc. Prior to the screening, the medium is replaced with a fresh medium or with an appropriate non-cytotoxic buffer, followed by culturing for a given period of time in the presence of a test compound, etc. Subsequently, the cells are extracted or the supernatant is recovered and the resulting product is quantified by appropriate procedures. Where it is difficult to detect the production of the indicator substance for the cell-stimulating activity (e.g., arachidonic acid, etc.) due to a degrading enzyme contained in the cells, an inhibitor against such a degrading enzyme may be added prior to the assay. For detecting activities such as the cAMP production suppression activity, the baseline production in the cells is increased by forskolin or the like and the suppressing effect on the increased baseline production can be detected.

To perform the screening by assaying the cell stimulating activity, cells in which an appropriate receptor is expressed are required. As the cells wherein the receptor is expressed, the above-described receptor-expressing cell lines, etc. are desirable.

Examples of the test compound include peptides, proteins, non-peptide compounds, synthetic compounds, fermentation products, cell extracts, plant extracts, animal tissue extracts, and the like.

The test compound used is preferably a compound which on the basis of the atomic coordinates of a receptor active site and the position of a ligand binding pocket, is designed to bind to the ligand binding pocket. The atomic coordinates of the receptor active site and the position of the ligand binding pocket can be measured by a known method or a modification thereof.

Further, the compound or its salt that alters the binding property between the peptide of the present application and the receptor can also be used in place of the peptide of the present application. The compound or its salt altering the binding property between the peptide of the present application and the receptor can be obtained by for example the screening method of the present application using the peptide of the present application as ligand, which will be described later.

The kit for screening the compound (the compound that promotes or inhibits the activity of the peptide of the present application) that alters the binding property between the peptide of the present application and the receptor, or salts thereof, comprises the preceptor or its salt, a partial peptide of the receptor or its salt, cells comprising the receptor, or a membrane fraction of cells comprising the receptor, and the peptide of the present application.
Examples of the screening kit of the present application include:

1. Screening reagents

[1] Measurement buffer and washing buffer

Hanks' balanced salt solution (GIBCO) plus 0.05% bovine serum albumin (Sigma).
The buffer may be filtered for sterilization through a filter having a pore diameter of 0.45 μM and stored at 4°C, or may be prepared just before use.

[2] Receptor preparation

Receptor-expressing CHO cells previously passaged at a density of $5 \times 10^5$ cells/well in a 12-well plate and cultured for 2 days at 37°C in 5% $CO_2$ and 95% air.

[3] Labeled ligand

A solution prepared by dissolving the peptide of the present application labeled with [$^3$H], [$^{125}$I], [$^{14}$C], [$^{35}$S] etc. in a suitable solvent or buffer is stored at 4°C or -20°C and diluted at 1 μM with the measurement buffer just before use.

[4] Ligand standard solution

The peptide of the present application is dissolved at 1 mM in PBS containing 0.1 % bovine serum albumin (Sigma) and stored at -20°C.

2. Measurement method

[1] The receptor-expressing cells cultured in a 12-well tissue culture plate are washed twice with 1 ml measurement buffer, and then 490 μl measurement buffer is added to each well.
[2] 5 μl of $10^{-3}$ to $10^{-10}$ M test compound solution is added to each well, and then 5 μl labeled peptide of the present application is added thereto, and the mixture is reacted at room temperature for 1 hour. To know the non-specific binding, 5 μl of $10^{-3}$ M peptide of the present application is added in place of the test compound.
[3] The reaction solution is removed, and the cells are washed 3 times with 1 ml washing buffer. The labeled peptide of the present application bound to the cells is lyzed with 0.2 N NaOH-1% SDS and mixed with 4 ml liquid scintillator A (Wako Pure Chemical Industries, Ltd.).
[4] The activity is measured with a liquid scintillation counter (Beckmann), and Percent Maximum Binding (PMB) is determined according to the following equation (1):

$$PMB = [(B - NSB)/B_0 - NSB)] \times 100 \qquad (1)$$

PMB: Percent maximum binding.
B: Binding when a sample was added.
NSB: Non-specific binding.
$B_0$: Maximum binding.

The compounds or salts thereof, which are obtainable using the screening methods or screening kits of the present application, are compounds (the compounds that promote or inhibit the activity of the peptide of the present application) that alter the binding (i.e. inhibit or promote the binding) between the peptide of the present application and the receptor, and are specifically compounds having the cell stimulating activities via the receptor, or salts thereof (so-called agonists of the receptor), or compounds having no such stimulating activities (so-called antagonists of the receptor).

Examples of these compounds include peptides, proteins, non-peptide compounds, synthetic compounds, fermentation products, and the like. These compounds may be novel compounds or publicly known compounds.

To specifically determine if the compound is the agonist or antagonist of the receptor, the following method (i) or (ii) may be used.

(i) After the compound that alters the binding property between the peptide of the present application and the receptor, especially the compound that inhibits the binding, is obtained by binding assays shown in the screening methods [1] to [3] described above, it is determined if the compound has the aforesaid cell-stimulating activity mediated by the receptor. The compound having the cell-stimulating activity or its salt is an agonist of the receptor, whereas the compound or its salt having no such activity is an antagonist of the receptor.

(ii)

(a) A test compound is brought into contact with cells comprising the receptor to assay the aforesaid cell-stimulating activity mediated by the receptor. The compound or its salt having the cell-stimulating activity is an agonist of the receptor.

(b) The cell-stimulating activity mediated by the receptor is measured, both when the compound that activates the receptor (e.g., an agonist of the peptide of the present application, etc.) is brought into contact with cells comprising the receptor and when the compound that activates the receptor and a test compound are brought into contact with cells comprising the receptor, and comparison is made on the cell-stimulating activity between the two cases. The compound capable of reducing the cell-stimulating activity exerted by the compound or its salt that activates the receptor is an antagonist of the receptor.

The agonists of the receptor exhibit similar activities to the physiological activities that the peptide of the present application has on the receptor, and are thus useful as safe and low toxic drugs, likewise the peptide of the present application.

Conversely, since the antagonists of the receptor can suppress the physiological activities that the peptide of the present application has on the receptor, they are useful as safe and low toxic drugs that suppress the physiological activities of the peptide of the present application.

Examples of the compounds or salts thereof, which are obtainable using the screening methods or screening kits of the present application, are compounds selected from, e.g., peptides, proteins, non-peptide compounds, synthetic compounds, fermentation products, cell extracts, plant extracts, animal tissue extracts, blood plasma, and the like, and are the compounds that promote or inhibit the functions of the peptide of the present application.

For salts of these compounds, the same salts as those given for the peptide of the present application above may be used.

When the compounds obtainable using the screening methods or screening kits of the present application are used as the therapeutic/prophylactic agents described above, a conventional manner may apply thereto. The compounds may be prepared in the form of tablets, capsules, elixirs, microcapsules, sterile solutions, suspensions, etc. in a manner similar to the method for preparing the pharmaceutical preparation comprising the peptide of the present application described hereinabove.

Since the thus obtained pharmaceutical preparation is safe and low toxic, the preparation can be administered to, for example, human and warm-blooded animal (e.g., mouse, rat, rabbit, sheep, swine, bovine, horse, chicken, cat, dog, monkey, chimpanzee, etc.).

The dose of the compound or its salt may vary depending on its action, target disease, subject to be administered, route for administration, etc. When the receptor antagonist is orally administered, the compound is administered to adult (as 60 kg) generally in a daily dose of approximately 0.1 mg to 100 mg, preferably approximately 1.0 mg to 50 mg, more preferably approximately 1.0 to 20 mg. When the receptor antagonist is parenterally administered, a single dose of the compound may vary depending on subject to be administered, target disease, etc. When the receptor antagonist is administered to adult (as 60 kg body weight) in the form of injection, it is convenient to administer the compound by intravenous injection, generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

Specifically, when the peptide of the present application is the peptide F, G, H or I of the present application, the receptor used is:

(1) G protein-conjugated receptor protein or its salt (hereinafter referred to sometimes as AQ27 receptor) comprising the same or substantially the same amino acid sequence as the amino acid sequence represented by SEQ ID NO: 31, SEQ ID NO: 44 or SEQ ID NO: 46, or

(2) G protein-conjugated receptor protein or its salt (hereinafter referred to sometimes as OT7T022 receptor) comprising the same or substantially the same amino acid sequence as the amino acid sequence represented by SEQ ID NO: 33 or SEQ ID NO: 35.

The amino acid sequence which is substantially the same amino acid sequence as the amino acid sequence represented by SEQ ID NO: 31, SEQ ID NO: 44, SEQ ID NO: 46, SEQ ID NO: 33 or SEQ ID NO: 35 includes, for example, an amino acid sequence having at least about 70% homology, preferably at least about 80% homology, more preferably at least about 90% homology, still more preferably at least about 95% homology, to the amino acid sequence represented by SEQ ID NO: 31, SEQ ID NO: 44, SEQ ID NO: 46, SEQ ID NO: 33 or SEQ ID NO: 35.

The protein containing the amino acid sequence which is substantially the same amino acid sequence as the amino acid sequence represented by SEQ ID NO: 31, SEQ ID NO: 44, SEQ ID NO: 46, SEQ ID NO: 33 or SEQ ID NO: 35 is preferably a protein having substantially the same amino acid sequence as the amino acid sequence represented by SEQ ID NO: 31, SEQ ID NO: 44, SEQ ID NO: 46, SEQ ID NO: 33 or SEQ ID NO: 35 and having an activity substantially equivalent to that of the protein consisting of the amino acid sequence represented by SEQ ID NO: 31, SEQ ID NO: 44, SEQ ID NO: 46, SEQ ID NO: 33 or SEQ ID NO: 35.

The substantially equivalent activity includes, for example, a ligand binding activity, signal information transmitting action, and the like. The term substantially equivalent means that the activity is equivalent qualitatively. Thus, the activities such as a ligand binding activity, signal information transmitting action etc. are preferably equivalent in strength (e.g., about 0.5 to about 2 times), and even quantitative differences such as the strength of these activities, molecular weight of proteins, etc, may be present.

The activities such as a ligand binding activity, signal information transmitting action, etc. can be measured in the same manner as described above.

As AQ27 receptor or OT7T022 receptor, a protein is used which comprises [1] an amino acid sequence wherein in the amino acid sequence represented by SEQ ID NO: 31, SEQ ID NO: 44, SEQ ID NO: 46, SEQ ID NO: 33 or SEQ ID NO: 35, one or more (preferably about 1 to 30, more preferably about 1 to 10, still more preferably a few (1 or 2)) amino acids were deleted, [2] an amino acid sequence wherein in the amino acid sequence represented by SEQ ID NO: 31, SEQ ID NO: 44, SEQ ID NO: 46, SEQ ID NO: 33 or SEQ ID NO: 35, one or more (preferably about 1 to 30, more preferably about 1 to 10, still more preferably a few (1 or 2)) amino acids were added, [3] an amino acid sequence wherein in the amino acid sequence represented by SEQ ID NO: 31, SEQ ID NO: 44, SEQ ID NO: 46, SEQ ID NO: 33 or SEQ ID NO: 35, one or more (preferably about 1 to 30, more preferably about 1 to 10, still more preferably a few (1 or 2)) amino acids were replaced by other amino acids, or [4] an amino acid sequence wherein the deletion, addition and replacement described above were combined.

AQ27 receptor or OT7T022 receptor is represented in accordance with the conventional way of describing peptides, that is, the N-terminus (amino terminus) at the left hand and the C-terminus (carboxyl terminus) at the right hand. In AQ27 receptor or OT7T022 receptor including the protein comprising the amino acid sequence shown by SEQ ID NO: 31, SEQ m NO: 44, SEQ ID NO: 46, SEQ ID NO: 33 or SEQ ID NO: 35, the C-terminus is usually in the form of a carboxyl group (-COOH) or a carboxylate ($-COO^-$) but may be in the form of an amide ($-CONH_2$) or an ester (-COOR).

Examples of R in the ester group include a $C_{1-6}$ alkyl group such as methyl, ethyl, n-propyl, isopropyl, n-butyl, etc.; a $C_{3-8}$ cycloalkyl group such as cyclopentyl, cyclohexyl. etc.; a $C_{6-12}$ aryl group such as phenyl, α-naphthyl, etc.; a $C_{7-14}$ aralkyl such as a phenyl-$C_{1-2}$ alkyl group, e.g., benzyl, phenethyl, etc.; an α-naphthyl-$C_{1-2}$ alkyl group such as α-naphthylmethyl, etc.; and the like. In addition, a pivaloyloxymethyl group or the like which is used widely as an ester for oral administration may also be used.

Where AQ27 receptor or OT7T022 receptor has a carboxyl group (or a carboxylate) at a position other than the C-terminus, it may be amidated or esterified and such an amide or ester is also included within the protein of the present application. As the ester group herein, the same esters as those described with respect to the above C-terminal are used.

Furthermore, examples of AQ27 receptor or OT7T022 receptor include those wherein the amino group at the N-terminus (e.g., methionine residue) of the protein is protected with a protecting group (e.g., a $C_{1-6}$ acyl group such as a $C_{2-6}$ alkanoyl group e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group , e.g., formyl group, acetyl group, etc.), or conjugated proteins such as glycoproteins having sugar chains.

Examples of AQ27 receptor include, for example, human-derived AQ27 receptor consisting of the amino acid sequence represented by SEQ ID NO: 31, rat-derived AQ27 receptor consisting of the amino acid sequence represented by SEQ ID NO: 44 and mouse-derived AQ27 receptor consisting of the amino acid sequence represented by SEQ ID NO: 46.

Examples of OT7T022 receptor include, for example, rat-derived OT7T022 receptor consisting of the amino acid sequence represented by SEQ ID NO: 33 and human-derived OT7T022 receptor consisting of the amino acid sequence represented by SEQ ID NO: 35.

The partial peptide of AQ27 receptor or OT7T022 receptor may be any partial peptide of AQ27 receptor or OT7T022 receptor, and for example, a partial peptide which is a part of the protein molecule of AQ27 receptor or OT7T022 receptor, is exposed to the outside of a cell membrane and has a receptor binding activity is used.

The partial peptide of AQ27 receptor or OT7T022 receptor is a peptide containing a region analyzed to be an extracellular region (hydrophilic site) in hydrophobicity plotting analysis. A peptide partially containing the hydrophobic site can also be used as well. A peptide containing each of individual domains can also be used, but a peptide containing a plurality of domains may also be used.

The partial peptide of AQ27 receptor or OT7T022 receptor is preferably a peptide having an amino acid sequence of at least 20 amino acids, preferably at least 50 amino acids, more preferably at least 100 amino acids in the amino acid sequence constituting AQ27 receptor or OT7T022 receptor.

Substantially the same amino acid sequence refers to an amino acid sequence having at least about 50% homology, preferably at least about 70% homology, more preferably at least about 80% homology, still more preferably at least about 90% homology and most preferably at least about 95% homology, to the above amino acid sequence.

The "substantially equivalent activity" has the same meaning as defined above. The "substantially equivalent activity" can be measured in the same manner as described above.

The partial peptide of AQ27 receptor or OT7T022 receptor may be a peptide wherein in the amino acid sequence described above, one or more (preferably about 1 to 10, more preferably a few (1 or 2)) amino acids were deleted, or in the amino acid sequence described above, one or more (preferably about 1 to 20, more preferably about 1 to 10, still more preferably a few (1 or 2)) amino acids were added, or in the amino acid sequence described above, one or more (preferably about 1 to 10, more preferably about 1 to 5, still more preferably a few (1 or 2)) amino acids were replaced by other amino acids.

In the partial peptide of AQ27 receptor or OT7T022 receptor, the C-terminus is usually in the form of a carboxyl group (-COOH) or a carboxylate ($-COO^-$) but may be in the form of an amide ($-CONH_2$) or an ester (-COOR).

Furthermore, examples of the partial peptide of AQ27 receptor or OT7T022 receptor, similar to the above-mentioned AQ27 receptor or OT7T022 receptor, include variants of the above partial peptide wherein the amino group at the N-terminus (e.g., methionine residue) of the peptide is protected with a protecting group; those wherein the N-terminal region is cleaved in vivo and the glutamyl group thus formed is pyroglutaminated; those wherein a substituent on the side chain of an amino acid in the molecule is protected with a suitable protecting group, or conjugated proteins such as glycoproteins having sugar chains.

The salt of AQ27 receptor or OT7T022 receptor or the salt of its partial peptide is particularly preferably a physiologically acceptable acid addition salt. As the salts, there may be used salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The DNA encoding AQ27 receptor or OT7T022 receptor may be any DNA so long as it comprises the nucleotide sequence encoding the AQ27 receptor or OT7T022 receptor described above. Such a DNA may be any of genomic DNA, genomic DNA library, cDNA derived from the cells or tissues described above, cDNA library derived from the cells or tissues described above, and synthetic DNA. The vector to be used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

Specifically, the DNA encoding AQ27 receptor may be any DNA insofar as it is DNA comprising the nucleotide sequence represented by, for example, SEQ ID NO: 32, SEQ ID NO: 45 or SEQ ID NO: 47, or DNA hybridizing under high stringent conditions with DNA having the nucleotide sequence represented by SEQ ID NO: 32, SEQ ID NO: 45 or SEQ ID NO: 47 and encoding a receptor having an activity (for example, ligand binding activity, signal information transmitting action etc.) substantially equivalent to that of AQ27 receptor consisting of the amino acid sequence represented by SEQ ID NO: 31, SEQ ID NO: 44 or SEQ ID NO: 46.

The DNA hybridizing under high stringent conditions with DNA comprising the nucleotide sequence represented by SEQ ID NO: 32, SEQ ID NO: 45 or SEQ ID NO: 47 is for example DNA comprising a nucleotide sequence having at least about 70% homology, preferably at least about 80% homology, more preferably at least about 90% homology and most preferably at least about 95% homology, to the nucleotide sequence represented by SEQ ID NO: 32, SEQ ID NO: 45 or SEQ ID NO: 47.

More specifically, the DNA encoding human AQ27 receptor consisting of the amino acid sequence represented by SEQ ID NO: 31 includes, for example, DNA consisting of the nucleotide sequence represented by SEQ ID NO: 32, etc.

More specifically, the DNA encoding rat AQ27 receptor consisting of the amino acid sequence represented by SEQ ID NO: 44 includes, for example, DNA consisting of the nucleotide sequence represented by SEQ ID NO: 45, etc.

More specifically, the DNA encoding mouse AQ27 receptor consisting of the amino acid sequence represented by SEQ ID NO: 46 includes, for example, DNA consisting of the nucleotide sequence represented by SEQ ID NO: 47, etc.
The DNA encoding OT7T022 receptor may, for example be:

(1) DNA comprising the nucleotide sequence represented by SEQ ID NO: 34, or DNA hybridizing under high stringent conditions with DNA having the nucleotide sequence represented by SEQ ID NO: 34 and encoding a receptor having an activity (for example, ligand binding activity, signal information transmitting action etc.) substantially equivalent to that of OT7T022 receptor consisting of the amino acid sequence represented by SEQ ID NO: 33, or
(2) DNA comprising the nucleotide sequence represented by SEQ ID NO: 36 or SEQ ID NO: 37, or DNA hybridizing under high stringent conditions with DNA having the nucleotide sequence represented by SEQ ID NO: 36 or SEQ ID NO: 37 and encoding a receptor having an activity (for example, ligand binding activity, signal information transmitting action etc.) substantially equivalent to that of OT7T022 receptor consisting of the amino acid sequence represented by SEQ ID NO: 35.

The DNA hybridizing under high stringent conditions with DNA having the nucleotide sequence represented by SEQ ID NO: 34, SEQ ID NO: 36 or SEQ ID NO: 37 is for example DNA comprising a nucleotide sequence having at least about 70% homology, preferably at least about 80% homology, more preferably at least about 90% homology and most preferably at least about 95% homology, to the nucleotide sequence represented by SEQ ID NO: 34, SEQ ID NO: 36 or SEQ ID NO: 37.
The hybridization can be carried out by publicly known methods or by a modification thereof, for example, according to the method described in Molecular Cloning, 2nd Ed., J. Sambrook et al., Cold Spring Harbor Lab. Press, (1989). A commercially available library may also be used according to the instructions of the attached manufacturer's protocol. The hybridization can be carried out preferably under high stringent conditions.
The high stringent conditions used herein are, for example, those in a sodium concentration of about 19 mM to about 40 mM, preferably about 19 mM to about 20 mM at a temperature of about 50°C to about 70°C, preferably about 60°C to about 65°C. The high stringent conditions are most preferably those in a sodium concentration of about 19 mM at a temperature of about 65°C.
More specifically, as DNA encoding AQ27 receptor comprising the amino acid sequence represented by SEQ ID NO: 31, DNA comprising the nucleotide sequence represented by SEQ ID NO: 32 is used.
As DNA encoding rat OT7T022 receptor consisting of the amino acid sequence represented by SEQ ID NO: 33, DNA consisting of the nucleotide sequence represented by SEQ ID NO: 34 is used.
As DNA encoding human OT7T022 receptor consisting of the amino acid sequence represented by SEQ ID NO: 35, DNA consisting of the nucleotide sequence represented by SEQ ID NO: 36 or SEQ ID NO: 37 is used.
Human AQ27 receptor or its partial peptide or salt can be produced according to a method described in WO01/16316.
OT7T022 receptor or its partial peptide or salt can be produced according to a method described in WO00/29441.
The rat or mouse AQ27 receptor, its partial peptide or its salt can be produced according to a method described in WO01/16316.
The compound or its salt that alters the binding property between the peptide F, G, H or I of the present application and AQ27 receptor, which is obtainable using the screening method or the screening kit of the present application, is useful as a safe and low toxic pharmaceutical preparation, for example, a hormone secretion regulator (for example, a promoter for secretion of adrenal cortical hormone such as corticosterone), an eating regulator, a sleep regulator, an arousal regulator, a pain regulator, a stress response regulator, a spontaneous behavior regulator or an emotional behavior regulator.
Further, the AQ27 receptor agonist obtainable using the screening method or screening kit of the present application is useful as a safe and low toxic pharmaceutical preparation, for example, a prophylactic/therapeutic agent for diseases in the central nerves (for example, Alzheimer's disease/dementia/difficulty in eating (anorexia)/epilepsy, etc.), diseases in the hormone system (for example, faint labor, flaccid bleeding, before and after, placenta, expulsion, insufficiency in uterine restoration, Caesarean operation, artificial abortion, milk statis etc.), diseases in liver/gall/pancreas/internal secretion (for example, diabetes/difficulty in eating, etc.), inflammatory diseases (allergy/asthma/rheumatism etc.), circulatory organ diseases (for example, high blood pressure/cardiac hypertrophy/angina/arteriosclerosis; etc.) and as an agent for promoting secretion of adrenal cortical hormone, etc.
On the other hand, the AQ27 receptor antagonist obtainable using the screening method or screening kit of the present application is useful as a safe and low toxic pharmaceutical preparation, for example a prophylactic/therapeutic agent for diseases caused by excessive production of the peptide F of the present application, such as diseases in the central nerves, diseases in the hormone system, diseases in liver/gall/pancreas/internal secretion (for example, an anti-obesity drug/excessive eating, etc.), inflammatory diseases, circulatory organ diseases and as an agent for inhibiting secretion of adrenal cortical hormone, etc.

The OT7T022 receptor agonist or OT7T022 receptor antagonist obtainable using the screening method or screening kit of the present application is useful as an agent for regulating prolactin secretion. That is, when the OT7T022 receptor agonist or OT7T022 receptor antagonist has a promoting action on secretion of prolactin, it is useful as a prolactin secretion promoter for diseases related to insufficiency in secretion of prolactin, for example a prophylactic/ therapeutic agent for diseases related to prolactin secretion, such as depression of ovarian functions, deficiency in growth of seminal vesicle, osteoporosis, menopausal disorders, deficiency in secretion of milk, hypothyroidism, renal insufficiency, etc. Further, when the OT7T022 receptor agonist or OT7T022 receptor antagonist has a promoting action on secretion of prolactin, it has a promoting action on sexual desire (pheromone-like action) based on the promoting action on secretion of prolactin, and is thus useful as an agent for promoting sexual desire.

When the OT7T022 receptor agonist or OT7T022 receptor antagonist has an inhibitory action on secretion of prolactin, it is useful as a prolactin secretion inhibitor, for example a prophylactic/therapeutic agent for various diseases related to excessive secretion of prolactin, such as hyperprolactinemia, pituitary gland tumor, interbrain tumor, abnormal menstruation, stress, autoimmune disease, prolactinoma, sterility, impotence, amenorrhea, galactorrhea, acromegaly, Chiari-Frommel syndrome, Argonz-del Castilo syndrome, Forbes-Albright syndrome, breast cancer lymphoma, Sheehan's syndrome or abnormality in spermatogenesis. Further, when the OT7T022 receptor agonist or OT7T022 receptor antagonist has an inhibitory action on secretion of prolactin, it is useful as a contraceptive agent based on the inhibitory action on secretion of prolactin.

In addition, the OT7T022 receptor agonist or OT7T022 receptor antagonist is also useful as a diagnostic drug for examining an ability to secrete prolactin or as an animal drug such as a milk secretion promoting agent for domestic mammals such as bovine, goat, swine etc., and can be applied to production of a useful substance by producing the useful substance in the mammals and secreting it in milk.

Further, the OT7T022 receptor agonist or OT7T022 receptor antagonist has a regulatory action on the functions of placenta, and is thus useful as a prophylactic/therapeutic agent for choriocarcinoma, hydatidiform mole, invasive mole, abortion, insufficiency in fetal growth, abnormality in sugar metabolism, abnormality in lipid metabolism or delivery induction.

The regulatory activity on secretion of prolactin can be measured according to a method described in Neuroendocrinology, 62,198-206, 1995 or Neuroscience Letters, 203, 164-170, 1996, or by a modification thereof.

The OT7T022 receptor agonist or OT7T022 receptor antagonist which is obtainable using the screening method or screening kit of the present application is a safe and low toxic pharmaceutical preparation, for example a prophylactic/ therapeutic agent for high blood pressure, autoimmune disease, cardiac insufficiency, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, bone fracture, breast cancer, bulimia, [bulimia], burn healing, uterine cervix cancer, chronic lymphatic leukemia, chronic myeloid leukemia, chronic pancreatitis, hepatocirrhosis, large-intestine cancer (colon/rectum cancer), Crohn's disease, dementia, diabetic complications, diabetic nephritis, diabetic neuropathy, diabetic retinitis, gastritis, helicobacter pylori infection, renal insufficiency, type A hepatitis, type B hepatitis, type C hepatitis, hepatitis, herpes simplex virus infection, varicella zoster virus infection, Hodgkin's disease, AIDS infection, human papilloma virus infection, hypercalcemia, hypercholesterolemia, hyperglyceridemia, hyperlipemia, infections, influenza infection, insulin-dependent diabetes (type I), invasive Staphylococcus infection, malignant melanoma, cancer metastasis, multiple myeloma, allergic rhinitis, nephritis, non-Hodgkin lymphoma, insulin-independent diabetes (type II), non-small cell lung cancer, organ transplant operation, bone arthritis, bone softening, bone decrease, osteoporosis, ovary cancer, bone Behcet's disease, digestive ulcer, peripheral blood vessel disease, prostate cancer, reflux esophagitis, renal insufficiency, rheumatoid arthritis, schizophrenia, septicemia, septicemic shock, severe systemic fungal infection, small cell lung cancer, spinal damage, stomach cancer, systemic lupus erythematosus, transient cerebral ischemia attack, tuberculosis, valvular heart disease, vascular/multiple infarct dementia, wound healing, insomnia, arthritis, insufficiency in secretion of pituitary hormone, frequent urination; uremia, or nerve degeneration disease.

Further, the OT7T022 receptor agonist or OT7T022 receptor antagonist can be used as a pharmaceutical preparation such as a therapeutic/prophylactic agent for a disease macular edema cystoid.

Further, the OT7T022 receptor agonist or OT7T022 receptor antagonist is useful for example as (1) a therapeutic agent for tumors such as acromegaly, TSH-producing tumor, non-secretory (non-functional) pituitary tumor, ectopic ACTH (adrenocorticotropine)-producing tumor, marrow-like thyroid cancer, VIP-producing tumor, glucagon-producing tumor, gastrin-producing tumor, insulinoma, carcinoid etc., (2) a therapeutic agent for insulin dependent or independent diabetes, or various diseases related to such diabetes, that is, diabetic complications (for example, diabetic retinitis, diabetic nephritis, diabetic neuropathy, down syndrome, erectile low blood pressure, etc.), (3) a therapeutic agent for obesity, bulimia etc. by ameliorating hyperinsulinemia or suppressing appetite, (4) a therapeutic agent for acute pancreatitis, chronic pancreatitis, pancreas/intestine physter, bleeding ulcer, digestive ulcer, gastritis, gastric hyperacidity, reflux esophagitis, etc., (5) an agent for ameliorating various symptoms caused by infection with helicobacter pylori bacteria (for example, an agent for inhibiting promotion of gastrin secretion, etc.), (6) an agent for suppressing amylase secretion caused by endoscopic cholangio-pancreatography, and for prognosis of surgical operation of pancreas, (7) a therapeutic agent for diarrhea attributable to a reduction of the absorbing ability of small intestine, acceleration of secretion, or abnormality in mobility of digestive tract (for example, short bowel syndrome, etc.), diarrhea attributable to chemicals in chemotherapy of cancer, diarrhea attributable to congenital shrinkage of small intestine, diarrhea attributable to neuroendocrine tumor such as VIP-producing tumor, diarrhea attributable to AIDS, diarrhea attributable to graft versus host reaction accompanying a marrow transplant, diarrhea attributable to diabetes, diarrhea attributable to blockage of abdominal nerve plexuses, diarrhea attributable to systematic sclerosis, diarrhea attributable to eosinophilia, etc., (8) a therapeutic agent for damping syndrome, hypersensitive colitis, Crohn's disease, inflammatory intestinal diseases, etc., (9) a therapeutic agent for tumors or cancers (for example, thyroid cancer, large intestine cancer, breast cancer, prostate cancer, small cell lung cancer, non-small cell lung cancer, pancreas cancer, stomach cancer, cholangioma, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (for example, basophil leukemia/chronic lymphatic leukemia, chronic myeloid leukemia, Hodgkin's disease, non-Hodgkin lymphoma, etc.); the therapeutic agent can be used alone or in combination with other anticancer drugs (for example, tamoxifen, LHRH agonist, LHRH antagonist, interferon-$\alpha$,$\beta$ and $\gamma$, interleukin-2, etc.), (10) a prophylactic/therapeutic agent for hypertrophic myocardial disease, arteriosclerosis, valvular heart disease, myocardial infarction (particularly, myocardial infarction after percutaneous transluminal coronary angioplasty), revascularization, (11) a therapeutic agent for esophagus vein cancer bleeding, hepatocirrhosis, peripheral blood vessel disease, (12) a therapeutic agent for diseases accompanying systemic or topical inflammations (for example, multiple arteritis, rheumatoid anhritis, psoriasis, sunburn, eczema, allergies (for example, asthma, atopic dermatitis, allergic rhinitis, etc.)), based on the secretion-regulating action of phsyoloigcally active substance acting on the immune system (for example, substance P, tachykinin, cytokine, etc.), (13) a therapeutic agent for dementia (for example, Alzheimer's disease, Alzheimer type senile dementia, vascular/multiple dementia, etc.), schizophrenia, epilepsy, depression, general anxiety, difficult sleep, multiple sclerosis, etc., because of its influence on production and secretion of nerve-regulating factor, (14) a therapeutic agent for eye diseases (for example, glaucoma, etc.), (15) a prophylactic/therapeutic agent for acute bacterial meningitis, acute viral encephalitis, adult respiratory distress syndrome, bacterial pneumonia, severe systemic fungal infection, tuberculosis, spinal damage, bone fracture, renal insufficiency, pneumonia, alcoholic hepatitis, type A hepatitis, type B hepatitis, type C hepatitis, AIDS infection, human papilloma virus infection, influenza infection, cancer metastasis, multiple myeloma, bone softening, osteoporosis, bone Behcet's disease, nephritis, renal insufficiency, septicemia, septicemic shock, hypercalcemia, hypercholesterolemia, hyperglyceridemia, hyperlipemia, systemic lupus erythematosus, transient cerebral ischemia attack, alcoholic hepatitis, etc. (16) a therapeutic agent for organ transplant operation, burn, wound, alopecia, etc., and (17) an analgesic for suppressing and relieving chronic or acute pains (for example, acute pain after operation, inflammatory acute pain, toothache, bone diseases (for example, acute pains accompanying arthritis, rheumatism, osteoporosis, etc.)).

These pharmaceutical preparations can be produced and used in the same manner as described above.

(2-2) Method of screening a compound that promotes or suppresses the expression of the protein of the present application

The protein of the present application, the oligonucleotide of the present application, the transformant of the present application or the antibody of the present application can be used in the method of screening a compound that promotes or suppresses the expression of the protein of the present application.

That is, the present application discloses:

(i) a method of screening a compound that promotes or suppresses the expression of the protein of the present application, wherein cells or tissues capable of expressing the protein of the present appliction are cultured in the presence or absence of a test compound, and the amount of the expressed protein of the present application or the amount of mRNA encoding the protein of the present application in the presence of the test compound is compared with that in the absence of the test compound.

The cells or tissues capable of expressing the protein of the present application any cells of human and other warm-blooded animals (e.g., guinea pig, rat, mouse, chicken, rabbit, swine, sheep, bovine, monkey, etc.) such as nerve cell, endocrine cells, neuroendocrine cells, glial cells, $\beta$ cells of pancreas, bone marrow cells, liver cells, splenocytes, mesangial cells, epidermic cells, epithelial cells, endothelial cells, fibroblasts, fibrocytes, myocytes, fat cells, immune cells (e.g., macrophage, T cells, B cells, natural killer cells, mast cells, neutrophils, basophils, eosinophils, monocytes, dendritic cells), megakaryocyte, synovial cells, chondrocytes, bone cells, osteoblasts, osteoclasts, mammary gland cells, interstitial cells, etc., the corresponding precursor cells, stem cells, cancer cells, etc., or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, pituitary, stomach, pancreas, kidney, liver, gonad, thyroid, gall-bladder, bone marrow, adrenal gland, skin, muscle, lung, gastrointestinal tract (e.g.,

large intestine and small intestine), blood vessels, heart, thymus, spleen, submandibular gland, peripheral blood, prostate, testis, ovary, placenta, uterus, bone, cartilage, joint, skeletal muscle, etc. The cells or tissues may be those of an established cell line or in a primary culture system. Further, the transformant of the present application be used.

The cells capable of expressing the peptide of the present application can be cultured in the same manner as in the above-described method of culturing the transformant of the present application.

As the test compound, not only the above-described test compound but also a DNA library etc. can be used.

The amount of the present peptide expressed can be measured by publicly known method such as immunochemical methods using antibody or by publicly known methods using Northern hybridization with mRNA encoding the peptide of the present RT-PCR, or TaQMan PCR.

Comparison of the amount of expressed RNA by hybridization methods can be performed by known methods or a modification thereof, for example, methods described by J. Sambrook et al, in Molecular Cloning, 2nd, Cold Spring Harbor Lab. Press (1989).

Specifically, the amount of mRNA encoding the peptide of the present application is carried out according to a known method by bringing RNA extracted from cells into contact with the polynucleotide of the present application or a part thereof or the antisense polynucleotide of the present application and measuring the amount of mRNA bound to the polynucleotide of the present application or a part thereof or the antisense polynucleotide of the present application. The polynucleotide of the present application or a part thereof or the antisense polynucleotide of the present application is labeled with, for example, a radioactive isotope, a colorant etc., whereby the amount of mRNA bound to the polynucleotide of the present application or a part thereof or the antisense polynucleotide of the present application can be easily measured. The radioactive isotope used includes, for example, $^{32}P$, $^{3}H$ etc., and the colorant used includes fluorescence colorants such as, for example, fluorescein, FAM (PE Biosystems), JOE (PE Biosystems), TAMRA (PE Biosystems), ROX (PE Biosystems), Cy5 (Amersham), Cy3 (Amersham) etc.

The amount of mRNA can be determined by converting RNA extracted from cells into cDNA by reverse transcriptase, and then measuring the amount of cDNA amplified by PCR using the polynucleotide of the present application a part thereof or the antisense polynucleotide of the present application as primers.

A test compound increasing the amount of mRNA encoding the peptide of the present application can be selected as a compound having an activity of promoting the expression of the peptide of the present application, while a test compound decreasing the amount of mRNA encoding the peptide of the present application can be selected as a compound having an activity of suppressing the expression of the peptide of the present application.

Further, the present application discloses:

(ii) a method of screening a compound that promotes or suppresses a promoter activity, which comprises measuring and comparing the activity of a reporter in a transformant in the presence and absence of a test compound, the transformant being transformed with a recombinant DNA comprising a reporter gene ligated downstream of a promoter or enhancer region for the gene encoding the peptide of the present application.

The reporter gene used includes, for example, lacZ (β-galactosidase gene), chloramphenicol acetyltransferase (CAT), luciferase, growth factor, β-glucronidase, alkali phosphatase, green fluorescent protein (GFP), β-lactamase etc.

By measuring the amount of the reporter gene product (for example, mRNA, protein) by a known method, a test compound increasing the amount of the reporter gene product can be selected as a compound having an action of regulating (particularly promoting) the activity of a promoter or enhancer for the peptide of the present application, that is, a compound having an activity of promoting the expression of the peptide of the present application. On the other hand, a test compound decreasing the amount of the reporter gene product can be selected as a compound having an action of regulating (particularly inhibiting) the activity of a promoter or enhancer for the peptide of the present application, that is, a compound having an activity of inhibiting the expression of the peptide of the present application.

The test compound used includes those described above.

The transformant can be cultured in the same manner as for the above transformant of the present application.

The construction of a reporter gene vector and assay methods can be carried out according to known techniques (for example, Molecular Biotechnology 13, 29-43, 1999).

The compound having the activity of promoting expression of the peptide of the present application can enhance the action of the peptide of the present application and is thus useful as a safe and low toxic drug, likewise the peptide of the present application.

Conversely, since the compound having the activity of inhibiting expression of the peptide of the present application can suppress the physiological activities possessed by the peptide of the present application, the compound is useful as a safe and low toxic drug that suppresses the physiological activities of the peptide of the present application.

The compounds or salts thereof which are obtainable using the screening methods or screening kits of the present application, are compounds selected from, e.g., peptides, proteins, non-peptide compounds, synthetic compounds, fermentation products, cell extracts, plant extracts, animal tissue extracts, plasma, and the like. For salts of these compounds, the same salts as those given for the peptide of the present application above may be used.

When the compounds obtainable using the screening methods or screening kits of the present application are used as the therapeutic/prophylactic agents described above, a conventional manner may apply thereto. The compounds may be prepared in the form of, for example, tablets, capsules, elixirs, microcapsules, sterile solutions, suspensions, etc. in a manner similar to the method for preparing the pharmaceutical preparation comprising the peptide of the present application described hereinabove.

Since the thus obtained pharmaceutical preparation is safe and low toxic, the preparation can be administered to, for example, human or warm-blooded animal (e.g., mouse, rat, rabbit, sheep, swine, bovine, horse, chicken, cat, dog, monkey, chimpanzee etc.).

The dose of the compound or its salt may vary depending on its action, target disease, subject to be administered, route for administration, etc. When the compound promoting expression of the peptide of the present application is orally administered, the compound is administered to adult (as 60 kg) generally in a daily dose of approximately 0.1 mg to 100 mg, preferably approximately 1.0 mg to 50 mg, more preferably approximately 1.0 to 20 mg. When the compound is parenterally administered, a single dose of the compound may vary depending on subject to be administered, target disease, etc. When the compound promoting expression of the peptide of the present application is administered to adult (as 60 kg body weight) in the form of injection, it is convenient to administer the compound by intravenous injection, generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

More specifically, when the peptide of the present application is the peptide F, G, H or I of the present application, a compound or its salt that promotes or suppresses expression of the peptide F, G, H or I of the present application is useful as a safe and low toxic pharmaceutical preparation, for example, a hormone secretion regulator (for example, a regulator for secretion of adrenal cortical hormone such as corticosterone), an eating regulator, a sleep regulator, an arousal regulator, a pain regulator, a stress response regulator, a spontaneous behavior regulator, an emotional behavior regulator, etc.

The compound or its salt that promotes expression of the peptide F, G, H or I of the present application is useful as a safe and low toxic pharmaceutical preparation, for example a prophylactic/therapeutic agent for diseases in the central nerves (for example, Alzheimer's disease/dementia/difficulty in eating (anorexia)/epilepsy, etc.), diseases in the hormone system (for example, faint labor, flaccid bleeding, before and after placenta expulsion, insufficiency in uterine restoration, Caesarean operation, artificial abortion, milk statis etc.), diseases in liver/gall/pancreas/internal secretion (for example, diabetes/difficulty in eating, etc.), inflammatory diseases (allergy/asthma/rheumatism etc.), circulatory organ diseases (for example, high blood pressure/cardiac hypertrophy/angina/arteriosclerosis, etc.) and as an agent for promoting secretion of adrenal cortical hormone, etc.

On the other hand, the compound or its salt that inhibits expression of the peptide F, G, H or I of the present application is useful as a safe and low toxic pharmaceutical preparation, for example a prophylactic/therapeutic agent for diseases caused by excessive production of the peptide F of the present application, such as diseases in the central nerves, diseases in the hormone system, diseases in liver/gall/pancreas/internal secretion (for example, an anti-obesity drug/excessive eating, etc.), inflammatory diseases, circulatory organ diseases and as an agent for inhibiting secretion of adrenal cortical hormone, etc.

The compound or its salt that promotes or inhibits expression of peptide F, G, H or I of the present application is useful as a secretion regulator for prolactin secretion. That is, when the compound or its salt that promotes or inhibits expression of peptide F, G, H or I of the present application has a promoting action on secretion of prolactin, it is useful as a prolactin secretion promoter for various diseases with which deficiency in prolactin secretion is associated, for example a prophylactic/therapeutic agent for diseases related to prolactin secretion, such as depression of ovarian functions, deficiency in growth of seminal vesicle, osteoporosis, menopausal disorders, deficiency in secretion of milk, hypothyroidism, renal insufficiency, etc. Further, when the compound or its salt that promotes or inhibits expression of the peptide F of the present application has a promoting action on secretion of prolactin, it has a promoting action on sexual desire (pheromone-like action) based on the promoting action on secretion of prolactin, and is thus useful as an agent for promoting sexual desire.

When the compound or its salt that promotes or inhibits expression of peptide F, G, H or I of the present application has an inhibitory action on secretion of prolactin, it is useful as a prolactin secretion inhibitor for diseases with which excessive secretion of prolactin is associated, for example a prophylactic/therapeutic agent for diseases related to prolactin secretion, such as hyperprolactinemia, pituitary gland tumor, interbrain tumor, abnormal menstruation, stress, autoimmune disease, prolactinoma, sterility, impotence, amenorrhea, galactorrhea, acromegaly, Chiari-Frommel syndrome, Argonz-del Castilo syndrome, Forbes-Albright syndrome, breast cancer lymphoma, Sheehan's syndrome or abnormality in spernatogenesis. Further, when the compound or its salt that promoters or inhibits expression of peptide F, G, H or I of the present application has an inhibitory action on secretion of prolactin, it is useful as a contraceptive agent based on the inhibitory action on secretion of prolactin.

In addition, the compound or its salt that promotes or inhibits the expression of peptide F, G, H or I of the present application is also useful as a diagnostic drug for examining an ability to secrete prolactin or as an animal drug such as a milk secretion promoting agent for domestic mammals such as bovine, goat, swine etc., and can be applied to production of a useful substance by producing the useful substance in the mammals and secreting it in milk.

Further, the compound or its salt that promotes or inhibits expression of the peptide F, G, H or I of the present application has a regulatory action on the functions of placenta and is thus useful as a prophylactic/therapeutic agent for choriocarcinoma, hydatidiform mole, invasive mole, abortion, insufficiency in fetal growth, abnormality in sugar metabolism, abnormality in lipid metabolism or delivery induction.

Further, the compound or its salt that promotes or inhibits expression of the peptide F, G, H or I of the present application can be used as a safe and low toxic pharmaceutical preparation, for example as a therapeutic/prophylactic agent for diseases such as high blood pressure, autoimmune disease, cardiac insufficiency, cataract, glaucorna, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, bone fracture, breast cancer, bulimia, [bulimia], burn healing, uterine cervix cancer, chronic lymphatic leukemia, chronic myeloid leukemia, chronic pancreatitis, hepatociahosis, large-intestine cancer (colon/rectum cancer), Crohn's disease, dementia, diabetic complications, diabetic nephritis, diabetic neuropathy, diabetic retinitis, gastritis, helicobacter pylori infection, renal insufficiency, type A hepatitis, type B hepatitis, type C hepatitis, hepatitis, herpes simplex virus infection, varicella zoster virus infection, Hodgkin's disease, AIDS infection, human papilloma virus infection, hypercalcemia, hypercholesterolemia, hyperglyceridernia, hyperlipemia, infections, influenza infection, insulin-dependent diabetes (type I), invasive Staphylococcus infection, malignant melanoma, cancer metastasis, multiple myeloma, allergic rhinitis, nephritis, non-Hodgkin lymphoma, insulin-independent diabetes (type II), non-small cell lung cancer, organ transplant operation, bone arthritis, bone softening, bone decrease, osteoporosis, ovary cancer, bone Behcet's disease, digestive ulcer, peripheral blood vessel disease, prostate cancer, reflux esophagitis, renal insufficiency, rheumatoid arthritis, schizophrenia, septicemia, septicemic shock, severe systemic fungal infection, small cell lung cancer, spinal damage, stomach cancer, systemic lupus erythematosus, transient cerebral ischemia attack, tuberculosis, valvular heart disease, vascular/multiple infarct dementia, wound healing, insomnia, arthritis, insufficiency in secretion of pituitary hormone, frequent urination, uremia, or nerve degeneration disease.

The compound or its salt that promotes or inhibits expression of peptide F, G, H or I of the present application can be used as a pharmaceutical preparation such as a therapeutic/prophylactic drug for a disease macular edema cystoid.

The compound or its salt that promotes or inhibits expression of peptide F, G, H or I of the present application is useful for example as (1)'a therapeutic agent for tumors such as acromegaly, TSH-producing tumor, non-secretory (non-functional) pituitary tumor, ectopic ACTH (adrenocorticotropine)-producing tumor, marrow-like thyroid cancer, VIP-producing tumor, glucagon-producing tumor, gastrin-producing tumor, insulinoma, carcinoid etc., (2) a therapeutic agent for insulin dependent or independent diabetes, or various diseases related to such diabetes, that is, diabetic complications (for example, diabetic retinitis, diabetic nephritis, diabetic neuropathy, down syndrome, erectile low blood pressure, etc.), (3) a therapeutic agent for obesity, bulimia etc. by ameliorating hyperinsulinemia or suppressing appetite, (4) a therapeutic agent for acute pancreatitis, chronic pancreatitis, pancreas/intestine physter, bleeding ulcer, digestive ulcer, gastritis, gastric hyperacidity, reflux esophagitis, etc., (5) an agent for ameliorating various symptoms caused by infection with helicobacter pylori bacteria (for example, an agent for inhibiting promotion of gastrin secretion, etc.), (6) an agent for suppressing amylase secretion caused by endoscopic cholangio-pancreatography, and for prognosis of surgical operation of pancreas, (7) a therapeutic agent for diarrhea attributable to a reduction of the absorbing ability of small intestine, acceleration of secretion, or abnormality in mobility of digestive tract (for example, short bowel syndrome, etc.), diarrhea attributable to chemicals in chemotherapy of cancer, diarrhea attributable to congenital shrinkage of small intestine, diarrhea attributable to neuroendocrine tumor such as VIP-producing tumor, diarrhea attributable to AIDS, diarrhea attributable to graft versus host reaction accompanying a marrow transplant, diarrhea attributable to diabetes, diarrhea attributable to blockage of abdominal nerve plexuses, diarrhea attributable to systematic sclerosis, diarrhea attributable to eosinophilia, etc., (8) a therapeutic agent for damping syndrome, hypersensitive colitis, Crohn's disease, inflammatory intestinal diseases, etc., (9) a therapeutic agent for tumors or cancers (for example, thyroid cancer, large intestine cancer, breast cancer, prostate cancer, small cell lung cancer, non-small cell lung cancer, pancreas cancer, stomach cancer, cholangioma, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (for example, basophil leukemia/ chronic lymphatic leukemia, chronic myeloid leukemia, Hodgkin's disease, non-Hodgkin lymphoma, etc.); the therapeutic agent can be used alone or in combination with other anticancer drugs (for example, tamoxifen, LHRH agonist, LHRH antagonist, interferon-$\alpha$,$\beta$ and $\gamma$, interleukin-2, etc.), (10) a prophylactic/therapeutic agent for hypertrophic myocardial disease, arteriosclerosis, valvular heart disease, myocardial infarction (particularly, myocardial infarction after percutaneous transluminal coronary angioplasty), revascularization, (11) a therapeutic agent for esophagus vein cancer bleeding, hepatocirrhosis, peripheral blood vessel disease, (12) a therapeutic agent for diseases accompanying systemic or topical inflammations (for example, multiple arteritis, rheumatoid arthritis, psoriasis, sunburn, eczema, allergies (for example, asthma, atopic dermatitis, allergic rhinitis, etc.)), based on the secretion-regulating action of phsyoloigcally active substance acting on the immune system (for example, substance P, tachykinin, cytokine, etc.), (13) a therapeutic agent for dementia (for example, Alzheimer's disease, Alzheimer type senile dementia, vascular/multiple dementia, etc.), schizophrenia, epilepsy, depression, general anxiety, difficult sleep, multiple sclerosis, etc., because of its influence on production and secretion of nerve-regulating factor, (14) a therapeutic agent for eye diseases (for example, glaucoma, etc.), (15) a prophylactic/therapeutic agent for acute bacterial meningitis, acute viral encephalitis, adult respiratory distress syndrome, bacterial pneumonia, severe systemic fungal infection, tuberculosis, spinal damage, bone fracture, renal insufficiency, pneumonia, alcoholic hepatitis, type A hepatitis, type B hepatitis, type C hepatitis, AIDS infection, human papilloma virus infection, influenza infection, cancer metastasis, multiple myeloma, bone softening, osteoporosis, bone Behcet's disease, nephritis, renal insufficiency, septicemia, septicemic shock, hypercalcemia, hypercholesterolemia, hyperglyceridemia, hyperlipemia, systemic lupus erythematosus, transient cerebral ischemia attack, alcoholic hepatitis, etc. (16) a therapeutic agent for organ transplant operation, burn, wound, alopecia, etc., and (17) an analgesic for suppressing and relieving chronic or acute pains (for example, acute pain after operation, inflammatory acute pain, toothache, bone diseases (for example, acute pains accompanying arthritis, rheumatism, osteoporosis, etc.)).

These pharmaceutical preparations can be produced and used in the same manner as above.

**(2-3) Method of screening a compound that promotes or inhibits the enzyme activity of the peptide of the present application**

The peptide of the present application the polynucleotide of the present application can be used in the method of screening a compound that promotes or inhibits the enzyme activity of the peptide of the present application

That is, the present application discloses:

(i) a method of screening a compound or its salt having the activity of promoting or inhibiting the enzyme activity of the peptide of the application which comprises measuring and comparing the amount or activity of a product formed upon addition of a substrate to the peptide of the present application in the presence and absence of a test compound, and

(ii) a method of screening a compound or its salt that promotes or inhibits the enzyme activity of the peptide of the present application, which comprises measuring and comparing the amount or activity of a product formed in the case [1] where cells capable of expressing the peptide of the present application are cultured while being contacted with an RI-labeled substrate and the case [2] where cells capable of expressing the peptide of the present application are cultured while being contacted with an RI-labeled substrate and a test compound.

The method of culturing cells capable of expressing the peptide of the present application is the same as the above-described method of culturing the transformant of the present application.

As the test compound, the same compound as described above can be used.

The enzyme activity can be measured by a method known in the art.

For example, a test compound by which the enzyme activity in the case (ii) is made higher by at least about 20%, preferably at least about 30%, more preferably at least about 50%, than in the case (i) can be selected as a compound promoting the enzyme activity of the peptide of the present application, while a test compound by which the enzyme activity in the case (ii) is made lower by at least about 20%, preferably at least about 30%, more preferably at least about 50%, than in the case (i) can be selected as a compound inhibiting the enzyme activity of the peptide of the present application.

The compound having the activity of promoting the enzymatic activity of the peptide of the present application can enhance the action of the peptide of the present application and is thus useful as a safe and low toxic drug, likewise the peptide of the present application.

Conversely, the compound having the activity of inhibiting the enzymatic activity of the peptide of the present application is useful as a safe and low toxic drug that suppresses the physiological activities of the peptide of the present application.

The compounds or salts thereof, which are obtainable using the screening methods or screening kits of the present application, are compounds selected from, e.g., peptides, proteins, non-peptide compounds, synthetic compounds, fermentation products, cell extracts, plant extracts, animal tissue extracts, blood plasma, and the like. For salts of these compounds, the same salts as those given for the peptide of the present application above may be used.

When the compounds obtainable using the screening methods or screening kits of the present application are used as the therapeutic/prophylactic agents described above, a conventional manner may apply thereto. The compounds may be prepared in the form of tablets, capsules, elixirs, microcapsules, sterile solutions, suspensions, etc. in the same manner for the pharmaceutical preparation comprising the peptide of the present application

Since the thus obtained pharmaceutical preparation is safe and low toxic, the preparation can be administered to, for example, human warm-blooded animal (e.g., mouse, rat, rabbit, sheep, swine, bovine, horse, chicken, cat, dog, monkey, chimpanzee, etc.).

The dose of the compound or its salt may vary depending on its action, target disease, subject to be administered, route for administration, etc. When the compound promoting the enzymatic activity of the peptide of the present application is orally administered, the compound is administered to adult (as 60 kg) generally in a daily dose of approximately 0.1 mg to 100 mg, preferably approximately 1.0 mg to 50 mg, more preferably approximately 1.0 to 20 mg. When the compound is parenterally administered, a single dose of the compound may vary depending on subject to be administered, target disease, etc. When the compound promoting the enzymatic activity of the peptide of the present application is administered to adult (as 60 kg body weight), it is convenient to administer the compound by intravenous injection, generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

**(2-4) Method of screening a compound that promotes or inhibits the activity of a transcriptional factor for the peptide of the present application**

application can be used in the method of screening a compound that promotes or inhibits the activity of a transcriptional factor for the peptide of the present application.

That is, the present application discloses:

(i) a method of screening a compound that promotes or inhibits the activity of a transcriptional factor for the peptide of the present application, which comprises measuring and comparing the activity of a selection marker between the case where a transformant transformed with a vector containing DNA having a selection marker gene bound to a downstream region of a DNA sequence (e.g., a promoter region, enhancer region) to which a sequence encoding the peptide of the present application was bound is cultured in the presence of the peptide of the present application and the case where the transformant is cultured in the presence of the peptide of the present application and a test compound.

As the selection marker gene, the reporter gene or chemical resistance gene described above is used (Shin Seikagaku Jikken Koza (New Biochemical Experimental Lecture) 2, Kakusan (Nucleic acid) III, 3.6 Animal Cell Expression Vector, pp. 84-103).

A combination of a chemical resistance gene and a chemical includes:

(1) combination of puromycin-N-acetyltransferase gene and puromycin;
(2) combination of aminoglycoside phosphotransferase gene (APH) and G418;
(3) combination of hygromycin B phosphotransferase gene (HPH) and hygromycin B, and
(4) combination of xanthine-guanine phosphoribosyl transferase (XGPRT) and mycophenolic acid.

When a parent cell strain is a strain deficient in hypoxanthine-guanine phosphoribosyl transferase (HGPRT) or thymidine kinase (TK), a combination of such gene and HAT (hypoxanthine, aminopterin, thymidine) can be used.

As the selection marker gene, a dihydrofolate reductase gene or an ampicillin resistance gene can also be used.

In addition, an enhancer, a splicing signal, a poly A addition signal, another selection marker, SV40 replication origin (hereinafter sometimes abbreviated as SV40ori) etc. may be added to a vector comprising the first and second gene constructs.

As the test compound, the same compound as described above can be used.

The reporter activity can be measured by a method known in the art.

For example, a test compound by which the reporter activity in the case (ii) is made higher by at least about 20%, preferably at least about 30%, more preferably at least about 50%, than in the case (i) can be selected as a compound promoting the activity of the transcriptional factor for the peptide of the present application, while a test compound by which the reporter activity in the case (ii) is made lower by at least about 20%, preferably at least about 30%, more preferably at least about 50%, than in the case (i) can be selected as a compound inhibiting the activity of the transcriptional factor for the peptide of the present application.

The compound having the activity of promoting the transcriptional factor activity for the peptide of the present application can enhance the action of the peptide of the present application and is thus useful as a safe and low toxic drug, likewise the peptide of the present application.

Conversely, the compound having the activity of inhibiting the transcriptional factor activity for the peptide of the present application is useful as a safe and low toxic drug that suppresses the physiological activities of the peptide of the present application.

The compounds or salts thereof which are obtainable using the screening methods or screening kits of the present application, are compounds selected from, e.g., peptides, proteins, non-peptide compounds, synthetic compounds, fermentation products, cell extracts, plant extracts, animal tissue extracts, blood plasma, and the like. For salts of these compounds, the same salts as those given for the peptide of the present application above may be used.

When the compounds obtainable using the screening methods or screening kits of the present application are used as the therapeutic/prophylactic agents described above, a conventional manner may apply thereto. The compounds may be prepared in the form of tablets, capsules, elixirs, microcapsules, sterile solutions, suspensions, etc. in a manner similar to the method for preparing the pharmaceutical preparation comprising the peptide of the present application described hereinabove.

Since the thus obtained pharmaceutical preparation is safe and low toxic, the preparation can be administered to, for example, human or warm-blooded animal (e.g., mouse, rat, rabbit, sheep, swine, bovine, horse, chicken, cat, dog, monkey, chimpanzee, etc.).

The dose of the compound or its salt may vary depending on its action, target disease, subject to be administered, route for administration, etc. When the compound promoting the transcriptional factor activity for the peptide of the present application is orally administered, the compound is administered to adult (as 60 kg) generally in a daily dose of approximately 0.1 mg to 100 mg, preferably approximately 1.0 mg to 50 mg, more preferably approximately 1.0 to 20 mg. When the compound is parenterally administered, a single dose of the compound may vary depending on subject to be administered, target disease, etc. When the compound promoting the transcriptional factor activity for the peptide of the present application is administered to adult (as 60 kg body weight) in the form of injection, it is convenient to administer the compound by intravenous injection, generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

### (2-5) Method of screening a compound that promotes or inhibits the protein-binding activity of the peptide of the present application

The peptide of the present application, the oligonucleotide of the present application or the transformant of the present application can be used in the method of screening a compound that promotes or inhibits the protein-binding activity of the peptide of the present application.

That is, the present application discloses:

(i) a method of screening a compound that promotes or inhibits the protein-binding activity of the peptide of the present application, which comprises bringing the peptide of the present application or cells comprising the peptide into contact, in the presence or absence of a test compound, with a protein to which the peptide of the present application binds or cells comprising the protein, and [1] measuring the binding activity of the two or [2] observing and comparing a change in the form of the two kinds of cells.

As the cells comprising the peptide of the present application, the transformant of the present application can be used.

The protein to which the peptide of the present application binds, or cells comprising the same, can also be produced in the same manner as described above.

As the test compound, the same compound as described above can be used.

The binding activity of the two can be measured by using a method known in the art.

A change in the form of the two kinds of cells can be observed by using a method known in the art.

For example, a test compound by which the protein-binding activity is made higher by at least about 20%, preferably at least about 30%, more preferably at least about 50% can be selected as a compound promoting the protein-binding activity of the peptide of the present application, while a test compound by which the protein-binding activity is made lower by at least about 20%, preferably at least about 30%, more preferably at least about 50% can be selected as a compound inhibiting the protein-binding activity of the peptide of the present application.

The compound having the activity of promoting the protein-binding activity of the peptide of the present application can enhance the action of the peptide of the present application and is thus useful as a safe and low toxic drug, likewise the peptide of the present application.

Conversely, the compound having the activity of inhibiting the protein-binding activity of the peptide of the present application is useful as a safe and low toxic drug that suppresses the physiological activities of the peptide of the present application.

The compounds or salts thereof, which are obtainable using the screening methods or screening kits of the present application, are compounds selected from, e.g., peptides, proteins, non-peptide compounds, synthetic compounds, fermentation products, cell extracts, plant extracts, animal tissue extracts, blood plasma, and the like. For salts of these compounds, the same salts as those given for the peptide of the present application above may be used.

When the compounds obtainable using the screening methods or screening kits of the present application are used as the therapeutic/prophylactic agents described above, a conventional manner may apply thereto. The compounds may be prepared in the form of tablets, capsules, elixirs, microcapsules, sterile solutions, suspensions, etc. in a manner similar to the method for preparing the pharmaceutical preparation comprising the peptide of the present application described hereinabove.

Since the thus obtained pharmaceutical preparation is safe and low toxic, the preparation can be administered to, for example, human warm-blooded animal (e.g., mouse, rat, rabbit, sheep, swine, bovine, horse, chicken, cat, dog, monkey, chimpanzee etc.).

The dose of the compound or its salt may vary depending on its action, target disease, subject to be administered, route for administration, etc. When the compound promoting the protein-binding activity of the peptide of the present application is orally administered, the compound is administered to adult (as 60 kg) generally in a daily dose of approximately 0.1 mg to 100 mg, preferably approximately 1.0 mg to 50 mg, more preferably approximately 1.0 to 20 mg. When the compound is parenterally administered, a single dose of the compound may vary depending on subject to be administered, target disease, etc. When the compound promoting the protein-binding activity of the peptide of the present application is administered to adult (as 60 kg body weight) in the form of injection, it is convenient to administer the compound by intravenous injection, generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

**(3) Quantification of the peptide of the presents application**

The antibody of the present application is capable of specifically recognizing the peptide of the present application and can thus be used for quantification of the peptide of the present application in a test sample fluid, in particular, for quantification by the sandwich immunoassay.

That is, the present application discloses:

(i) a method for quantification of the peptide of the present application in a test sample fluid, which comprises competitively reacting the antibody of the present application, a test sample fluid and a labeled form of the peptide of the present application, and measuring the ratio of the labeled peptide of the present application bound to the antibody; and

(ii) a method for quantification of the peptide of the present application in a test sample fluid, which comprises reacting the test sample fluid simultaneously or continuously with the antibody of the present application immobilized on a carrier and a labeled form of the antibody of the present application, and then measuring the activity of the labeling agent on the insoluble carrier.

In the quantification method in the above-mentioned (ii), it is desirable that one antibody is an antibody recognizing the N-terminal region of the peptide of the present application, and the other antibody is an antibody reacting with the C-terminal region of the peptide of the present application.

The monoclonal antibody to the peptide of the present application may be used to quantify the peptide of the present application. Besides, the peptide of the present application may also be detected by means of tissue staining. For these purposes, the antibody molecule per se may be used or $F(ab')_2$, Fab' or Fab fractions of the antibody molecule may be used as well.

There is no particular limitation to the method of quantifying the peptide of the present application using the antibody of the present application any method may be used so far as it relates to a method in which the amount of antibody, antigen or antibody-antigen complex can be detected by a chemical or a physical means, depending on or corresponding to the amount of antigen (e.g., the amount of the peptide) in a test sample fluid to be assayed, and then calculated using a standard curve prepared by a standard solution containing the known amount of antigen. Advantageously used are, for example, nephrometry, competitive method, immunometric method and sandwich method; in terms of sensitivity and specificity, the sandwich method, which will be later described, is particularly preferred.

Examples of the labeling agent used in the assay method using the labeling substance are radioisotopes, enzymes, fluorescent substances, luminescent substances, etc. Examples of the radioisotope are [$^{125}I$], [$^{131}I$], [$^{3}H$], [$^{14}C$], etc. Preferred examples of the enzyme are those that are stable and have a high specific activity, which include β-galactosidase, β-glucosidase, alkaline phosphatase, peroxidase, malate dehydrogenase, etc. Examples of the fluorescent substance are fluorescamine, fluorescein isothiocyanate, etc. Examples of the luminescent substance are luminol, a luminol derivative, luciferin, lucigenin, etc. Furthermore, the biotin-avidin system may also be used for binding of an antibody or antigen to a labeling agent.

In the immobilization of antigens or antibodies, physical adsorption may be used. Alternatively, chemical binding conventionally used for immobilization of peptides or enzymes may be used as well. Examples of the carrier include insoluble polysaccharides such as agarose, dextran and cellulose; synthetic resins such as polystyrene, polyacrylamide, silicone, etc.; glass; and the like.

In the sandwich method, a test sample fluid is reacted with an immobilized form of the monoclonal antibody of the present application (Primary reaction), then reacted with another labeled form of the monoclonal antibody of the present application (secondary reaction) and the activity of the labeling agent on the insoluble carrier is assayed, whereby the amount of the peptide of the present application in the test sample fluid can be quantified. The primary and secondary reactions may be carried out in a reversed order, simultaneously or sequentially with an interval. The type of the labeling agent and the method for immobilization may be effected by modifications of those described hereinabove. In the immunoassay by the sandwich method, it is not always necessary that the antibody used for the labeled antibody and for the solid phase should be one type or one species but a mixture of two or more antibodies may be used as well, for the purpose of improving the measurement sensitivity, etc.

In the method for assaying the peptide of the present application by the sandwich method according to the present application, preferred monoclonal antibodies of the present application used for the primary and the secondary reactions are antibodies whose binding sites to the peptide of the present application are different from one another. Thus, the antibodies used in the primary and the secondary reactions are those wherein, when the antibody used in the secondary reaction recognizes the C-terminal region of the peptide of the present application, the antibody recognizing the site other than the C-terminal region, e.g., recognizing the N-terminal region, is preferably used in the primary reaction.

The monoclonal antibody of the present application may be used in an assay system other than the sandwich method, such as a competitive method, an immunometric method, nephrometry, etc.

In the competitive method, an antigen in a test sample fluid and a labeled antigen are competitively reacted with an antibody, then the unreacted labeled antigen (F) and the labeled antigen bound to the antibody (B) are separated (i.e., B/F separation) and the labeled amount of either B or F is measured to determine the amount of the antigen in the test sample fluid. In the reactions for such a method, there are a liquid phase method in which a soluble antibody is used as the antibody and the B/F separation is effected by polyethylene glycol while a second antibody to the antibody described above is used, and a solid phase method in which an immobilized antibody is used as the first antibody or a soluble antibody is used as the first antibody while an immobilized antibody is used as the second antibody.

In the immunometric method, an antigen in a test sample fluid and an immobilized antigen are competitively reacted with a given amount of a labeled antibody followed by separating the solid phase from the liquid phase; or an antigen in a test sample fluid and an excess amount of labeled antibody are reacted, then an immobilized antigen is added to bind an unreacted labeled antibody to the solid phase, and the solid phase is separated from the liquid phase. Thereafter, the labeled amount of any of the phases is measured to determine the antigen amount in the test sample fluid.

In the nephrometry, the amount of insoluble sediment, which is produced as a result of the antigen-antibody reaction in a gel or in a solution, is measured. Even when the amount of an antigen in a test sample fluid is small and only a small amount of the sediment is obtained, laser nephrometry utilizing laser scattering can be suitably used.

In applying each of those immunoassays to the quantification method of the present application, any special conditions or operations are not required to set forth. The assay system for the peptide of the present application may be constructed in addition to conditions or operations conventionally used for each of the methods, taking the technical consideration by one skilled in the art into account. For the details of such conventional technical means, a variety of reviews, reference books, etc. may be referred to (for example, Hiroshi Irie (ed.): "Radioimmunoassay" (published by Kodansha, 1974); Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immuochemical Techniques (Part A)); ibid., Vol. 73 (Immunochemical Techniques. (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)) (published by Academic Press); etc.)

As described above, the peptide of the present application can be quantified with high sensitivity, using the antibody of the present application.

Furthermore, when a decrease in the concentration of the peptide of the present application detected by quantifying the concentration of the peptide of the present application using the antibody of the present application, it can be diagnosed that for example a disease related to deficiency in the functions of the peptide of the present application is involved or it is highly likely to suffer from such a disease in the future.

When an increase in the concentration of the peptide of the present application is detected, it can be diagnosed that for example a disease related to excessive expression of the peptide of the present application is involved or it is highly likely to suffer from such a disease in the future.

The diseases with which deficiency in the functions of peptide F, G, H or I of the present application or the diseases attributable to overexpression of the peptide include, for example:

(i) diseases in the central nerves (for example, Alzheimer's disease/dementia/difficulty in eating (anorexia)/epilepsy, etc.), diseases in the hormone system (for example, faint labor, flaccid bleeding, before and after placenta expulsion, insufficiency in uterine restoration, Caesarean operation, artificial abortion, milk statis etc.), diseases in liver/gall/pancreas/internal secretion (for example, diabetes/difficulty in eating, etc.), inflammatory diseases (allergy/asthma/rheumatism etc.), circulatory organ diseases (for example, high blood pressure/cardiac hypertrophy/angina/arteriosclerosis, etc.),
(ii) diseases related to deficiency in secretion of prolactin, for example, depression of ovarian functions, deficiency in growth of seminal vesicle, osteoporosis, menopausal disorders, deficiency in secretion of milk, hypothyroidism, renal insufficiency,
(iii) diseases related to excessive secretion of prolactin, such as hyperprolactinemia, pituitary gland tumor, interbrain tumor, abnormal menstruation, stress, autoimmune disease, prolactinoma, sterility, impotence, amenorrhea, galactorrhea, acromegaly, Chiari-Frommel syndrome, Argonz-del Castilo syndrome, Forbes-Albright syndrome, breast cancer lymphoma, Sheehan's syndrome or abnormality in spermatogenesis,
(iv) choriocarcinoma, hydatidiform mole, invasive mole, abortion, insufficiency in fetal growth, abnormality in sugar metabolism, abnormality in lipid metabolism or delivery induction,
(v) high blood pressure, autoimmune disease, cardiac insufficiency, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, bone fracture, breast cancer, bulimia, [bulimia], burn healing, uterine cervix cancer, chronic lymphatic leukemia, chronic myeloid leukemia, chronic pancreatitis, hepatocirrhosis, large-intestine cancer (colon/rectum cancer), Crohn's disease, dementia, diabetic complications, diabetic nephritis, diabetic neuropathy, diabetic retinitis, gastritis, helicobacter pylori infection, renal insufficiency, type A hepatitis, type B hepatitis, type C hepatitis, hepatitis, herpes simplex virus infection, varicella zoster virus infection, Hodgkin's disease, AIDS infection, human papilloma virus infection, hypercalcemia, hypercholesterolemia, hyperglyceridemia, hyperlipemia, infection, influenza infection, insulin-dependent diabetes (type I), invasive Staphylococcus infection, malignant melanoma, cancer metastasis, multiple myeloma, allergic rhinitis, nephritis, non-Hodgkin lymphoma, insulin-independent diabetes (type II), non-small cell lung cancer, organ transplant operation, bone arthritis, bone softening, bone decrease, osteoporosis, ovary cancer, bone Behcet's disease, digestive ulcer, peripheral blood vessel disease, prostate cancer, reflux esophagitis, renal insufficiency, rheumatoid arthritis, schizophrenia, septicemia, septicemic shock, severe systemic fungal infection, small cell lung cancer, spinal damage, stomach cancer, systemic lupus erythematosus, transient cerebral ischemia attack, tuberculosis, valvular heart disease, vascular/multiple infarct dementia, wound healing, insomnia, arthritis, insufficiency in secretion of pituitary hormone, frequent urination, uremia, or nerve degeneration disease,
(vi) macular edema cystoid,
(vii) acromegaly, TSH-producing tumor, non-secretory (non-functional) pituitary tumor, ectopic ACTH (adrenocorticotropine)-producing tumor, marrow-like thyroid cancer, VIP-producing tumor, glucagon-producing tumor, gastrin-producing tumor, insulinoma, carcinoid etc.,
(viii) insulin dependent or independent diabetes, or various diseases related to such diabetes, that is, diabetic complications (for example, diabetic retinitis, diabetic nephritis, diabetic neuropathy, down syndrome, erectile low blood pressure, etc.),
(ix) obesity, bulimia etc. to be treated by ameliorating hyperinsulinemia or suppressing appetite,
(x) acute pancreatitis, chronic pancreatitis, pancreas/intestine physter, bleeding ulcer, digestive ulcer, gastritis, gastric hyperacidity, reflux esophagitis, etc.,
(xi) various symptoms caused by infection with helicobacter pylori bacteria (for example, promotion of gastrin secretion, etc.),
(xii) suppression of amylase secretion by endoscopic cholangio-pancreatography, (xiii) diarrhea attributable to a reduction of the absorbing ability of small intestine, acceleration of secretion, or abnormality in mobility of digestive tract (for example, short bowel syndrome, etc.), diarrhea attributable to chemicals in chemotherapy of cancer, diarrhea attributable to congenital shrinkage of small intestine, diarrhea attributable to neuroendocrine tumor such as VIP-producing tumor, diarrhea attributable to AIDS, diarrhea attributable to graft versus host reaction accompanying a marrow transplant, diarrhea attributable to diabetes, diarrhea attributable to blockage of abdominal nerve plexuses, diarrhea attributable to systematic sclerosis, diarrhea attributable to eosinophilia, etc.,
(xiv) damping syndrome, hypersensitive colitis, Crohn's disease, inflammatory intestinal diseases, etc.,
(xv) tumors or cancers (for example, thyroid cancer, large intestine cancer, breast cancer, prostate cancer, small cell lung cancer, non-small cell lung cancer, pancreas cancer, stomach cancer, cholangioma, liver cancer, bladder cancer, ovary cancer, melanoma, osteosarcoma, chondrosarcoma, malignant pheochromocytoma, neuroblastoma,

brain tumor, thymoma, kidney cancer, etc.), leukemia (for example, basophil leukemia/chronic lymphatic leukemia, chronic myeloid leukemia, Hodgkin's disease, non-Hodgkin lymphoma, etc.),
(xvi) hypertrophic myocardial disease, arteriosclerosis, valvular heart disease, myocardial infarction (particularly, myocardial infarction after percutaneous transluminal coronary angioplasty), revascularization,
(xvii) esophagus vein cancer bleeding, hepatocirrhosis, peripheral blood vessel disease, (xviii) diseases accompanying systemic or topical inflammations (for example, multiple arteritis, rheumatoid arthritis, psoriasis, sunburn, eczema, allergies (for example, asthma, atopic dermatitis, allergic rhinitis, etc.)),
(xix) dementia (for example, Alzheimer's disease, Alzheimer type senile dementia, vascular/multiple dementia, etc.), schizophrenia, epilepsy, depression, general anxiety, difficult sleep, multiple sclerosis, etc.,
(xx) eye diseases (for example, glaucoma, etc.),
(xxi) acute bacterial meningitis, acute viral encephalitis, adult respiratory distress syndrome, bacterial pneumonia, severe systemic fungal infection, tuberculosis, spinal damage, bone fracture, renal insufficiency, pneumonia, alcoholic hepatitis, type A hepatitis, type B hepatitis, type C hepatitis, AIDS infection, human papilloma virus infection, influenza infection, cancer metastasis, multiple myeloma, bone softening, osteoporosis, bone Behcet's disease, nephritis, renal insufficiency, septicemia, septicemic shock, hypercalcemia, hypercholesterolemia, hyperglyceridemia, hyperlipemia, systemic lupus erythematosus, transient cerebral ischemia attack, alcoholic hepatitis, (xxii) organ transplant operation, burn, wound, alopecia, etc.,
(xxiii) chronic or acute pains (for example, acute pain after operation, inflammatory acute pain, toothache, bone diseases (for example, acute pains accompanying arthritis, rheumatism, osteoporosis, etc.)) and
(xxiv) pain.

The antibody of the present application can be employed for detecting the peptide of the present application which may be present in a test sample fluid such as a body fluid, a tissue, etc. The antibody can also be used to prepare an antibody column for purification of the peptide of the present application, detection of the peptide of the present application in the fractions when purified, and analysis of the behavior of the peptide of the present application in the cells under investigation, etc.

(4) Gene diagnostic agent

By using the polynucleotide of the present application or the antisense polynucleotide of the present application, e.g., as a probe, an abnormality (gene abnormality) of the DNA or mRNA encoding the peptide of the present application in human or warm-blooded animal (e.g., rat, mouse, guinea pig, rabbit, chicken, sheep, swine, bovine, horse, cat, dog, monkey, etc.) can be detected. Therefore, the polynucleotide or antisense polynucleotide of the present application is useful as a gene diagnostic agent for the damage to the DNA or mRNA, its mutation, or its decreased expression, or increased expression or overexpression of the DNA or mRNA.
The gene diagnosis described above using the polynucleotide of the present application or or the antisense polynucleotide of the present application can be performed by, for example, the publicly known Northern hybridization assay, the PCR-SSCP assay (Genomics, 5, 874-879 (1989); Proceedings of the National Academy of Sciences of the United States of America, 86, 2766-2770 (1989)).
Furthermore, when decreased expression is detected by Northern hybridization, it can be diagnosed that for example a disease related to deficiency in the functions of the peptide of the present application is involved or it is highly likely to suffer from such a disease in the future.
When overexpression is detected by Northern hybridization, it can be diagnosed that for example a disease related to overexpression of the peptide of the present application is involved or it is highly likely to suffer from such a disease in the future.
The diseases related to decreased expression or overexpression of the peptide F, G, H or I of the present application include, for example:

(i) diseases in the central nerves (for example, Alzheimer's disease/dementia/difficulty in eating (anorexia)/epilepsy, etc.), diseases in the hormone system (for example, faint labor, flaccid bleeding, before and after placenta expulsion, insufficiency in uterine restoration, Caesarean operation, artificial abortion, milk statis etc.), diseases in liver/gall/pancreas/internal secretion (for example, diabetes/difficulty in eating, etc.), inflammatory diseases (allergy/asthma/rheumatism etc.), circulatory organ diseases (for example, high blood pressure/cardiac hypervophy/angina/arteriosclerosis, etc.),
(ii) diseases related to deficiency in secretion of prolactin, for example, depression of ovarian functions, deficiency in growth of seminal vesicle, osteoporosis, menopausal disorders, deficiency in secretion of milk, hypothyroidism, renal insufficiency,
(iii) diseases related to excessive secretion of prolactin, such as hyperprolactinemia, pituitary gland tumor, interbrain tumor, abnormal menstruation, stress, autoimmune disease, prolactinoma, sterility, impotence, amenorrhea, galactorrhea, acromegaly, Chiari-Frommel syndrome, Argonz-del Castilo syndrome, Forbes-Albright syndrome, breast cancer lymphoma, Sheehan's syndrome or abnormality in spermatogenesis,

(iv) choriocarcinoma, hydatidiform mole, invasive mole, abortion, insufficiency in fetal growth, abnormality in sugar metabolism, abnormality in lipid metabolism or delivery induction,

(v) high blood pressure, autoimmune disease, cardiac insufficiency, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, bone fracture, breast cancer, bulimia, [bulimia], burn healing, uterine cervix cancer, chronic lymphatic leukemia, chronic myeloid leukemia, chronic pancreatitis, hepatocirrhosis, large-intestine cancer (colon/rectum cancer), Crohn's disease, dementia, diabetic complications, diabetic nephritis, diabetic neuropathy, diabetic retinitis, gastritis, helicobacter pylori infection, renal insufficiency, type A hepatitis, type B hepatitis, type C hepatitis, hepatitis, herpes simplex virus infection, varicella zoster virus infection, Hodgkin's disease, AIDS infection, human papilloma virus infection, hypercalcemia, hypercholesterolemia, hyperglyceridemia, hyperlipemia, infections, influenza infection, insulin-dependent diabetes (type I), invasive Staphylococcus infection, malignant melanoma, cancer metastasis, multiple myeloma, allergic rhinitis, nephritis, non-Hodgkin lymphoma, insulin-independent diabetes (type II), non-small cell lung cancer, organ transplant operation, bone arthritis, bone softening, bone decrease, osteoporosis, ovary cancer, bone Behcet's disease, digestive ulcer, peripheral blood vessel disease, prostate cancer, reflux esophagitis, renal insufficiency, rheumatoid arthritis, schizophrenia, septicemia, septicemic shock, severe systemic fungal infection, small cell lung cancer, spinal damage, stomach cancer, systemic lupus erythematosus, transient cerebral ischemia attack, tuberculosis, valvular heart disease, vascular/multiple infarct dementia, wound healing, insomnia, arthritis, insufficiency in secretion of pituitary hormone, frequent urination, uremia, or nerve degeneration disease,

(vi) macular edema cystoid,

(vii) acromegaly, TSH-producing tumor, non-secretory (non-functional) pituitary tumor, ectopic ACTH (adrenocorticotropine)-producing tumor, marrow-like thyroid cancer, VIP-producing tumor, glucagon-producing tumor, gastrin-producing tumor, insulinoma, carcinoid etc.,

(viii) insulin dependent or independent diabetes, or various diseases related to such diabetes, that is, diabetic complications (for example, diabetic retinitis, diabetic nephritis, diabetic neuropathy, down syndrome, erectile low blood pressure, etc.),

(ix) obesity, bulimia etc. to be treated by ameliorating hyperinsulinemia or suppressing appetite,

(x) acute pancreatitis, chronic pancreatitis, pancreas/intestine physter, bleeding ulcer, digestive ulcer, gastritis, gastric hyperacidity, reflux esophagitis, etc.,

(xi) various symptoms caused by infection with helicobacter pylori bacteria (for example, promotion of gastrin secretion, etc.),

(xii) suppression of amylase secretion by endoscopic cholangio-pancreatography, (xiii) diarrhea attributable to a reduction of the absorbing ability of small intestine, acceleration of secretion, or abnormality in mobility of digestive tract (for example, short bowel syndrome, etc.), diarrhea attributable to chemicals in chemotherapy of cancer, diarrhea attributable to congenital shrinkage of small intestine, diarrhea attributable to neuroendocrine tumor such as VIP-producing tumor, diarrhea attributable to AIDS, diarrhea attributable to graft versus host reaction accompanying a marrow transplant, diarrhea attributable to diabetes, diarrhea attributable to blockage of abdominal nerve plexuses, diarrhea attributable to systematic sclerosis, diarrhea attributable to eosinophilia, etc.,

(xiv) damping syndrome, hypersensitive colitis, Crohn's disease, inflammatory intestinal diseases, etc.,

(xv) tumors or cancers (for example, thyroid cancer, large intestine cancer, breast cancer, prostate cancer, small cell lung cancer, non-small cell lung cancer, pancreas cancer, stomach cancer, cholangioma, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (for example, basophil leukemia/chronic lymphatic leukemia, chronic myeloid leukemia, Hodgkin's disease, non-Hodgkin lymphoma, etc.),

(xvi) hypertrophic myocardial disease, arteriosclerosis, valvular heart disease, myocardial infarction (particularly, myocardial infarction after percutaneous transluminal coronary angioplasty), revascularization,

(xvii) esophagus vein cancer bleeding, hepatocirrhosis, peripheral blood vessel disease,

(xviii) diseases accompanying systemic or topical inflammations (for example, multiple arteritis, rheumatoid arthritis, psoriasis, sunburn, eczema, allergies (for example, asthma, atopic dermatitis, allergic rhinitis, etc.)),

(xix) dementia (for example, Alzheimer's disease, Alzheimer type senile dementia, vascular/multiple dementia. etc.), schizophrenia, epilepsy, depression, general anxiety, difficult sleep, multiple sclerosis, etc.,

(xx) eye diseases (for example, glaucoma, etc.),

(xxi) acute bacterial meningitis, acute viral encephalitis, adult respiratory distress syndrome, bacterial pneumonia, severe systemic fungal infection, tuberculosis, spinal damage, bone fracture, renal insufficiency, pneumonia, alcoholic hepatitis, type A hepatitis, type B hepatitis, type C hepatitis, AIDS infection, human papilloma virus infection, influenza infection, cancer metastasis, multiple myeloma, bone softening, osteoporosis, bone Behcet's disease, nephritis, renal insufficiency, septicemia, septicemic shock, hypercalcemia, hypercholesterolemia, hyperglyceridemia, hyperlipemia, systemic lupus erythematosus, transient cerebral ischemia attack, alcoholic hepatitis, (xxii) organ transplant operation, burn, wound, alopecia, etc.,

(xxiii) chronic or acute pains (for example, acute pain after operation, inflammatory acute pain, toothache, bone diseases (for example, acute pains accompanying arthritis, rheumatism, osteoporosis, etc.)) and

(xxiv) pain.

**(5) Pharmaceutical preparation** (pharmaceutical, drug or medicine) comprising the **antisense polynucleotide**

The antisense polynucleotide of the present application that binds to the polynucleotide (e.g., DNA) of the present application complementarily to inhibit expression of the polynucleotide (e.g., DNA) is low-toxic and can suppress the functions of the receptor protein of the present application or the polynucleotide (e.g., DNA) of the present application in the body, and is thus used as the agent for the treatment/prevention of diseases caused by, e.g., overexpression of the peptide of the present application.

Specifically, the antisense polynucleotide to the DNA encoding the peptide F, G, H or I of the present application can inhibit the functions of peptide F, G, H or I of the present application, and is thus useful as a safe and low toxic pharmaceutical preparation suppressing the activity of peptide F, G, H or I of the present application, for example, a hormone secretion regulator (for example, an inhibitory for secretion of adrenal cortical hormone such as corticosterone), an eating regulator, a sleep regulator, an arousal regulator, a pain regulator, a stress response regulator, a spontaneous behavior regulator or an emotional behavior regulator, and as a prophylactic/therapeutic agent for diseases caused by excessive production of the peptide F, G, H or I of the present application, such as diseases in the central nerves, diseases in the hormone system, diseases in liver/gall/pancreas/internal secretion (for example, an anti-obesity drug/excessive eating, etc.), inflammatory diseases, circulatory organ diseases, etc.

Further, the antisense polynucleotide to the DNA encoding the peptide F, G, H or I of the present application is useful as a regulator for secretion of prolactin. That is, when the antisense polynucleotide to the DNA encoding the peptide F, G, H or I of the present application has a promoting action on secretion of prolactin, it is useful as a prolactin secretion promoter for diseases with which deficiency in secretion of prolactin is associated, for example, a prophylactic/therapeutic agent for diseases related to prolactin secretion, such as depression of ovarian functions, deficiency in growth of seminal vesicle, osteoporosis, menopausal disorders, deficiency in secretion of milk, hypothyroidism, renal insufficiency, etc.

Further, when the antisense polynucleotide to the DNA encoding the peptide F, G, H or I of the present application has a promoting action on secretion of prolactin, it has a promoting action on sexual desire (pheromone-like action) based on the promoting action on secretion of prolactin, and is thus useful as an agent for promoting sexual desire.

When the antisense polynucleotide to the DNA encoding the peptide F, G, H or I of the present application has an inhibitory action on secretion of prolactin, it is useful as a prolactin secretion inhibitor, for example a prophylactic/therapeutic agent for various diseases related to excessive secretion of prolactin, such as hyperprolactinemia, pituitary gland tumor, interbrain tumor, abnormal menstruation, stress, autoimmune disease, prolactinoma, sterility, impotence, arnenorrhea, galactorrhea, acromegaly, Chiari-Frommel syndrome, Argonz-del Castilo syndrome, Forbes-Albright syndrome, breast cancer lymphoma, Sheehan's syndrome or abnormality in spermatogenesis. Further, when the antisense polynucleotide to the DNA encoding the peptide F, G, H or I of the present application has an inhibitory action on secretion of prolactin, it is useful as a contraceptive agent based on the inhibitory action on secretion of prolactin.

In addition, the antisense polynucleotide to the DNA encoding the peptide F, G, H or I of the present application is also useful as a diagnostic drug for examining an ability to secrete prolactin or as an animal drug such as a milk secretion promoting agent for mammals such as bovine, goat, swine etc., and can be applied to production of a useful substance by producing the useful substance in the mammals and secreting it in milk.

Further, the antisense polynucleotide to the DNA encoding the peptide F, G, H or I of the present application useful as a prophylactic/therapeutic agent for choriocarcinoma, hydatidiform mole, invasive mole, abortion, insufficiency in fetal growth, abnormality in sugar metabolism, abnormality in lipid metabolism or delivery induction.

Further, the antisense polynucleotide to the DNA encoding the peptide F, G, H or I of the present application is useful as a prophylactic/therapeutic agent for diseases such as high blood pressure, autoimmune disease, cardiac insufficiency, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, bone fracture, breast cancer, bulimia, [bulimia], burn healing, uterine cervix cancer, chronic lymphatic leukemia, chronic myeloid leukemia, chronic pancreatitis, hepatocirrhosis, large-intestine cancer (colon/rectum cancer), Crohn's disease, dementia, diabetic complications, diabetic nephritis, diabetic neuropathy, diabetic retinitis, gastritis, helicobacter pylori infection, renal insufficiency, type A hepatitis, type B hepatitis, type C hepatitis, hepatitis, herpes simplex virus infection, varicella zoster virus infection, Hodgkin's disease, AIDS infection, human papilloma virus infection, hypercalcemia, hypercholesterolemia, hyperglyceridemia, hyperlipemia, infections, influenza infection, insulin-dependent diabetes (type I), invasive Staphylococcus infection, malignant melanoma, cancer metastasis, multiple myeloma, allergic rhinitis, nephritis, non-Hodgkin lymphoma, insulin-independent diabetes (type II), non-small cell lung cancer, organ transplant operation, bone arthritis, bone softening, bone decrease, osteoporosis, ovary cancer, bone Behcet's disease, digestive ulcer, peripheral blood vessel disease, prostate cancer, reflux esophagitis, renal insufficiency, rheumatoid arthritis, schizophrenia, septicemia, septicemic shock, severe systemic fungal infection, small cell lung cancer, spinal damage, stomach cancer, systemic lupus erythematosus, transient cerebral ischemia attack, tuberculosis, valvular heart disease, vascular/multiple infarct dementia, wound healing, insomnia, arthritis, insufficiency in secretion of pituitary hormone, frequent urination, uremia, or nerve degeneration disease.

Further, the antisense polynucleotide to the DNA encoding the peptide F, G, H or I of the present application can be used as a pharmaceutical preparation such as a therapeutic/prophylactic agent for a disease macular edema cystoid.

Further, the antisense polynucleotide to the DNA encoding the peptide F, G, H or I of the present application is useful for example as (1) a therapeutic agent for tumors such as acromegaly, TSH-producing tumor, non-secretory (non-functional) pituitary tumor, ectopic ACTH (adrenocorticotropine)-producing tumor, marrow-like thyroid cancer, VIP-producing tumor, glucagon-producing tumor, gastrin-producing tumor, insulinoma, carcinoid etc., (2) a therapeutic agent for insulin dependent or independent diabetes, or various diseases related to such diabetes, that is, diabetic complications (for example, diabetic retinitis, diabetic nephritis, diabetic neuropathy, down syndrome, erectile low blood pressure, etc.), (3) a therapeutic, agent for obesity, bulimia etc. by ameliorating hyperinsulinemia or suppressing appetite, (4) a therapeutic agent for acute pancreatitis, chronic pancreatitis, pancreas/intestine physter, bleeding ulcer, digestive ulcer, gastritis, gastric hyperacidity, reflux esophagitis, etc., (5) an agent for ameliorating various symptoms caused by infection with helicobacter pylori bacteria (for example, an agent for inhibiting promotion of gastrin secretion, etc.), (6) an agent for suppressing amylase secretion caused by endoscopic cholangio-pancreatography, and for prognosis of surgical operation of pancreas, (7) a therapeutic agent for diarrhea attributable to a reduction of the absorbing ability of small intestine, acceleration of secretion, or abnormality in mobility of digestive tract (for example, short bowel syndrome, etc.), diarrhea attributable to chemicals in chemotherapy of cancer, diarrhea attributable to congenital shrinkage of small intestine, diarrhea attributable to neuroendocrine tumor such as VIP-producing tumor, diarrhea attributable to AIDS, diarrhea attributable to graft versus host reaction accompanying a marrow transplant, diarrhea attributable to diabetes, diarrhea attributable to blockage of abdominal nerve plexuses, diarrhea attributable to systematic sclerosis, diarrhea attributable to eosinophilia, etc., (8) a therapeutic agent for damping syndrome, hypersensitive colitis, Crohn's disease, inflammatory intestinal diseases, etc., (9) a therapeutic agent for tumors or cancers (for example, thyroid cancer, large intestine cancer, breast cancer, prostate cancer, small cell lung cancer, non-small cell lung cancer, pancreas cancer, stomach cancer, cholangioma, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (for example, basophil leukemia/chronic lymphatic leukemia, chronic myeloid leukemia, Hodgkin's disease, non-Hodgkin lymphoma, etc.); the therapeutic agent can be used alone or in combination with other anticancer drugs (for example, tamoxifen, LHRH agonist, LHRH antagonist, interferon-$\alpha$,$\beta$ and $\gamma$, interleukin-2, etc.), (10) a prophylactic/therapeutic agent for hypertrophic myocardial disease, arteriosclerosis, valvular heart disease, myocardial infarction (particularly, myocardial infarction after percutaneous transluminal coronary angioplasty), revascularization, (11) a therapeutic agent for esophagus vein cancer bleeding, hepatocirrhosis, peripheral blood vessel disease, (12) a therapeutic agent for diseases accompanying systemic or topical inflammations (for example, multiple arteritis, rheumatoid arthritis, psoriasis, sunburn, eczema, allergies (for example, asthma, atopic dermatitis, allergic rhinitis, etc.)), based on the secretion-regulating action of phsyoloigcally active substance acting on the immune system (for example, substance P, tachykinin, cytokine, etc.), (13) a therapeutic agent for dementia (for example, Alzheimer's disease, Alzheimer type senile dementia, vascular/multiple dementia, etc.), schizophrenia, epilepsy, depression, general anxiety, difficult sleep, multiple sclerosis, etc., because of its influence on production and secretion of nerve-regulating factor, (14) a therapeutic agent for eye diseases (for example, glaucoma, etc.), (15) a prophylactic/therapeutic agent for acute bacterial meningitis, acute viral encephalitis, adult respiratory distress syndrome, bacterial pneumonia, severe systemic fungal infection, tuberculosis, spinal damage, bone fracture, renal insufficiency, pneumonia, alcoholic hepatitis, type A hepatitis, type B hepatitis, type C hepatitis, AIDS infection, human papilloma virus infection, influenza infection, cancer metastasis, multiple myeloma, bone softening, osteoporosis, bone Behcet's disease, nephritis, renal insufficiency, septicemia, septicemic shock, hypercalcemia, hypercholesterolemia, hyperglyceridemia, hyperlipemia, systemic lupus erythematosus, transient cerebral ischemia attack, alcoholic hepatitis, etc., (16) a therapeutic agent for organ transplant operation, burn, wound, alopecia, etc., and (17) an analgesic for suppressing and relieving chronic or acute pains (for example, acute pain after operation, inflammatory acute pain, toothache, bone diseases (for example, acute pains accompanying arthritis, rheumatism, osteoporosis, etc.)).

When the antisense polynucleotide is used as the above therapeutic/prophylactic agent, the antisense polynucleotide can be formed into a pharmaceutical preparation in the same manner as for the above polynucleotide of the present application.

Since the thus obtained pharmaceutical preparation is safe and low toxic, the preparation can be administered orally or parenterally to human and warm-blooded animal (e.g., rat, rabbit, sheep, swine, bovine, cat, dog, monkey, etc.).

The antisense polynucleotide may be administered as it stands, or prepared into pharmaceutical preparations together with physiologically acceptable carriers such as adjuvants to assist its uptake, and such preparations are administered by gene gun or through a catheter like a hydrogel catheter.

The dose of the antisense polynucleotide may vary depending upon target disease, subject to be administered, route for administration, etc. When the antisense nucleotide is administered topically to an organ (e.g., liver, lung, heart, kidney etc.) for the purpose of treatment of cancers, the antisense nucleotide is administered to adult (60 kg body weight) in a daily dose of approximately 0.1 to 100 mg.

In addition, the antisense polynucleotide may also be employed as an oligonucleotide probe for diagnosis to examine the presence of the DNA of the present application in tissues or cells, or the states of its expression.

Further, the present application discloses:

[1] double-stranded RNA comprising a part of RNA encoding the peptide of the present application and RNA complementary thereto,
[2] a pharmaceutical preparation comprising the double-stranded RNA,
[3] ribozyme comprising a part of RNA encoding the peptide of the present application, and
[4] a pharmaceutical preparation comprising the ribozyme.

The double-stranded RNA (RNAi; RNA interference method) or the ribozyme, similar to the antisense polynucleotide described above, can suppress the expression of the polynucleotide of the present application (for example DNA) and can, in vivo, suppress the functions of the peptide G, H or I of the present application or the polynucleotide of the present application (for example, DNA) encoding the same, and can thus be used as a prophylactic/therapeutic agent for diseases attributable to overexpression of the peptide G, H or I of the present application (for example, diseases caused by excessive production of the peptide F, G, H or I of the present invention), such as diseases in the central nerves, diseases in the hormone system, diseases in liver/gall/pancreas/internal secretion (for example, an anti-obesity drug/excessive eating, etc.), inflammatory diseases, circulatory organ diseases, etc. or as a hormone secretion regulator (for example, an inhibitor for secretion of adrenal cortical hormone such as corticosterone), an eating regulator, a sleep regulator, an arousal regulator, a pain regulator, a stress response regulator, a spontaneous behavior regulator or an emotional behavior regulator.

Further, the double-stranded RNA (RNAi; RNA interference method) or the ribozyme is useful as a regulator for secretion of prolactin. That is, when the double-stranded RNA (RNAi; RNA interference method) or the ribozyme has a promoting action on secretion of prolactin, it is useful as a prolactin secretion promoter for diseases with which deficiency in secretion of prolactin is associated, for example a prophylactic/therapeutic agent for diseases related to prolactin secretion, such as depression of ovarian functions, deficiency in growth of seminal vesicle, osteoporosis, menopausal disorders, deficiency in secretion of milk, hypothyroidism, renal insufficiency, etc. When the double-stranded RNA (RNAi; RNA interference method) or the ribozyme has a promoting action on secretion of prolactin, it has a promoting action on sexual desire (pheromone-like action) based on the promoting action on secretion of prolactin, and is thus useful as an agent for promoting sexual desire.

When the double-stranded RNA (RNAi; RNA interference method) or the ribozyme has an inhibitory action on secretion of prolactin, it is useful as a prolactin secretion inhibitor for diseases with which excessive secretion of prolactin is associated, for example a prophylactic/therapeutic agent for diseases related to secretion of prolactin, such as hyperprolactinemia, pituitary gland tumor, interbrain tumor, abnormal menstruation, stress, autoimmune disease, prolactinoma, sterility, impotence, amenorrhea, galactorrhea, acromegaly, Chiari-Frommel syndrome, Argonz-del Castilo syndrome, Forbes-Albright syndrome, breast cancer lymphoma, Sheehan's syndrome or abnormality in spermatogenesis. Further, when the double-stranded RNA (RNAi; RNA interference method) or the ribozyme has an inhibitory action on secretion of prolactin, it is useful as a contraceptive agent based on the inhibitory action on secretion of prolactin.

In addition, the double-stranded RNA (RNAi; RNA interference method) or the ribozyme is also useful as a diagnostic drug for examining an ability to secrete prolactin or as an animal drug such as a milk secretion promoting agent for domestic mammals such as bovine, goat, swine etc., and can be applied to production of a useful substance by producing the useful substance in the mammals and secreting it in milk.

Further, the double-stranded RNA (RNAi; RNA interference method) or the ribozyme is useful as a prophylactic/therapeutic agent for choriocarcinoma, hydatidiform mole, invasive mole, abortion, insufficiency in fetal growth, abnormality in sugar metabolism, abnormality in lipid metabolism or delivery induction.

Further, the double-stranded RNA (RNAi ; RNA interference method) or the ribozyme is useful as a prophylactic/therapeutic agent for diseases such as high blood pressure, autoimmune disease, cardiac insufficiency, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, bone fracture, breast cancer, bulimia, [bulimia], burn healing, uterine cervix cancer, chronic lymphatic leukemia, chronic myeloid leukemia, chronic pancreatitis, hepatocirrhosis, large-intestine cancer (colon/rectum cancer), Crohn's disease, dementia, diabetic complications, diabetic nephritis, diabetic neuropathy, diabetic retinitis, gastritis, helicobacter pylori infection, renal insufficiency, type A hepatitis, type B hepatitis, type C hepatitis, hepatitis, herpes simplex virus infection, varicella zoster virus infection, Hodgkin's disease, AIDS infection, human papilloma virus infection, hypercalcemia, hypercholesterolemia, hyperglyceridemia, hyperlipernia, infections; influenza infection, insulin-dependent diabetes (type I), invasive Staphylococcus infection, malignant melanoma, cancer metastasis, multiple myeloma, allergic rhinitis, nephritis, non-Hodgkin lymphoma, insulin-independent diabetes (type II), non-small cell lung cancer, organ transplant operation, bone arthritis, bone softening, bone decrease, osteoporosis, ovary cancer, bone Behcet's disease, digestive ulcer, peripheral blood vessel disease, prostate cancer, reflux esophagitis, renal insufficiency, rheumatoid arthritis, schizophrenia, septicemia, septicemic shock, severe systemic fungal infection, small cell lung cancer, spinal damage, stomach cancer, systemic lupus erythematosus, transient cerebral ischemia attack, tuberculosis, valvular heart disease, vascular/multiple infarct dementia, wound healing, insomnia, arthritis, insufficiency in secretion of pituitary hormone, frequent urination, uremia, or nerve degeneration disease.

The double-stranded RNA (RNAi; RNA interference method) or the ribozyme can be used as a pharmaceutical preparation such as a therapeutic/prophylactic agent for macular edema cystoid.

In addition, the double-stranded RNA (RNAi; RNA interference method) or the ribozyme is useful for example as (1) a therapeutic agent for tumors such as acromegaly, TSH-producing tumor, non-secretory (non-functional) pituitary tumor, ectopic ACTH (adrenocorticotropine)-producing tumor, marrow-like thyroid cancer, VIP-producing tumor, glucagon-producing tumor, gastrin-producing tumor, insulinoma, carcinoid etc., (2) a therapeutic agent for insulin dependent or independent diabetes, or various diseases related to such diabetes, that is, diabetic complications (for example, diabetic retinitis, diabetic nephritis, diabetic neuropathy, down syndrome, erectile low blood pressure, etc.), (3) a therapeutic agent for obesity, bulimia etc. by ameliorating hyperinsulinemia or suppressing appetite, (4) a therapeutic agent for acute pancreatitis, chronic pancreatitis, pancreas/intestine physter, bleeding ulcer, digestive ulcer, gastritis, gastric hyperacidity, reflux esophagitis, etc., (5) an agent for ameliorating various symptoms caused by infection with helicobacter pylori bacteria (for example, an agent for inhibiting promotion of gastrin secretion, etc.), (6) an agent for suppressing amylase secretion caused by endoscopic cholangio-pancreatography, and for prognosis of surgical operation of pancreas, (7) a therapeutic agent for diarrhea attributable to a reduction of the absorbing ability of small intestine, acceleration of secretion, or abnormality in mobility of digestive tract (for example, short bowel syndrome, etc.), diarrhea attributable to chemicals in chemotherapy of cancer, diarrhea attributable to congenital shrinkage of small intestine, diarrhea attributable to neuroendocrine tumor such as VIP-producing tumor, diarrhea attributable to AIDS, diarrhea attributable to graft versus host reaction accompanying a marrow transplant, diarrhea attributable to diabetes, diarrhea attributable to blockage of abdominal nerve plexuses, diarrhea attributable to systematic sclerosis, diarrhea attributable to eosinophilia, etc., (8) a therapeutic agent for damping syndrome, hypersensitive colitis, Crohn's disease, inflammatory intestinal diseases, etc., (9) a therapeutic agent for tumors or cancers (for example, thyroid cancer, large intestine cancer, breast cancer, prostate cancer, small cell lung cancer, non-small cell lung cancer, pancreas cancer, stomach cancer, cholangioma, liver cancer, bladder cancer, ovary cancer, melanoma, osteosarcoma, chondrosarcoma, malignant pheochromocytoma, neuroblastoma; brain tumor, thymoma, kidney cancer, etc.), leukemia (for example, basophil leukemia/chronic lymphatic leukemia, chronic myeloid leukemia, Hodgkin's disease, non-Hodgldn lymphoma, etc.); the therapeutic agent can be used alone or in combination with other anticancer drugs (for example, tamoxifen, LHRH agonist, LHRH antagonist, interferon-$\alpha$,$\beta$ and $\gamma$, interleukin-2, etc.), (10) a , prophylactic/therapeutic agent for hypertrophic myocardial disease, arteriosclerosis, valvular heart disease, myocardial infarction, (particularly, myocardial infarction after percutaneous transluminal coronary angioplasty), revascularization, (11) a therapeutic agent for esophagus vein cancer bleeding, hepatocirrhosis, peripheral blood vessel disease, (12) a therapeutic agent for diseases accompanying systemic or topical inflammations (for example, multiple arteritis, rheumatoid arthritis, psoriasis, sunburn, eczema, allergies (for example, asthma, atopic dermatitis, allergic rhinitis, etc.)), based on the secretion-regulating action of phsyoloigcally active substance acting on the immune system (for example, substance P, tachykinin, cytokine, etc.), (13) a therapeutic agent for dementia (for example, Alzheimer's disease, Alzheimer type senile dementia, vascular/multiple dementia, etc.), schizophrenia, epilepsy, depression, general anxiety, difficult sleep, multiple sclerosis, etc., because of its influence on production and secretion of nerve-regulating factor, (14) a therapeutic agent for eye diseases (for example, glaucoma, etc.), (15) a prophylactic/therapeutic agent for acute bacterial meningitis, acute viral encephalitis, adult respiratory distress syndrome, bacterial pneumonia, severe systemic fungal infection, tuberculosis, spinal damage, bone fracture, renal insufficiency, pneumonia, alcoholic hepatitis, type A hepatitis, type B hepatitis, type C hepatitis, AIDS infection, human papilloma virus infection, influenza infection, cancer metastasis, multiple myeloma, bone softening, osteoporosis, bone Behcet's disease, nephritis, renal insufficiency, septicemia, septicemic shock, hypercalcemia, hypercholesterolemia, hyperglyceridemia, hyperlipemia, systemic lupus erythematosus, transient cerebral ischemia attack, alcoholic hepatitis, etc. (16) a therapeutic agent for organ transplant operation, burn, wound, alopecia, etc., and (17) an analgesic for suppressing and relieving chronic or acute pains (for example, acute pain after operation, inflammatory acute pain, toothache, bone diseases (for example, acute pains accompanying arthritis, rheumatism, osteoporosis, etc.)).

According to known methods (for example, Nature, vol. 411, p. 494, 2001), the double-stranded RNA can be produced by designing it on the basis of the sequence of the polynucleotide of the present application.

According to known methods (for example, TRENDS in Molecular Medicine, vol. 7, p. 221, 2001), the ribozyme can be produced by designing it on the basis of the sequence of the polynucleotide of the present application. The pharmaceutical preparation of the present application can be produced for example by linking a known ribozyme to a part of RNA encoding the peptide of the present application. A part of RNA encoding the peptide of the present application includes a region (RNA fragment) adjacent to that cleavage site of the RNA of the present application which can be cleaved with a known ribozyme.

When the double-stranded RNA or the ribozyme is used as the aforesaid prophylactic/therapeutic agent, it can be formed into a pharmaceutical preparation and administered in the same manner as for the antisense polynucleotide.

(6) Pharmaceutical preparations comprising the antibody of the present application

The antibody of the present application which has the effect of neutralizing the activities of the peptide of the present application can be used as drugs for the treatment/prevention of diseases caused by overexpression of the peptide of the present application.

Specifically, the antibody to the peptide F, G, H or I of the present application can inhibit the functions of peptide F and is thus useful as a pharmaceutical preparation suppressing the activity of peptide F, G, H or I of the present application, for example a hormone secretion regulator (for example, am inhibitor for secretion of adrenal cortical hormone such as corticosterone), an eating regulator, a sleep regulator, an arousal regulator, a pain regulator, a stress response regulator, a spontaneous behavior regulator or an emotional behavior regulator, and is useful as a prophylactic/therapeutic agent for diseases caused by excessive production of the peptide F, G, H or I of the present application, such as diseases in the central nerves, diseases in the hormone system, diseases in liver/gall/pancreas/internal secretion (for example, an anti-obesity drug/excessive eating, etc.), inflammatory diseases, circulatory organ diseases etc.

Further, the antibody to the peptide F of the present application is useful as an agent for regulating prolactin secretion. That is, when the antibody to the peptide F, G, H or I of the present application has a promoting action on secretion of prolactin, it is useful as a prolactin secretion promoter for various diseases with which deficiency in secretion of prolactin is associated, for example a prophylactic/therapeutic agent for diseases related to secretion of prolactin, such as depression of ovarian functions, deficiency in growth of seminal vesicle, osteoporosis, menopausal disorders, deficiency in secretion of milk, hypothyroidism, renal insufficiency etc. Further, when the antibody to the peptide F, G, H or I of the present application has a promoting action on secretion of prolactin, it has a promoting action on sexual desire (pheromone-like action) based on the promoting action on secretion of prolactin, and is thus useful as an agent for promoting sexual desire.

When the antibody to the peptide F, G, H or I of the present application has an inhibitory action on secretion of prolactin, it is useful as a prolactin secretion inhibitor for diseases with which excessive secretion of prolactin is associated, for example a prophylactic/therapeutic agent for diseases related to secretion of prolactin, such as hyperprolactinemia, pituitary gland tumor, interbrain tumor, abnormal menstruation, stress, autoimmune disease, prolactinoma, sterility, impotence, amenorrhea, galactorrhea, acromegaly, Chiari-Frommel syndrome, Argonz-del Castilo syndrome, Forbes-Albright syndrome, breast cancer lymphoma, Sheehan's syndrome or abnormality in spermatogenesis. Further, when the antibody to the peptide F, G, H or I of the present application has an inhibitory action on secretion of prolactin, it is useful as a contraceptive agent based on the inhibitory action on secretion of prolactin.

In addition, the antibody to the peptide F, G, H or I of the present application is also useful as a diagnostic drug for examining an ability to secrete prolactin or as an animal drug such as a milk secretion promoting agent for mammals such as bovine, goat, swine etc., and can be applied to production of a useful substance by producing the useful substance in the mammals and secreting it in milk.

Further, the antibody to the peptide F, G, H or I of the present application has a regulatory action on the functions of placenta and is thus useful as a prophylactic/therapeutic agent for choriocarcinoma, hydatidiform mole, invasive mole, abortion, insufficiency in fetal growth, abnormality in sugar metabolism, abnormality in lipid metabolism or delivery induction.

Further, the antibody to the peptide F, G, H or I of the present application is useful as pharmaceutical preparation, for example, a therapeutic/prophylactic agent for diseases such as high blood pressure, autoimmune disease, cardiac insufficiency, cataract, glaucoma, acute bacterial meningitis, acute myocardial infarction, acute pancreatitis, acute viral encephatitis, adult respiratory distress syndrome, alcoholic hepatitis, Alzheimer's disease, asthma, arteriosclerosis, atopic dermatitis, bacterial pneumonia, bladder cancer, bone fracture, breast cancer, bulimia, [bulimia], burn healing, uterine cervix cancer, chronic lymphatic leukemia, chronic myeloid leukemia, chronic pancreatitis, hepatocirrhosis, large-intestine cancer (colon/rectum cancer), Crohn's disease, dementia, diabetic complications, diabetic nephritis, diabetic neuropathy, diabetic retinitis, gastritis, helicobacter pylori infection, renal insufficiency, type A hepatitis, type B hepatitis, type C hepatitis, hepatitis, herpes simplex virus infection, varicella zoster virus infection, Hodgkin's disease, AIDS infection, human papilloma virus infection, hypercalcemia, hypercholesterolemia, hyperglyceridemia, hyperlipemia, infections, influenza infection, insulin-dependent diabetes (type I), invasive Staphylococcus infection, malignant melanoma, cancer metastasis, multiple myeloma, allergic rhinitis, nephritis, non-Hodgkin lymphoma, insulin-independent diabetes (type II), non-small cell lung cancer, organ transplant operation, bone arthritis, bone softening, bone decrease, osteoporosis, ovary cancer, bone Behcet's disease, digestive ulcer, peripheral blood vessel disease, prostate cancer, reflux esophagitis, renal insufficiency, rheumatoid arthritis, schizophrenia, septicemia, septicemic shock, severe systemic fungal infection, small cell lung cancer, spinal damage, stomach cancer, systemic lupus erythematosus, transient cerebral ischemia attack, tuberculosis, valvular heart disease, vascular/multiple infarct dementia, wound healing, insomnia, arthritis, insufficiency in secretion of pituitary hormone, frequent urination, uremia, or nerve degeneration disease.

Further, the antibody to the peptide F, G, H or I of the present application can be used as a pharmaceutical preparation such as a therapeutic/prophylactic agent for a disease macular edema cystoid.

Further, the antibody to the peptide F, G, H or I of the present application is useful for example as (1) a therapeutic agent for tumors such as acromegaly, TSH-producing tumor, non-secretory (non-functional) pituitary tumor, ectopic ACTH (adrenocorticotropine)-producing tumor, marrow-like thyroid cancer, VIP-producing tumor, glucagon-producing tumor, gastrin-producing tumor, insulinoma, carcinoid etc., (2) a therapeutic agent for insulin dependent or independent diabetes, or various diseases related to such diabetes, that is, diabetic complications (for example, diabetic retinitis, diabetic nephritis, diabetic neuropathy, down syndrome, erectile low blood pressure, etc.), (3) a therapeutic agent for obesity, bulimia etc. by ameliorating hyperinsulinemia or suppressing appetite, (4) a therapeutic agent for acute pancreatitis, chronic pancreatitis, pancreas/intestine physter, bleeding ulcer, digestive ulcer, gastritis, gastric hyperacidity, reflux esophagitis, etc., (5) an agent for ameliorating various symptoms caused by infection with helicobacter pylori bacteria (for example, an agent for inhibiting promotion of gastrin secretion, etc.), (6) an agent for suppressing amylase secretion caused by endoscopic cholangio-pancreatography, and for prognosis of surgical operation of pancreas, (7) a therapeutic agent for diarrhea attributable to a reduction of the absorbing ability of small intestine, acceleration of secretion, or abnormality in mobility of digestive tract (for example, short bowel syndrome, etc.), diarrhea attributable to chemicals in chemotherapy of cancer, diarrhea attributable to congenital shrinkage of small intestine, diarrhea attributable to neuroendocrine tumor such as VIP-producing tumor, diarrhea attributable to AIDS, diarrhea attributable to graft versus host reaction accompanying a marrow transplant, diarrhea attributable to diabetes, diarrhea attributable to blockage of abdominal nerve plexuses, diarrhea attributable to systematic sclerosis, diarrhea attributable to eosinophilia, etc., (8) a therapeutic agent for damping syndrome, hypersensitive colitis, Crohn's disease, inflammatory intestinal diseases, etc., (9) a therapeutic agent for tumors or cancers (for example, thyroid cancer, large intestine cancer, breast cancer, prostate cancer, small cell lung cancer, non-small cell lung cancer, pancreas cancer, stomach cancer, cholangioma, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (for example, basophil leukemia/chronic lymphatic leukemia, chronic myeloid leukemia, Hodgkin's disease, non-Hodgkin lymphoma, etc.); the therapeutic agent can be used alone or in combination with other anticancer drugs (for example, tamoxifen, LHRH agonist, LHRH antagonist, interferon-$\alpha$,$\beta$ and $\gamma$, interleukin-2, etc.), (10) a prophylactic/therapeutic agent for hypertrophic myocardial disease, arteriosclerosis, valvular heart disease, myocardial infarction (particularly, myocardial infarction after percutaneous transluminal coronary angioplasty), revascularization, (11) a therapeutic agent for esophagus vein cancer bleeding, hepatocirrhosis, peripheral blood vessel disease, (12) a therapeutic agent for diseases accompanying systemic or topical inflammations (for example, multiple arteritis, rheumatoid arthritis, psoriasis, sunburn, eczema, allergies (for example, asthma, atopic dermatitis, allergic rhinitis, etc.)), based on the secretion-regulating action of phsyoloigcally active substance acting on the immune system (for example, substance P, tachykinin, cytokine, etc.), (13) a therapeutic agent for dementia (for example, Alzheimer's disease, Alzheimer type senile dementia, vascular/multiple dementia, etc.), schizophrenia, epilepsy, depression, general anxiety, difficult sleep, multiple sclerosis, etc., because of its influence on production and secretion of nerve-regulating factor, (14) a therapeutic agent for eye diseases (for example, glaucoma, etc.), (15) a prophylactic/therapeutic agent for acute bacterial meningitis, acute viral encephalitis, adult respiratory distress syndrome, bacterial pneumonia, severe systemic fungal infection, tuberculosis, spinal damage, bone fracture, renal insufficiency, pneumonia, alcoholic hepatitis, type A hepatitis, type B hepatitis, type C hepatitis, AIDS infection, human papilloma virus infection, influenza infection, cancer metastasis, multiple myeloma, bone softening, osteoporosis, bone Behcet's disease, nephritis, renal insufficiency, septicemia, septicemic shock, hypercalcemia, hypercholesterolemia, hyperglyceridemia, hyperlipemia, systemic lupus erythematosus, transient cerebral ischemia attack, alcoholic hepatitis, etc. (16) a therapeutic agent for organ transplant operation, burn, wound, alopecia, etc., and (17) an analgesic for suppressing and relieving chronic or acute pains (for example, acute pain after operation, inflammatory acute pain, toothache, bone diseases (for example, acute pains accompanying arthritis, rheumatism, osteoporosis, etc.)).

The therapeutic/prophylactic agent comprising the antibody of the present application described above may be administered orally or parenterally to human or mammal (e.g., rat, rabbit, sheep, swine, bovine, cat, dog, monkey, etc.).as a liquid preparation in its original form, or as a pharmaceutical composition in an appropriate drug form. The dose varies depending on subject to be administered, target disease, symptom, route for administration, etc.; for example, it is convenient to intravenously administer the antibody of the present application to adult normally in a single dose of approximately 0.01 to 20 mg/kg body weight, preferably approximately 0.1 to 10 mg/kg body weight, and more preferably approximately 0.1 to 5 mg/kg per day approximately 1 to 5 times a day, preferably approximately 1 to 3 times. In parenteral administration in other route and in oral administration, a dose similar to those given above can be administered. Where conditions are serious, the dose may be increased depending on the conditions.

The antibody of the present application may be administered in itself or as an appropriate pharmaceutical composition. The pharmaceutical composition used for the administration described above contains a pharmacologically acceptable carrier with the aforesaid compounds or salts thereof, a diluent or excipient. Such a composition is provided in the preparation suitable for oral or parenteral administration.

That is, examples of the composition for oral administration include solid or liquid preparations, specifically tablets (including dragees and film-coated tablets), pills, granules, powdery preparations, capsules (including soft capsules), syrup, emulsions, suspensions, etc. Such a composition is manufactured by publicly known methods and comprises a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration that can be used are injections, suppositories, etc. and the injections include the form of intravenous, subcutaneous, transcutaneous, intramuscular and drip injections. Such injections are prepared by publicly known methods, e.g., by dissolving, suspending or emulsifying the aforesaid antibody or its salts in a sterile aqueous or oily liquid medium. For the aqueous medium for injection, for example, physiological saline and isotonic solutions containing glucose and other adjuvant, etc. are used. Appropriate dissolution aids, for example, an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. may be used in combination. For the oily solution, for example, sesame oil, soybean oil, etc. are used, and dissolution aids such as benzyl benzoate, benzyl alcohol, etc. may be used in combination. The thus prepared liquid for injection is normally filled in an appropriate ampoule. The suppository used for rectal administration is prepared by mixing the aforesaid antibody or its salts with conventional suppository base.

The oral or parenteral pharmaceutical composition described above is advantageously prepared in a unit dosage form suitable for the dose of the active ingredient. Examples of such unit dosage form include tablets, pills, capsules, injections (ampoules), suppositories, etc. It is preferred that the antibody described above is comprised generally in a dose of approximately 5 to 500 mg per unit dosage form, approximately 5 to 100 mg especially for injections and approximately 10 to 250 mg for other preparations.

Each composition described above may further comprise other active components unless formulation with the antibody causes any adverse interaction.

**(7) DNA transgenic animal**

The present application disclosed a non-human mammal having the DNA encoding the peptide of the present application, which is exogenous (hereinafter simply referred to as the exogenous DNA of the present application) or its mutant DNA (sometimes simply referred to as the exogenous mutant DNA of the present application

Thus, the present application discloseds:

(i) a non-human mammal having the exogenous DNA of the present application or its mutant DNA;
(ii) the mammal according to (i), wherein the non-human mammal is a rodent;
(iii) the mammal according to (ii), wherein the rodent is mouse or rat; and
(iv) a recombinant vector comprising the exogenous DNA of the present application or its mutant DNA and capable of expression in a mammal.

The non-human mammal having the exogenous DNA of the present application or its mutant DNA (hereinafter simply referred to as the DNA transgenic animal of the present application can be created by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase) by standard means such as the calcium phosphate method, the electric pulse method, the lipofection method, the agglutination method, the microinjection method, the particle gun method, the DEAE-dextran method, etc. Also, it is possible to transfect the exogenous DNA of the present application into a somatic cell, a living organ, a tissue cell or the like, by the DNA transfection methods, and utilize the transformant for cell culture, tissue culture, etc. In addition, these cells may be fused with the above-described germinal cell by a publicly known cell fusion method to create the transgenic animal of the present application.

Examples of the non-human mammal that can be used include bovine, swine, sheep, goat, rabbits, dogs, cats, guinea pigs, hamsters, mice, rats and the like. Above all, preferred are rodents, especially mice (e.g., C57BL/6 strain, DBA2 strain, etc. for a pure line and for a cross line, $B6C3F_1$ strain, $BDF_1$ strain, $B6D2F_1$ strain, BALB/c strain, ICR strain, etc.) or rats (Wistar, SD, etc.) and the like, since they are relatively short in ontogeny and life cycle from a standpoint of creating model disease animals, and are easy in breeding.

"Mammals" in a recombinant vector that can be expressed in mammals include, in addition to the aforesaid non-human mammals, human, etc.

The exogenous DNA of the present application refers to the DNA of the present application that is once isolated and extracted from mammals, not the DNA of the present application inherently possessed by the non-human mammals.

The mutant DNA of the present application includes mutants resulting from variation (e.g., mutation, etc.) in the nucleotide sequence of the original DNA of the present application, specifically DNAs resulting from base addition, deletion, substitution with other bases, etc. and further including abnormal DNA.

The abnormal DNA is intended to mean the DNA that expresses the abnormal peptide of the present application and exemplified by such a DNA that expresses a polypeptide capable of suppressing the functions of the normal peptide of the present application or the like.

The exogenous DNA of the present application may be any one of those derived from a mammal of the same species as, or a different species from, the mammal as the target animal. In transfecting the DNA of the present application to the target animal, it is generally advantageous to use the DNA as a DNA construct in which the DNA is ligated downstream a promoter capable of expressing the DNA in the target animal. For example, in the case of transfecting the human DNA of the present application, a DNA transgenic mammal that has the DNA of the present application to a high level can be prepared by microinjecting a DNA construct (e.g., vector, etc.) ligated with the human DNA of the present application into a fertilized egg of the target mammal, e.g., a fertilized egg of mouse, downstream the various promoters capable of expressing the DNA derived from various mammals (e.g., rabbits, dogs, cats, guinea pigs, hamsters, rats, mice, etc.) bearing the DNA of the present application highly homologous to the human DNA.

As expression vectors for the peptide of the present application, there are Escherichia coli-derived plasmids, Bacillus subtilis-derived plasmids, yeast-derived plasmids, bacteriophages such as λ phage, etc., retroviruses such as Moloney leukemia virus, etc., animal viruses such as vaccinia virus, baculovirus, etc. Of these vectors, Escherichia coli-derived plasmids, Bacillus subtilis-derived plasmids, yeast-derived plasmids, etc. are preferably used.

Examples of these promoters for regulating the DNA expression include [1] promoters for DNA derived from viruses (e.g., simian virus, cytomegalovirus, Moloney leukemia virus, JC virus, breast cancer virus, poliovirus, etc.), and [2] promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na, K-ATPase), neurofilament light chain, metallothioneins I and IIA, metalloproteinase 1 tissue inhibitor, MHC class I antigen (H-2L), H-ras, renin, dopamine β-hydroxylase, thyroid peroxidase (TPO), peptide chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human peptide elongation factor 1α (EP-1α) promoters, human and chicken β actin promoters etc., which can achieve high expression in the whole body, are preferred.

It is preferred that the vectors described above have a sequence for terminating the transcription of the desired messenger RNA in the DNA transgenic animal (generally called a terminator); for example, a sequence of each DNA derived from viruses and various mammals. SV40 terminator of the simian virus, etc. are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The normal translational region of the peptide of the present application can be prepared as the whole or a part of genomic DNA from DNA derived from liver, kidney, thyroid cells, fibroblasts etc. derived from humans or mammals (for example, rabbit, dog, cat, guinea pig, hamster, rat, mouse etc.) and a wide variety of commercial DNA libraries, or from complementary DNA as a starting material prepared by a known method from RNA derived from liver, kidney, thyroid cells, fibroblasts etc. As the extraneous abnormal DNA, a translational region can be prepared by point mutation of the normal translational region of the peptide of the present application obtained from the above cells or tissues.

The translational region can be prepared, as a DNA construct capable of being expressed in the transgenic animal, by a conventional DNA engineering technique, in which the DNA is ligated downstream the aforesaid promoter and if desired, upstream the translation termination site.

The exogenous DNA of the present application is transfected at the fertilized egg cell stage in a manner such that the DNA is certainly present in all the terminal cells and somatic cells of the target mammal. The fact that the exogenous DNA of the present application is present in the germinal cells of the animal prepared by DNA transfection means that all offspring of the prepared animal will maintain the exogenous DNA of the present application in all of the germinal cells and somatic cells thereof. The offspring of the animal that inherits the exogenous DNA of the present application also have the exogenous DNA of the present application in all of the germinal cells and somatic cells thereof.
The non-human mammal, in which the normal exogenous DNA of the present application has been transfected can be passaged as the DNA-bearing animal under ordinary rearing environment, by confirming that the exogenous DNA is stably retained by mating.
By the transfection of the exogenous DNA of the present application at the fertilized egg cell stage, the DNA is retained to be excess in all of the germinal and somatic cells of the target mammal. The fact that the exogenous DNA of the present application is excessively present in the germinal cells of the prepared animal after transfection means that all of the offspring of the animal prepared have the exogenous DNA of the present application excessively in all of the germinal cells and somatic cells thereof. The offspring of the animal of this kind that inherits the exogenous DNA of the present application excessively have the DNA of the present application in all of the germinal cells and somatic cells thereof.
By obtaining a homozygotic animal having the transfected DNA in both of homologous chromosomes and mating a male and female of the animal, all offspring can be passaged to excessively retain the DNA.
In a non-human mammal having the normal DNA of the present application, the normal DNA of the present application is expressed to a high level, and may eventually develop the hyperfunction of the peptide of the present application by promoting the functions of endogenous normal DNA. Therefore, the animal can be utilized as a pathologic model animal for such a disease. Specifically, using the normal DNA transgenic animal of the present application, it becomes possible to elucidate the hyperfunction of the peptide of the present application and to clarify the pathological mechanism of the disease associated with the peptide of the present and to determine how to treat these diseases.
Furthermore, since a mammal transfected the exogenous normal DNA of the present application has an increasing symptom of the librated peptide of the present application, the animal is usable for screening of therapeutic agents agent for the disease associated with the peptide of the present application.
On the other hand, non-human mammal having the exogenous abnormal DNA of the present application can be passaged under normal breeding conditions as the DNA-bearing animal by confirming the stable retaining of the exogenous DNA via crossing. In addition, the objective exogenous DNA can be utilized as a starting material by inserting the objective exogenous DNA into the plasmid described above. The DNA construct with a promoter can be prepared using conventional DNA engineering techniques. The transfection of the abnormal DNA of the present application at the fertilized egg cell stage is preserved to be present in all of the germinal and somatic cells of the mammals to be targeted. The fact that the abnormal DNA of the present application is present in the germinal cells of the animal after DNA transfection means that all of the offspring of the prepared animal have the abnormal DNA of the present application in all of the germinal and somatic cells. The offspring of such an animal that inherits the exogenous DNA of the present application has the abnormal DNA of the present application in all the germinal and somatic cells. A homozygous animal having the introduced DNA on both of homologous chromosomes can be acquired and then by mating these male and female animals, all the offspring can be bred to have the DNA.
Since the non-human mammal having the abnormal DNA of the present application expresses the abnormal DNA of the present application at a high level, the animal may cause the function inactive type inadaptability of the peptide of the present application by inhibiting the functions of the endogenous normal DNA, and can be utilized as its disease model animal. For example, using the abnormal DNA-transferred animal of the present application it is possible to elucidate the mechanism of the function inactive type inadaptability of the peptide of the present application and to study a method for treatment of this disease.
In its specific applicability, the transgenic animal of the present application expressing the abnormal DNA of the present application to a high level is also expected to serve as a model for the elucidation of the mechanism of the functional inhibition (dominant negative effect) of a normal peptide by the abnormal peptide of the present application in the function inactive type inadaptability of the peptide of the present application.
A mammal bearing the abnormal exogenous DNA of the present application is also expected to serve for screening a candidate drug for the treatment of the function inactive type inadaptability of the peptide of the present application, since the peptide of the present application is increased in such an animal in its free form.
Other potential applicability of the two kinds of the transgenic animals described above includes:

[1] use as a cell source for tissue culture;
[2] elucidation of the association with a peptide that is specifically expressed or activated by the peptide of the present application, through direct analysis of DNA or RNA in tissue of the DNA transgenic animal of the present application or by analysis of the peptide tissue expressed by the DNA;

[3] research in the function of cells derived from tissues that are cultured usually only with difficulty, using cells of tissue having the DNA cultured by a standard tissue culture technique;
[4] screening for a drug that enhances the functions of cells using the cells described in [3] above; and,
[5] isolation and purification of the variant peptide of the present application and preparation of an antibody thereto.

Furthermore, clinical conditions of a disease associated with the peptide of the present application, including the function inactive type inadaptability of the peptide of the present application can be determined using the DNA transgenic animal of the present application. Also, pathological findings on each organ in a disease model associated with the peptide of the present application can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present application, mincing the organ and degrading with a proteinase such as trypsin, etc., followed by establishing the line of culturing or cultured cells. Furthermore, the DNA transgenic animal of the present application can serve as identification of cells capable of producing the peptide of the present application, and as studies on association with apoptosis, differentiation or propagation or on the mechanism of signal transduction in these properties to inspect any abnormality therein. Thus, the DNA transgenic animal of the present application provide an effective research material for the peptide of the present application and for elucidating the function and effect thereof.

To develop pharmaceuticals for the treatment of diseases associated with the peptide of the present application, including the function inactive type inadaptability of the peptide of the present application, using the DNA transgenic animal of the present application, an effective and rapid method for screening the pharmaceuticals for the treatment of diseases can be provided by using the method for inspection and the method for quantification, etc. described above. It is also possible to investigate and develop a method for DNA therapy for the treatment of diseases associated with the peptide of the present application, using the DNA transgenic animal of the present application or a vector capable of expressing the exogenous DNA of the present application (8) Knockout animal

The present application discloses a non-human mammal embryonic stem cell bearing the DNA of the present application inactivated and a non-human mammal deficient in expressing the DNA of the present application.

Thus, the present application discloses:

(i) a non-human mammal embryonic stem cell in which the DNA of the present application inactivated;
(ii) the embryonic stem cell according to (i), wherein the DNA is inactivated by introducing a reporter gene (e.g., β-galactosidase gene derived from Escherichia coli);
(iii) the embryonic stem cell according to (i), which is resistant to neomycin;
(iv) the embryonic stem cell according to (i), wherein the non-human mammal is a rodent;
(v) an embryonic stem cell according to (iv), wherein the rodent is mouse;
(vi) a non-human mammal deficient in expressing the DNA of the present application, wherein the DNA of the present application inactivated;
(vii) the non-human mammal according to (vi), wherein the DNA is inactivated by inserting a reporter gene (e.g., β-galactosidase derived from Escherichia coli) therein and the reporter gene is capable of being expressed under the control of a promoter for the DNA of the present application;
(viii) the non-human mammal according to (vi), which is a rodent;
(ix) the non-human mammal according to (viii), wherein the rodent is mouse; and
(x) a method for screening a compound or its salt that promotes or inhibits the promoter activity for the DNA of the present application, which comprises administering a test compound to the animal of (vii) and detecting expression of the reporter gene.

The non-human mammalian embryonic stem cell, in which the DNA of the present application is inactivated, refers to a non-human mammalian embryonic stem cell that suppresses the ability of the non-human mammalian to express the DNA by artificially mutating the DNA of the present application possessed in the non-human mammal, or the DNA has no substantial ability to express the peptide of the present application (hereinafter sometimes referred to as the knockout DNA of the present application) by substantially inactivating the activities of the peptide of the present application encoded by the DNA (hereinafter merely referred to as ES cell).

As the non-human mammalian, the same examples as described above apply.

Techniques for artificially mutating the DNA of the present application include deletion of a part or all of the DNA sequence and insertion of, or substitution with, other DNA, e.g., by genetic engineering. By these variations, the knockout DNA of the present application may be prepared, for example, by shifting the reading frame of a codon or by disrupting the function of a promoter or exon.

Specifically, the non-human mammalian embryonic stem cell, in which the DNA of the present application is inactivated (hereinafter merely referred to as the ES cell with the DNA of the present application inactivated or the knockout ES cell of the present application), can be obtained by, for example, isolating the DNA of the present application possessed by the target non-human mammal, inserting a DNA strand (hereinafter simply referred to as targeting vector) having a DNA sequence constructed so as to eventually destroy the gene by inserting into its exon site a chemical resistant gene such as a neomycin resistant gene or a hygromycin resistant gene, or a reporter gene such as lacZ (β-galactosidase gene) or cat (chloramphenicol acetyltransferase gene), etc. thereby destroying the functions of exon, or by inserting into the intron site between exons a DNA sequence which terminates gene transcription (e.g., polyA-added signal, etc.) thereby disabling the synthesis of complete messenger RNA, into a chromosome of the animal cells by, e.g., homologous recombination. The thus obtained ES cells are analyzed by the Southern hybridization using as a probe a DNA sequence on or near the DNA of the present application or by PCR using as primers a DNA sequence on the targeting vector and another DNA sequence near the DNA of the present application which is not included in the targeting vector, and the knockout ES cell of the present application is selected.

The parent ES cells to inactivate the DNA of the present application by homologous recombination, etc. may be of a strain already established as described above, or may be originally established in accordance with a modification of the known method by Evans and Kaufman supra. For example, in the case of mouse ES cells, currently it is common practice to use ES cells of the 129 strain. However, since their immunological background is obscure, the C57BL/6 mouse or the $BDF_1$ mouse ($F_1$ hybrid between C57BL/6 and DBA/2), wherein the low ovum collection per C57BL/6 mouse or C57BL/6 has been improved by crossing with DBA/2, may be preferably used, instead of obtaining a pure line of ES cells with the clear immunological genetic background. The $BDF_1$ mouse is advantageous in that when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes of 3.5 days after fertilization are commonly used. A large number of early stage embryos may be acquired more efficiently, by collecting the embryos of the 8-cell stage and using the same after culturing until the blastocyte stage.

Although the ES cells used may be of either sex, male ES cells are generally more convenient for generation of a germ cell line chimera and are therefore preferred. It is desirable to identify sexes as soon as possible also in order to save painstaking culture time.

As an example of the method for sex identification of the ES cell, mention may be made of a method in which a gene in the sex-determining region on the Y-chromosome is amplified by PCR and detected. When this method is used, ES cells (about 50 cells) corresponding to almost 1 colony are sufficient, whereas karyotype analysis hitherto required about $10^6$ cells; therefore, the first selection of ES cells at the early stage of culture can be based on sex identification, and male cells can be selected early, which saves a significant amount of time at the early stage of culture.

Second selection can be achieved by, for example, number of chromosome confirmation by the G-banding method. It is usually desirable that the chromosome number of the obtained ES cells be 100% of the normal number. However, when it is difficult to obtain the cells having the normal number of chromosomes due to physical operation etc. in cell establishment, it is desirable that the ES cell be again cloned to a normal cell (e.g., in mouse cells having the number of chromosomes being 2n = 40) after the gene of the ES cells is rendered knockout.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and about 90% air) in the presence of LIF (1 to 10000 U/ml) on appropriate feeder cells such as STO fibroblasts, treated with a trypsin/EDTA solution (normally about 0.001 to about 0.5% trypsin/about 0.1 to 5 mM EDTA, preferably about 0.1% trypsin/about 1 mM EDTA) at the time of passage to obtain separate single cells, which are then seeded on freshly prepared feeder cells. This passage is normally conducted every 1 to 3 days; it is desirable that cells be observed at passage and cells found to be morphologically abnormal in culture, if any, be abandoned.

By allowing ES cells to reach a high density in mono-layers or to form cell aggregates in suspension under appropriate conditions, it is possible to differentiate them to various cell types, for example, parietal and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present application, which are obtainable from the differentiated ES cells of the present application, are useful for studying the functions of the peptide of the present application or the receptor protein of the present application in vitro cytologically or molecular biologically.

The non-human mammal deficient in expression of the DNA of the present application can be identified from a normal animal by measuring the amount of mRNA in the subject animal by a publicly known method, and indirectly comparing the levels of expression.

As the non-human mammal, the same examples supra apply.

With respect to the non-human mammal deficient in expression of the DNA of the present application, the DNA of the present application can be made knockout by transfecting a targeting vector, prepared as described above, to mouse embryonic stem cells or mouse oocytes thereof, and conducting homologous recombination in which a targeting vector DNA sequence, wherein the DNA of the present application is inactivated by the transfection, is replaced with the DNA of the present application on a chromosome of a mouse embryonic stem cell or mouse oocyte.

The cells with the DNA of the present application in which the DNA of the present application is rendered knockout can be identified by the Southern hybridization analysis using as a probe a DNA sequence on or near the DNA of the present application, or by PCR analysis using as primers a DNA sequence on the targeting vector and another DNA sequence which is not included in the DNA of the present application derived from mouse, which is used as the targeting vector. When non-human mammalian embryonic stem cells are used, the cell line wherein the DNA of the present application is inactivated is cloned by homologous recombination; the resulting cloned cell line is injected to, e.g., a non-human mammalian embryo or blastocyte, at an appropriate stage such as the 8-cell stage. The resulting chimeric embryos are transplanted to the uterus of the pseudo-pregnant non-human mammal. The resulting animal is a chimeric animal composed of both cells having the normal locus of the DNA of the present application and those having an artificially mutated locus of the DNA of the present application.

When some germ cells of the chimeric animal have a mutated locus of the DNA of the present application, an individual, in which all tissues are composed of cells having an artificially mutated locus of the DNA of the present application, can be selected from a series of offspring obtained by crossing between such a chimeric animal and a normal animal, e.g., by coat color identification, etc. The individuals thus obtained are normally deficient in heterozygous expression of the peptide of the present application. The individuals deficient in homozygous expression of the peptide of the present application can be obtained from offspring of the intercross between the heterozygotes.

When an oocyte is used, a DNA solution may be injected, e.g., to the prenucleus by microinjection thereby obtaining a transgenic non-human mammal having a targeting vector introduced into its chromosome. From such transgenic non-human mammals, those having a mutation at the locus of the DNA of the present application can be obtained by selection based on homologous recombination.

As described above, individuals wherein the DNA of the present application is rendered knockout permit passage rearing under ordinary rearing conditions, after it is confirmed that in the animal individuals obtained by their crossing, the DNA has been knockout.

Furthermore, the genital system may be obtained and maintained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammalian embryonic stem cell, in which the DNA of the present application is inactivated, is very useful for preparing a non-human mammal deficient in expression of the DNA of the present application.

Since the non-human mammal, in which the DNA of the present application fails to express, lacks various biological activities induced by the peptide of the present application, such an animal can be a disease model suspected of inactivated biological activities of the peptide of the present application and thus, offers an effective study to investigate causes for and therapy for these diseases.

### (8a) Method for screening of compounds having therapeutic/prophylactic effects for diseases caused by deficiency, damages, etc. of the DNA of the present application

The non-human mammal deficient in expression of the DNA of the present application can be used to screen compounds having therapeutic/prophylactic effects for diseases caused by deficiency, damages, etc. of the DNA of the present application.

That is, the present application discloses a method for screening of a compound or its salt having therapeutic/prophylactic effects for diseases caused by deficiency, damages, etc. of the DNA of the present application, which comprises administering a test compound to the non-human mammal deficient in expression of the DNA of the present application, and observing and measuring a change having occurred in the animal.

As the non-human mammal deficient in expression of the DNA of the present application used for the screening method, the same examples as given hereinabove apply.

Examples of the test compounds include peptides, proteins, non-peptide compounds, synthetic compounds, fermentation products, cell extracts, plant extracts, animal tissue extracts, blood plasma, etc. and these compounds may be novel compounds or publicly known compounds.

Specifically, the non-human mammal deficient in the expression of the DNA of the present application is treated with a test compound, comparison is made with an intact animal for control and a change in each organ, tissue, disease conditions, etc. of the animal is used as an indicator to assess the therapeutic/prophylactic effects of the test compound.

For treating an animal to be tested with a test compound, for example, oral administration, intravenous injection, etc. are applied and the treatment is appropriately selected depending upon conditions of the test animal, properties of the test compound, etc. Furthermore, the amount of a test compound administered can be appropriately selected depending on administration route, nature of the test compound, or the like.

In the screening method, a test compound can be selected as a compound having therapeutic/prophylactic effects on the disease when functions of a test animal upon administering the test compound are reduced by preferably at least about 10%, preferably at least about 30%, more preferably at least about 50%.

Specifically, when the peptide is peptide F, G, H or I of the present application, the compound can be used for example as an eating regulator, a sleep regulator, an arousal regulator, a pain regulator, a stress response regulator, a spontaneous behavior regulator or an emotional behavior regulator or as a prophylactic/therapeutic agent for diseases in the central nerves (for example, Alzheimer's disease/dementia/difficulty in eating (anorexia)/epilepsy, etc.), diseases in the hormone system (for example, faint labor, flaccid bleeding, before and after placenta expulsion, insufficiency in uterine restoration, Caesarean operation, artificial abortion, milk statis etc.), diseases in liver/gall/pancreas/internal secretion (for example, diabetes/difficulty in eating, etc.), inflammatory diseases (allergy/asthma/rheumatism etc.), circulatory organ diseases (for example, high blood pressure/cardiac hypertrophy/angina/arteriosclerosis, etc.).

Further, the compound is useful as an agent for regulating prolactin secretion. That is, when the compound has a promoting action on secretion of prolactin, it is useful as a prolactin secretion promoter for various diseases with which deficiency in secretion of prolactin is associated, for example a prophylactic/therapeutic agent for diseases related to secretion of prolactin, such as depression of ovarian functions, deficiency in growth of seminal vesicle, osteoporosis, menopausal disorders, deficiency in secretion of milk, hypothyroidism, renal insufficiency, etc. Further, when the compound has a promoting action on secretion of prolactin, it has a promoting action on sexual desire (pheromone-like action) based on the promoting action on secretion of prolactin, and is thus useful as an agent for promoting sexual desire.

When the compound has an inhibitory action on secretion of prolactin, it is useful as a prolactin secretion inhibitor for diseases with which excessive secretion of prolactin is associated, for example a prophylactic/therapeutic agent for diseases related to prolactin secretion, such as hyperprolactinemia, pituitary gland tumor, interbrain tumor, abnormal menstruation, stress, autoimmune disease, prolactinoma, sterility, impotence, amenorrhea, galactorrhea, acromegaly, Chiari-Frommel syndrome, Argonz-del Castilo syndrome, Forbes-Albright syndrome, breast cancer lymphoma, Sheehan's syndrome or abnormality in spermatogenesis. Further, when the compound has an inhibitory action on secretion of prolactin, it is useful as a contraceptive agent based on the inhibitory action on secretion of prolactin.

In addition, the compound is also useful as a diagnostic drug for examining an ability to secrete prolactin or as an animal drug such as a milk secretion promoting agent for domestic mammals such as bovine, goat, swine etc., and can be applied to production of a useful substance by producing the useful substance in the mammals and secreting it in milk.

Further, the compound is useful as a prophylactic/therapeutic agent for choriocarcinoma, hydatidiform mole, invasive mole, abortion, insufficiency in fetal growth, abnormality in sugar metabolism, abnormality in lipid metabolism or delivery induction.

Further, the compound is useful as a prophylactic/therapeutic agent for diseases such as high blood pressure, autoimmune disease, cardiac insufficiency, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, bone fracture, breast cancer, bulimia, [bulimia], burn healing, uterine cervix cancer, chronic lymphatic leukemia, chronic myeloid leukemia, chronic pancreatitis, hepatocirrhosis, large-intestine cancer (colon/rectum cancer), Crohn's disease, dementia, diabetic complications, diabetic nephritis, diabetic neuropathy, diabetic retinitis, gastritis, helicobacter pylori infection, renal insufficiency, type A hepatitis, type B hepatitis, type C hepatitis, hepatitis, herpes simplex virus infection, varicella zoster virus infection, Hodgkin's disease, AIDS infection, human papilloma virus infection, hypercalcemia, hypercholesterolemia, hyperglyceridemia, hyperlipemia, infections, influenza infection, insulin-dependent diabetes (type I), invasive Staphylococcus infection, malignant melanoma, cancer metastasis, multiple myeloma, allergic rhinitis, nephritis, non-Hodgkin lymphoma, insulin-independent diabetes (type II), non-small cell lung cancer, organ transplant operation, bone arthritis, bone softening, bone decrease, osteoporosis, ovary cancer, bone Behcet's disease, digestive ulcer, peripheral blood vessel disease, prostate cancer, reflux esophagitis, renal insufficiency, rheumatoid arthritis, schizophrenia, septicemia, septicemic shock, severe systemic fungal infection, small cell lung cancer, spinal damage, stomach cancer, systemic lupus erythematosus, transient cerebral ischemia attack, tuberculosis, valvular heart disease, vascular/multiple infarct dementia, wound healing, insomnia, arthritis, insufficiency in secretion of pituitary hormone, frequent urination, uremia, or nerve degeneration disease.

Further, the compound can be used as a pharmaceutical preparation such as a therapeutic/prophylactic agent for a disease macular edema cystoid.

Further, the compound is useful for example as (1) a therapeutic agent for tumors such as acromegaly, TSH-producing tumor, non-secretory (non-functional) pituitary tumor, ectopic ACTH (adrenocorticotropine)-producing tumor, marrow-like thyroid cancer, VIP-producing tumor, glucagon-producing tumor, gastrin-producing tumor, insulinoma, carcinoid etc., (2) a therapeutic agent for insulin dependent or independent diabetes, or various diseases related to such diabetes, that is, diabetic complications (for example, diabetic retinitis, diabetic nephritis, diabetic neuropathy, down syndrome, erectile low blood pressure, etc.), (3) a therapeutic agent for obesity, bulimia etc. by ameliorating hyperinsulinemia or suppressing appetite, (4) a therapeutic agent for acute pancreatitis, chronic pancreatitis, pancreas/intestine physter, bleeding ulcer, digestive ulcer, gastritis, gastric hyperacidity, reflux esophagitis, etc., (5) an agent for ameliorating various symptoms caused by infection with helicobacter pylori bacteria (for example, an agent for inhibiting promotion of gastrin secretion, etc.), (6) an agent for suppressing amylase secretion caused by endoscopic cholangio-pancreatography, and for prognosis of surgical operation of pancreas, (7) a therapeutic agent for diarrhea attributable to a reduction of the absorbing ability of small intestine, acceleration of secretion, or abnormality in mobility of digestive tract (for example, short bowel syndrome, etc.), diarrhea attributable to chemicals in chemotherapy of cancer, diarrhea attributable to congenital shrinkage of small intestine, diarrhea attributable to neuroendocrine tumor such as VIP-producing tumor, diarrhea attributable to AIDS, diarrhea attributable to graft versus host reaction accompanying a marrow transplant, diarrhea attributable to diabetes, diarrhea attributable to blockage of abdominal nerve plexuses, diarrhea attributable to systematic sclerosis, diarrhea attributable to eosinophilia, etc., (8) a therapeutic agent for damping syndrome, hypersensitive colitis, Crohn's disease, inflammatory intestinal diseases, etc., (9) a therapeutic agent for tumors or cancers (for example, thyroid cancer, large intestine cancer, breast cancer, prostate cancer, small cell lung cancer, non-small cell lung cancer, pancreas cancer, stomach cancer, cholangioma, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (for example, basophil leukemia/chronic lymphatic leukemia, chronic myeloid leukemia, Hodgkin's disease, non-Hodgkin lymphoma, etc.); the therapeutic agent can be used alone or in combination with other anticancer drugs (for example, tamoxifen, LHRH agonist, LHRH antagonist, interferon-$\alpha$,$\beta$ and $\gamma$, interleukin-2, etc.), (10) a prophylactic/therapeutic agent for hypertrophic myocardial disease, arteriosclerosis, valvular heart disease, myocardial infarction (particularly, myocardial infarction after percutaneous transluminal coronary angioplasty), revascularization, (11) a therapeutic agent for esophagus vein cancer bleeding, hepatocirrhosis, peripheral blood vessel disease, (12) a therapeutic agent for diseases accompanying systemic or topical inflammations (for example, multiple arteritis, rheumatoid arthritis, psoriasis, sunburn, eczema, allergies (for example, asthma, atopic dermatitis, allergic rhinitis, etc.)), based on the secretion-regulating action of phsyoloigcally active substance acting on the immune system (for example, substance P, tachykinin, cytokine, etc.), (13) a therapeutic agent for dementia (for example, Alzheimer's disease, Alzheimer type senile dementia, vascular/multiple dementia, etc.), schizophrenia, epilepsy, depression, general anxiety, difficult sleep, multiple sclerosis, etc., because of its influence on production and secretion of nerve-regulating factor, (14) a therapeutic agent for eye diseases (for example, glaucoma, etc.), (15) a prophylactic/therapeutic agent for acute bacterial meningitis, acute viral encephalitis, adult respiratory distress syndrome, bacterial pneumonia, severe systemic fungal infection, tuberculosis, spinal damage, bone fracture, renal insufficiency, pneumonia, alcoholic hepatitis, type A hepatitis, type B hepatitis, type C hepatitis, AIDS infection, human papilloma virus infection, influenza infection, cancer metastasis, multiple myeloma, bone softening, osteoporosis, bone Behcet's disease, nephritis, renal insufficiency, septicemia, septicemic shock, hypercalcemia, hypercholesterolemia, hyperglyceridemia, hyperlipemia, systemic lupus erythematosus, transient cerebral ischemia attack, alcoholic hepatitis, etc. (16) a therapeutic agent for organ transplant operation, burn, wound, alopecia, etc., and (17) an analgesic for suppressing and relieving chronic or acute pains (for example, acute pain after operation, inflammatory acute pain, toothache, bone diseases (for example, acute pains accompanying arthritis, rheumatism, osteoporosis, etc.)).

The compound obtained using the above screening methods is a compound selected from the test compounds described above and has a therapeutic/prophylactic effect for diseases caused by deficiency, damages, etc. of the peptide of the present application, and therefore, the compound can be used as a safe and low toxic drug for the treatment/ prevention, etc. for these diseases. Furthermore, compounds derived from such a compound obtained by the screening supra can be used as well.

The compound obtained by the screening above may be in the form of salts. As the salts of the compound, there may be used salts with physiologically acceptable acids (e.g., inorganic acids or organic acids) or bases (e.g., alkali metal salts), preferably physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

A pharmaceutical preparation comprising the compound or salts thereof obtained by the above screening methods may be manufactured in a manner similar to the method for preparing the pharmaceutical preparation comprising the peptide of the present application described hereinabove.

Since the pharmaceutical preparation thus obtained is safe and low toxic, it can be administered to human and mammal (e.g., rat, mouse, guinea pig, rabbit, sheep, swine, bovine, horse, cat, dog, monkey, etc.).

The dose of the compound or its salt may vary depending on target disease, subject to be administered, route for administration, etc. When the compound is orally administered, the compound is administered to adult (as 60 kg) generally in a daily dose of approximately 0.1 mg to 100 mg, preferably approximately 1.0 mg to 50 mg, more preferably approximately 1.0 to 20 mg. When the compound is parenterally administered, a single dose of the compound may vary depending on subject to be administered, target disease/etc. When the compound is administered to adult (as 60 kg body weight) in the form of injection, it is desired to intravenously administer the compound, generally in a daily dose of approximately 0.01 to 30 mg, preferably approximately 0.1 to 20 mg, and more preferably approximately 0.1 to 10 mg. For other animal species, the corresponding dose as converted per 60 kg weight can be administered.

**(8b) Method of screening a compound that promotes or inhibits the activities of a promoter for the DNA of the present application**

The present application discloses a a method of screening a compound or its salt that promotes or inhibits the activities of a promoter for the DNA of the present application, which comprises administering a test compound to a non-human mammal deficient in expression of the DNA of the present application and detecting expression of the reporter gene.

In the screening method described above, the non-human mammal deficient in expression of the DNA of the present application is selected from the aforesaid non-human mammal deficient in expression of the DNA of the present application for an animal, in which the DNA of the present application is inactivated by introducing a reporter gene and the reporter gene can be expressed under the control of a promoter for the DNA of the present application.

The same examples given above for the test compound apply to the test compound.

As the reporter gene, the same specific examples given above apply to the reporter gene, with β-galactosidase (lacZ), soluble alkaline phosphatase gene, luciferase gene, etc. being preferred.

In the non-human mammal deficient in expression of the DNA of the present application wherein the DNA of the present application is substituted with a reporter gene, the reporter gene is present under the control of a promoter for the DNA of the present application. Thus, the activity of the promoter can be detected by tracing the expression of a substance encoded by the reporter gene.

For example, when a part of the DNA region encoding the peptide of the present application is substituted with, e.g., β-galactosidase gene (lacZ) derived from Escherichia coli, β-galactosidase is expressed in a tissue where the peptides of the present application should originally be expressed, in place of the peptide of the present application. Thus, the expression state of the peptide of the present application can be readily observed in vivo in an animal, by staining with a reagent, e.g., 5-bromo-4-chloro-3-indolyl-β-D-galactopyranoside (X-gal), which is a substrate for β-galactosidase. Specifically, a mouse deficient in the peptide of the present application, or its tissue section, is fixed with glutaraldehyde, etc. After washing with phosphate buffered saline (PBS), the system is reacted with a staining solution containing X-gal at room temperature or about 37°C for approximately 30 minutes to 1 hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening methods supra are compounds selected from the test compounds described above, which promote or inhibit the promoter activity for the DNA of the present application.

The compound obtained by the screening methods may be in the form of salts. The salts of the compound used are salts with physiologically acceptable acids (e.g., inorganic acids) or bases (e.g., organic acids), and physiologically acceptable acid addition salts are preferred. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The compound or its salt that promotes the activity of the promoter for the DNA of the present application can promote the expression of the peptide of the present application and promote the function of the peptide, and therefore, the compound can be used as a pharmaceutical preparation such as a drug for the treatment/prevention, etc. for diseases related to e.g. deficiency in the function of the peptide of the present application.

More specifically, when the peptide of the present application is the peptide F, G, H or I of the present application, a compound or its salt that promotes or inhibits the activity of the promoter for the DNA of the present application is useful as a safe and low toxic pharmaceutical preparation, for example, a hormone secretion regulator (for example, a regulator for secretion of adrenal cortical hormone such as corticosterone), an eating regulator, a sleep regulator, an arousal regulator, a pain regulator, a stress response regulator, a spontaneous behavior regulator or an emotional behavior regulator.

Further, the compound or its salt that promotes the activity of a promoter for the DNA encoding the peptide F, G, H or I of the present application is useful as a safe and low toxic pharmaceutical preparation promoting the activity of the peptide F of the present application, for example a prophylactic/therapeutic agent for diseases in the central nerves (for example, Alzheimer's disease/dementia/difficulty in eating (anorexia)/epilepsy, etc.), diseases in the hormone system (for example, faint labor, flaccid bleeding, before and after placenta expulsion, insufficiency in uterine restoration, Caesarean operation, artificial abortion, milk statis etc.), diseases in liver/gall/pancreas/internal secretion (for example, diabetes/difficulty in eating, etc.), inflammatory diseases (allergy/asthma/rheumatism etc.), circulatory organ diseases (for example, high blood pressure/cardiac hypertrophy/angina/arteriosclerosis, etc.) and as an agent for promoting secretion of adrenal cortical hormone, etc.

On the other hand, the compound or its salt that inhibits the activity of a promoter for the DNA encoding the peptide F, G, H or I of the present application is a safe and low toxic pharmaceutical preparation suppressing the activity of the peptide F of the present application, for example a prophylactic/therapeutic agent for diseases caused by excessive production of the peptide F of the present application, such as diseases in the central nerves, diseases in the hormone system, diseases in liver/gall/pancreas/internal secretion (for example, an and-obesity drug/excessive eating, etc.), inflammatory diseases, circulatory organ diseases, etc. and as an agent for inhibiting secretion of adrenal cortical hormone, etc.

In addition, the compound or its salt that promotes or inhibits the activity of a promoter for the DNA encoding the peptide F, G, H or I of the present application useful as a pharmaceutical preparation for regulating secretion of prolactin. That is, when the compound or its salt that promotes or inhibits the activity of a promoter for the DNA encoding the peptide F, G, H or I of the present application has a promoting action on secretion of prolactin, it is useful as a prolactin secretion promoter for diseases with which deficiency in secretion of prolactin is associated, for example a prophylactic/therapeutic agent for diseases related to prolactin secretion, such as depression of ovarian functions, deficiency in growth of seminal vesicle, osteoporosis, menopausal disorders, deficiency in secretion of milk, hypothyroidism, renal insufficiency etc. Further, when the compound or its salt that promotes or inhibits the activity of a promoter for the DNA encoding the peptide F, G, H or I of the present application has a promoting action on secretion of prolactin, it has a promoting action on sexual desire (pheromone-like action) based on the promoting action on secretion of prolactin, and is thus useful as an agent for promoting sexual desire.

When the compound or its salt that promotes or inhibits the activity of a promoter for the DNA encoding the peptide F, G, H or I of the present application has an inhibitory action on secretion of prolactin, it is useful as a prolactin secretion inhibitor for diseases with which excessive secretion of prolactin is associated, for example a prophylactic/therapeutic agent for diseases related to secretion of prolactin, such as hyperprolactinemia, pituitary gland tumor, interbrain tumor, abnormal menstruation, stress, autoimmune disease, prolactinoma, sterility, impotence, amenorrhea, galactorrhea, acromegaly, Chiari-Frommel syndrome, Argonz-del Castilo syndrome, Forbes-Albright syndrome, breast cancer lymphoma, Sheehan's syndrome or abnormality in spermatogenesis. Further, when the compound or its salt that promotes or inhibits the activity of a promoter for the DNA encoding the peptide F, G, H or I of the present application has an inhibitory action on secretion of prolactin, it is useful as a contraceptive agent based on the inhibitory action on secretion of prolactin.

In addition, the compound or its salt that promotes or inhibits the activity of a promoter for the DNA encoding peptide F, G, H or I of the present application is also useful as a diagnostic drug for examining an ability to secrete prolactin or as an animal drug such as a milk secretion promoting agent for domestic mammals such as bovine, goat, swine etc., and can be applied to production of a useful substance by producing the useful substance in the mammals and secreting it in milk.

Further, the compound or its salt that promotes or inhibits the activity of a promoter for the DNA encoding peptide F, G, H or I of the present application is also useful as a prophylactic/therapeutic agent for choriocarcinoma, hydatidiform mole, invasive mole, abortion, insufficiency, in fetal growth, abnormality in sugar metabolism, abnormality in lipid metabolism or delivery induction.

In addition, the compound or its salt that promotes or inhibits the activity of a promoter for the DNA encoding peptide F, G, H or I of the present application is useful as a pharmaceutical preparation, for example a prophylactic/therapeutic agent for diseases such as high blood pressure, autoimmune disease, cardiac insufficiency, cataract, glaucoma, acute bacterial meningitis, acute myocardial infarction, acute pancreatitis, acute viral encephalitis, adult respiratory distress syndrome, alcoholic hepatitis, Alzheimer's disease, asthma, arteriosclerosis, atopic dermatitis, bacterial pneumonia, bladder cancer, bone fracture, breast cancer, bulimia, [bulimia], burn healing, uterine cervix cancer, chronic lymphatic leukemia, chronic myeloid leukemia, chronic pancreatitis, hepatocirrhosis, large-intestine cancer (colon/rectum cancer), Crohn's disease, dementia, diabetic complications, diabetic nephritis, diabetic neuropathy, diabetic retinitis, gastritis, helicobacter pylori infection, renal insufficiency, type A hepatitis, type B hepatitis, type C hepatitis, hepatitis, herpes simplex virus infection, varicella zoster virus infection, Hodgkin's disease, AIDS infection, human papilloma virus infection, hypercalcemia, hypercholesterolemia, hyperglyceridemia, hyperlipemia, infections, influenza infection, insulin-dependent diabetes (type I), invasive Staphylococcus infection, malignant melanoma, cancer metastasis, multiple myeloma, allergic rhinitis, nephritis, non-Hodgkin lymphoma, insulin-independent diabetes (type II), non-small cell lung cancer, organ transplant operation, bone arthritis, bone softening, bone decrease, osteoporosis, ovary cancer, bone Behcet's disease, digestive ulcer, peripheral blood vessel disease, prostate cancer, reflux esophagitis, renal insufficiency, rheumatoid arthritis, schizophrenia, septicemia, septicemic shock, severe systemic fungal infection, small cell lung cancer, spinal damage, stomach cancer, systemic lupus erythematosus, transient cerebral ischemia attack, tuberculosis, valvular heart disease, vascular/multiple infarct dementia, wound healing, insomnia, arthritis, insufficiency in secretion of pituitary hormone, frequent urination, uremia, or nerve degeneration disease.

Further, the compound or its salt promoting or inhibiting the activity of a promoter for the DNA encoding the peptide F, G, H or I of the present application can be used as a pharmaceutical preparation such as a therapeutic/prophylactic agent for a disease macular edema cystoid.

Further, the compound or its salt promoting or inhibiting the activity of a promoter for the DNA encoding the peptide F, G, H or I of the present application is useful for example as (1) a therapeutic agent for tumors such as acromegaly, TSH-producing tumor, non-secretory (non-functional) pituitary tumor, ectopic ACTH (adrenocorticotropine)-producing tumor, marrow-like thyroid cancer, VIP-producing tumor, glucagon-producing tumor, gastrin-producing tumor, insulinoma, carcinoid etc., (2) a therapeutic agent for insulin dependent or independent diabetes, or various diseases related to such diabetes, that is, diabetic complications (for example, diabetic retinitis, diabetic nephritis, diabetic neuropathy, down syndrome, erectile low blood pressure, etc.), (3) a therapeutic agent for obesity, bulimia etc. by ameliorating hyperinsulinemia or suppressing appetite, (4) a therapeutic agent for acute pancreatitis, chronic pancreatitis, pancreas/intestine physter, bleeding ulcer, digestive ulcer, gastritis, gastric hyperacidity, reflux esophagitis, etc., (5) an agent for ameliorating various symptoms caused by infection with helicobacter pylori bacteria (for example, an agent for inhibiting promotion of gastrin secretion, etc.), (6) an agent for suppressing amylase secretion caused by endoscopic cholangio-pancreatography, and for prognosis of surgical operation of pancreas, (7) a therapeutic agent for diarrhea attributable to a reduction of the absorbing ability of small intestine, acceleration of secretion, or abnormality in mobility of digestive tract (for example, short bowel syndrome, etc.), diarrhea attributable to chemicals in chemotherapy of cancer, diarrhea attributable to congenital shrinkage of small intestine, diarrhea attributable to neuroendocrine tumor such as VIP-producing tumor, diarrhea attributable to AIDS, diarrhea attributable to graft versus host reaction accompanying a marrow transplant, diarrhea attributable to diabetes, diarrhea attributable to blockage of abdominal nerve plexuses, diarrhea attributable to systematic sclerosis, diarrhea attributable to eosinophilia, etc., (8) a therapeutic agent for damping syndrome, hypersensitive colitis, Crohn's disease, inflammatory intestinal diseases, etc., (9) a therapeutic agent for tumors or cancers (for example, thyroid cancer, large intestine cancer, breast cancer, prostate cancer, small cell lung cancer, non-small cell lung cancer, pancreas cancer, stomach cancer, cholangioma, liver cancer, bladder cancer, ovary cancer, melanoma, osteosarcoma, chondrosarcoma, malignant pheochromocytoma, neuroblastoma, brain tumor, thymoma, kidney cancer, etc.), leukemia (for example, basophil leukemia/chronic lymphatic leukemia, chronic myeloid leukemia, Hodgkin's disease, non-Hodgkin lymphoma, etc.); the therapeutic agent can be used alone or in combination with other anticancer drugs (for example, tamoxifen, LHRH agonist, LHRH antagonist, interferon-$\alpha$,$\beta$ and $\gamma$, interleukin-2, etc.), (10) a prophylactic/therapeutic agent for hypertrophic myocardial disease, arteriosclerosis, valvular heart disease, myocardial infarction (particularly, myocardial infarction after percutaneous transluminal coronary angioplasty), revascularization, (11) a therapeutic agent for esophagus vein cancer bleeding, hepatocirrhosis, peripheral blood vessel disease, (12) a therapeutic agent for diseases accompanying systemic or topical inflammations (for example, multiple arteritis, rheumatoid arthritis, psoriasis, sunburn, eczema, allergies (for example, asthma, atopic dermatitis, allergic rhinitis, etc.)), based on the secretion-regulating action of phsyoloigcally active substance acting on the immune system (for example, substance P, tachykinin, cytokine, etc.), (13) a therapeutic agent for dementia (for example, Alzheimer's disease, Alzheimer type senile dementia, vascular/multiple dementia, etc.), schizophrenia, epilepsy, depression, general anxiety, difficult sleep, multiple sclerosis, etc., because of its influence on production and secretion of nerve-regulating factor, (14) a therapeutic agent for eye diseases (for example, glaucoma, etc.), (15) a prophylactic/therapeutic agent for acute bacterial meningitis, acute viral encephalitis, adult respiratory distress syndrome, bacterial pneumonia, severe systemic fungal infection, tuberculosis, spinal damage, bone fracture, renal insufficiency, pneumonia, alcoholic hepatitis, type A hepatitis, type B hepatitis, type C hepatitis, AIDS infection, human papilloma virus infection, influenza infection, cancer metastasis, multiple myeloma, bone softening, osteoporosis, bone Behcet's disease, nephritis, renal insufficiency, septicemia; septicemic shock, hypercalcemia, hypercholesterolemia, hyperglyceridemia, hyperlipemia, systemic lupus erythematosus, transient cerebral ischemia attack, alcoholic hepatitis, etc. (16) a therapeutic agent for organ transplant operation, burn, wound, alopecia, etc., and (17) an analgesic for suppressing and relieving chronic or acute pains (for example, acute pain after operation, inflammatory acute pain, toothache, bone diseases (for example, acute pains accompanying arthritis, rheumatism, osteoporosis, etc.)).

Further, a compound derived from the compound obtained in the above screening can also be used similarly.

The pharmaceutical preparation comprising the compound or its salt obtained by the screening method can be produced in a manner similar to the method for preparing the pharmaceutical preparation comprising the peptide of the present application or its salt described hereinabove.

Since the thus obtained pharmaceutical preparation is safe and low toxic, and can be administered to, for example, human and warm-blooded animal (e.g., rat, mouse, guinea pig, rabbit, sheep, swine, bovine, horse, cat, dog, monkey, etc.).

The dose of the compound or its salt may vary depending on target disease, subject to be administered, route for administration, etc. When the compound promoting the promoter activity for the DNA of the present application is orally administered, the compound is administered to adult (as 60 kg) generally in a daily dose of approximately 0.1 mg to 100 mg, preferably approximately 1.0 mg to 50 mg, more preferably approximately 1.0 to 20 mg. When the compound is parenterally administered, a single dose of the compound may vary depending on subject to be administered, target disease, etc. When the compound promoting the promoter activity for the DNA of the present application is administered to adult (as 60 kg body weight) in the form of injection, it is convenient to administer the compound by intravenous injection, in a daily dose of approximately 0.01 to 30 mg, preferably approximately 0.1 to 20 mg, more preferably approximately 0.1 to 10 mg. For other animal species, the corresponding dose as converted per 60 kg weight can be administered.

On the other hand, when the compound inhibiting the promoter activity for the DNA of the present applications is orally administered, the compound is administered to adult (as 60 kg) generally in a daily dose of approximately 0.1 mg to 100 mg, preferably approximately 1.0 mg to 50 mg, more preferably approximately 1.0 to 20 mg. When the compound is parenterally administered, a single dose of the compound may vary depending on subject to be administered, target disease, etc. When the compound inhibiting the promoter activity for the DNA of the present application is administered to adult (as 60 kg body weight) in the form of injection, it is convenient to administer the compound by intravenous injection, in a daily dose of approximately 0.01 to 30 mg, preferably approximately 0.1 to 20 mg, more preferably approximately 0.1 to 10 mg. For other animal species, the corresponding dose as converted per 60 kg weight can be administered.

Thus, the non-human mammal deficient in expression of the DNA of the present application extremely useful in screening a compound or its salt that promotes or inhibits the activity of a promoter for the DNA of the present application, and can contribute significantly to elucidation of causes for various diseases attributable to deficient expression of the DNA of the present application and development of a prophylactic/therapeutic agent for the diseases.

Further, genes encoding various proteins are ligated downstream DNA comprising a promoter region for the peptide of the present application and injected into a fertilized egg of an animal to create a transgenic animal by which the peptide of the present application can be specifically synthesized and examined for its action in the living body. When a suitable reporter gene is ligated to the promoter region to establish a cell strain expressing the same, the cell strain can be used as a system of searching for a low-molecular compound having an action of specifically promoting or suppressing the ability of the cell strain to produce the peptide of the present application in vivo.

The antibody to AQ27 receptor, and a nucleotide sequence complementary to DNA encoding AQ27 receptor, or an antisense polynucleotide (antisense DNA) comprising a part of the DNA can be produced according to a method described in WO01/16313.

AQ27 receptor, the DNA encoding AQ27 receptor (hereinafter sometimes referred to as AQ27 receptor DNA), the antibody to AQ27 receptor (hereinafter sometimes referred to as the antibody of the present application), the antisense DNA to AQ27 receptor DNA (hereinafter sometimes referred to as the antisense DNA of the present application) have the following uses.

**(1) Prophylactic/therapeutic agent for diseases with which deficiency in the functions of AQ27 receptor is associated**

a) AQ27 receptor or b) DNA encoding AQ27 receptor can be used as a pharmaceutical preparation such as a prophylactic/therapeutic agent for diseases with which deficiency in the functions of AQ27 receptor is associated.

For example, when there is a patient who cannot expect the physiological action of fatty acid as ligand because of a decrease in AQ27 receptor in the living body (deficiency in AQ27 receptor), a) AQ27 receptor is administered into the patient to supply the AQ27 receptor, or b) (i) DNA encoding AQ27 receptor is administered into the patient to express it or (ii) DNA encoding AQ27 receptor is inserted into target cells to express it and the cells are transplanted to the patient, whereby the amount of AQ27 receptor is increased in the body of the patient to demonstrate the action of the ligand sufficiently. That is, the DNA encoding AQ27 receptor is useful as a safe and low toxic prophylactic and/or therapeutic agent for diseases with which deficiency in the functions of AQ27 receptor is associated.

Specifically, the AQ27 receptor or AQ27 receptor DNA is useful as a safe and low toxic pharmaceutical preparation, for example, a hormone secretion regulator (for example, a promoter for secretion of adrenal cortical hormone such as corticosterone), an eating regulator, a sleep regulator, an arousal regulator, a pain regulator, a stress response regulator, a spontaneous behavior regulator or an emotional behavior regulator.

Further, the AQ27 receptor or the AQ27 receptor DNA is useful as a prophylactic/therapeutic agent for diseases in the central nerves (for example, Alzheimer's disease/dementia/difficulty in eating (anorexia)/epilepsy, etc.), diseases in the hormone system (for example, faint labor, flaccid bleeding, before and after placenta expulsion, insufficiency in uterine restoration, Caesarean operation, artificial abortion, milk statis etc.), diseases in liver/gall/pancreas/internal secretion (for example, diabetes/difficulty in eating, etc.), inflammatory diseases (allergy/asthma/rheumatism etc.), circulatory organ diseases (for example, high blood pressure/cardiac hypertrophy/angina/arteriosclerosis, etc.).

When the AQ27 receptor is used as the therapeutic/prophylactic agent, the AQ27 receptor can be formed into a pharmaceutical preparation in a usual manner.

Where the AQ27 receptor DNA is used as the prophylactic/therapeutic agent described above, the AQ27 receptor DNA itself is inserted into an appropriate vector such as retrovirus vector, adenovirus vector, adenovirus-associated virus vector, etc. and then administered to human or other warm-blooded animal in a conventional manner. The AQ27 receptor DNA may also be administered as an intact DNA, or prepared into pharmaceutical preparations together with physiologically acceptable carriers such as adjuvants to assist its uptake, which are administered by gene gun or through a catheter such as a hydrogel catheter.

For example, a) AQ27 receptor or b)AQ27 receptor DNA can be used orally, for example, in the form of tablets which may be sugar coated if necessary and desired, capsules, elixirs, microcapsules etc., or parenterally in the form of injectable preparations such as a sterile solution and a suspension in water or with other pharmaceutically acceptable liquid. These preparations can be manufactured for example by mixing a) AQ27 receptor or b)AQ27 receptor DNA with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin, alginic acid, etc., a lubricant such as magnesium stearate, a sweetening agent such as sucrose, lactose or saccharin, and a flavoring agent such as peppermint, akamono oil or cherry. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredients in a vehicle such as water for injection with a naturally occurring vegetable oil such as sesame oil, coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil, soybean oil, etc., which may also be used in combination with a dissolution aid such as benzyl benzoate, benzyl alcohol, etc.

The prophylactic/therapeutic agent described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

The thus obtained pharmaceutical preparation is safe and low toxic, and can thus be administered to, for example, human and warm-blooded animals (e.g., rat, mouse, rabbit, sheep, swine, bovine, cat, dog, monkey, etc.).

The dose of AQ27 receptor may vary depending on subject to be administered, target organ, symptom, administration method, etc. When the AQ27 receptor is orally administered, the AQ27 receptor is administered to a diabetic (as 60 kg) generally in a daily dose of approximately 0.1 mg to 100 mg, preferably approximately 1.0 mg to 50 mg, more preferably approximately 1.0 to 20 mg. When the AQ27 receptor is parenterally administered, a single dose of the AQ27 receptor may vary depending on subject to be administered, target organ, symptom, administration method, etc. When the AQ27 receptor is administered to a diabetic (as 60 kg body weight) in the form of injection, it is convenient to administer the AQ27 receptor by intravenous injection, generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

The dose of AQ27 receptor DNA may vary depending on subject to be administered, target organ, symptom, administration method, etc. When the AQ27 receptor DNA is orally administered, the AQ27 receptor DNA is administered to a diabetic (as 60 kg) generally in a daily dose of approximately 0.1 mg to 100 mg, preferably approximately 1.0 mg to 50 mg, more preferably approximately 1.0 to 20 mg. When the AQ27 receptor DNA is parenterally administered, a single dose of the AQ27 receptor DNA may vary depending on subject to be administered, target organ, symptom, administration method etc. When the AQ27 receptor DNA is administered to a diabetic (as 60 kg body weight) in the form of injection, it is convenient to administer the AQ27 receptor DNA by intravenous injection, generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

(2) Gene diagnostic agent

By using the AQ27 receptor DNA and antisense DNA as a probe, an abnormality (gene abnormality) of the DNA or mRNA encoding the AQ27 receptor or its partial peptide in humans or mammals (e.g., rat, mouse, rabbit, sheep, swine, bovine, cat, dog, monkey, etc.) can be detected. Therefore, the AQ27 receptor DNA and antisense DNA is useful as a gene diagnostic agent for the damage to the DNA or mRNA, its mutation, or its decreased expression, or increased expression or overexpression of the DNA or mRNA.

The gene diagnosis described above using the AQ27 receptor DNA or antisense DNA can be performed by, for example, the publicly known Northern hybridization assay, the PCR-SSCP assay (Genomics, 5, 874-879 (1989); Proceedings of the National Academy of Sciences of the United States of America, 86, 2766-2770 (1989)); and the like.

For example, when a decrease in the expression of the AQ27 receptor is detected by Northern hybridization, it can be diagnosed that for example a disease related to deficiency in the functions of the AQ27 receptor is involved or it is highly likely to suffer from such a disease in the future.

When overexpression of the AQ27 receptor is detected by Northern hybridization, it can be diagnosed that for example a disease related to overexpression of the AQ27 receptor is involved or it is highly likely to suffer from such a disease in the future.

The diseases with which deficiency in the functions of AQ27 receptor is associated include diseases in the central nerves (for example, Alzheimer's disease/dementia/difficulty in eating (anorexia)/epilepsy, etc.), diseases in the hormone system (for example, faint labor, flaccid bleeding, before and after placenta expulsion, insufficiency in uterine restoration, Caesarean operation, artificial abortion, milk statis etc.), diseases in liver/gall/pancreas/internal secretion (for example, diabetes/difficulty in eating, etc.), inflammatory diseases (allergy/asthma/rheumatism etc.), circulatory organ diseases (for example, high blood pressure/cardiac hypertrophy/angina/arteriosclerosis, etc.) etc.

The diseases attributable to overexpression of AQ27 receptor include diseases caused by excessive production of AQ27 receptor, such as diseases in the central nerves, diseases in the hormone system, diseases in liver/gall/pancreas/internal secretion (for example, an anti-obesity drug/excessive eating, etc.), inflammatory diseases, circulatory organ diseases.

**(3) Pharmaceutical preparation comprising a compound that alters the amount of AQ27 receptor expressed**

The AQ27 receptor DNA can be used as a probe in screening of a compound that alters the amount of AQ27 receptor expressed.

That is, the present application discloses, for example, a method of screening a compound that alters the amount of AQ27 receptor expressed, which comprises measuring the amount of AQ27 receptor mRNA in, for example, (i) a) blood, b) specific organs or c) tissues or cells isolated from organs in non-human mammals or (ii) transformants, etc.

Specifically, the amount of the AQ27 receptor mRNA is measured in the following manner.

(i) Normal or morbid non-human mammals (for example, mice, rats, rabbits, sheep, swine, bovine, cats, dogs, monkeys etc., specifically rats with dementia, mice with obesity, rabbits with arteriosclerosis, mice bearing cancer, etc.) are given a chemical (for example, an anti-dementia drug, blood pressure depressant, anti-cancer drug, anti-obesity drug, etc.) or physical stress (for example, water immersion stress, electrical shock, brightening/darkening, low temperature, etc.), and after a predetermined time, blood, a specific organ (for example, brain, liver, kidney, etc.) or tissues or cells isolated from organs are obtained.

The AQ27 receptor mRNA contained in the resulting cells can be quantified by techniques such as, for example, TaQMan PCR of the mRNA extracted in a usual manner from the cells, and can be analyzed by Northern blotting by a means known per se.

(ii) The transformant expressing AQ27 receptor is prepared according to the method described above, and the AQ27 receptor mRNA in the transformant can be quantified and analyzed in the same manner as described above.

Screening of the compound that alters the amount of AQ27 receptor expressed can be carried out by:

(i) administering a test compound into normal or morbid non-human mammals before a predetermined time, that is, 30 minutes to 24 hours before, more preferably 30 minutes to 12 hours before, still more preferably 1 hour to 6 hours before giving chemical or physical stress to the mammals, or after a predetermined time, that is, 30 minutes to 3 days after, preferably 1 hour to 2 days after, more preferably 1 hour to 24 hours after giving chemical or physical stress, or simultaneously with the chemical or physical stress, and quantifying and analyzing the amount of AQ27 receptor mRNA in the cells after a predetermined time, that is, 30 minutes to 3 days after, preferably 1 hour to 2 days after, more preferably 1 hour to 24 hours after the administration, or

(ii) mixing a test compound with a medium for culturing the transformant in a usual manner and quantifying and analyzing the amount of AQ27 receptor mRNA in the transformant after culture, that is, 1 to 7 days later, preferably 1 to 3 days later, more preferably 2 to 3 days later.

The compound or its salt obtainable using the screening method of the present application is a compound having an action of altering the amount of AQ27 receptor expressed, and is specifically a compound (a) increasing the amount of AQ27 receptor expressed thereby enhancing cell stimulating activities via the AQ27 receptor (e.g., the activity that promotes or suppresses arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.) or a compound (b) decreasing the amount of AQ27 receptor expressed thereby reducing the cell stimulating activities.

The compound includes peptides, proteins, non-peptide compounds, synthetic compounds, fermentation products, etc., and these compounds may be novel compounds or known compounds.

The compound or its salt that alters the amount of AQ27 receptor expressed, which is obtainable using the aforementioned screening method, is a safe and low toxic pharmaceutical preparation such as a hormone secretion regulator (for example, a regulator for secretion of adrenal cortical hormone such as corticosterone), an eating regulator, a sleep regulator, an arousal regulator, a pain regulator, a stress response regulator, a spontaneous behavior regulator or an emotional behavior regulator.

The compound increasing the amount of AQ27 receptor expressed thereby enhancing the cell-stimulating activities is useful as a safe and low toxic prophylactic/therapeutic agent for diseases with which deficiency in the functions of AQ27 receptor is associated, for example diseases in the central nerves (for example, Alzheimer's disease/dementia/difficulty in eating (anorexia)/epilepsy, etc.), diseases in the hormone system (for example, faint labor, flaccid bleeding, before and after placenta expulsion, insufficiency in uterine restoration, Caesarean operation, artificial abortion, milk statis etc.), diseases in liver/gall/pancreas/internal secretion (for example, diabetes/difficulty in eating, etc.), inflammatory diseases (allergy/asthma/rheumatism etc.) and circulatory organ diseases (for example, high blood pressure/cardiac hypertrophy/angina/arteriosclerosis, etc.) and as an agent for promoting secretion of adrenal cortical hormone, etc.

The compound decreasing the amount of AQ27 receptor expressed thereby reducing the cell stimulating activities is useful as a safe and low toxic prophylactic/therapeutic agent for diseases with which overexpression of AQ27 receptor is associated, for example, diseases induced by excessive production of AQ27 receptor, such as diseases in the central system, diseases in the hormone system, diseases in liver/gall/panereas/internal secretion (for example, an anti-obesity drug/excessive eating, etc.), inflammatory diseases and circulatory organ diseases, and as a an agent for inhibiting secretion of adrenal cortical hormone, etc.

When the compound or its salt obtained by using the screening method of the present application is used as a pharmaceutical composition, the compound or its salt can be formed into a pharmaceutical preparation in a usual manner.

The compound can be used orally, for example, in the form of tablets which may be sugar coated if necessary and desired, capsules, elixirs, microcapsules etc., or parenterally in the form of injectable preparations such as a sterile solution and a suspension in water or with other pharmaceutically acceptable liquid. These preparations can be manufactured by mixing the compound with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin, alginic acid, etc., a lubricant such as magnesium stearate, a sweetening agent such as sucrose, lactose or saccharin, and a flavoring agent such as peppermint, akamono oil or cherry. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredients in a vehicle such as water for injection with a naturally occurring vegetable oil such as sesame oil, coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80™ and HCO-50), etc. Examples of the oily medium include sesame oil, soybean oil, etc., which may also be used in combination with a dissolution aid such as benzyl benzoate, benzyl alcohol, etc.

The prophylactic/therapeutic agent described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

The thus obtained pharmaceutical preparation is safe and low toxic, and can thus be administered to, for example, human and warm-blooded animals (e.g., rat, mouse, rabbit, sheep, swine, bovine, cat, dog, monkey, etc.).

The dose of the compound or its salt may vary depending on subject to be administered, target organ, symptom, administration method, etc. When the compound is orally administered, the compound is administered to a diabetic (as 60 kg) generally in a daily dose of approximately 0.1 mg to 100 mg, preferably approximately 1.0 mg to 50 mg, more preferably approximately 1.0 to 20 mg. When the compound is parenterally administered, a single dose of the compound may vary depending on subject to be administered, target organ, symptom, administration method etc. When the compound is administered to a diabetic (as 60 kg body weight) in the form of injection, it is convenient to administer the compound by intravenous injection, generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

**(4) Method of quantifying AQ27 receptor and method of diagnosing the same**

The antibody of the present application is capable of specifically recognizing AQ27 receptor and can thus be used for quantification of AQ27 receptor in a test sample fluid, in particular, for quantification by the sandwich immunoassay.

That is, the present application discloses:

(i) a method for quantification of AQ27 receptor in a test sample fluid, which comprises competitively reacting the antibody of the present application, a test sample fluid and a labeled form of AQ27 receptor, and measuring the ratio of the labeled AQ27 receptor bound to said antibody; and,

(ii) a method for quantification of AQ27 receptor in a test sample fluid, which comprises reacting the test sample fluid simultaneously or continuously with the antibody of the present application application immobilized on a carrier and a labeled form of the antibody of the present application, and then measuring the activity of the labeling agent on the insoluble carrier.

In the quantification method in the above-mentioned (ii), it is desirable that one antibody is an antibody recognizing the N-terminal region of AQ27 receptor, and the other antibody is an antibody reacting with the C-terminal region of AQ27 receptor.

The monoclonal antibody to AQ27 receptor may be used to quantify AQ27 receptor. Besides, AQ27 receptor may also be detected by means of tissue staining. For these purposes, the antibody molecule per se may be used or F $(ab)_2$, Fab' or Fab fractions of the antibody molecule may be used as well.

There is no particular limitation to the method of quantifying AQ27 receptor using the antibody of the present application; any method may be used so far as it relates to a method in which the amount of antibody, antigen or antibody-antigen complex can be detected by a chemical or a physical means, depending on or corresponding to the amount of antigen (e.g., the amount of AQ27 receptor) in a test sample fluid to be assayed, and then calculated using a standard curve prepared by a standard solution containing the known amount of antigen. Advantageously used are, for example, nephrometry, competitive method, immunometric method and sandwich method; in terms of sensitivity and specificity, the sandwich method, which will be later described, is particularly preferred.

Examples of the labeling agent used in the assay method using the labeling substance are radioisotopes, enzymes, fluorescent substances, luminescent substances, etc. Examples of the radioisotope are [$^{125}I$], [$^{131}I$], [$^{3}H$], [$^{14}C$], etc. Preferred examples of the enzyme are those that are stable and have a high specific activity, which include β-galactosidase, β-glucosidase, alkaline phosphatase, peroxidase, malate dehydrogenase, etc. Examples of the fluorescent substance are fluorescamine, fluorescein isothiocyanate, etc. Examples of the luminescent substance are luminol, a luminol derivative, luciferin, lucigenin, etc. Furthermore, the biotin-avidin system may also be used for binding of an antibody or antigen to a labeling agent.

In the immobilization of antigens or antibodies, physical adsorption may be used. Alternatively, chemical binding conventionally used for immobilization of AQ27 receptor or enzymes may be used as well. Examples of the carrier include insoluble polysaccharides such as agarose, dextran and cellulose; synthetic resins such as polystyrene, polyacrylamide, silicone, etc.; glass; and the like.

In the sandwich method, a test sample fluid is reacted with an immobilized form of the monoclonal antibody of the present application (primary reaction), then reacted with another labeled form of the monoclonal antibody of the present application (secondary reaction) and the activity of the labeling agent on the insoluble carrier is assayed, whereby the amount of AQ27 receptor in the test sample fluid can be quantified. The primary and secondary reactions may be carried out in a reversed order, simultaneously or sequentially with an interval. The type of the labeling agent and the method for immobilization may be effected by modifications of those described hereinabove. In the immunoassay by the sandwich method, it is not always necessary that the antibody used for the labeled antibody and for the solid phase should be one type or one species but a mixture of two or more antibodies may be used as well, for the purpose of improving the measurement sensitivity, etc.

In the method for assaying AQ27 receptor by the sandwich method according to the present application, preferred monoclonal antibodies of the present application used for the primary and the secondary reactions are antibodies whose binding sites to AQ27 receptor are different from one another. Thus, the antibodies used in the primary and the secondary reactions are those wherein when the antibody used in the secondary reaction recognizes the C-terminal region of AQ27 receptor, the antibody recognizing the site other than the C-terminal region, e.g., recognizing the N-terminal region, is preferably used in the primary reaction.

The monoclonal antibody of the present application may be used in an assay system other than the sandwich method, such as a competitive method, an immunometric method, nephrometry, etc.

In the competitive method, an antigen in a test sample fluid and a labeled antigen are competitively reacted with an antibody, then the unreacted labeled antigen (F) and the labeled antigen bound to the antibody (B) are separated (i.e., B/F separation) and the labeled amount of either B or F is measured to determine the amount of the antigen in the test sample fluid. In the reactions for such a method, there are a liquid phase method in which a soluble antibody is used as the antibody and the B/F separation is effected by polyethylene glycol while a second antibody to the antibody described above is used, and a solid phase method in which an immobilized antibody is used as the first antibody or a soluble antibody is used as the first antibody while an immobilized antibody is used as the second antibody.

In the immunometric method, an antigen in a test sample fluid and an immobilized antigen are competitively reacted with a given amount of a labeled antibody followed by separating the solid phase from the liquid phase; or an antigen in a test sample fluid and an excess amount of labeled antibody are reacted, then an immobilized antigen is added to bind an unreacted labeled antibody to the solid phase, and the solid phase is separated from the liquid phase. Thereafter, the labeled amount of any of the phases is measured to determine the antigen amount in the test sample fluid.

In the nephrometry, the amount of insoluble sediment, which is produced as a result of the antigen-antibody reaction in a gel or in a solution, is measured. Even when the amount of an antigen in a test sample fluid is small and only a small amount of the sediment is obtained, laser nephrometry utilizing laser scattering can be suitably used.

In applying each of those immunoassays to the quantification method of the present application, any special conditions or operations are not required to set forth. The assay system for AQ27 receptor may be constructed in addition to conditions or operations conventionally used for each of the methods, taking the technical consideration by one skilled in the art into account. For the details of such conventional technical means, a variety of reviews, reference books, etc. may be referred to (for example, Hiroshi Irie (ed.): "Radioimmunoassay" (published by Kodansha, 1974); Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzynie Immunoassay" (Second Edition) (published by Igaku Shoin, 1982); Eiji Ishikawa, et al. (ed.): "enzyme Immunoassay" (Third Edition) (published by Igaku Shoin, 1997); "Methods in Enzymology" Vol. 70 (Immuochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)) (published by Academic Press); etc.)

As described above, AQ27 receptor can be quantified with high sensitivity, using the antibody of the present application.

Furthermore, when a decrease in the concentration of AQ27 receptor is detected by quantifying the concentration of AQ27 receptor using the antibody of the present application, it can be diagnosed that for example a disease related to deficiency in the functions of AQ27 receptor is involved or it is highly likely to suffer from such a disease in the future.

When an increase in the concentration of AQ27 receptor is detected, it can be diagnosed that for example a disease related to overexpression of AQ27 receptor is involved or it is highly likely to suffer from such a disease in the future.

The diseases with which deficiency in the function of AQ27 receptor is associated include diseases in the central nerves (for example, Alzheimer's disease/dementia/difficulty in eating (anorexia)/epilepsy, etc.), diseases in the hormone system (for example, faint labor, flaccid bleeding, before and after placenta expulsion, insufficiency in uterine restoration, Caesarean operation, artificial abortion, milk statis etc.), diseases in liver/gall/pancreas/internal secretion (for example, diabetes/difficulty in eating, etc.), inflammatory diseases (allergy/asthmalrheumatism etc.), circulatory organ diseases (for example, high blood pressure/cardiac hypertrophy/angina/arteriosclerosis, etc.) etc.

The diseases attributable to overexpression of AQ27 receptor include diseases caused by excessive production of AQ27 receptor, for example diseases in the central nerves, diseases in the hormone system, diseases in liver/gall/panereas/internal secretion (for example, an anti-obesity drug/excessive eating, etc.), inflammatory diseases, circulatory organ diseases, etc.

(5) Pharmaceutical preparation comprising a compound that alters the amount of AQ27 receptor or its partial peptide in cell membranes

The antibody of the present application can specifically recognize AQ27 receptor, and can thus be used in screening a compound that alters the amount of AQ27 receptor in cell membranes.

That is, the present application discloses, for example:

(i) a method of screening a compound that alters the amount of AQ27 receptor in cell membranes, which comprises disrupting a) blood, b) specific organs, or c) tissues or cells isolated from organs, then isolating a cell membrane fraction and quantifying AQ27 receptor contained in the cell membrane fraction;

(ii) a method of screening a compound that alters the amount of AQ27 receptor in cell membranes, which comprises disrupting an AQ27 receptor-expressing transformant etc., then isolating a cell membrane fraction and quantifying AQ27 receptor contained in the cell membrane fraction;

(iii) a method of screening a compound that alters the amount of AQ27 receptor in cell membrane, which comprises preparing a section of a) blood, b) specific organs, or c) tissues or cells of organs isolated from a non-human mammal and quantifying the degree of staining of the receptor protein on a cell surface layer by immune staining thereby confirming the protein on the cell surface layer, and

iv) a method of screening a compound that alters the amount of AQ27 receptor in cell membranes, which comprises preparing a section of a AQ27 receptor-expressing transformant etc. and quantifying the degree of staining of the receptor protein on a cell surface layer by immune staining thereby confirming the protein on the cell surface layer.

Specifically, AQ27 receptor contained in the cell membrane fraction is quantified as follows:

(i) Normal or morbid non-human mammals (for example, mice, rats, rabbits, sheep, swine, bovine, cats, dogs, monkeys etc., specifically rats with dementia, mice with obesity, rabbits with arteriosclerosis, mice bearing cancer, etc.) are given a chemical (for example, an anti-dementia drug, blood pressure depressant, anti-cancer drug, anti-obesity drug, etc.) or physical stress (for example, water immersion stress, electrical shock, brightening/darkening, low temperature, etc.), and after a predetermined time, blood, a specific organ (for example, brain, liver, kidney, etc.) or tissues or cells isolated from organs are obtained. The resulting organ, tissues or cells are suspended for example in a suitable buffer (for example, Tris-HCl buffer, phosphate buffer, HEPES buffer etc.) and the organ, tissues or cells are disrupted, and a cell membrane fraction is obtained by using a surfactant (for example. Triton X100™, Tween 20™ etc.) and by techniques such as centrifugation, column fractionation, etc.

The cell membrane fraction is a fraction abundant in cell membrane obtained by cell disruption and subsequent fractionation by a publicly known method. Cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugadon. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the AQ27 receptor expressed and in membrane components such as cell-derived phospholipids and membrane proteins.

The AQ27 receptor mRNA contained in the cell membrane fraction can be quantified by, for example, sandwich immunoassays using the antibody of the present application, Western blotting analysis, etc.

The sandwich immunoassays can be performed in the same manner as in the method described above, and Western blotting can be performed by a means known per se.

(ii) The transformant expressing AQ27 receptor is created according to the method descried above, and the AQ27 receptor contained in its cell membrane fraction can be quantified.

Screening of the compound that alters the amount of AQ27 receptor in the cell membrane can be carried out by:

(i) administering a test compound into normal or morbid non-human mammals before a predetermined time, that is, 30 minutes to 24 hours before, more preferably 30 minutes to 12 hours before, still more preferably 1 hour to 6 hours before giving chemical or physical stress to the mammals, or after a predetermined time, that is, 30 minutes to 3 days after, preferably 1 hour to 2 days after, more preferably 1 hour to 24 hours after giving chemical or physical stress, or simultaneously with the chemical or physical stress, and quantifying and analyzing the amount of AQ27 receptor in a cell membrane after a predetermined time, that is, 30 minutes to 3 days after, preferably 1 hour to 2 days after, more preferably 1 hour to 24 hours after the administration, or

(ii) mixing a test compound with a medium for culturing the transformant in a usual manner and quantifying and analyzing the amount of AQ27 receptor in a cell membrane of the transformant after culture, that is, 1 to 7 days later, preferably 1 to 3 days later, more preferably 2 to 3 days later.

Specifically, AQ27 receptor contained in the cell membrane fraction is confirmed in the following manner.

(iii) Normal or morbid non-human mammals (for example, mice, rats, rabbits, sheep, swine, bovine, cats, dogs, monkeys etc., specifically rats with dementia, mice with obesity, rabbits with arteriosclerosis, mice bearing cancer, etc.) are given a chemical (for example, an anti-dementia drug, blood pressure depressant, anti-cancer drug, anti-obesity drug, etc.) or physical stress (for example, water immersion stress, electrical shock, brightening/darkening, low temperature, etc.), and after a predetermined time, blood, a specific organ (for example, brain, liver, kidney, etc.) or tissues or cells isolated from organs are obtained. The resulting organ, tissues or cells are formed in a usual manner into a tissue section and subjected to immune staining with the antibody of the present application. The degree of staining of the receptor protein in the cell surface layer is quantified to confirm the protein on the cell membrane, whereby the amount of AQ27 receptor on the cell membrane can be quantitatively or qualitatively.

(iv) AQ27 receptor can also be confirmed in an analogous manner by using the AQ27 receptor-expressing transformant etc.

The compound or its salt obtainable using the screening method of the present application is a compound having an action of altering the amount of AQ27 receptor in the cell membrane, and is specifically a compound (A) increasing the amount of AQ27 receptor in the cell membrane thereby enhancing cell-stimulating activities via G protein-conjugated receptor (e.g., the activity that promotes or suppresses arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.) or a compound (B) decreasing the amount of AQ27 receptor in the cell membrane thereby reducing the cells-stimulating activities.

The compound includes peptides, proteins, non-peptide compounds, synthetic compounds, fermentation products, etc., and these compounds may be novel compounds or known compounds.

The compound can be used as a safe and low toxic pharmaceutical preparation, for example, a hormone secretion regulator (for example, a regulator for secretion of adrenal cortical hormone such as corticosterone), an eating regulator, a sleep regulator, an arousal regulator, a pain regulator, a stress response regulator, a spontaneous behavior regulator or an emotional behavior regulator.

The compound increasing the amount of AQ27 receptor in the cell membrane thereby enhancing the cell-stimulating activities is useful as a safe and low toxic prophylactic/therapeutic agent for diseases related to deficiency in the functions of AQ27 receptor or as a promoter for secretion of adrenal cortical hormone.

The compound decreasing the amount of AQ27 receptor in the cell membrane thereby reducing the cell-stimulating activities is useful as a safe and low toxic prophylactic/therapeutic agent for diseases attributable to overexpression of AQ27 receptor or as an inhibitor for secretion of adrenal cortical hormone.

The diseases with which deficiency in the functions of AQ27 receptor include diseases in the central nerves (for example, Alzheimer's disease/dementia/difficulty in eating (anorexia)/epilepsy, etc.), diseases in the hormone system (for example, faint labor, flaccid bleeding, before and after placenta expulsion, insufficiency in uterine restoration, Caesarean operation, artificial abortion, milk statis etc.), diseases in liver/gall/pancreas/internal secretion (for example, diabetes/difficulty in eating, etc.), inflammatory diseases (allergy/asthma/rheumatism etc.), circulatory organ diseases (for example, high blood pressure/cardiac hypertrophy/angina/arteriosclerosis, etc.) etc.

The diseases attributable to overexpression of AQ27 include diseases caused by excessive production of AQ27 receptor, such as diseases in the central nerves, diseases in the hormone system, diseases in liver/gall/pancreas/internal secretion (for example, an anti-obesity drug/excessive eating, etc.), inflammatory diseases, circulatory organ diseases, etc.

When the compound or its salt obtained by using the screening method of the present application is used as a pharmaceutical composition, the compound or its salt can be formed into a pharmaceutical preparation in a usual manner.

The compound can be used orally, for example, in the form of tablets which may be sugar coated if necessary and desired, capsules, elixirs, microcapsules etc., or parenterally in the form of injectable preparations such as a sterile solution and a suspension in water or with other pharmaceutically acceptable liquid. These preparations can be manufactured by mixing the compound with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin, alginic acid, etc., a lubricant such as magnesium stearate, a sweetening agent such as sucrose, lactose or saccharin, and a flavoring agent such as peppermint, akamono oil or cherry. When the unit dosage is in the form of capsules, liquid carriers such as oils and fats may further used together with the additives described above. A sterile composition for injection may be formulated according to a conventional manner used to make pharmaceutical compositions, e.g., by dissolving or suspending the active ingredients in a vehicle such as water for injection with a naturally occurring vegetable oil such as sesame oil, coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil, soybean oil, etc., which may also be used in combination with a dissolution aid such as benzyl benzoate, benzyl alcohol, etc.

The prophylactic/therapeutic agent described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

The thus obtained pharmaceutical preparation is safe and low toxic, and can thus be administered to, for example, human and warm-blooded animals (e.g., rat, mouse, rabbit, sheep, swine, bovine, cat, dog, monkey, etc.).

The dose of the compound or its salt may vary depending on subject to be administered, target organ, symptom, administration method, etc. When the compound is orally administered, the compound is administered to a diabetic (as 60 kg) generally in a daily dose of approximately 0.1 mg to 100 mg, preferably approximately 1.0 mg to 50 mg, more preferably approximately 1.0 to 20 mg. When the compound is parenterally administered, a single dose of the compound may vary depending on subject to be administered, target organ, symptom, administration method etc. When the compound is administered to a diabetic (as 60 kg body weight) in the form of injection, it is convenient to administer the compound by intravenous injection, generally in a daily dose of approximately 0.01 to 30 mg, preferably approximately 0.1 to 20 mg, more preferably approximately 0.1 to 10 mg. For other animal species, the corresponding dose as converted per 60 kg weight can be administered. (6) Pharmaceutical preparation comprising the antibody to AQ27 receptor

The neutralizing activity of the antibody to AQ27 receptor means an activity of inactivating signal transmission functions in which the AQ27 receptor is involved. Accordingly, when the anti-body has a neutralizing activity, the antibody can inactivate signal transmission in which the AQ27 receptor is involved, for example, cell stimulating activities via the AQ27 receptor (e.g., the activity that promotes or suppresses arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.).

Accordingly, the neutralizing antibody to AQ27 receptor can be used as a safe and low toxic pharmaceutical preparation, for example, a hormone secretion regulator (for example, an inhibitor for secretion of adrenal cortical hormone such as corticosterone), an eating regulator, a sleep regulator, an arousal regulator, a pain regulator, a stress response regulator, a spontaneous behavior regulator or an emotional behavior regulator, or as a prophylactic/therapeutic agent for diseases attributable to overexpression of AQ27 receptor, for example, diseases caused by excessive production of the peptide F of the present application, such as diseases in the central nerves, diseases in the hormone system, diseases in liver/gall/pancreas/internal secretion (for example, an anti-obesity drug/excessive eating, etc.), inflammatory diseases, circulatory organ diseases etc.

(7) Pharmaceutical preparation comprising the antisense DNA of the present application

The antisense DNA of the present application can be used as a safe and low toxic pharmaceutical preparation, for example, a hormone secretion regulator (for example, an inhibitor for secretion of adrenal cortical hormone such as corticosterone), an eating regulator, a sleep regulator, an arousal regulator, a pain regulator, a stress response regulator, a spontaneous behavior regulator or an emotional behavior regulator or as a prophylactic/therapeutic agent for diseases attributable to overexpression of AQ27 receptor, for example diseases caused by excessive production of the peptide F of the present application, such as diseases in the central nerves, diseases in the hormone system, diseases in liver/gall/pancreas/internal secretion (for example, an anti-obesity drug/excessive eating, etc.), inflammatory diseases, circulatory organ diseases, etc.

Where the antisense DNA for example is used, the antisense DNA itself is inserted into an appropriate vector such as retrovirus vector, adenovirus vector, adenovirus-associated virus vector, etc. and then administered to human or other warm-blooded animal in a conventional manner. The antisense DNA may also be administered as an intact DNA, or prepared into pharmaceutical preparations together with physiologically acceptable carriers such as adjuvants to assist its uptake, which are administered by gene gun or through a catheter such as a hydrogel catheter.

Further, the antisense DNA can also be used as a diagnostic oligonucleotide probe for examining the presence of AQ27 receptor DNA in tissues or cells or its expression.

(8) Preparation of animals bearing the AQ27 receptor DNA

The present application discloses a non-human mammal having the extraneous AQ27 receptor DNA (hereinafter simply referred to as the extraneous DNA of the present application or its mutant DNA (sometimes simply referred to as the extraneous mutant DNA of the present application).

Thus, the present application discloses:

[1] a non-human mammal having the extraneous DNA of the present application or its mutant DNA;
[2] the mammal according to [1], wherein the non-human mammal is a rodent;
[3] the mammal according to [2]. wherein the rodent is mouse or rat; and
[4] a recombinant vector comprising the extraneous DNA of the present application or its mutant DNA and capable of expression in a mammal.

The non-human mammal having the extraneous DNA of the present application or its mutant DNA (hereinafter simply referred to as the AQ27 receptor DNA transgenic animal of the present application) can be created by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the extraneous DNA of the present application into a somatic cell, a living organ, a tissue cell or the like, by the DNA transfection methods, and utilize the transformant for cell culture, tissue culture, etc. In addition, these cells may be fused with the above-described germinal cell by a publicly known cell fusion method to create the AQ27 receptor DNA transgenic animal of the present application.

Examples of the non-human mammal that can be used include bovine, swine, sheep, goat, rabbits, dogs, cats, guinea pigs, hamsters, mice, rats and the like. Above all, preferred are rodents, especially mice (e.g., C57BL/6 strain, DBA2 strain, etc. for a pure line and for a cross line, $B6C3F_1$ strain, $BDF_1$ strain $B6D2F_1$ strain, BALB/c strain, ICR strain, etc.) or rats (Wistar, SD, etc.) and the like, since they are relatively short in ontogeny and life cycle from a standpoint of creating model disease animals, and are easy in bleeding.

"Mammals" in a recombinant vector that can be expressed in mammals include, in addition to the aforesaid non-human mammals, human, etc.

The extraneous DNA of the present application refers to the AQ27 receptor DNA of the present application that is once isolated and extracted from mammals, not the AQ27 receptor DNA of the present application inherently had by the non-human mammals.

The mutant DNA of the present application includes mutants resulting from variation (e.g., mutation, etc.) in the nucleotide sequence of the original AQ27 receptor DNA of the present application, specifically DNAs resulting from base addition, deletion, substitution with other bases, etc. and further including abnormal DNA.

The abnormal DNA is intended to mean the DNA that expresses the abnormal AQ27 receptor and exemplified by such a DNA that expresses AQ27 receptor suppressing the functions of the normal AQ27 receptor, or the like.

The extraneous DNA of the present application may be any one of those derived from a mammal of the same species as, or a different species from, the mammal as the target animal. In transfecting the AQ27 receptor DNA to the target animal, it is generally advantageous to use the DNA as a DNA construct in which the DNA is ligated downstream a promoter capable of expressing the DNA in the target animal. For example, in the case of transfecting the human DNA of the present application, a DNA transgenic mammal that expresses the DNA of the present application to a high level, can be prepared by microinjecting a DNA construct (e.g., vector, etc.) ligated with the human DNA of the present application into a fertilized egg of the target mammal, e.g., a fertilized egg of mouse, downstream the various promoters capable of expressing the DNA derived from various mammals (e.g., rabbits, dogs, cats, guinea pigs, hamsters, rats, mice, etc.) having the AQ27 receptor DNA highly homologous to the human DNA.

As expression vectors for AQ27 receptor, there are Escherichia coli-derived plasmids, Bacillus subtilis-derived plasmids, yeast-derived plasmids, bacteriophages such as $\lambda$ phage, etc., retroviruses such as Moloney leukemia virus, etc., animal viruses such as vaccinia virus, baculovirus, etc. Of these vectors, Escherichia coli-derived plasmids, Bacillus subtilis-derived plasmids, yeast-derived plasmids, etc. are preferably used.

Examples of these promoters for regulating the DNA expression include [1] promoters for DNA derived from viruses (e.g., simian virus, cytomegalovirus, Moloney leukemia virus, JC virus, breast cancer virus, poliovirus, etc.), and [2] promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor $\beta$, keratins K1, K10 and K14, collagen types I and II, cyclic AMP-dependent protein kinase $\beta$I subunit, dystrophin, tartarate-resistant alkaline phosphatase, atrial natriuretic factor, endothelial receptor tyrosine kinase (generally abbreviated as Tie2), sodium-potassium adenosine triphosphorylase (Na, K-ATPase), neurofilament light chain, metallothioneins I and IIA, metalloproteinase I tissue inhibitor, MHC class I antigen (H-2L), H-ras, renin, dopamine $\beta$-hydroxylase, thyroid peroxidase (TPO), peptide chain elongation factor 1$\alpha$ (EF-1$\alpha$), $\beta$ actin, $\alpha$ and $\beta$ myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human peptide elongation factor 1α (EF-1α) promoters, human and chicken β actin promoters etc., which can achieve high expression in the whole body, are preferred.

It is preferred that the vectors described above have a sequence for terminating the transcription of the desired messenger RNA in the DNA transgenic animal (generally called a terminator); for example, a sequence of each DNA derived from viruses and various mammals. SV40 terminator of the simian virus, etc. are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The normal translational region of AQ27 receptor can be prepared as the whole or a part of genomic DNA from DNA derived from liver, kidney, thyroid cells, fibroblasts etc. derived from humans or mammals (for example, rabbit, dog, cat, guinea pig, hamster, rat, mouse etc.) and a wide variety of commercial DNA libraries, or from complementary DNA as a starting material prepared by a known method from RNA derived from liver, kidney, thyroid cells, fibroblasts etc. As the extraneous abnormal DNA, a translational region can be prepared by point mutation of the normal translational region of AQ27 receptor obtained from the above cells or tissues.

The translational region can be prepared, as a DNA construct capable of being expressed in the transgenic animal, by a conventional DNA engineering technique, in which the DNA is ligated downstream the aforesaid promoter and if desired, upstream the translation termination site.

The extraneous DNA of the present application is transfected at the fertilized egg cell stage in a manner such that the DNA is certainly present in all the germinal cells and somatic cells of the target mammal. The fact that the extraneous DNA of the present application is present in the germinal cells of the animal prepared by DNA transfection means that all offspring of the prepared animal will maintain the extraneous DNA of the present application in all of the germinal cells and somatic cells thereof. The offspring of the animal that inherits the extraneous DNA of the present application also have the extraneous DNA of the present application in all of the germinal cells and somatic cells thereof.

The non-human mammal, in which the normal extraneous DNA of the present application has been transfected can be passaged as the DNA-bearing animal under ordinary rearing environment, by confirming that the extraneous DNA is stably retained by mating.

By the transfection of the extraneous DNA of the present application at the fertilized egg cell stage, the DNA is retained to be excess in all of the germinal and somatic cells of the target mammal. The fact that the extraneous DNA of the present application is excessively present in the germinal cells of the prepared animal after transfection means that all of the offspring of the animal prepared have the extraneous DNA of the present application excessively in all of the germinal cells and somatic cells thereof. The offspring of the animal of this kind that inherits the extraneous DNA of the present application excessively have the DNA of the present application in all of the germinal cells and somatic cells thereof.

By obtaining a homozygotic animal having the transfected DNA in both of homologous chromosomes and mating a male and female of the animal, all offspring can be passaged to excessively have the DNA.

In a non-human mammal having the normal DNA of the present application, the normal DNA of the presents application is expressed to a high level, and may eventually develop the hyperfunction of AQ27 receptor by promoting the functions of endogenous normal DNA. Therefore, the animal can be utilized as a pathologic model animal for such a disease. Specifically, using the normal DNA transgenic animal of the present application, it becomes possible to elucidate the hyperfunction of AQ27 receptor and to clarify the pathological mechanism of the disease associated with AQ27 receptor and to determine how to treat these diseases.

Furthermore, since a mammal transfected the extraneous normal DNA of the present application has an increasing symptom of the liberated AQ27 receptor, the animal is usable for screening of therapeutic agents agent for the disease associated with AQ27 receptor.

On the other hand, non-human mammal having the extraneous abnormal DNA of the present application can be passaged under normal breeding conditions as the DNA-bearing animal by confirming the stable retaining of the extraneous DNA via crossing. In addition, the objective extraneous DNA can be utilized as a starting material by inserting the objective extraneous DNA into the plasmid described above. The DNA construct with a promoter can be prepared using conventional DNA engineering techniques. The transfection of the abnormal DNA of the present application at the fertilized egg cell stage is preserved to be present in all of the germinal and somatic cells of the mammals to be targeted. The fact that the abnormal DNA of the present application is present in the germinal cells of the animal after DNA transfection means that all of the offspring of the prepared animal have the abnormal DNA of the present application in all of the germinal and somatic cells. The offspring of such an animal that inherits the extraneous DNA of the present application has the abnormal DNA of the present application in all the germinal and somatic cells. A homozygous animal having the introduced DNA on both of homologous chromosomes can be acquired and then by mating these male and female animals, all the offspring can be bred to have the DNA.

Since the non-human mammal having the abnormal DNA of the present application expresses the abnormal DNA of the present application at a high level, the animal may cause the function inactive type inadaptability of AQ27 receptor by inhibiting the functions of the endogenous normal DNA, and can be utilized as its disease model animal. For example, using the abnormal DNA-transferred animal of the present application, it is possible to elucidate the mechanism of the function inactive type inadaptability of AQ27 receptor and to study a method for treatment of this disease.
In its specific applicability, the transgenic animal of the present application expressing the abnormal DNA of the present application to a high level is also expected to serve as an experimental model for the elucidation of the mechanism of the functional inhibition (dominant negative effect) of normal AQ27 receptor by the abnormal AQ27 receptor of the present application in the function inactive type inadaptability of AQ27 receptor.
A mammal bearing the abnormal extraneous DNA of the present application is also expected to serve for screening a candidate drug for the treatment of the function inactive type inadaptability of AQ27 receptor, since AQ27 receptor is increased in such an animal in its free form.
Other potential applicability of the two kinds of the AQ27 receptor transgenic animals described above includes:

[1] use as a cell source for tissue culture;
[2] elucidation of the association with AQ27 receptor that is specifically expressed or activated by AQ27 receptor, through direct analysis of DNA or RNA in tissue of the AQ27 receptor DNA transgenic animal or by analysis of the AQ27 receptor tissue expressed by the DNA;
[3] research in the function of cells derived from tissues that are cultured usually only with difficulty, using cells of tissue having the DNA cultured by a standard tissue culture technique;
[4] screening for a drug that enhances the functions of cells using the cells described in [3] above; and,
[5] isolation and purification of the variant AQ27 receptor of the present application and preparation of an antibody thereto.

Furthermore, clinical conditions of a disease associated with AQ27 receptor, including the function inactive type inadaptability of AQ27 receptor can be determined using the AQ27 receptor DNA transgenic animal of the present application. Also, pathological findings on each organ in a disease model associated with AQ27 receptor can be obtained in more detail, leading to the development of a new method for treatment as well as the research and therapy of any secondary diseases associated with the disease.
It is also possible to obtain a free DNA-transfected cell by withdrawing each organ from the AQ27 receptor DNA transgenic animal, mincing the organ and degrading with a proteinase such as trypsin, etc., followed by establishing the line of culturing or cultured cells. Furthermore, the DNA transgenic animal of the present application can serve as identification of cells capable of producing AQ27 receptor, and as studies on association with apoptosis, differentiation or propagation or on the mechanism of signal transduction in these properties to inspect any abnormality therein. Thus, the AQ27 receptor DNA transgenic animal can provide an effective research material for AQ27 receptor and for elucidating the function and effect thereof.
To develop pharmaceuticals for the treatment of diseases associated with AQ27 receptor, including the function inactive type inadaptability of AQ27 receptor, using the AQ27 receptor DNA transgenic animal, an effective and rapid method for screening the pharmaceuticals for the treatment of diseases can be provided by using the method for inspection and the method for quantification, etc. described above. It is also possible to investigate and develop a method for DNA therapy for the treatment of diseases associated with AQ27 receptor, using the AQ27 receptor DNA transgenic animal or a vector capable of expressing the extraneous DNA of the present application.

**(9) Knockout animal**

The present application disclsoes a non-human mammal embryonic stem cell bearing the AQ27 receptor DNA inactivated and a non-human mammal deficient in expressing the AQ27 receptor DNA.
Thus, the present application discloses:

[1] a non-human mammal embryonic stem cell in which the AQ27 receptor DNA is inactivated; .
[2] the embryonic stem cell according to [1], wherein the DNA is inactivated by introducing a reporter gene (e.g., β-galactosidase gene derived from Escherichia coli);
[3] the embryonic stem cell according to [1], which is resistant to neomycin;
[4] the embryonic stem cell according to [1], wherein the non-human mammal is a rodent;
[5] the embryonic stem cell according to [4], wherein the rodent is mouse;
[6] a non-human mammal deficient in expressing AQ27 receptor DNA, wherein the DNA is inactivated;
[7] the non-human mammal according to [6], wherein the DNA is inactivated by inserting a reporter gene (e.g., β-galactosidase derived from Escherichia coli) therein and the reporter gene is capable of being expressed under the control of a promoter for the AQ27 receptor DNA;
[8] the non-human mammal according to [6], which is a rodent;
[9] the non-human mammal according to [8], wherein the rodent is mouse; and,
[10] a method for screening a compound or its salt that promotes or inhibits the promoter activity for AQ27 receptor DNA, which comprises administering a test compound to the mammal of [7] and detecting expression of the reporter gene.

The non-human mammalian embryonic stem cell, in which the AQ27 receptor DNA is inactivated, refers to a non-human mammalian embryonic stem cell that suppresses the ability of the non-human mammalian to express the DNA by artificially mutating the AQ27 receptor DNA had in the non-human mammal, or the DNA has no substantial ability to express the AQ27 receptor (hereinafter sometimes referred to as the knockout DNA of the present application by substantially inactivating the activities of the AQ27 receptor encoded by the DNA (hereinafter merely referred to as ES cell).
As the non-human mammalian, the same examples as described above apply.
Techniques for artificially mutating the AQ27 receptor DNA include deletion of a part or all of the DNA sequence and insertion of or substitution with other DNA, e.g., by genetic engineering. By these variations, the knockout DNA of the present application may be prepared, for example, by shifting the reading frame of a codon or by disrupting the function of a promoter or exon.
Specifically, the non-human mammalian embryonic stem cell, in which the AQ27 receptor DNA is inactivated (hereinafter merely referred to as the ES cell with the AQ27 receptor DNA inactivated or the knockout ES cell of the present application), can be obtained by, for example, isolating the AQ27 receptor DNA had by the target non-human mammal, inserting a DNA strand (hereinafter simply referred to as targeting vector) having a DNA sequence constructed so as to eventually destroy the gene by inserting into its exon site a chemical resistant gene such as a neomycin resistant gene or a hygromycin resistant gene, or a reporter gene such as lacZ (β-galactosidase gene) or cat (chloramphenicol acetyltransferase gene), etc. thereby destroying the functions of exon, or by inserting into the intron site between exons a DNA sequence which terminates gene transcription (e.g., polyA-added signal, etc.) thereby disabling the synthesis of complete messenger RNA, into a chromosome of the animal cells by, e.g., homologous recombination. The thus obtained ES cells are analyzed by the Southern hybridization using as a probe a DNA sequence on or near the AQ27 receptor DNA, or by PCR using as primers a DNA sequence on the targeting vector and another DNA sequence near the AQ27 receptor DNA which is not included in the targeting vector, and the knockout ES cell of the present application is selected.
The parent ES cells to inactivate the AQ27 receptor DNA by homologous recombination, etc. may be of a strain already established as described above, or may be originally established in accordance with a modification of the known method by Evans and Kaufman supra. For example, in the case of mouse ES cells, currently it is common practice to use ES cells of the 129 strain. However, since their immunological background is obscure, the C57BL/6 mouse or the $BDF_1$ mouse ($F_1$ hybrid between C57BL/6 and DBA/2), wherein the low ovum collection per C57BL/6 mouse or C57BL/6 has been improved by crossing with DBA/2, may be preferably used, instead of obtaining a pure line of ES cells with the clear immunological genetic background. The $BDF_1$ mouse is advantageous in that when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.
In establishing ES cells, blastocytes of 3.5 days after fertilization are commonly used. A large number of early stage embryos may be acquired more efficiently, by collecting the embryos of the 8-cell stage and using the same after culturing until the blastocyte stage.
Although the ES cells used may be of either sex, male ES cells are generally more convenient for generation of a germ cell line chimera and are therefore preferred. It is desirable to identify sexes as soon as possible also in order to save painstaking culture time.
As an example of the method for sex identification of the ES cell, mention may be made of a method in which a gene in the sex-determining region on the Y-chromosome is amplified by PCR and detected. When this method is used, ES cells (about 50 cells) corresponding to almost 1 colony are sufficient, whereas karyotype analysis hitherto required about $10^6$ cells; therefore, the first selection of ES cells at the early stage of culture can be based on sex identification, and male cells can be selected early, which saves a significant amount of time at the early stage of culture.
Second selection can be achieved by, for example, number of chromosome confirmation by the G-banding method. It is usually desirable that the chromosome number of the obtained ES cells be 100% of the normal number. However, when it is difficult to obtain the cells having the normal number of chromosomes due to physical operation etc. in cell establishment, it is desirable that the ES cell be again cloned to a normal cell (e.g., in mouse cells having the number of chromosomes being 2n = 40) after the gene of the ES cells is rendered knockout.
Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and about 90% air) in the presence of LIF (1 to 10000 U/ml) on appropriate feeder cells such as STO fibroblasts, treated with a trypsin/EDTA solution (normally 0.001 to 0.5% trypsin/0.1 to 5 mM EDTA, preferably 0.1 % trypsin/1 mM EDTA) at the time of passage to obtain separate single cells, which are then seeded on freshly prepared feeder cells. This passage is normally conducted every 1 to 3 days; it is desirable that cells be observed at passage and cells found to be morphologically abnormal in culture, if any, be abandoned.

By allowing ES cells to reach a high density in mono-layers or to form cell aggregates in suspension under appropriate conditions, it is possible to differentiate them to various cell types, for example, parietal and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the AQ27 receptor DNA, which are obtainable from the differentiated ES cells of the present application, are useful for studying the functions of AP27 receptor in vitro cytologically or molecular biologically.

The non-human mammal deficient in expression of the AQ27 receptor DNA can be identified from a normal animal by measuring the amount of mRNA in the subject animal by a publicly known method, and indirectly comparing the levels of expression.

As the non-human mammal, the same examples supra apply.

With respect to the non-human mammal deficient in expression of the AQ27 receptor DNA, the AQ27 receptor DNA can be made knockout by transfecting a targeting vector, prepared as described above, to mouse embryonic stem cells or mouse oocytes thereof, and conducting homologous recombination in which a targeting vector DNA sequence, wherein the AQ27 receptor DNA is inactivated by the transfection, is replaced with the AQ27 receptor DNA on a chromosome of a mouse embryonic stem cell or mouse oocyte.

The cells with the AQ27 receptor DNA in which the AQ27 receptor DNA is rendered knockout can be identified by the Southern hybridization analysis using as a probe a DNA sequence on or near the AQ27 receptor DNA, or by PCR analysis using as primers a DNA sequence on the targeting vector and another DNA sequence which is not included in the AQ27 receptor DNA derived from mouse, which is used as the targeting vector. When non-human mammalian embryonic stem cells are used, the cell line wherein the AQ27 receptor DNA is inactivated is cloned by homologous recombination; the resulting cloned cell line is injected to, e.g., a non-human mammalian embryo or blastocyte, at an appropriate stage such as the 8-cell stage. The resulting chimeric embryos are transplanted to the uterus of the pseudopregnant non-human mammal. The resulting animal is a chimeric animal composed of both cells having the normal locus of the AQ27 receptor DNA and those having an artificially mutated locus of the AQ27 receptor DNA.

When some germ cells of the chimeric animal have a mutated locus of the AQ27 receptor DNA, an individual, in which all tissues are composed of cells having an artificially mutated locus of the AQ27 receptor DNA, can be selected from a series of offspring obtained by crossing between such a chimeric animal and a normal animal, e.g., by coat color identification, etc. The individuals thus obtained are normally deficient in heterozygous expression of the AQ27 receptor. The individuals deficient in homozygous expression of the AQ27 receptor can be obtained from offspring of the intercross between the heterozygotes.

When an oocyte is used, a DNA solution may be injected, e.g., to the prenucleus by microinjection thereby to obtain a transgenic non-human mammal having a targeting vector introduced into its chromosome. From such transgenic non-human mammals, those having a mutation at the locus of the AQ27 receptor DNA can be obtained by selection based on homologous recombination.

As described above, individuals wherein the AQ27 receptor DNA is rendered knockout permit passage rearing under ordinary rearing conditions, after it is confirmed that in the animal individuals obtained by their crossing, the DNA has been knockout.

Furthermore, the genital system may be obtained and maintained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammalian embryonic stem cell, in which the AQ27 receptor DNA is inactivated, is very useful for preparing a non-human mammal deficient in expression of the AQ27 receptor DNA.

Since the non-human mammal, in which the AQ27 receptor DNA fails to express, lacks various biological activities induced by the AQ27 receptor, such an animal can be a disease model suspected of inactivated biological activities of the AQ27 receptor and thus, offers an effective study to investigate causes for and therapy for these diseases.

(9a) Method for screening of compounds having therapeutic/prophylactic effects for diseases caused by deficiency, damages, etc. of the AQ27 receptor DNA

The non-human mammal deficient in expression of the AQ27 receptor DNA can be used to screen compounds having therapeutic/prophylactic effects for diseases caused by deficiency, damages, etc. of the AQ27 receptor DNA.

That is, the present application discloses a method for screening of a compound or its salt having therapeutic/prophylactic effects for diseases caused by deficiency, damages, etc. of the AQ27 receptor DNA, which comprises administering a test compound to the non-human mammal deficient in expression of the AQ27 receptor DNA, and observing and measuring a change having occurred in the animal.

As the non-human mammal deficient in expression of the AQ27 receptor DNA used for the screening method, the same examples as given hereinabove apply.

Examples of the test compounds include peptides, proteins, non-peptide compounds, synthetic compounds, fermentation products, cell extracts, plant extracts, animal tissue extracts, blood plasma, etc. and these compounds may be novel compounds or publicly known compounds.

Specifically, the non-human mammal deficient in the expression of the AQ27 receptor DNA is treated with a test compound, comparison is made with an intact animal for control and a change in each organ, tissue, disease conditions, etc. of the animal is used as an indicator to assess the therapeutic/prophylactic effects of the test compound.

For treating an animal to be tested with a test compound, for example, oral administration, intravenous injection, etc. are applied and the treatment is appropriately selected depending upon conditions of the test animal, properties of the test compound, etc. Furthermore, the amount of a test compound administered can be appropriately selected depending on administration route, nature of the test compound, or the like.

In the screening method, a test compound can be selected as a compound having a therapeutic/prophylactic effect on the disease when the blood sugar level of a test animal upon administering the test compound is reduced by preferably at least about 10%, preferably at least about 30%, more preferably at least about 50%.

The compound obtainable using the screening method is a compound selected from the above test compounds, and is useful as a safe and low toxic prophylactic/therapeutic agent for diseases caused by deficiency or damage of AQ27 receptor (for example, diseases in the central nerves (for example, Alzheimer's disease/dementia/difficulty in eating (anorexia)/epilepsy, etc.), diseases in the hormone system (for example, faint labor, flaccid bleeding, before and after placenta expulsion, insufficiency in uterine restoration, Caesarean operation, artificial abortion, milk statis etc.), diseases in liver/gall/pancreas/internal secretion (for example, diabetes/difficulty in eating, etc.), inflammatory diseases (allergy/asthma/rheumatism etc.), circulatory organ diseases (for example, high blood pressure/cardiac hypertrophy/angina/arteriosclerosis, etc.)), a hormone secretion regulator (for example, a promoter for secretion of adrenal cortical hormone such as corticosterone), an eating regulator, a sleep regulator, an arousal regulator, a pain regulator, a stress response regulator, a spontaneous behavior regulator or an emotional behavior regulator. Further, compounds derived from the compound obtained by the aforesaid screening can also be used in a similar manner.

The compound obtained in the screening method may form a salt, and the salt of the compound may be used in the form of salts with physiologically acceptable acids (e.g., inorganic acids or organic acids) or bases (e.g., alkali metals etc.), preferably in the form of physiologically acceptable acid addition salts. Examples of such salts are salts with inorganic acids (e.g., hydrochloric acid, phosphoric acid, hydrobromic acid, sulfuric acid etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid etc.) and the like.

The pharmaceutical preparation comprising the compound or its salt obtained by the screening method can be produced in the same manner as for the pharmaceutical preparation comprising the compound altering the binding property between the AQ27 receptor and the ligand.

Since the thus obtained pharmaceutical preparation is safe and low toxic, and can be administered to, for example, human and warm-blooded animals (e.g., rat, mouse, guinea pig, rabbit, sheep, swine, bovine, horse, cat, dog, monkey, etc.).

The dose of the compound or its salt may vary depending on target disease, subject to be administered, route for administration, etc. When the compound is orally administered, the compound is administered to a diabetic (as 60 kg) generally in a daily dose of approximately 0. 1 mg to 100 mg, preferably approximately 1.0 mg to 50 mg, more preferably approximately 1.0 to 20 mg. When the compound is parenterally administered, a single dose of the compound may vary depending on subject to be administered, target disease etc. When the compound is administered to a diabetic (as 60 kg body weight) in the form of injection, it is convenient to administer the compound by intravenous injection, generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

(9b) Method of screening a compound that promoters or inhibits the activity of a promoter for AQ27 receptor DNA

The present application discloses a method of screening a compound or its salt that promotes or inhibits the activity of a promoter for AQ27 receptor DNA, which comprises administering a test compound into a non-human mammal deficient in expression of AQ27 receptor DNA and detecting the expression of a reporter gene.

In the screening method, the non-human mammal deficient in expression of AQ27 receptor DNA is particularly a non-human mammal deficient in expression of AQ27 receptor DNA, wherein the AQ27 receptor DNA is inactivated by introducing a reporter gene, and the reporter gene can be expressed under the control of a promoter for AQ27 receptor DNA.

The test compound includes those described above.

The reporter gene used is the same as described above, and is preferably β-galactosidase gene (lacZ), soluble alkali phosphatase gene or luciferase gene.

In the non-human mammal deficient in expression of AQ27 reporter DNA wherein AQ27 receptor DNA is substituted with a reporter gene, the reporter gene is present under the control of a promoter for AQ27 receptor DNA, and thus the activity of the promoter can be detected by tracing the expression of a substance encoded by the reporter gene.

For example, when a part of the DNA region encoding AQ27 receptor is substituted with β-galactosidase gene (lacZ) derived from E. coli, β-galactosidase is expressed in place of AQ27 receptor, in tissues originally expressing AQ27 receptor. Thus, the expression state of AQ27 receptor can be readily observed in vivo in an animal, by staining with a reagent, e.g., 5-bromo-4-chloro-3-indolyl-β-D-galactopyranoside " (X-gal), which is a substrate for β-galactosidase. Specifically, a mouse deficient in AQ27 receptor, or its tissue section, is fixed with glutaraldehyde, etc. After washing with phosphate buffered saline (PBS), the system is reacted with a staining solution containing X-gal at room temperature or about 37°C for approximately 30 minutes to 1 hour. After the β-galactosidase reaction is terminated by washing the tissue preparation with 1 mM EDTA/PBS solution, the color formed is observed. Alternatively, mRNA encoding lacZ may be detected in a conventional manner.

The compound or its salt obtained by using the above screening method is a compound selected from the test compounds described above, and is a compound promoting or inhibiting the activity of a promoter for AQ27 receptor DNA.

The compound obtained in the screening method may form a salt, and the salt of the compound may be used in the form of salts with physiologically acceptable acids (e.g., inorganic acids etc.) or bases (e.g., organic acids [sic] etc.), preferably in the form of physiologically acceptable acid addition salts. Examples of such salts are salts with inorganic acids (e.g., hydrochloric acid, phosphoric acid, hydrobromic acid, sulfuric acid etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid etc.) and the like.

The compound or its salt that promotes or inhibits the activity of a promoter for AQ27 receptor DNA is useful as a safe and low toxic pharmaceutical preparation, for example, a hormone secretion regulator (for example, a regulator for secretion of adrenal cortical hormone such as corticosterone), an eating regulator, a sleep regulator, an arousal regulator, a pain regulator, a stress response regulator, a spontaneous behavior regulator or an emotional behavior regulator.

A compound or its salt promoting the activity of a promoter for AQ27 receptor DNA can promote expression of AQ27 receptor and promote the functions of AQ27 receptor, and is thus useful as a prophylactic/therapeutic agent for diseases with which deficiency in the functions of AQ27 receptor is associated, or as a pharmaceutical preparation such as an agent for promoting secretion of adrenal cortical hormone, etc.

A compound or its salt inhibiting the activity of a promoter for AQ27 receptor DNA can inhibit expression of AQ27 receptor and inhibit the functions of AQ27 receptor, and is thus useful as a prophylactic/therapeutic agent for diseases with which overexpression of AQ27 receptor is associated, or as a pharmaceutical preparation such as an agent for inhibiting secretion of adrenal cortical hormone, etc.

The diseases with which deficiency in the functions of AQ27 receptor include diseases in the central nerves (for example, Alzheimer's disease/dementia/difficulty in eating (anorexia)/epilepsy, etc.), diseases in the hormone system (for example, faint labor, flaccid bleeding, before and after placenta expulsion, insufficiency in uterine restoration, Caesarean operation, artificial abortion, milk statis etc.), diseases in liver/gall/pancreas/internal secretion (for example, diabetes/difficulty in eating, etc.), inflammatory diseases (allergy/asthma/rheumatism etc.), circulatory organ diseases (for example, high blood pressure/cardiac hypertrophy/angina/arteriosclerosis, etc.) etc.

The diseases attributable to overexpression of AQ27 receptor include, for example, diseases caused by excessive production of the peptide F of the present application, such as diseases in the central nerves, diseases in the hormone system, diseases in liver/gall/pancreas/internal secretion (for example, an anti-obesity drug/excessive eating, etc.), inflammatory diseases, circulatory organ diseases etc.

Further, a compound derived from the compound obtained in the above screening can also be used similarly.

The pharmaceutical preparation comprising the compound or its salt obtained by the screening method can be produced in the same manner as for the pharmaceutical preparation comprising the compound altering the binding property between the AQ27 receptor or its salt and the ligand.

Since the thus obtained pharmaceutical preparation is safe and low toxic, and can be administered to, for example, human and warm-blooded animals (e.g., rat, mouse, guinea pig, rabbit, sheep, swine, bovine, horse, cat, dog, monkey, etc.).

The dose of the compound or its salt may vary depending on target disease, subject to be administered, route for administration, etc. When the compound promoting the activity of a promoter for AQ27 receptor DNA is orally administered, the compound is administered to a diabetic (as 60 kg) generally in a daily dose of approximately 0.1 mg to 100 mg, preferably approximately 1.0 mg to 50 mg, more preferably approximately 1.0 to 20 mg. When the compound is parenterally administered, a single dose of the compound may vary depending on subject to be administered, target disease, etc. When the compound promoting the activity of a promoter for AQ27 receptor DNA is administered to a diabetic (as 60 kg body weight) in the form of injection, it is convenient to administer the compound by intravenous injection, generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

Thus, the non-human mammal deficient in expression of AQ27 receptor DNA is extremely useful in screening a compound or its salt that promotes or inhibits the activity of a promoter for AQ27 receptor DNA, and can contribute significantly to elucidation of causes for various diseases attributable to deficient expression of AQ27 receptor DNA and development of a prophylactic/therapeutic agent for the diseases.

Further, genes encoding various proteins are ligated downstream DNA comprising a promoter region of AQ27 receptor and injected into a fertilized egg of an animal to create a transgenic animal, whereby the AQ27 receptor can be specifically synthesized and examined for its action in the living body. When a suitable reporter gene is ligated to the promoter region to establish a cell strain expressing the same, the cell strain can be used as a system of searching for a low-molecular compound having an action of specifically promoting or suppressing the ability of the cell strain to produce AQ27 receptor in vivo.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA : | deoxyribonucleic acid |
| cDNA : | complementary deoxyribonucleic acid |
| A : | adenine |
| T : | thymine |
| G : | guanine |
| C : | cytosine |
| I : | inosine |
| R : | adenine (A) or guanine (G) |
| Y : | thymine (T) or cytosine (C) |
| M : | adenine (A) or cytosine (C) |
| K : | guanine (G) or thymine (T) |
| S : | guanine (G) or cytosine (C) |
| W : | adenine (A) or thymine (T) |
| B : | guanine (G), guanine (G) or thymine (T) |
| D : | adenine (A), guanine (G) or thymine (T) |
| V : | adenine (A), guanine (G) or cytosine (C) |
| N : | adenine (A), guanine (G), cytosine (C) or thymine (T), or unrevealed or other base |

| | |
|---|---|
| RNA : | ribonucleic acid |
| mRNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP : | adenosine triphosphate |
| EDTA : | ethylenediaminetetraacetic acid |
| SDS : | sodium dodecyl sulfate |
| BHA : | benzhydrylamine |
| pMBHA: | p-methylbenzhydrylamine |
| Tos : | p-toluenesulfonyl |
| Bzl : | benzyl |
| Bom : | benzyloxymethyl |
| Boc : | t-butyloxycarbonyl |
| DCM : | dichloromethane |
| HOBt : | 1-hydroxybenztriazole |
| DCC : | N,N'-dicyclohexylcarbodiimide |
| TFA : | trifluoroacetic acid |
| DIEA : | diisopropylethylamine |
| Gly or G: | glycine |
| Ala or A: | alanine |
| Val or V : | valine |
| Leu or L: | leucine |
| Ile or I : | isoleucine |
| Ser or S : | serine |
| Thr or T : | threonine |
| Cys or C: | cysteine |
| Met or M: | methionine |

Glu or E: glutamic acid

Asp or D: aspartic acid

Lys or K: lysine

Arg or R: arginine

His or H: histidine

Phe or F : phenylalanine

Tyr or Y : tyrosine

Trp or W: tryptophan

Pro or P : proline

Asn or N: asparagine

Gln or Q: glutamine

pGlu : pyroglutamic acid

Tyr (I) : 3-iodotyrosine

DMF : N,N-dimethylformamide

Fmoc : N-9-fluorenylmethoxycarbonyl

Trt : trityl

Pbf : 2,2,4,6,7-pentamethyldihydrobenzofuan-5-sulfonyl

Clt : 2-chlorotrityl

$Bu^t$ : t-butyl

Met(O) : methioninesulfoxide

The sequence identification numbers in the sequence listing of the specification indicates the following sequence, respectively.

[SEQ ID NO: 1]

This shows the amino acid sequence of secretory peptide A.

[SEQ ID NO: 2]

This shows the amino acid sequence of precursor protein A.

[SEQ ID NO: 3]

This shows the amino acid sequence of precursor protein B.

[SEQ ID NO: 4]

This shows the amino acid sequence of precursor protein C.

[SEQ ID NO: 5]

This shows the amino acid sequence of precursor protein D.

[SEQ ID NO: 6]

This shows the amino acid sequence of precursor protein E.

[SEQ ID NO: 7]

This shows the nucleotide sequence of DNA encoding secretory peptide A.

[SEQ ID NO: 8]

This shows the nucleotide sequence of DNA encoding precursor protein A.

[SEQ ID NO: 9]

This shows the nucleotide sequence of DNA encoding precursor protein B.

[SEQ ID NO: 10]

This shows the nucleotide sequence of DNA encoding precursor protein C.

[SEQ ID NO: 11]

This shows the nucleotide sequence of DNA encoding precursor protein D.

[SEQ ID NO: 12]

This shows the nucleotide sequence of DNA encoding precursor protein E.

[SEQ ID NO: 13]

This shows the synthetic DNA employed in screening cDNA encoding precursor protein A in Example 1.

[SEQ ID NO: 14]

This shows the synthetic DNA employed in screening cDNA encoding precursor protein A in Example 1.

[SEQ ID NO: 15]

This shows the synthetic DNA employed in screening cDNA encoding precursor protein B in Example 2.

[SEQ ID NO: 16]

This shows the synthetic DNA employed in screening cDNA encoding precursor protein B in Example 2.

[SEQ ID NO: 17]

This shows the synthetic DNA employed in screening cDNA encoding precursor protein C in Example 3.

[SEQ ID NO: 18]

This shows the synthetic DNA employed in screening cDNA encoding precursor proteins C in Example 3.

[SEQ ID NO: 19]

This shows the synthetic DNA employed in screening cDNA encoding precursor protein D in Example 4.

[SEQ ID NO: 20]

This shows the synthetic DNA employed in screening cDNA encoding precursor protein D in Example 4.

[SEQ ID NO: 21]

This shows the synthetic DNA employed in screening cDNA encoding precursor protein E in Example 5.

[SEQ ID NO: 22]

This shows the synthetic DNA employed in screening cDNA encoding precursor protein E in Example 5.

[SEQ ID NO: 23]

This shows the amino acid sequence of precursor protein F.

[SEQ ID NO: 24]

This shows the nucleotide sequence of DNA encoding precursor protein F.

[SEQ ID NO: 25]

This shows the synthetic DNA employed in screening cDNA encoding precursor protein F in Example 6.

[SEQ ID NO: 26]

This shows the synthetic DNA employed in screening cDNA encoding precursor protein F in Example 6.

[SEQ ID NO: 27]

This shows the amino acid sequence of precursor protein G.

[SEQ ID NO: 28]

This shows the nucleotide sequence of DNA encoding precursor protein G.

[SEQ ID NO: 29]

This shows the synthetic DNA employed in screening cDNA encoding precursor protein G in Example 7.

[SEQ ID NO: 30]

This shows the synthetic DNA employed in screening cDNA encoding precursor protein G in Example 7.

[SEQ ID NO: 31]

This shows the amino acid sequence of human AQ27 receptor.

[SEQ ID NO: 32]

This shows the nucleotide sequence of DNA encoding human AQ27 receptor.

[SEQ ID NO: 33]

This shows the amino acid sequence of rat OT7T022 receptor.

[SEQ ID NO: 34]

This shows the nucleotide sequence of DNA encoding rat OT7T022 receptor.

[SEQ ID NO: 35]

This shows the amino acid sequence of human OT7T022 receptor.

[SEQ ID NO: 36]

This shows the nucleotide sequence of DNA encoding human OT7T022 receptor.

[SEQ ID NO: 37]

This shows the nucleotide sequence of DNA encoding human OT7T022 receptor.

[SEQ ID NO: 38]

This shows the amino acid sequence of precursor protein H.

[SEQ ID NO: 39]

This shows the nucleotide sequence of DNA encoding precursor protein H.

[SEQ ID NO: 40]

This shows the synthetic DNA employed in screening cDNA encoding precursor protein H in Example 10.

[SEQ ID NO: 41]

This shows the synthetic DNA employed in screening cDNA encoding precursor protein H in Example 10.

[SEQ ID NO: 42]

This shows the amino acid sequence of precursor protein I.

[SEQ ID NO: 43]

This shows the nucleotide sequence of DNA encoding precursor protein I.

[SEQ ID NO: 44]

This shows the amino acid sequence of rat AQ27 receptor.

[SEQ ID NO: 45]

This shows the nucleotide sequence of DNA encoding rat AQ27 receptor.

[SEQ ID NO: 46]

This shows the amino acid sequence of mouse AQ27 receptor.

[SEQ ID NO: 47]

This shows the nucleotide sequence of DNA encoding mouse AQ27 receptor.

[SEQ ID NO: 48]

This shows the synthetic DNA employed in screening cDNA encoding precursor protein I in Example 21.

[SEQ ID NO: 49]

This shows the synthetic DNA employed in screening cDNA encoding precursor protein I in Example 21.

[SEQ ID NO: 50]

This shows the synthetic DNA employed in screening cDNA encoding rat AQ27 in Example 22.

[SEQ ID NO: 51]

This shows the synthetic DNA employed in screening cDNA encoding rat AQ27 in Example 22.

[SEQ ID NO: 52]

This shows the synthetic DNA employed in screening cDNA encoding mouse AQ27 in Example

[SEQ ID NO: 53]

This shows the synthetic DNA employed in screening cDNA encoding mouse AQ27 in Example 22.

[SEQ ID NO: 54]

This shows the nucleotide sequence of the primer used in Example 24.

[SEQ ID NO: 55]

This shows the nucleotide sequence of the primer used in Example 24.

[SEQ ID NO: 56]

This shows the nucleotide sequence of the probe used in Example 24.

[SEQ ID NO: 57]

This shows the nucleotide sequence of the primer used in Example 25.

[SEQ ID NO: 58]

This shows the nucleotide sequence of the primer used in Example 25.

[SEQ ID NO: 59]

This shows the nucleotide sequence of the probe used in Example 25.

[SEQ ID NO: 60]

This shows the nucleotide sequence of the primer used in Example 26.

[SEQ ID NO: 61]

This shows the nucleotide sequence of the primer used in Example 26.

[SEQ ID NO: 62]

This shows the nucleotide sequence of the probe used in Example 26.

[SEQ ID NO: 63]

This shows the nucleotide sequence of the primer used in Example 26.

[SEQ ID NO: 64]

This shows the nucleotide sequence of the primer used in Example 26.

[SEQ ID NO: 65]

This shows the nucleotide sequence of the probe used in Example 26.

Transformant Escherichia coli IM109/pTA-S65 obtained in Example 1 later described has been deposited with International Patent Organism Depositary (IPOD), National Institute of Advanced Industrial Science and Technology (AIST) at Chuo No. 6, 1, Higashi 1-chome, Tsukuba-shi, Ibaraki-ken, Japan (zip code: 305-8566), under the Accession Number FERM BP-7742 since September 19,2001, and with Institute for Fermentation, Osaka (IFO) at No. 17-85, Juso-honmachi 2-chome, Yodogawa-ku, Osaka, Japan (zip code: 532-8686), under the Accession Number IFO 16697 since September 6, 2001.

Transformant Escherichia coli JM109/pTA-S66 obtained in Example 2 later described has been deposited with International Patent Organism Depositary (IPOD), National Institute of Advanced Industrial Science and Technology (AIST) at Chuo No. 6, 1, Higashi 1-chome, Tsukuba-shi, Ibaraki-ken, Japan (zip code: 305-8566), under the Accession Number FERM BP-7743 since September 19, 2001, and with Institute for Fermentation, Osaka (IFO) at No. 17-85, Juso-honmachi 2-chome, Yodogawa-ku, Osaka, Japan (zip code: 532-8686), under the Accession Number IFO 16698 since September 6, 2001.

Transformant Escherichia coli JM109/pTA-S67 obtained in Example 3 later described has been deposited with International Patent Organism Depositary (IPOD), National Institute of Advanced Industrial Science and Technology (AIST) at Chuo No. 6, 1, Higashi 1-chome, Tsukuba-shi, Ibaraki-ken, Japan (zip code: 305-8566), under the Accession Number FERM BP-7744 since September 19, 2001, and with Institute for Fermentation, Osaka (IFO) at No. 17-85, Juso-honmachi home, Yodogawa-ku, Osaka, Japan (zip code: 532-8686), under the Accession Number IFO 16699 since September 11, 2001.

Transformant Escherichia coli JM109/pTA-S68 obtained in Example 4 later described has been deposited with International Patent Organism Depositary (IPOD), National Institute of Advanced Industrial Science and Technology (AIST) at Chuo No. 6, 1, Higashi 1-chome, Tsukuba-shi, Ibaraki-ken, Japan (zip code: 305-8566), under the Accession Number FERM BP-7745 since September 19, 2001, and with Institute for Fermentation, Osaka (IFO) at No. 17-85, Juso-honmachi 2-chome, Yodogawa-ku, Osaka, Japan (zip code: 532-8686), under the Accession Number IFO 16700 since September 11, 2001.

Transformant Escherichia coli JM109/pTA-S69 obtained in Example 5 later described has been deposited with International Patent Organism Depositary (IPOD), National Institute of Advanced Industrial Science and Technology (AIST) at Chuo No. 6, 1, Higashi 1-chome, Tsukuba-shi, Ibaraki-ken, Japan (zip code: 305-8566), under the Accession Number FERM BP-7746 since September 19, 2001, and with Institute for Fermentation, Osaka (IFO) at No. 17-85, Juso-honmachi 2-chome, Yodogawa-ku, Osaka, Japan (zip code: 532-8686), under the Accession Number IFO 16701 since September 11, 2001.

Transformant Escherichia coli JM109/pTAhFRF-1 obtained in Example 6 later described has been deposited with International Patent Organism Depositary (IPOD), National Institute of Advanced Industrial Science and Technology (AIST) at Chuo No. 6, 1, Higashi 1-chome, Tsukuba-shi, Ibaraki-ken, Japan (zip code: 305-8566), under the Accession Number FERM BP-7903 since February 18, 2002, and with Institute for Fermentation, Osaka (IFO) at No. 17-85, Juso-honmachi 2-chome, Yodogawa-ku, Osaka, Japan (zip code: 532-8686), under the Accession Number IFO 16752 since February 6, 2002.

Transformant Escherichia coli JM109/pTArFRF-1 obtained in Example 7 later described has been deposited with International Patent Organism Depositary (IPOD), National Institute of Advanced Industrial Science and Technology (AIST) at Chuo No. 6, 1, Higashi 1-chome, Tsukuba-shi, Ibaraki-ken, Japan (zip code: 305-8566), under the Accession Number FERM BP-7905 since February 18, 2002, and with Institute for Fermentation, Osaka (IFO) at No. 17-85, Juso-honmachi 2-chome, Yodogawa-ku, Osaka, Japan (zip code: 532-8686), under the Accession Number IFO 16754 since February 6, 2002.

Transformant Escherichia coli IM109/pTAmFRF-1 obtained in Example 10 later described has been deposited with International Patent Organism Depositary (IPOD), National Institute of Advanced Industrial Science and Technology (AIST) at Chuo No. 6, 1, Higashi 1-chome, Tsukuba-shi, Ibaraki-ken, Japan (zip code: 305-8566), under the Accession Number FERM BP-7904 since February 18, 2002, and with Institute for Fermentation, Osaka (IFO) at No. 17-85, Juso-honmachi 2-chome, Yodogawa-ku, Osaka, Japan (zip code: 532-8686), under the Accession Number IFO 16753 since February 6, 2002.

Transformant Escherichia coli JM109/pTAbFRF-1 obtained in Example 21 later described has been deposited with International Patent Organism Depositary (IPOD), National Institute of Advanced Industrial Science and Technology (AIST) at Chuo No. 6, 1, Higashi 1-chome, Tsukuba-shi, Ibaraki-ken, Japan (zip code: 305-8566), under the Accession Number FERM BP-8162 since August 21, 2002.

Transformant Escherichia coli JM109/pCR2.1-rat AQ27 obtained in Example 22 later described has been deposited with International Patent Organism Depositary (IPOD), National Institute of Advanced Industrial Science and Technology (AIST) at Chuo No. 6, 1, Higashi 1-chome, Tsukuba-shi, Ibaraki-ken, Japan (zip code: 305-8566), under the Accession Number FERM BP-8163 since August 21, 2002.

Transformant Escherichia coli JM109/pCR2.1-mouse AQ27 obtained in Example 23 later described has been deposited with International Patent Organism Depositary (IPOD), National Institute of Advanced Industrial Science and Technology (AIST) at Chuo No. 6, 1, Higashi 1-chome, Tsukuba-shi, Ibaraki-ken, Japan (zip code: 305-8566), under the Accession Number FERM BP-8164 since August 21, 2002.

## EXAMPLES

Hereinafter, the present invention will be specifically described with reference to the Examples, but is not limited thereto.

### Example 1: Acquisition of a secretory protein gene from human brain cDNA bp PCR (Comparative example)

Amplification by PCR was carried out using human brain cDNA as a template purchased from Clontech and the following 2 synthetic DNAs:

C216F: 5'-GTCGACCATGGGGCCTGGACGATGCCTCCTGACG-3' (SEQ ID NO: 13) and
C216R: 5'-GCTAGCCTATCTTGCGGCCAGGTGTCGAGTAGT-3' (SEQ ID NO: 14).

A PCR reaction solution, 25 μl, was prepared by mixing 1 μl cDNA solution, 0.5 μl C216F (10 μM), 0.5 μl C216R (10 μM, 2.5 μl of an attached 10 × reaction solution, 2.5 μl dNTP (10 mM), 0.5 μl KlenTaQ (Clontech) and 17.5 μl Ohtsuka distilled water. The reaction solution was subjected to PCR reaction using Thermal Cycler 9600. PCR involved denaturation at 95°C for 2 minutes and 40 cycles each consisting of a reaction at 98°C for 10 seconds, at 63°C for 20 seconds and at 72°C for 60 seconds. By electrophoresis of a part of the PCR product, amplification of about 300-bp PCR product was confirmed, and then the PCR product was purified by using a Quiagen PCR purification kit and directly sequenced to give the sequence shown in Fig. 1. The amino acid sequence deduced from the DNA sequence in Fig. 1 is shown in Fig. 2. An amino acid sequence in positions 1 to 17 of the amino acid sequence in Fig. 2 was estimated to be a secretory signal. As the peptide formed, there was estimated Pro Pro Pro Glu Ala Ser Gln Tyr Cys Gly Arg Leu Glu Tyr Trp Asn Pro Asp Asn Lys Cys Cys Ser Ser Cys Leu Gln Arg Phe Gly Pro Pro Pro Cys Pro Glu Leu Ser Ser Leu Ala Ser Gln Pro Leu Ser Arg Leu Leu Asp Glu Leu Glu Val Leu Glu Glu Leu De Val Leu Leu Asp Pro Glu Pro Gly Pro Gly Gly Gly Met Ala His Gly Thr Thr Arg His Leu Ala Ala Arg (SEQ ID NO: 1). The PCR product recovered from the gel was subcloned into E. coli JM 109 by a TA cloning kit (Invitrogen, Inc.) to give E. coli JM109/pTA-S65. From the E. coli obtained by subcloning, plasmid pTA-S65 was extracted by a plasmid extractor (Kurabo) to determine the nucleotide sequence of the inserted sequence, and it was confirmed that the sequence is the same as the human secretory protein gene cDNA in Fig. 1.

### Example 2: Acquisition of a secretory protein gene from human placenta cDNA by PCR (Comparative example)

Amplification by PCR was carried out using human placenta cDNA as a template purchased from Clontech and the following 2 synthetic DNAs:

C2008F: 5'-GTCGACC.ATGTGGCCCCGAAAACrCGCCrGGA7T-3- (SEQ ID NO: 15) and

C2008R: 5'-GCTAGCCTAGTATCCCCATCCTCTCCCTAATTC-3' (SEQ ID NO: 16).

A PCR reaction solution, 25 μl, was prepared by mixing 1 μl cDNA solution, 0.5 μl C2008F (10 μM, 0.5 μl C2008R (10 μM), 2.5 μl of an attached 10 × reaction solution, 2.5 μl dNTP (10 mM), 0.5 μl KlenTaQ (Clontech) and 17.5 μl Ohtsuka distilled water. The reaction solution was subjected to PCR reaction using Thermal Cycler 9600. PCR involved denaturation at 95°C for 2 minutes and 40 cycles each consisting of a reaction at 98°C for 10 seconds, at 63°C for 20 seconds and at 72°C for 60 seconds. By electrophoresis of a part of the PCR product, amplification of about 600-bp PCR product was confirmed, and then the PCR product was purified by using a Quiagen PCR purification kit and directly sequenced to give the sequence shown in Fig. 3. The amino acid sequence deduced from the DNA sequence in Fig. 3 is shown in Fig. 4 An amino acid sequence in positions 1 to 21 of the amino acid sequence in Fig. 4 was estimated to be a secretory signal. As the peptide formed, there were estimated an amide of a peptide having an amino acid sequence in positions 22 to 37 of the amino acid sequence in Fig. 4, an amide of a peptide having an amino acid sequence in positions 40 to 55 of the amino acid sequence in Fig. 4, etc. The PCR product recovered from the gel was subcloned into E. coli JM109 by a TA cloning kit (Invitrogen, Inc.) to give E. coli JM109/pTA-S66. From the E. coli obtained by subcloning, plasmid pTA-S66 was extracted by a plasmid extractor (Kurabo) to determine the nucleotide sequence of the inserted sequence, and it was confirmed that the sequence is the same as the human secretory protein gene cDNA in Fig. 3.

**Example 3: Acquisition of a secretory protein gene from human brain cDNA by PCR (Comparative example)**

Amplification by PCR was carried out using human brain cDNA as a template purchased from Clontech and the following 2 synthetic DNAs:

C196F:5'-GTCGACCATGCGGGCGCCACTCTGCCTGCTCCTG-3' (SEQ ID NO: 17) and
C196R: 5'-GCTAGCTCAGGGCTGCAGGCCGGGCTGGCGCGG-3' (SEQ ID NO: 18).

A PCR reaction solution, 25 μl, was prepared by mixing 1 μl cDNA solution, 0.5 μl C196F (10 μM), 0.5 μl C196R (10 μM), 2.5 μl of an attached 10 × reaction solution, 2.5 μl dNTP (10 mM), 0.5 μl KlenTaQ (Clontech) and 17.5 μl Ohtsuka distilled water. The reaction solution was subjected to PCR reaction using Thermal Cycler 9600. PCR involve denaturation at 95°C for 2 minutes and 40 cycles each consisting of a reaction at 98°C for 10 seconds, at 63°C for 20 seconds and at 72°C for 60 seconds. By electrophoresis of a part of the PCR product, amplification of about 100-bp PCR product was confirmed, and then the PCR product was purified by using a Quiagen PCR purification kit and directly sequenced to give the sequence shown in Fig. 5. The amino acid sequence deduced from the DNA sequence in Fig. 5 is shown in Fig. 6. An amino acid sequence in positions 1 to 14 of the amino acid sequence in Fig. 6 was estimated to be a secretory signal. As the peptide formed, there were estimated a peptide having an amino acid sequence in positions 15 to 21 of the amino acid sequence in Fig. 6, a peptide having an amino acid sequence in positions 26 to 58 of the amino acid sequence in Fig. 6, a peptide having an amino acid sequence in positions 60 to 88 of the amino acid sequence in Fig. 6, a peptide having an amino acid sequence in positions 90 to 107 of the amino acid sequence in Fig. 6, a peptide having an amino acid sequence in positions 110 to 187 of the amino acid sequence in Fig. 6, etc. The PCR product recovered from the gel was subcloned into E. coli JM109 by a TA cloning kit (Invitrogen, Inc.) to give E. coli JM109/pTA-S67. From the E. coli obtained by subcloning, plasmid pTA-S67 was extracted by a plasmid extractor (Kurabo) to determine the nucleotide sequence of the inserted sequence, and it was confirmed that the sequence is the same as the human secretory protein gene cDNA in Fig. 5.

**Example 4: Acquisition of a secretory protein gene from human brain cDNA by PCR (Comparative Example)**

Amplification by PCR was carried out using human brain cDNA as a template purchased from Clontech and the following 2 synthetic DNAs:

C296F: 5'-GTCGACCATGGGGGCCCCGCTCGCCGTAGCGCTG-3' (SEQ ID NO: 19) and
C296R: 5'-GCTAGCTCACTGTCCACACCCCCAGGTCCAAGT-3' (SEQ ID NO: 20).

A PCR reaction solution, 25 μl, was prepared by mixing 1 μl cDNA solution, 0.5 μl C296F (10 μM), 0.5 μl C296R (10 μM), 2.5 μl of an attached 10 × reaction solution, 2.5 μl dNTP (10 mM), 0.5 μl KlenTaQ (Clontech) and 17.5 μl Ohtsuka distilled water. The reaction solution was subjected to PCR reaction using Thermal Cycler 9600. PCR involved denaturation at 95°C for 2 minutes and 40 cycles each consisting of a reaction at 98°C for 10 seconds, at 63°C for 20 seconds and at 72°C for 60 seconds. By electrophoresis of a part of the PCR product, amplification of about 500-bp PCR product was confirmed, and then the PCR product was purified by using a Quiagen PCR purification kit and directly sequenced to give the sequence shown in Fig. 7. The amino acid sequence deduced from the DNA sequence in Fig. 7 is shown in Fig. 8. An amino acid sequence in positions 1 to 24 of the amino acid sequence in Fig. 8 was estimated to be a secretory signal. As the peptide formed, there was estimated a peptide having an amino acid sequence in positions 25 to 85 of the amino acid sequence in Fig. 8, a peptide having an amino acid sequence in positions 86 to 114 of the amino acid sequence in Fig. 8 or a peptide having an amino acid sequence in positions 115 to 166 of the amino acid sequence in Fig. 8. The PCR product recovered from the gel was subcloned into E. coli IM109 by a TA cloning kit (Invitrogen, Inc.) to give E. coli JM109/pTA-S68. From the E. coli obtained by subloning, plasmid pTA-S68 was extracted by a plasmid extractor (Kurabo) to determine the nucleotide sequence of the inserted sequence, and it was confirmed that the sequence is the same as the human secretory protein gene cDNA in Fig. 7.

**Example 5: Acquisition of a secretory protein gene from human brain cDNA by PCR (Comparative example)**

Amplification by PCR was carried out using human brain cDNA as a template purchased from Clontech and the following 2 synthetic DNAs:

C118Batg: 5'-GTCGACCATGGCCCCAGCCTTCCTGCTGCTGCTG-3' (SEQ ID NO: 21) and
C118Bstop: 5'-GCTAGCCTAGTACCGGCCGCCAGTGTGCTGGAA-3' (SEQ ID NO: 22).

A PCR reaction solution, 25 μl, was prepared by mixing 1 μl cDNA solution, 0.5 μl C216F (10 μM), 0.5 μl C216R (10 μM), 2.5 μl of an attached 10 × reaction solution, 2.5 μl dNTP (10 mM), 0.5 μl KlenTaQ (Clontech) and 17.5 μl Ohtsuka distilled water. The reaction solution was subjected to PCR reaction using Thermal Cycler 9600. PCR involved denaturation at 95°C for 2 minutes and 40 cycles each consisting of a reaction at 98°C for 10 seconds, at 63°C for 20 seconds and at 72°C for 60 seconds. By electrophoresis of a part of the PCR product, amplification of about 300-bp PCR product was confirmed, and then the PCR product was purified by using a Quiagen PCR purification kit and directly sequenced to give the sequence shown in Fig. 9. The amino acid sequence deduced from the DNA sequence in Fig. 9 is shown in Fig. 10. An amino acid sequence in positions 1 to 18 of the amino acid sequence in Fig. 10 was estimated to be a secretory signal. As the peptide formed, there was estimated a peptide having an amino acid sequence in positions 19 to 58 of the amino acid sequence in Fig. 10, a peptide having an amino acid sequence in positions 63 to 202 of the amino acid sequence in Fig. 10 or a peptide having an amino acid sequence in positions 205 to 241 of the amino acid sequence in Fig. 10. The PCR product recovered from the gel was subcloned into E. coli JM109 by a TA cloning kit (Invitrogen, Inc.) to give E. coli JM109/pTA-S69. From the E: coli obtained by subcloning, plasmid pTA-S69 was extracted by a plasmid extractor (Kurabo) to determine the nucleotide sequence of the inserted sequence, and it was .. confirmed that the sequence is the same as the human secretory protein gene cDNA in Fig. 9.

**Example 6: Acquisition of a secretory protein gene from human cDNA by PCR**

Amplification by PCR was carried out using human universal cDNA as a template purchased from Clontech and the following 2 synthetic DNAs:

RFF2: 5'-ATGGTAAGGCCTTACCCCCTGATCTAC-3' (SEQ ID NO: 25) and
RFR1:5'-CAAATCCTTCCAAGGCGTCCTGGCCCT-3' (SEQ ID NO: 26).

A PCR reaction solution, 25 μl, was prepared by mixing 1 μl cDNA solution, 0.5 μl RFF2 (10 μM), 0.5 μl RFR1 (10 μM, 2.5 μl of an attached 10 × reaction solution, 2.5 μl dNTP (10 mM), 0.25 μl ExTaq (Takara) and 17.75 μl Ohtsuka distilled water. The reaction solution was subjected to PCR reaction using Thermal Cycler 9600. PCR involved denaturation at 95°C for 2 minutes and 40 cycles each consisting of a reaction at 98°C for 10 seconds, at 63°C for 20 seconds and at 72°C for 30 seconds. By electrophoresis of a part of the PCR product, amplification of about 400-bp PCR product was confirmed, and then the PCR product was purified by using a Quiagen PCR purification kit and directly sequenced to give the sequence shown in Fig. 11. The amino acid sequence deduced from the DNA sequence in Fig. 11 is shown in Fig. 12. An amino acid sequence in positions 1 to 18 of the amino acid sequence in Fig. 12 was estimated to be a secretory signal. The peptides formed would be:

(1) a peptide comprising an amino acid sequence in positions 26 to 88 of the amino acid sequence represented by Fig. 12 (SEQ ID NO: 23) having an amidated C terminus;
(2) a peptide comprising an amino acid sequence in positions 80 to 88 of the amino acid sequence represented by Fig. 12 (SEQ ID NO: 23) having an amidated C terminus;
(3) a peptide comprising an amino acid sequence in positions 91 to 133 of the amino acid sequence represented by Fig. 12 (SEQ ID NO: 23) having an amidated C terminus; and

(4) a peptide comprising an amino acid sequence in positions 127 to 133 of the amino acid sequence represented by Fig. 12 (SEQ NO: 23) having an amidated C terminus.

The PCR product recovered from the gel was subcloned into E. coli JM109 by a TA cloning kit (Invitrogen, Inc.) to give E. coli JM109/pTAhFRF-1. From the E. coli obtained by subcloning, plasmid pTAhFRF-1 was extracted by a plasmid extractor (Kurabo) to determine the nucleotide sequence of the inserted sequence, and it was confirmed that the sequence is the same as the human secretory protein gene cDNA in Fig. 11.

**Example 7: Acquisition of a ligand candidate gene from rat genomic DNA by PCR (Comparative example)**

Amplification by PCR was carried out using rat genomic DNA as a template purchased from Clontech and the following 2 synthetic DNAs:

F1: 5'-CCTCCTCTCTCTCCCTCCTCTGCTCAG-3' (SEQ ID NO: 29) and
R1: 5'-ACGGGGCAGAGTCCACGCAGGCCCTCA-3' (SEQ ID NO: 30).

A PCR reaction solution, 25 μl, was prepared by mixing 1 μl DNA solution, 0.5 μl F1 (10 μM), 0.5 μl R1 (10 μM), 2.5 μl of an attached 10 × reaction solution, 2.5 μl dNTP (10 mM), 0.25 μl ExTaq (Takara) and 17.75 μl Ohtsuka distilled water. The reaction solution was subjected to PCR reaction using Thermal Cycler 9600. PCR involved denaturation at 95°C for 2 minutes and 30 cycles each consisting of a reaction at 98°C for 10 seconds, at 60°C for 20 seconds and at 72°C for 30 seconds. By electrophoresis of a part of the PCR product, amplification of about 400-bp PCR product was confirmed, and then the PCR product was purified by using a Quiagen PCR purification kit and directly sequenced to give the sequence shown in Fig. 13. The amino acid sequence deduced from the DNA sequence in Fig. 13 is shown in Fig. 14. An amino acid sequence in positions 1 to 17 of the amino acid sequence in Fig. 14 was estimated to be a secretory signal.

Comparison between the human and rat amino acid sequences is shown in Fig. 15, and particularly the sequence (Arg Phe Gly Arg) of a C-terminal RF amide motif was stored. As the peptide formed, there were estimated peptides having an amidated C terminus, such as Gly Gly Phe Ser Phe Arg Phe-$NH_2$ (positions 116 to 122 in SEQ ID NO: 27), Lys Gly Gly Phe Ser Phe Arg Phe-$NH_2$ (positions 115 to 122 in SEQ ID NO: 27) etc.

The PCR product recovered from the gel was subcloned into E. coli JM 109 by a TA cloning kit (Invitrogen, Inc.) to give E. coli JM109/pTArFRF-1. From the E. coli obtained by subcloning, plasmid pTArFRF-1 was extracted by a plasmid extractor (Kurabo) to determine the nucleotide sequence of the inserted sequence, and it was confirmed that the sequence is the same as the rat secretory protein gene cDNA in Fig. 13.

**Example 8:**

(1) Synthesis of Gly-Gly-Phe-Ser-Phe-Arg-Phe-NHi (positions 127 to 133 in SEQ ID NO: 23) and Glu-HB-Ala-Gly-cp-Arg-Phe-Arg-Phe-$NH_2$ (positions 80 to 88 in SEQ ID NO: 23)
Gly-Gly-Phe-Ser-Phe-Arg-Phc-$NH_2$ (positions 127 to 133 in SEQ ID NO: 23) and Glu-HB-Ala-Gly-cys-Arg-Phe.Arg-Phe-$NH_2$ (positions 80 to 88 in SEQ ID NO: 23) were produced by a peptide synthesis method known per se.
(2) Synthesis of Arg-Lys-Lys-Gly-Gly-Phe-Ser-Phe.Arg-Phe-$NH_2$ (positions 124 to 133 in SEQ ID NO: 23)
Arg-Lys-Lys-Gly-Gly-Pbe-Ser-Phe-Arg-Phe-$NH_2$ (positions 124 to 133 in SEQ ID NO: 23) was produced by a peptide synthesis method known per se.

**Example 9:** Activity of polypeptides F: Gly-Gly-Phe-Ser-Phe-Arg-Phe-$NH_2$ (positions 127 to 133 in SEQ ID NO: 23) and Glu-His-Ala-Gly-Cys-Arg-Pbe-Arg-Phe-$NH_2$ (positions in 80 to 88 of SEQ ID NO: 23) in increasing the amount of an expressed reporter gene in HEK 293 cells transiently expressing AQ27 receptor and OT7T022 receptor

The specific stimulating activity of polypeptides F synthesized in Example 8 on AQ27 receptor (SEQ ID NO: 31) and human OT7T022 receptor (SEQ ID NO: 35) was detected by using, as an indicator, the amount of a reporter gene product (luciferase) produced by induction of expression of a cAMP response element (CRE) promoter.

The HEK 293 cells were suspended in a growth medium (DMEM [Dulbecco's Modified Eagle Medium] (Gibco BRL) containing 10% fetal bovine serum (Gibco BRL)), and plated at a density of 1 × $10^5$ cells/well on a black 96-well plate (Becton Dickinson Company, Ltd.) coated with collagen. After the cells were cultured overnight at 37°C in 5% $CO_2$, the cells were transfected in the following manner with a reporter gene-containing plasmid pCRE-Luc (Clontech) and simultaneously with an expression vector plasmid prepared by inserting AQ27 gene (SEQ ID NO: 32) and human OT7T022 gene (SEQ ID NO: 36) by a known method into an animal cell expression vector pAKKO-111H (the same plasmid vector as pAKKO-111H described in Biochem. Biophys. Acta, Hinuma, S. et al., 1219, 251-259, 1994) or with the intact pAKKO-111H not containing AQ27 gene.

OPTI-MEM-I (Gibco BRL) and Lipofectamine™ 2000 Reagent (Gibco BRL) were mixed in a ratio of 24 : 1 to prepare a Lipofectamine dilution. Separately, OPTI-MEM-I, AQ27 expression vector plasmid or the intact vector plasmid (240 ng/μl) and pCRE-Luc (240 ng/μl) were mixed in a ratio of 24: 0.9 : 0.1 to prepare a DNA dilution. The Lipofectamine dilution and the DNA dilution were mixed in equal amounts and left for 20 minutes at room temperature, to form a DNA/ Lipofectamine complex, and 25 μl of the complex was added to the plate where the HEK 293 cells were cultured, and the cells were further cultured overnight at 37°C in 5% $CO_2$.

The HEK 293 cells thus transfected were washed with an assay medium (DMEM containing 0. 1% bovine serum albumin), and then each of polypeptides F synthesized in Example 8, diluted with the assay buffer, was added at concentrations of 10, 1 and 0.1 μM respectively to the cells which were then cultured at 37°C in 5% $CO_2$ for 4 hours. The culture supernatant was discarded, and 50 μl Pikka Gene LT2.0 (Toyo Ink Mfg. Co., Ltd.), that is, a substrate for measurement of luciferase activity, was added thereto, and the fluorescence of luciferase was measured with a plate reader (ARVO sx multilabel counter, Wallac).

As a result, the 2 polypeptides F were detected as an enhancer for AQ27 receptor, which depending on the concentration, enhanced the activity of luciferase stimulated by adding forskolin (FSK). On the other hand, the polypeptides F were detected as a suppressor for human OT7T022 receptor, which suppressed the activity of luciferase stimulated by adding FSK. This promoting or suppressing reaction was not detected in cells expressing the intact vector pAKKO-111H not containing the receptor gene. It was thus confirmed that the 2 polypeptides F caused enhancement of luciferase activity specifically for AQ27 receptor or suppression of luciferase activity specifically for human OT7T022 receptor (Figs. 16, 17 and 18).

**Example 10: Acquisition of a ligand candidate gene from mouse genomic DNA by PCR**

Amplification by PCR was carried out using mouse genomic DNA as a template purchased from Clontech and the following 2 synthetic DNAs:

F2: 5'-ATGAGGGGCTTCCGGCCTTTGCTTTCC-3'(SEQ ID NO: 40) and
R2: 5'-TCACCGTCCAAAGCGGAAGCTGAAGCC-3'(SEQ ID NO: 41).

A PCR reaction solution, 25 μl, was prepared by mixing 1 μl DNA solution, 0.5 μl F2 (10 μM, 0.5 μl R2 (10 μM), 2.5 μl of an attached 10 × reaction solution, 2.5 μl dNTP (10 mM), 0.25 μl ExTaq (Takara) and 17.75 μl Ohtsuka distilled water. The reaction solution was subjected to PCR reaction using Thermal Cycler 9600. PCR involved denaturation at 95°C for 2 minutes and 30 cycles each consisting of a reaction at 98°C for 10 seconds, at 60°C for 20 seconds and at 72°C for 30 seconds. By electrophoresis of a part of the PCR product, amplification of about 400-bp PCR product was confirmed, and then the PCR product was purified by using a Quiagen PCR purification kit and directly sequenced to give the sequence shown in Fig. 19. The amino acid sequence deduced from the DNA sequence in Fig. 19 is shown in Fig. 20. An amino acid sequence in positions 1 to 17 of the amino acid sequence in Fig. 20 was estimated to be a secretory signal. As the peptide formed, there were estimated peptides having an amidated C terminus, such as Gly Gly Phe Ser Phe Arg Phe-$NH_2$ (positions 116 to 122 in SEQ ID NO: 40), Lys Gly Gly Phe Ser Phe Arg Phe-$NH_2$ (positions 115 to 122 in SEQ ID NO: 40) etc.

The PCR product recovered from the gel was subcloned into E. coli JM109 by a TA cloning kit (Invitrogen, Inc.) to give E. coli JM109/pTAmFRF-1. From the E. coli obtained by subcloning, plasmid pTAmFRF-1 was extracted by a plasmid extractor (Kurabo) to determine the nucleotide sequence of the inserted sequence, and it was confirmed that the sequence is the same as the mouse secretory protein gene cDNA in Fig. 19.

**Example 11: Activity of polypeptide F:**

Arg-Lys-Lys-Gly-Gly-Phe-Ser-Phe-Arg-Phe-$NH_2$ (positions 124 to 133 in SEQ ID NO: 23) in increasing the amount of an expressed reporter gene in HEK 293 cells transiently expressing AQ27 receptor and OT7T022 receptor

The activity of polypeptide F:
Arg-Lys-Lys-Gly-Gly-Phe-Ser-Phe-Arg-Phe-$NH_2$ (positions 124 to 133 in SEQ ID NO: 23) in increasing the amount of an expressed reporter gene in (1) HEK 293 cells not transiently expressing AQ27 receptor and OT7T022 receptor (Fig. 21), (2) HEK 293 cells transiently expressing AQ27 receptor (Fig. 22) and (3) HEK 293 cells transiently expressing OT7T022 receptor (Fig. 23) was measured by the same method as in Example 9. As a result, the polypeptide caused an action of increasing the luciferase activity for AQ27 (Fig. 22) or an action of suppressing the luciferase activity for hOT7T022 (Fig. 23).

**Example 12: Detection of a cAMP production-suppressing activity in human AQ27-expressing CHO cells**

AQ27-expressing CHO cells (Fig. 24) established by a method known per se and mock CHO cells (Fig. 25) serving as a control were plated on a 96-well plate (Becton Dickinson Company, Ltd.) at a density of $4 \times 10^4$ cells/well and cultured overnight. The assay buffer used was a buffer prepared by adding 0.1 % bovine serum albumin and 0.2 mM 3-isobutyl-1-methylxanthine to a Hanks' balanced salt solution. The cells were washed twice with the assay buffer and pre-incubated at 37°C for 30 minutes. The cells were washed again twice with the assay buffer, and a sample prepared in the assay buffer was added thereto, and the cells were incubated at 37°C for 30 minutes. To evaluate the cAMP production-suppressing activity, a sample composed exclusively of 2 μM forskolin (FSK, Wako Pure Chemical Industries, Ltd.) promoting production of cAMP and a sample containing 2μM FSK and $10^{-7}$ to $10^{-5}$ M polypeptide F: Arg-Lys-Lys-Gly-Gly-Phe-Ser-Phe-Arg-Phe-$NH_2$ (positions 124 to 133 in SEQ ID NO: 23) were compared. A supernatant of the cells was discarded, and the amount of cAMP produced in the cells was measured by cAMP Screen System (Applied Biosystems). The result indicated that by adding the peptide, cAMP production induced by FSK was suppressed in the AQ27-expressing CHO, as shown in Fig. 24.

**Example 13: Detection of an intracellular calcium ion release-promoting activity in AQ27-expressing CHO cells**

AQ27-expressing CHO cells established by a method known per se were plated on a 96-well black culture plate (Costar) at a density of $4 \times 10^4$ cells/well and cultured overnight. The assay buffer used was a buffer prepared by adding 2.0 mM HEPES, pH 7.4 (Dojin Kagaku) and 2.5 mM probenecid (Sigma) to a Hanks' balanced salt solution (HBSS). The medium for the cells were removed, and a solution prepared by adding 4 μM Fluo 3-AM (Dojindo) and 0.04% pluronic acid (Molecular Probes) to the assay buffer was added thereto and incubated at 37°C for 1 hour. The cells were washed with the assay buffer to remove an excess of Fluo 3 and set in FLIPR (Molecular Devices). A sample dissolved in the assay buffer was also set in FLIPR, and the sample was added to the cells by a built-in manipulator, and a change in fluorescence depending on the concentration of intracellular calcium ions generated by irradiation with an excitation light was measured. As a result, the reactions shown in Figs. 26 and 27 were detected respectively in the presence of peptide F:
Arg-Lys-Lys-Gly-Gly-Phe-Ser-Phe-Arg-Phe-$NH_2$ (positions 124 to 133 (RKKGGFSFRF-$NH_2$) in SEQ ID NO: 23) and polypeptide F:
Gly-Gly-Phe-Ser-Phe-Arg-Phe-$NH_2$ (positions 127 to 133 (GGFSFRF-$NH_2$) in SEQ ID NO: 23) in an amount of 10 uM (▲) and 1 μM (□), as compared with the absence of the sample (○), and the activity of each peptide in promoting intracellular calcium ion release in the AQ27-expressing CHO cells was detected.

**Example 14:**

(1) Synthesis of
Thr-Ser-Gly-Pro-Leu-Gly-Asn-Leu-Ala-Glu-Glu-Leu-Asn-Gly-Tyr-Ser-Arg-Lys-Lys-Gly-Gly-Phe-Ser-Phe Arg-Phe-$NH_1$ (positions 108 to 133 (T1-F26-$NH_2$) in SEQ ID NO: 23)
T1-F26-$NH_2$ was produced by a peptide synthesis method known per se.
(2) Synthesis of
Pyr-Asp-Glu-Gly-Ser-Glu-Ala-Thr-Gly-Phe-Leu-Pro-Ala-Ala-Gly-Glu-Lys-Thr-Ser-Gly-P ro-Leu-Gly-Asn-Leu-Ala-Glu-Glu-Leu-Asn-Gly-Tyr-Ser-Arg-Lys-Lys-Gly-Gly-Phe-Ser-P he Arg-Phe-$NH_2$ (positions 91 to 133 (Pyr1-F43-$NH_2$) in SEQ ID NO: 23)
Pyr1-F43-$NH_2$ was produced by a peptide synthesis method known per se.
(3) Synthesis of
Ala-Ser-Gln-Pto-Gln-Ala-L,eu-Leu-Val-Ile-Ala-Arg-Gly-Leu-Gln-Thr-Ser-Gly-Arg-Glu-H is-Ala-Gly-Cys-Arg-Phe-$NH_2$ (positions 61 to 86 (A1-F28-$NH_2$) SEQ ID NO: 23)
A1-F28-$NH_2$ was produced by a peptide synthesis method known per se.

**Example 15:** Activity of polypeptide F:

Thr-Ser-Gly-Pro-Leu-Gly-Asn-Leu-Ala-Glu-Glu-Leu-Asn-Gly-Tyr-Ser-Arg-Lys-Lys-Gly-Gly-Phe-Ser-Phe Arg-Phe-$NH_2$ (positions 108 to 133 (T1-F26-$NH_2$) in SEQ ID NO: 23) and
Pyr-Asp-Glu-Gly-Ser-Glu-Ala-Thr-Gly-Phe-Leu-Pro-Ala-Ala-Gly-Glu-Lys-Thr-Ser-Gly-P ro-Leu-Gly-Asn-Leu-Ala-Glu-Glu-Leu-Asn-Gly-Tyr-Ser-Arg-Lys-Lys-Gly-Gly-Phe-Ser-P he Arg-Phe-$NH_2$ (positions 91 to 133 (Pyr1-F43-$NH_2$) in SEQ ID NO: 23) in increasing the amount of an expressed reporter gene in HEK 293 cells transiently expressing human AQ27 receptor

The activity of polypeptides T1-F26-$NH_2$ and Pyr1-F43-$NH_2$ synthesized in Example 14 in increasing the amount of an expressed reporter gene in (1) HEK 293 cells not transiently expressing AQ27 receptor (Fig. 28) and (2) HEK 293 cells transiently expressing AQ27 receptor (Fig. 29) was measured by the same method as in Example 9. As a result, an increase in luciferase activity in the AQ27 receptor-expressing HEK 293 cells by each peptide was detected.

**Example 16: Detection of the AQ27-expressing CHO cell-specific activity of polypeptide Pyr1-F43-$NH_2$ in mobilizing intracellular calcium ions**

The AQ27-expressing CHO cell-specific activity of polypeptide Pyr1-F43-$NH_2$ in mobilizing intracellular calcium ions was examined by the same method as in Example 13. As a result, the CHO-AQ27-specific activity of polypeptide Pyr1-F43-$NH_2$ in mobilizing intracellular calcium ions was recognized by adding at least $10^{-8}$ M polypeptide, as shown in Fig. 30. On the other hand, the activity was not recognized in the mock CHO cells as the control as shown in Fig. 31, and it was thus confirmed that the peptide exhibits a specific agonistic activity as AQ27 ligand.

**Example 17: AQ27-expressing CHO cell-specific activity of polypeptide Pyr1-F43-$NH_2$ in suppressing production of cAMP**

The AQ27-expressing CHO cell-specific activity of polypeptide Pyr1-F43-$NH_2$ in suppressing production of cAMP was detected by the same method as in Example 12. As a result, cAMP production induced by FSK was suppressed specifically in AQ27-expressing CHO by adding polypeptide Pyr1-F43-$NH_2$, as shown in Fig. 32. On the other hand, the activity was not recognized in the mock CHO cells as the control as shown in Fig. 33, and it was thus confirmed that the peptide exhibits a specific agonistic activity as AQ27 ligand.

**Example 18:**

Activity of polypeptide Ala-Ser-Gln-Pro-Gln-Ala-Leu-Leu-Val-Ile-Ala-Arg-Gly-Leu-Gln-Thr-Ser-Gly-Arg-Glu-His-Ala-Gly-Cys-Arg-Phe-$NH_2$ (positions 61 to 86 (A1-F28-$NH_2$) in SEQ ID NO: 23) in increasing the amount of an expressed reporter gene in HEK 293 cells transiently expressing human AQ27 receptor

The activity of polypeptide F: A1-F28-$NH_2$ synthesized in Example 14 in increasing the amount of an expressed reporter gene in (1) HEK 293 cells not transiently expressing AQ27 receptor (Fig. 34) and (2) HEK 293 cells transiently expressing AQ27 receptor (Fig. 35) was measured by the same method as in Example 9. As a result, an increase in luciferase activity in the AQ27 receptor-expressing HEK 293 cells by A 1-F28-$NH_2$ was detected.

**Example 19: Detection of the AQ27-expressing CHO cell-specific activity of polypeptide A1-F28-$NH_2$ in mobilizing intracellular calcium ions**

The AQ27-expressing CHO cell-specific activity of polypeptide A1-F28-$NH_2$ in mobilizing intracellular calcium ions was examined by the same method as in Example 13. As a result, the CHO-AQ27-specific activity of polypeptide A 1-F28-$NH_2$ in mobilizing intracellular calcium ions was recognized by adding at least $10^{-6}$ M polypeptide, as shown in Fig. 36. On the other hand, the activity was not recognized in the mock CHO cells as the control as shown in Fig. 37, and it was thus confirmed that the peptide exhibits a specific agonistic activity as AQ27 ligand.

**Example 20: AQ27-expressing CHO cell-specific activity of polypeptide A1-F28-$NH_2$ in suppressing production of cAMP**

The AQ27-expressing CHO cell-specific activity of polypeptide A1-F28-$NH_2$ in suppressing production of cAMP was detected by the same method as in Example 12. As a result, cAMP production induced by FSK was suppressed specifically in AQ27-expressing CHO by adding at least $10^{-6}$ M polypeptide, as shown in Fig. 38. On the other hand, the activity was not recognized in the mock CHO cells as the control as shown in Fig. 39, and it was thus confirmed that the peptide exhibits a specific agonistic activity as AQ27 ligand.

**Example 21: Acquisition of a secretory protein I gene from bovine genomic DNA by PCR (Comparative example)**

Amplification by PCR was carried out using bovine genomic DNA as a template purchased from Clontech and the following 2 synthetic DNAs:

bF: 5'- ATGCGGAGCCCTTACTCCCTGCCCTAC -3' (SEQ ID NO: 48) and

bR: 5'- TCACCGCCGACCGAAGCGGAAGCTGAA -3' (SEQ ID NO: 49).

A PCR reaction solution, 25 μl, was prepared by mixing 1 μl DNA solution, 0.5 μl bF (10 μM), 0.5 μl bR (10 μM), 2.5 μl of an attached 10 × reaction solution, 2.5 μl dNTP (10 mM), 0.25 μl ExTaq (Takara) and 17.75 μl Ohtsuka distilled water. The reaction solution was subjected to PCR reaction using Thermal Cycler 9600. PCR involved denaturation at 95°C for 2 minutes and 30 cycles each consisting of a reaction at 98°C for 10 seconds, at 60°C for 20 seconds and at 72°C for 30 seconds. By electrophoresis of a part of the PCR product, amplification of about 400-bp PCR product was confirmed, and then the PCR product was purified by using a Quiagen PCR purification kit and directly sequenced to give the sequence shown in Fig. 40. The amino acid sequence deduced from the DNA sequence in Fig. 40 is shown in Fig. 41. An amino acid sequence in positions 1 to 17 of the amino acid sequence in Fig. 41 was estimated to be a secretory signal. As the peptide formed, there were estimated a peptide having an amino acid sequence in positions 124 to 131 of the amino acid sequence in Fig. 41 having an amidated C terminus, a peptide having an amino acid sequence in positions 125 to 131 of the amino acid sequence in Fig. 41 having an amidated C terminus, etc.

The PCR product recovered from the gel was subcloned into E. coli JM109 by a TA cloning kit (Invitrogen, Inc.) to give E. coli JM109/pTAbFRF-1. From the E. coli obtained by subcloning, plasmid pTAbFRF-1 was extracted by a plasmid extractor (Kurabo) to determine the nucleotide sequence of the inserted sequence, and it was confumed that the sequence is the same as the human secretary protein gene cDNA in Fig. 40.

**Example 22: Acquisition of AQ27 receptor gene from rat adrenal cDNA by PCR (Comparative example)**

Using random primers and a reverse transcriptase Super Script II (GIBCO BRL), cDNA was obtained from 1 μg rat adrenal polyA+RNA by reaction at 42°C according to an attached manual.

Amplification by PCR was carried out using the following 2 synthetic DNAs:

F1: 5'-CGTCGACGCATGCAGGCGCTCAACATCACCGCG-3' (SEQ ID NO: 50) and
R2: 5'-CACTAGTTTACAGTTCATGGCCACTACCAAAAGTA-3' (SEQ ID NO: 51).

A PCR reaction solution, 25 μl, was prepared by mixing 1 μl cDNA solution, 0.5 μl F1 (10 μM, 0.5 μl R1 (10 μM), 5 μl of an attached 5 × reaction solution, 2.5 μl Gemelt. 2.5 μl dNTP (10 mM), 0.5 μl Advantage Gcmelt polymerase Mix (Clontech) and 12.5 μl Ohtsuka distilled water. The reaction solution was subjected to PCR reaction using Thermal Cycler 9600. PCR involved denaturation at 95°C for 2 minutes and 35 cycles each consisting of a reaction at 98°C for 10 seconds, at 61°C for 20 seconds and at 72°C for 120 seconds. By electrophoresis of a part of the PCR product, amplification of about 1300-bp PCR product was confirmed, and then the PCR product was purified by using a Quiagen PCR purification kit and subcloned into E. coli JM109 by a TA cloning kit (Invitrogen, Inc.) to give E. coli JM 109/pCR2.1-rat AQ27. From the E. coli obtained by subcloning, plasmid pCR2.1-rat AQ27 was extracted by Quiagen QIAwell 8 Ultra Plasmid Kit to determine the nucleotide sequence of the inserted sequence, and the sequence shown in Fig. 42 was obtained. The amino acid sequence deduced from the DNA sequence in Fig. 42 is shown in Fig. 43. The homology between human and mouse AQ27 is shown in Fig. 46, and it was confirmed to be rat AQ27 receptor.

**Example 23: Acquisition of AQ27 receptor gene from mouse brain cDNA by PCR (comparative example)**

Amplification by PCR was carried out using Clontech mouse MTC panel brain cDNA as a template and the following 2 synthetic DNAs:

F1: 5'-CGTCGACGCATGCAGGCGCTCAACATCACCGCG-3' (SEQ ID NO: 52) and
R2: 5'-CATCGATATTACAGTTCATGTCCACTGCCOAAAGTA-3' (SEQ ID NO: 53).

A PCR reaction solution, 25 μl, was prepared by mixing 1 μl cDNA solution, 0.5 μl F1 (10 μM), 0.5 μl R1 (10 μM), 5 μl of an attached 5 × reaction solution, 2.5 μl Gcmelt, 2.5 μl dNTP (10 mM), 0.5 μl Advantage Gcmelt polymerase Mix (Clontech) and 12.5 μl Ohtsuka distilled water. The reaction solution was subjected to PCR reaction using Thermal Cycle 9600. PCR involved denaturation at 95°C for 2 minutes and 35 cycles each consisting of a reaction at 98°C for 10 seconds, at 59°C for 20 seconds and at 72°C for 120 seconds. By electrophoresis of a part of the PCR product, amplification of about 1300-bp PCR product was confirmed, and then the PCR product was purified by using a Quiagen PCR purification kit and subcloned into E. coli JM109 by a TA cloning kit (Invitrogen, Inc.) to give E. coli JM109/pCR2.1-mouse AQ27. From the E. coli obtained by subcloning, plasmid pCR2.1-mouse AQ27 was extracted by Quiagen QIAwell 8 Ultra Plasmid Kit to determine the nucleotide sequence of the inserted sequence, and the sequence shown in Fig. 44 was obtained. The amino acid sequence deduced from the DNA sequence in Fig. 44 is shown in Fig. 45. The homology between human and rat AQ27 is shown in Fig. 46, and it was confirmed to be mouse AQ27 receptor.

**Example 24: Examination by RT-PCR of the distribution of AQ27 receptor mRNA in human tissus (Comparative example)**

The cDNA used as a template was synthesized by the following method from polyA+RNA (Clontech) derived from various human tissues. 1 μg RNA, random primers and a reverse transcriptase Super Script II (GIBCO BRL) were reacted at 42°C according to the attached manual, and after the reaction was completed, the product was precipitated with ethanol and then dissolved to give 100 μl solution. RT-PCR was conducted using Sequence Detection System Prism 7700 (PE Biosystems), and

5'-CCAGAACATTTCCGACAACTG-3' (SEQ ID NO: 54) and
5'-ACAGCGGTAGACTGGACAAA-3' (SEQ ID NO: 55) were used as the primers for amplification and detection, and: 5'-(Fam)-TGCTTTCATTTGCAAGATGGTGCC-(Tamra)-3' (SEQ ID NO: 56) was used as TaQMan probe. The RT-PCR reaction solution was prepared by adding 0.0.5 μl each of 100 μM primer solutions, 0.5 μl of 5μM TaQMan probe and 0.5 μl of the cDNA solution prepared above to 12.5 μl of TaQMan Universal PCR Master Mix (PE Biosystems) and then adjusting the total volume of the reaction solution to 25 μl with distilled water. PCR reaction involved a reaction at 50°C for 2 minutes and at 95°C for 10 minutes and 40 cycles each consisting of a reaction at 95°C for 15 seconds and at 60°C for I minute. The amount of AQ27 receptor mRNA expressed in various human tissues was calculated as copy number/25 ng total RNA (Fig. 47).

**Example 25: Examination by RT-PCR of the distribution of AQ27 receptor mRNA in rat tissues (Comparative example)**

The cDNA used as a template was synthesized by the following method from polyA+RNA derived from various rat tissues. 1 μg RNA, random primers and a reverse transcriptase Super Script II (GIBCO BRL) were reacted at 42°C according to the attached manual, and after the reaction was completed, the product was precipitated with ethanol and then dissolved to give 100 μl solution. RT-PCR was conducted using Sequence Detection System Prism 7700 (PE Biosystems), and 5'-CGGAAGCCTGGGAATTCTG-3' (SEQ ID NO: 57) and 5'-ATGTGTCTCCTTTGGTTTCTTCCA-3' (SEQ ID NO: 58) were used as the primers for amplification and detection, and
5'-(Fam)-AGCAAAGTTATCTCGACCACAGCGTCCA-(Tamra)-3' (SEQ ID NO: 59) was used as TaQMan probe. The RT-PCR reaction solution was prepared by adding 0.05 μl each of 100 μM primer solutions, 0.5 μl of 5μM TaQMan probe and 0.5 μl of the cDNA solution prepared above to 12.5 μl of TaQMan Universal PCR Master Mix (PE Biosystems) and then adjusting the total volume of the reaction solution to 25 μl with distilled water. PCR reaction involved a reaction at 50°C for 2 minutes and 95°C for 10 minutes and 40 cycles each consisting of a reaction at 95°C for 15 seconds and at 60°C for 1 minute. The amount of AQ27 receptor mRNA expressed in various rat tissues was calculated as copy number/25 ng total RNA (Fig. 48).

**Example 26: Examination by RT-PCR of the distribution of secretory protein G mRNA in rat times (Comparative example)**

Various organs were removed from Wister rats, and total RNA was prepared using Isogen (Nippon Gene), and poly(A)$^+$RNA was prepared using a mRNA purification kit (Pharmacia), according to the respective manuals. 1 μg of the resulting poly(A)$^+$RNA was treated with Dnase I (Amplification Grade, GIBCO BRL), and 160 ng of the RNA was used to synthesize cDNA by using RNA PCR Kit (Takara Co., Ltd.) at 42°C according to its manual. The synthesized cDNA was dissolved at a concentration of 4 ng/μl in terms of poly(A)$^+$RNA and used as the template in the following RT-PCR. RT-PCR was conducted using Sequence Detection System Prism 7700 (PE Biosystems), and
5'-AGCACACTGGCTTCCGTCTAG-3' (SEQ ID NO: 60) and
5'-CGCTGGCCTTCTCTGAGTCA-3' (SEQ ID NO: 61) were used as the primers for amplification and detection, and
5'-(Fam)AGGCAGGACAGTGGCAGTGAAGCC-(Tamar)-3' (SEQ ID NO: 62) was used as TaQMan probe. The RT-PCR reaction solution was prepared by adding 0.225 μl each of 100 μM primer solutions, 1.25 μl of 5μM TaQMan probe and 0.5 μl of the cDNA solution prepared above to 12.5 μl of TaQMan Universal PCR Master Mix (PE Biosystems) and then adjusting the total volume of the reaction solution to 25 μl with distilled water. PCR reaction involved a reaction at 50°C for 2 minutes and at 95°C for 10 minutes and 40 cycles each consisting of a reaction at 95°C for 15 seconds and at 60°C for 1 minute. The amount of the secretory protein G mRNA expressed in the various rat tissues was calculated as copy number/1 ng poly(A)$^+$RNA (Fig. 49).

**Example 27: Examination by RT-PCR of secretory protein F mRNA in human tissues (Comparative example)**

The cDNA used as a template in RT-PCR for detection of the amount of expressed mRNA was synthesized by the following method from polyA+RNA (Clontech) derived from various human tissues. 1 μg RNA, random primers and a reverse transcriptase Super Script II (GIBCO BRL) were reacted at 42°C according to the attached manual, and after the reaction was completed, the product was precipitated with ethanol and then dissolved to give 100 μl solution. Quantification of the amount of expressed mRNA was conducted using Sequence Detection System Prism 7700 (PE Biosystems).
5'-TGAGAGCTTCACAGCCACA-3' (SEQ ID NO: 63) and
5'-AGCTGAAGCCGCCTTTCTT-3' (SEQ ID NO: 64) were used as the primers for amplification and detection, and
5'-(Fam)AACCTGGCTGAGGAGCTCAATGGCTA-(Tamra)-3' (SEQ ID NO: 65) was used as TaQMan probe. The RT-PCR reaction was conducted in the same manner as in Example 24.

The amount of secretory protein F RNA expressed in various human tissues was calculated as copy number/ 1 ng poly(A)$^+$RNA (Fig. 50).

**Example 28: Examination of the distribution of secretory protein mRNA expressed in rat brain by in situ hybridization (Comparative example)**

The abdomen of a Wistar rat was opened under anesthesia with Nembutal, and the rat was perfused with 0.9% saline through the left ventricle for 5 minutes and then perfused with 4% paraformaldehyde for 5 minutes. The removed brain was immersed in the same solution for 24 hours at 4°C, and then immersed in 30% sucrose at 4°C for 3 days to give a brain sample to be analyzed.

Secretory protein antisense and sense probes were prepared by the following method.

First, rat AQ27 ligand cDNA was inserted into plasmid vector pCRII TOPO (Invitrogen, Inc.) by a method known per se. This cDNA was amplified and linearized by PCR using M13 primer (Invitrogen, Inc.) and Advantage GC2 polymerase (Clontech) and purified by precipitation with ethanol. Using DIG RNA Labeling KIT (SP6/T7) (Roche), the cDNA was subjected to in vitro transcription (40 μl scale) with SP6 or T7. The formed DIG-labeled riboprobe was purified by precipitation with ethanol. The purified probe was dissolved in 100 μl Ohtsuka purified water. From the direction of the cDNA inserted, it was determined that the DIG-labeled riboprobe formed with SP6 was an antisense probe, and the DIG-labeled riboprobe formed with T7 was a sense probe.

In in situ hybridization, a free floating method was used. First, a frozen forehead section of 40 μm in thickness was prepared using Cryostat CM3050 (Leica). Then, the section was washed with PBS and then treated with a protease by treatment in 10 mM Tris-HCl/1 mM EDTA (pH 8.0) (37°C, 15 minutes) containing 1 μg/ml proteinase K. Further, it was acetylated by treatment with 0.1 M triethanolamine (pH 8.0) containing 0.25 % acetic anhydride (room temperature, 10 minutes), and then subjected to pre-hybridization reaction by treatment (60°C, 20 minutes) with a hybridization buffer. (50% formaldehyde, 10 mM Tris-HCl, pH 7.5, 1 × Denhardt's solution, 200 μg/ml tRNA, 10% dextran sulfate, 600 mM NaCl, 0.25% SDS, 1 mM EDTA). In hybridization reaction, the antisense probe or sense probe was diluted 1000-fold with the hybridization buffer, denatured at 85°C for 3 minutes, added to the section and reacted at 60°C for 12 hours or more. Subsequently, the unspecifically hybridized probe was washed away by the following procedure: 1) treatment with 2 × SSC (SSC; 1 × SSC = 150 mM NaCl, 15 mM sodium citrate, pH 7.0)/50% formaldehyde (60°C, 15 minutes, twice), 2) treatment with TNE (0.5 M NaCl, 10 mM Tris-HCl (pH 7-6), 1 mM EDTA) (37°C, 10 minutes), 3) treatment with 20 mg/ml RNase A in TNE (37°C, 30 minutes), 4) treatment with TNE (37°C, 10 minutes), 5) treatment with 2 × SSC (60°C, 15 minutes, twice), 6) treatment with 0.5 × SSC (60°C, 15 minutes, twice). After the above procedure was carried out, immunohistochemistry was carried out to detect the DIG-labeled probe. First, the section was washed with DIG-1 (100 mM Tris-HCl, pH 7.5, 150 mM NaCl, 0.1% Tween 20), and after unspecific reaction was blocked by treatment with DIG-1 containing 1.5% blocking reagent (Roche) (37°C, 1 hour), the sample was reacted at room temperature for 1 hour with DIG-1 (1 : 1000) containing anti-DIG fab-fragment antibody conjugated with alkaline phosphatase (Roche). After sufficient washing with DIG-1, the sample was rinsed with DIG-3 (100 mM Tris-HCl, pH 9.5, 100 mM NaCl, 50 mM $MgCl_2$) and subjected to coloration reaction at room temperature with a solution wherein dimethylformamide containing 0.18 mg/ml 5-bromo-4-chloro-3-indolyl-phosphate (BCIP), and 70% dimethylformamide containing 0.34 mg/ml 4-nitroblue tetrazolium (NBT), were added in amounts of 3.5 ml and 4.5 ml respectively per ml of DIG-3. The coloration was terminated after a suitable period by washing with PBS, and the section was placed on a slide glass, mounted in PBS containing 90% glycerol and observed under an optical microscope.

The regions colored specifically with the antisense probe were mainly the hypothalamic retrochiasmatic area and a snout side of the arcuate nucleus, particularly the arcuate nucleus, dorsal and lateral parts. In these regions, coloration with the sense probe could not be detected.

It is known that in the above sites, cocaine- and amphetamine-regulated transcript (CART) and POMC are expressed (both suppressing eating), and also that leptin receptor occurs at high density. Accordingly, it is estimated that the AQ27 ligand regulates eating. It is known that the arcuate nucleus is a site where GHRH is present and there are nerves projecting to a median tuberal external layer. Accordingly, it is considered that the secretory protein participates in neurosecretion. Further, in consideration of the distribution of AQ27 mRNA shown in Example 29, the secretory protein is considered to regulate and modify sleep/arousal, pain, sympathetic system, spontaneous behavior/emotional behavior, etc.

**Example 29: Examination of the distribution of AQ27 mRNA expressed in rat brain by in situ hybridization (Comparative example)**

The abdomen of a Wistar rat was opened under anesthesia with Nembutal, and the rat was perfused with 0.9% saline through the left ventricle for 5 minutes and then perfused with 4% paraformaldehyde for 5 minutes. The removed brain was immersed in the same solution for 24 hours at 4°C, and then immersed in 30% sucrose at 4°C for 3 days to give a brain sample to be analyzed.

AQ27 antisense and sense probes were prepared by the following method.

First, rat AQ27 cDNA was inserted into plasmid vector pCRII TOPO (Invitrogen, Inc.) by a method known per se. This cDNA was amplified and linearized by PCR using M 13 primer (Invitrogen, Inc.) and Advantage GC2 polymerase (Clontech) and purified by precipitation with ethanol. Using DIG RNA Labeling KIT (SP6/T7) (Roche), the cDNA was subjected to in vitro transcription (40 μl scale) with SP6 or T7. The formed DIG-labeled riboprobe was subjected to alkali hydrolysis with 40 mM $NaHCO_3$, 60 mM $Na_2CO_3$, pH 10.2 to give 400 bp probe which was then purified by precipitation with ethanol. The purified probe was dissolved in 100 μl Ohtsuka purified water. From the direction of the cDNA inserted, it was determined that the DIG-labeled riboprobe formed with SP6 was an antisense probe, and the DIG-labeled riboprobe formed with T7 was a sense probe.

In in situ hybridization, a free floating method was used. First, a frozen forehead section of 40 μm in thickness was prepared using Cryostat CM3050 (Leica). Then, the section was washed with PBS and then treated with a protease by treatment in 10 mM Tris-HCl/1 mM EDTA (pH 8.0) (37°C, 15 minutes) containing 1 μg/ml proteinase K. Further, it was acetylated by treatment with 0.1 M triethanolamine (pH 8.0) containing 0.25 % acetic anhydride (room temperature, 10 minutes), and then subjected to pre-hybridization reaction by treatment (60°C, 20 minutes) with a hybridization buffer (50% formaldehyde, 10 mM Tris-HCl, pH 7.5, 1 × Denhardt's solution, 200 μg/ml tRNA, 10% dextran sulfate, 600 mM NaCl, 0.25% SDS, 1 mM EDTA). In hybridization reaction, the antisense probe or sense probe was diluted 1000-fold with the hybridization buffer, denatured at 85°C for 3 minutes, added to the section and reacted at 60°C for 12 hours or more. Subsequently, the unspecifically hybridized probe was washed away by the following procedure: 1) treatment with 2 × SSC (SSC; 1 × SSC = 150 mM NaCl, 15 mM sodium citrate, pH 7.0)/50% formaldehyde (60°C, 15 minutes, twice), 2) treatment with TNE (0.5 M NaCl, 10 mM Tris-HCl (pH 7.6), 1 mM EDTA) (37°C, 10 minutes), 3) treatment with 20 mg/ml RNase A in TNE (37°C, 30 minutes), 4) treatment with TNE (37°C, 10 minutes), 5) treatment with 2 × SSC (60°C, 15 minutes, twice), 6) treatment with 0.5 × SSC (60°C, 15 minutes, twice). After the above procedure was carried out, immunohistochemistry was carried out to detect the DIG-labeled probe. First, the section was washed with DIG-1 (100 mM Tris-HCl, pH 7.5,150 mM NaCl, 0.1 % Tween 20), and after unspecific reaction was blocked by treatment with DIG-1 containing 1.5% blocking reagent (Roche) (37°C, 1 hour), the sample was reacted with DIG-1 (1 : 1000) containing anti-DIG fab-fragment antibody conjugated with alkaline phosphatase (Roche) at room temperature for 1 hour. After sufficient washing with DIG-1, the sample was rinsed with DIG-3 (100 mM Tris-HCl, pH 9.5, 100 mM NaCl, 50 mM $MgCl_2$) and subjected to coloration reaction overnight at 4°C with a solution wherein dimethylforatamide containing 0.18 mg/ml 5-bromo-4-chloro-3-indolyl-phosphate (BCIP), and 70% dimethylformamide containing 0.34 mg/ml 4-nioroblue tetrazolium (NBT), were added in amounts of 3.5 ml and 4.5 ml respectively per ml of DIG-3. The coloration was terminated after a suitable time by washing with PBS, and the section was placed on a slide glass, mounted in PBS containing 90% glycerol and observed under an optical microscope.

The regions colored specifically with the antisense probe were piriform cortex, cortex-amygdara transition zone, ventral pallidum, lateral preoptic area, ventromedial hypothalamic nucleus, zona incerta, posteror hypothalamic area, marginal zone median geniculate, dorsal raphe nucleus, nucleus of brachium inferior colliculus, intergeniculate leaf, locus coeruleus, central gray, alpha-beta part etc. (Fig. 51). Strong coloration was recognized particularly in the dorsal raphe nucleus and locus coeruleus. In these regions, coloration with the sense probe was not detected.

From the above results, the secretory protein/AQ27 was considered to play a role in transmitting information integrated in hypothalamus, to cerebrum-brain stem. Specifically, its regulating and modifying action on 1) eating, 2) sleep/arousal, 3) pain, 4) stress response, 5) spontaneous behavior/emotional behavior, etc. was suggested.

**Example 30: Purification of an AQ27-specitic active component in a supernatant of CHO cells into which human secretory protein F gene was introduced (Comparative example)**

first, full-length human secretory protein F gene (SEQ ID NO: 24) was introduced in a usual manner into an expression vector pAKKO-H111. This vector was transfected with Lipofectamine 2000 (Gibco-BRL) to express human secretory protein F transiently in CHO cells, and a supernatant of the culture was obtained. In the supernatant of the culture, a stimulating activity specific to orphan receptor AQ27 expressed transiently in HEK cells was found (Fig. 52), as compared with a supernatant of a culture of CHO cells (mock CHO cells) into which the intact vector was introduced. The AQ27-specific activity was detected by using an indicator the amount of the reporter gene product (luciferase) produced by induction with expression of cAMP response element (CRE) promoter.

Then, CHOdhfr- cells were transfected with this expression vector by using Gene. Transfer (Wako Pure Chemical Industries, Ltd.) to obtain CHO cells constantly expressing the human AQ27 ligand gene: The CHO cells expressing the human secretory protein F gene were cultured to give a culture supernatant.

For purification from the culture supernatant, whether the antibody recognizing a known RF amide peptide can recognize secretory protein F was examined by competitive method in a liquid phase, and as a result, it was revealed that the anti-RFRP-1 antibody 1F3-1 recognizes a C-terminal structure of secretory protein F (Fig. 53).

2.4 L culture supernatant was obtained and purified by an affinity column using the 1F3-1 antibody. First, the supernatant was boiled, centrifuged to remove precipitates and applied onto an NHS-activated Sepharose column (Amersham Bioscience) to which the 1F3-1 antibody was bound. The column was washed with PBS (phosphate buffered saline) containing 1.0 M NaCl, and then the components bound onto the NHS-activated Sepharose column to which the 1F3-1 antibody was bound was eluted with 0.2 M glycine-HCl, pH 2.2, containing 0.5 M NaCl. This eluted fraction was applied onto a Vydac C18 218TP5415 column and eluted with a gradient from 20 to 35% acetonitrile, to detect 2 peaks of AQ27-specific stimulating activity (Fig. 54). The fraction of each activity peak was separated by μRPC C2/C18 SC2.1/10 column, and as a result, each activity peak was divided into two peaks, to give four activity peaks 1 to 4 in total (Figs. 55 and 56).

The molecular weights of the peptides contained in the four activity fractions were measured by MALDI-TOF-MS. The determined molecular weights of the peptides contained in the activity peaks as shown in Figs. 55 and 56, and their theoretical molecular weights and amino acid sequences deduced from the corresponding human AQ27 ligand gene, are shown in Fig. 57.

**Example 31: Test of the inhibitory action of peptides F different in length on production of cAMP in AQ27-expressing CHO cell strain**

AQ27-expressing CHO cells were plated in a 96-well plate (Falcon) at a density of $4 \times 10^4$ cells/well and cultured overnight at 37°C in 5% $CO_2$/95% air. Hanks' balanced salt solution (GIBCO) containing 0.1 % bovine serum albumin (BSA, Sigma), 200 μM 3-isobutyl-1-methylxanthine (Sigma), 20 mM HEPES (pH 7.4) (GIBCO) was prepared as the assay buffer. A sample dilution buffer was prepared by adding forskolin at a final concentration of 2 μM to the assay buffer, and a sample peptide shown in Fig. 58 was diluted in this dilution buffer to final concentrations of $2 \times 10^{-6}$ M, $2 \times 10^{-7}$ M, $2 \times 10^{-8}$ M, $2 \times 10^{-9}$ M, $2 \times 10^{-10}$ M and $2 \times 10^{-11}$ M, respectively. The cells cultured overnight were washed twice with 150 μl assay buffer and cultured for 30 minutes at 37°C in 100% air, and then washed twice in the same manner as above, and 50 μl assay buffer and 50 μl sample solution were added to the cells which were then stirred and cultured for 30 minutes at 37°C in 100% air. The amount of cAMP in the cells was measured by using cAMP-Screen™ System (ABI) according to the protocol of this kit. The difference between the maximum amount of cAMP and the amount of cAMP upon addition of each sample was calculated, and the percentage relative to the amount of cAMP whose production was promoted by forskolin was calculated and used as the degree of inhibition of cAMP production (Fig. 59). The $EC_{50}$ of each sample is shown in Fig. 58.

**Example 32: Stimolating activity of human secretory protein F in secretion of rat corticosterone**

AQ27 receptor was expressed at higher levels in rat adrenal glands, and thus there was the possibility that the peptide could act on secretion of adrenal cortical hormone. Accordingly, AQ27L-43 shown in Fig. 58 was administered into a rat to examine its influence on adrenal cortical hormone. A 8-week-old male Wistar rat was lightly fixed, and a solution of AQ27L-43 in physiological saline was administered in a dose of 0.5 mg/rat via a tail vein into the rat, and 30 minutes later, blood was collected by decapitation. Blood was also collected from a rat given physiological saline as the control. When serum components in these bloods were measured by using a rat corticosterone measurement kit (Amersham), an increase in blood corticosterone in the rat given AQ27 could be observed.

**INDUSTRIAL APPLICABILITY**

The secretory protein of the present application and its DNA are useful for example as prophylactic/therapeutic agents for diseases with which deficiency in the functions of the secretory protein of the present application is associated.

The application secretory protein of the present application is also useful for screening a compound that promotes or inhibits the functions of the secretory protein of the present application.

The DNA of the present application is also useful for screening a compound that promotes or inhibits the expression of the secretory protein of the present application.

## Claims

1. A peptide consisting of an amino acid sequence in positions 19 to 133, an amino acid sequence in positions 90 to 133, an amino acid sequence in positions 91 to 133, an amino acid sequence in positions 93 to 133, an amino acid sequence in positions 104 to 133, an amino acid sequence in positions 108 to 133, an amino acid sequence in positions 109 to 133, an amino acid sequence in positions 111 to 133, an amino acid sequence in positions 115 to 133, an amino acid sequence in positions 119 to 133, an amino acid sequence in positions 124 to 133 or an amino acid sequence in positions 127 to 133 of the amino acid sequence represented by SEQ ID NO: 23, its amide or ester, or a salt thereof.

2. The peptide according to claim 1, which consists of the amino acid sequence in positions 91 to 133 of SEQ ID NO: 23, its amide or ester, or a salt thereof.

3. The peptide according to claim 1, which consists of the amino acid sequence in positions 108 to 133 of SEQ ID NO: 23, its amide or ester, or a salt thereof.

4. The peptide according to claim 1, which consists of the amino acid sequence in positions 124 to 133 of SEQ ID NO: 23, its amide or ester, or a salt thereof.

5. The peptide according to claim 1, which consists of the amino acid sequence in positions 127 to 133 of SEQ ID NO: 23, its amide or ester, or a salt thereof.

6. A polynucleotide encoding the peptide according to claim 1.

7. The polynucleotide according to claim 6, which consists of a nucleotide sequence in positions 271 to 399 or a nucleotide sequence in positions 379 to 399 of the nucleotide sequence represented by SEQ ID NO: 24.

8. A recombinant vector comprising the polynucleotide according to claim 6 or 7.

9. A transformant transformed with the recombinant vector according to claim 8.

10. A process for producing the peptide according to claim 1, its amide or ester, or a salt thereof, which comprises culturing the transformant according to claim 9 to form the peptide according to claim 1.

11. An antibody to the peptide according to claim 1, its amide or ester, or a salt thereof.

12. The antibody according to claim 11, which is a neutralizing antibody inactivating the activity of the peptide according to claim 1, its amide or ester, or a salt thereof.

13. An antisense polynucleotide comprising the whole or a part of a nucleotide sequence complementary to the polynucleotide according to claim 6 or 7.

14. A method of screening a compound or its salt that promotes or suppresses the according to claim 1, its amide or ester, or a salt thereof, which comprises using the peptide according to claim 1, its amide or ester, or a salt thereof.

15. A kit which comprises the peptide according to claim 1, its amide or ester, or a salt thereof,

16. A method of screening a compound or its salt that promotes or suppresses the expression of the peptide according to claim 1, its amide or ester, or a salt thereof, which comprises using the polynucleotide according to claim 6 or 7.

17. A kit which comprises the polynucleotide according to claim 6 or 7, receptor-binding activity, inrtracellular $Ca^{2a}$ release or/and intracellular cAMP production of the peptide

18. A method of screening a compound or its salt that alters the binding property between (i) the peptide according to claim 1, its amide or ester, or a salt thereof and (ii) G protein-conjugated receptor protein comprising the amino acid sequence represented by SEQ ID NO: 31, SEQ ID NO: 44 or SEQ ID NO: 46 or a salt thereof, which comprises using (i) the peptide according to claim 1, its amide or ester, or a salt thereof and (ii) G protein-conjugated receptor protein comprising the amino acid sequence represented by SEQ ID NO: 31, SEQ ID NO: 44 or SEQ ID NO: 46 or a salt thereof.

**19.** A method of screening a compound or its salt that alters the binding property between (i) the peptide according to claim 1, its amide or ester, or a salt thereof and (ii) G protein-conjugated receptor protein comprising the amino acid sequence represented by SEQ ID NO: 33 or SEQ ID NO: 35 or a salt thereof, which comprises using (i) the peptide according to claim 1, its amide or ester, or a salt thereof and (ii) G protein-conjugated receptor protein comprising the amino acid sequence represented by SEQ ID NO: 33 or SEQ ID NO: 35 or a salt thereof.

## Patentansprüche

**1.** Peptid, bestehend aus einer Aminosäuresequenz in den Positionen 19 bis 133, einer Aminosäuresequenz in den Positionen 90 bis 133, einer Aminosäuresequenz in den Positionen 91 bis 133, einer Aminosäuresequenz in den Positionen 93 bis 133, einer Aminosäuresequenz in den Positionen 104 bis 133, einer Aminosäuresequenz in den Positionen 108 bis 133, einer Aminosäuresequenz in den Positionen 109 bis 133, einer Aminosäuresequenz in den Positionen 111 bis 133, einer Aminosäuresequenz in den Positionen 115 bis 133, einer Aminosäuresequenz in den Positionen 119 bis 133, einer Aminosäuresequenz in den Positionen 124 bis 133 oder einer Aminosäuresequenz in den Positionen 127 bis 133 der in SEQ ID NO: 23 dargestellten Aminosäuresequenz, sein Amid oder Ester, oder ein Salz hiervon.

**2.** Peptid nach Anspruch 1, bestehend aus der Aminosäuresequenz in den Positionen 91 bis 133 von SEQ ID NO: 23, sein Amid oder Ester, oder ein Salz hiervon.

**3.** Peptid nach Anspruch 1, bestehend aus der Aminosäuresequenz in den Positionen 108 bis 133 von SEQ ID NO: 23, sein Amid oder Ester, oder ein Salz hiervon.

**4.** Peptid nach Anspruch 1, bestehend aus der Aminosäuresequenz in den Positionen 124 bis, 133 von SEQ ID NO: 23, sein Amid oder Ester, oder ein Salz hiervon.

**5.** Peptid nach Anspruch 1, bestehend aus der Aminosäuresequenz in den Positionen 127 bis 133 von SEQ ID NO: 23, sein Amid oder Ester, oder ein Salz hiervon.

**6.** Polynukleotid, welches für das Peptid nach Anspruch 1 kodiert.

**7.** Polynukleotid nach Anspruch 6, bestehend aus einer Nukleotidsequenz in den Positionen 271 bis 399 oder einer Nukleotidsequenz in den Positionen 379 bis 399 der in SEQ ID NO: 24 dargestellten Nukleotidsequenz.

**8.** Rekombinanter Vektor, umfassend das Polynukleotid nach Anspruch 6 oder 7.

**9.** Transformante, welche mit dem rekombinanten Vektor nach Anspruch 8 transformiert ist.

**10.** Verfahren zur Herstellung des Peptids nach Anspruch 1, seines Amids oder Esters, oder eines Salzes hiervon, umfassend das Kultivieren der Transformante nach Anspruch 9 zur Bildung des Peptids nach Anspruch 1.

**11.** Antikörper gegen das Peptid nach Anspruch 1, sein Amid oder seinen Ester, oder ein Salz hiervon.

**12.** Antikörper nach Anspruch 11, bei welchem es sich um einen neutralisierenden Antikörper handelt, der die Aktivität des Peptids nach Anspruch 1, seines Amids oder Esters, oder eines Salzes hiervon inaktiviert.

**13.** Antisense-Polynukleotid, umfassend die Gesamtheit oder einen Teil einer Nukleotidsequenz, welche zu dem Polynukleotid nach Anspruch 6 oder 7 komplementär ist.

**14.** Verfahren zum Screenen einer Verbindung oder ihres Salzes, welche/welches die Rezeptorbindungsaktivität, die intrazelluläre $Ca^{2+}$-Freisetzung oder/und die intrazelluläre cAMP-Produktion des Peptids nach Anspruch 1, seines Amids oder Esters, oder eines Salzes hiervon fördert oder unterdrückt, umfassend das Verwenden des Peptids nach Anspruch 1, seines Amids oder Esters, oder eines Salzes hiervon.

**15.** Kit, umfassend das Peptid nach Anspruch 1, sein Amid oder seinen Ester, oder ein Salz hiervon.

**16.** Verfahren zum Screenen einer Verbindung oder ihres Salzes, welche/welches die Expression des Peptids nach Anspruch 1, seines Amids oder Esters, oder eines Salzes hiervon fördert oder unterdrückt, umfassend das Verwenden des Polynukleotids nach Anspruch 6 oder 7.

**17.** Kit, umfassend das Polynukleotid nach Anspruch 6 oder 7.

**18.** Verfahren zum Screenen einer Verbindung oder ihres Salzes, welche/welches die Bindungseigenschaft zwischen (i) dem Peptid nach Anspruch 1, seinem Amid oder Ester, oder einem Salz hiervon und (ii) G-Protein-konjugiertem Rezeptorprotein, das die in SEQ ID NO: 31, SEQ ID NO: 44 oder SEQ ID NO: 46 dargestellte Aminosäuresequenz umfasst, oder einem Salz hiervon, verändert, umfassend das Verwenden (i) des Peptids nach Anspruch 1, seines Amids oder Esters, oder eines Salzes hiervon und (ii) G-Protein-konjugiertem Rezeptorprotein, das die in SEQ ID NO: 31, SEQ ID NO: 44 oder SEQ ID NO: 46 dargestellte Aminosäuresequenz umfasst, oder einem Salz hiervon.

**19.** Verfahren zum Screenen einer Verbindung oder ihres Salzes, welche/welches die Bindungseigenschaft zwischen (i) dem Peptid nach Anspruch 1, seinem Amid oder Ester, oder einem Salz hiervon und (ii) G-Protein-konjugiertem Rezeptorprotein, das die in SEQ ID NO: 33 oder SEQ ID NO: 35 dargestellte Aminosäuresequenz umfasst, oder einem Salz hiervon, verändert, umfassend das Verwenden (i) des Peptids nach Anspruch 1, seines Amids oder Esters, oder eines Salzes hiervon und (ii) G-Protein-konjugiertem Rezeptorprotein, das die in SEQ ID NO: 33 oder SEQ ID NO: 35 dargestellte Aminosäuresequenz umfasst, oder einem Salz hiervon.

**Revendications**

**1.** Peptide consistant en la séquence des acides aminés en positions 19 à 133, la séquence des acides aminés en positions 90 à 133, la séquence des acides aminés en positions 91 à 133, la séquence des acides aminés en positions 93 à 133, la séquence des acides aminés en positions 104 à 133, la séquence des acides aminés en positions 108 à 133, la séquence des acides aminés en positions 109 à 133, la séquence des acides aminés en positions 111 à 133, la séquence des acides aminés en positions 115 à 133, la séquence des acides aminés en positions 119 à 133, la séquence des acides aminés en positions 124 à 133, ou la séquence des acides aminés en positions 127 à 133, de la séquence d'acides aminés présentée en tant que Séquence N° 23, amide ou ester d'un tel peptide, ou sel d'un tel peptide, amide ou ester.

**2.** Peptide conforme à la revendication 1, consistant en la séquence des acides aminés en positions 91 à 133 de la Séquence N° 23, amide ou ester d'un tel peptide, ou sel d'un tel peptide, amide ou ester.

**3.** Peptide conforme à la revendication 1, consistant en la séquence des acides aminés en positions 108 à 133 de la Séquence N° 23, amide ou ester d'un tel peptide, ou sel d'un tel peptide, amide ou ester.

**4.** Peptide conforme à la revendication 1, consistant en la séquence des acides aminés en positions 124 à 133 de la Séquence N° 23, amide ou ester d'un tel peptide, ou sel d'un tel peptide, amide ou ester.

**5.** Peptide conforme à la revendication 1, consistant en la séquence des acides aminés en positions 127 à 133 de la Séquence N° 23, amide ou ester d'un tel peptide, ou sel d'un tel peptide, amide ou ester.

**6.** Polynucléotide codant un peptide conforme à la revendication 1.

**7.** Polynucléotide conforme à la revendication 6, consistant en la séquence des nucléotides en positions 271 à 399, ou en la séquence des nucléotides en positions 379 à 399, de la séquence de nucléotides présentée en tant que Séquence N° 24.

**8.** Vecteur recombiné comprenant un polynucléotide conforme à la revendication 6 ou 7.

**9.** Organisme transformé avec un vecteur recombiné conforme à la revendication 8.

**10.** Procédé de production d'un peptide conforme à la revendication 1, d'un amide ou d'un ester d'un tel peptide, ou d'un sel d'un tel peptide, amide ou ester, qui comporte le fait de cultiver un organisme transformé conforme à la revendication 9, pour obtenir un peptide conforme à la revendication 1.

**11.** Anticorps dirigé contre un peptide conforme à la revendication 1, contre un amide ou un ester d'un tel peptide, ou contre un sel d'un tel peptide, amide ou ester.

**12.** Anticorps conforme à la revendication 11, qui est un anticorps neutralisant, inhibiteur de l'activité d'un peptide conforme à la revendication 1, d'un amide ou d'un ester d'un tel peptide, ou d'un sel d'un tel peptide, amide ou ester.

**13.** Polynucléotide antisens, comprenant tout ou partie d'une séquence de nucléotides complémentaire d'un polynucléotide conforme à la revendication 6 ou 7.

**14.** Procédé de recherche par criblage d'un composé, ou d'un sel de composé, qui favorise ou inhibe l'activité de liaison au récepteur, de libération intracellulaire d'ions $Ca^{2+}$ et/ou de production intracellulaire d'AMP cyclique d'un peptide conforme à la revendication 1, d'un amide ou d'un ester d'un tel peptide, ou d'un sel d'un tel peptide, amide ou ester, lequel procédé comporte le fait d'utiliser un peptide conforme à la revendication 1, un amide ou un ester d'un tel peptide, ou un sel d'un tel peptide, amide ou ester.

**15.** Trousse comprenant un peptide conforme à la revendication 1, un amide ou un ester d'un tel peptide, ou un sel d'un tel peptide, amide ou ester.

**16.** Procédé de recherche par criblage d'un composé, ou d'un sel de composé, qui favorise ou inhibe l'expression d'un peptide conforme à la revendication 1, d'un amide ou d'un ester d'un tel peptide, ou d'un sel d'un tel peptide, amide ou ester, lequel procédé comporte le fait d'utiliser un polynucléotide conforme à la revendication 6 ou 7.

**17.** Trousse comprenant un polynucléotide conforme à la revendication 6 ou 7.

**18.** Procédé de recherche par criblage d'un composé, ou d'un sel de composé, qui modifie les caractéristiques de liaison entre (i) un peptide conforme à la revendication 1, un amide ou un ester d'un tel peptide, ou un sel d'un tel peptide, amide ou ester, et (ii) une protéine récepteur conjuguée à une protéine G, comprenant la séquence d'acides aminés présentée en tant que Séquence N° 31, N° 44 ou N° 46, ou un sel d'une telle protéine, lequel procédé comporte le fait d'utiliser (i) un peptide conforme à la revendication 1, un amide ou un ester d'un tel peptide, ou un sel d'un tel peptide, amide ou ester, et (ii) une protéine récepteur conjuguée à une protéine G, comprenant la séquence d'acides aminés présentée en tant que Séquence N° 31, N° 44 ou N° 46, ou un sel d'une telle protéine.

**19.** Procédé de recherche par criblage d'un composé, ou d'un sel de composé, qui modifie les caractéristiques de liaison entre (i) un peptide conforme à la revendication 1, un amide ou un ester d'un tel peptide, ou un sel d'un tel peptide, amide ou ester, et (ii) une protéine récepteur conjuguée à une protéine G, comprenant la séquence d'acides aminés présentée en tant que Séquence N° 33 ou N° 35, ou un sel d'une telle protéine, lequel procédé comporte le fait d'utiliser (i) un peptide conforme à la revendication 1, un amide ou un ester d'un tel peptide, ou un sel d'un tel peptide, amide ou ester, et (ii) une protéine récepteur conjuguée à une protéine G, comprenant la séquence d'acides aminés présentée en tant que Séquence N° 33 ou N° 35, ou un sel d'une telle protéine.

Fig. 1

```
atggggcctg gacgatgcct cctgacggcc ttgttgcttc tggccctggc gccaccgccg    60
gaagcctccc agtactgcgg ccgccttgaa tactggaacc cagacaacaa gtgctgcagc   120
agctgcctgc aacgcttcgg gccgcccccc tgcccggaac tgtccagtct ggcgtcacaa   180
cccctgtctc gcctcctgga tgagctggag gtgctggaag agctgattgt actgctggac   240
cctgagcctg ggccaggtgg gggtatggcc catggcacta ctcgacacct ggccgcaaga   300
tag                                                                 303
```

# Fig. 2

| | | | | | | | | | | | | | | | |
|---|---|---|---|---|---|---|---|---|---|---|---|---|---|---|---|
| Met | Gly | Pro | Gly | Arg | Cys | Leu | Leu | Thr | Ala | Leu | Leu | Leu | Leu | Ala | Leu |
| | | | | 5 | | | | | 10 | | | | | 15 | |
| Ala | Pro | Pro | Pro | Glu | Ala | Ser | Gln | Tyr | Cys | Gly | Arg | Leu | Glu | Tyr | Trp |
| | | | 20 | | | | | 25 | | | | | 30 | | |
| Asn | Pro | Asp | Asn | Lys | Cys | Cys | Ser | Ser | Cys | Leu | Gln | Arg | Phe | Gly | Pro |
| | | 35 | | | | | 40 | | | | | 45 | | | |
| Pro | Pro | Cys | Pro | Glu | Leu | Ser | Ser | Leu | Ala | Ser | Gln | Pro | Leu | Ser | Arg |
| | 50 | | | | | 55 | | | | | 60 | | | | |
| Leu | Leu | Asp | Glu | Leu | Glu | Val | Leu | Glu | Glu | Leu | Ile | Val | Leu | Leu | Asp |
| 65 | | | | | 70 | | | | | 75 | | | | | 80 |
| Pro | Glu | Pro | Gly | Pro | Gly | Gly | Gly | Met | Ala | His | Gly | Thr | Thr | Arg | His |
| | | | | 85 | | | | | 90 | | | | | 95 | |
| Leu | Ala | Ala | Arg | End | | | | | | | | | | | |
| | | | 100 | | | | | | | | | | | | |

# Fig. 3

```
atgtggcccc gaaaactcgc ctggattttg gcggcggccg cgttggcgcc gggatcgtcc   60
agcgggatgt ctagaatgtc gtcgtccggc ttggagcagg cgttctcctg ggggcgaagc  120
aggggggttc ttgtggggtc actgggtctt gatccgcggg agtggggtag gaatagagcc  180
ctgagggaca gggactatgg cgacgcgggg gtgggaggta cagagagggg tgtcgagcct  240
ggagcagggc tatggcaggg acactcactg gggcatgggc agcgacaggc gaagaaggtt  300
cagggccaga tgcccagtca tcgatttcaa ttcagttaa                         339
```

# Fig. 4

Met Trp Pro Arg Lys Leu Ala Trp Ile Leu Ala Ala Ala Ala Leu Ala
5 10 15
Pro Gly Ser Ser Ser Gly Met Ser Arg Met Ser Ser Ser Gly Leu Glu
20 25 30
Gln Ala Phe Ser Trp Gly Arg Ser Arg Gly Val Leu Val Gly Ser Leu
35 40 45
Gly Leu Asp Pro Arg Glu Trp Gly Arg Asn Arg Ala Leu Arg Asp Arg
50 55 60
Asp Tyr Gly Asp Ala Gly Val Gly Gly Thr Glu Arg Gly Val Glu Pro
65 70 75 80
Gly Ala Gly Leu Trp Gln Gly His Ser Leu Gly His Gly Gln Arg Gln
85 90 95
Ala Lys Lys Val Gln Gly Gln Met Pro Ser His Arg Phe Gln Phe Ser
100 105 110
End

Fig. 5

```
atgcgggcgc cactctgcct gctcctgctc gtcgcccacg ccgtggacat gctcgccctg    60
aaccgaagga agaagcaagt gggcactggc ctggggggca actgcacagg ctgtatcatc   120
tgctcagagg agaacggctg ttccacctgc cagcagaggc tcttcctgtt catccgccgg   180
gaaggcatcc gccagtacgg caagtgcctg cacgactgtc cccctgggta cttcggcatc   240
cgcggccagg aggtcaacag gtgcaaaaaa tgtggggcca cttgtgagag ctgcttcagc   300
caggacttct gcatccggtg caagaggcag ttttacttgt acaaggggaa gtgtctgccc   360
acctgcccgc cgggcacttt ggcccaccag aacacacggg agtgccaggg ggagtgtgaa   420
ctgggtccct ggggcggctg gagcccctgc acacacaatg gaaagacctg cggctcggct   480
tggggcctgg agagccgggt acgagaggct ggccgggctg ggcatgagga ggcagccacc   540
tgccaggtgc tttctgagtc aaggaaatgt cccatccaga ggccctgccc aggagagagg   600
agccccggcc agaagaaggg caggaaggac cggcgcccac gcaaggacag gaagctggac   660
cgcaggctgg acgtgaggcc gcgccagccc ggcctgcagc cctga                   705
```

# Fig. 6

Met Arg Ala Pro Leu Cys Leu Leu Leu Leu Val Ala His Ala Val Asp
5 10 15

Met Leu Ala Leu Asn Arg Arg Lys Lys Gln Val Gly Thr Gly Leu Gly
20 25 30

Gly Asn Cys Thr Gly Cys Ile Ile Cys Ser Glu Glu Asn Gly Cys Ser
35 40 45

Thr Cys Gln Gln Arg Leu Phe Leu Phe Ile Arg Arg Glu Gly Ile Arg
50 55 60

Gln Tyr Gly Lys Cys Leu His Asp Cys Pro Pro Gly Tyr Phe Gly Ile
65 70 75 80

Arg Gly Gln Glu Val Asn Arg Cys Lys Lys Cys Gly Ala Thr Cys Glu
85 90 95

Ser Cys Phe Ser Gln Asp Phe Cys Ile Arg Cys Lys Arg Gln Phe Tyr
100 105 110

Leu Tyr Lys Gly Lys Cys Leu Pro Thr Cys Pro Pro Gly Thr Leu Ala
115 120 125

His Gln Asn Thr Arg Glu Cys Gln Gly Glu Cys Glu Leu Gly Pro Trp
130 135 140

Gly Gly Trp Ser Pro Cys Thr His Asn Gly Lys Thr Cys Gly Ser Ala
145 150 155 160

Trp Gly Leu Glu Ser Arg Val Arg Glu Ala Gly Arg Ala Gly His Glu
165 170 175

Glu Ala Ala Thr Cys Gln Val Leu Ser Glu Ser Arg Lys Cys Pro Ile
180 185 190

Gln Arg Pro Cys Pro Gly Glu Arg Ser Pro Gly Gln Lys Lys Gly Arg
195 200 205

Lys Asp Arg Arg Pro Arg Lys Asp Arg Lys Leu Asp Arg Arg Leu Asp
210 215 220

Val Arg Pro Arg Gln Pro Gly Leu Gln Pro End
225 230 235

# Fig. 7

```
atgggggccc cgctcgccgt agcgctgggc gccctccact acctggcact tttcctgcaa    60
ctcggcggcg ccacgcggcc cgccggccac gcgccctggg acaaccacgt ctccggccac   120
gccctgttca cagagacacc ccatgacatg acagcacgga cgggcgagga cgtggagatg   180
gcctgctcct tccgcggcag cggctccccc tcctactcgc tggagatcca gtggtggtat   240
gtacggagcc accgggactg gaccgacaag caggcgtggg cctcgaacca ggtggtcaag   300
gtggtggca gcaacatctc ccacaagctg cgcctgtccc gggtgaagcc cacggacgaa   360
ggcacctacg agtgccgcgt catcgacttc agcgacggca aggcccggca ccacaaggtc   420
aaggcctacc tgcgggtgca gccaggggag aactccctcc tcctaccatc cctcacttgg   480
acctgggggt gtggacagtg a                                             501
```

# Fig. 8

Met Gly Ala Pro Leu Ala Val Ala Leu Gly Ala Leu His Tyr Leu Ala
5 10 15
Leu Phe Leu Gln Leu Gly Gly Ala Thr Arg Pro Ala Gly His Ala Pro
20 25 30
Trp Asp Asn His Val Ser Gly His Ala Leu Phe Thr Glu Thr Pro His
35 40 45
Asp Met Thr Ala Arg Thr Gly Glu Asp Val Glu Met Ala Cys Ser Phe
50 55 60
Arg Gly Ser Gly Ser Pro Ser Tyr Ser Leu Glu Ile Gln Trp Trp Tyr
65 70 75 80
Val Arg Ser His Arg Asp Trp Thr Asp Lys Gln Ala Trp Ala Ser Asn
85 90 95
Gln Val Val Lys Val Val Gly Ser Asn Ile Ser His Lys Leu Arg Leu
100 105 110
Ser Arg Val Lys Pro Thr Asp Glu Gly Thr Tyr Glu Cys Arg Val Ile
115 120 125
Asp Phe Ser Asp Gly Lys Ala Arg His His Lys Val Lys Ala Tyr Leu
130 135 140
Arg Val Gln Pro Gly Glu Asn Ser Leu Leu Leu Pro Ser Leu Thr Trp
145 150 155 160
Thr Trp Gly Cys Gly Gln End
165

Fig. 9

```
atggccccag ccttcctgct gctgctgctg ctgtggccac agggttgcgt ctcaggcccc    60
tctgctgaca gtgtatacac aaaagtgagg ctccttgaag ggagactct gtctgtgcag    120
tgctcctata agggctacaa aaaccgcgtg gagggcaagg tttggtgcaa aatcaggaag   180
aagaagtgtg agcctggctt tgcccgagtc tgggtgaaag ggccccgcta cttgctgcag   240
gacgatgccc aggccaaggt ggtcaacatc accatggtgg ccctcaagct ccaggactca   300
ggccgatact ggtgcatgcg caacacctct gggatcctgt accccttgat gggcttccag   360
ctggatgtgt ctccagctcc ccaaactgag aggaacattc ctttcacaca tctggacaac   420
atcctcaaga gtggaactgt cacaactggc caagccccta cctcaggccc tgatgcccct   480
tttaccactg gtgtgatggt gttcacccca ggactcatca ccttgcctag gctcttagcc   540
tccgccagac ctgcctccaa gacaggctac agcttcactg ctaccagcac caccagccag   600
ggacccagga ggaccatggg gtcccagaca gtgaccgcgt ctcccagcaa tgccacctgc   660
ccgggcggcc gctcgagccc tatagtgagt aagggcgaat tccagcacac tggcggccgg   720
tactag                                                              726
```

# Fig. 10

Met Ala Pro Ala Phe Leu Leu Leu Leu Leu Leu Trp Pro Gln Gly Cys
5 10 15
Val Ser Gly Pro Ser Ala Asp Ser Val Tyr Thr Lys Val Arg Leu Leu
20 25 30
Glu Gly Glu Thr Leu Ser Val Gln Cys Ser Tyr Lys Gly Tyr Lys Asn
35 40 45
Arg Val Glu Gly Lys Val Trp Cys Lys Ile Arg Lys Lys Lys Cys Glu
50 55 60
Pro Gly Phe Ala Arg Val Trp Val Lys Gly Pro Arg Tyr Leu Leu Gln
65 70 75 80
Asp Asp Ala Gln Ala Lys Val Val Asn Ile Thr Met Val Ala Leu Lys
85 90 95
Leu Gln Asp Ser Gly Arg Tyr Trp Cys Met Arg Asn Thr Ser Gly Ile
100 105 110
Leu Tyr Pro Leu Met Gly Phe Gln Leu Asp Val Ser Pro Ala Pro Gln
115 120 125
Thr Glu Arg Asn Ile Pro Phe Thr His Leu Asp Asn Ile Leu Lys Ser
130 135 140
Gly Thr Val Thr Thr Gly Gln Ala Pro Thr Ser Gly Pro Asp Ala Pro
145 150 155 160
Phe Thr Thr Gly Val Met Val Phe Thr Pro Gly Leu Ile Thr Leu Pro
165 170 175
Arg Leu Leu Ala Ser Ala Arg Pro Ala Ser Lys Thr Gly Tyr Ser Phe
180 185 190
Thr Ala Thr Ser Thr Thr Ser Gln Gly Pro Arg Arg Thr Met Gly Ser
195 200 205
Gln Thr Val Thr Ala Ser Pro Ser Asn Ala Thr Cys Pro Gly Gly Arg
210 215 220
Ser Ser Pro Ile Val Ser Lys Gly Glu Phe Gln His Thr Gly Gly Arg
225 230 235 240
Tyr End

Fig. 11

```
atggtaaggc cttaccccct gatctacttc ctcttcctgc cgctgggcgc ctgcttccct    60
ctactggaca gaagagagcc cacagacgcc atgggtggcc tcggagctgg agaacgctgg   120
gccgacctgg ccatggggcc ccgaccccac tccgtgtggg gttcctctcg gtggctgaga   180
gcttcacagc cacaggccct gcttgtcata gccagggggc tgcagacatc gggcagagag   240
catgctggct gcagattccg cttcgggagg caggacgaag gcagtgaggc caccggcttc   300
ctccctgctg cgggggagaa gaccagcggc ccgttaggga acctggctga ggagctcaat   360
ggctacagca ggaagaaagg cggcttcagc ttccgcttcg gtcggcggtg a            411
```

# Fig. 12

Met Val Arg Pro Tyr Pro Leu Ile Tyr Phe Leu Phe Leu Pro Leu Gly
5 10 15
Ala Cys Phe Pro Leu Leu Asp Arg Arg Glu Pro Thr Asp Ala Met Gly
20 25 30
Gly Leu Gly Ala Gly Glu Arg Trp Ala Asp Leu Ala Met Gly Pro Arg
35 40 45
Pro His Ser Val Trp Gly Ser Ser Arg Trp Leu Arg Ala Ser Gln Pro
50 55 60
Gln Ala Leu Leu Val Ile Ala Arg Gly Leu Gln Thr Ser Gly Arg Glu
65 70 75 80
His Ala Gly Cys Arg Phe Arg Phe Gly Arg Gln Asp Glu Gly Ser Glu
85 90 95
Ala Thr Gly Phe Leu Pro Ala Ala Gly Glu Lys Thr Ser Gly Pro Leu
100 105 110
Gly Asn Leu Ala Glu Glu Leu Asn Gly Tyr Ser Arg Lys Lys Gly Gly
115 120 125
Phe Ser Phe Arg Phe Gly Arg Arg End
130 135

Fig. 13

```
atgaggtgcc tctgctcttg gctttgcctc ctcctgcctc tgagtgcctg ctttcctctg    60
ctggacagaa ggggacccac agacatcggt gacatcggag ccagaatgag ctgggtccag   120
ctgactgagg gacacacccc ccgctcagtt caaagtccac ggccacaggc cctgctcgtg   180
gtggccaagg agcagcaggc ctctcgcagg gagcacactg gcttccgtct agggaggcag   240
gacagtggca gtgaagccac ggggttcctg cccactgact cagagaaggc cagcggcccc   300
ctggggactc tggcagagga gctcagcagc tacagccggc ggaagggagg cttcagcttc   360
cgcttcggcc ggtga                                                    375
```

# Fig. 14

Met Arg Cys Leu Cys Ser Trp Leu Cys Leu Leu Leu Pro Leu Ser Ala
5 10 15
Cys Phe Pro Leu Leu Asp Arg Arg Gly Pro Thr Asp Ile Gly Asp Ile
20 25 30
Gly Ala Arg Met Ser Trp Val Gln Leu Thr Glu Gly His Thr Pro Arg
35 40 45
Ser Val Gln Ser Pro Arg Pro Gln Ala Leu Leu Val Val Ala Lys Glu
50 55 60
Gln Gln Ala Ser Arg Arg Glu His Thr Gly Phe Arg Leu Gly Arg Gln
65 70 75 80
Asp Ser Gly Ser Glu Ala Thr Gly Phe Leu Pro Thr Asp Ser Glu Lys
85 90 95
Ala Ser Gly Pro Leu Gly Thr Leu Ala Glu Glu Leu Ser Ser Tyr Ser
100 105 110
Arg Arg Lys Gly Gly Phe Ser Phe Arg Phe Gly Arg End
115 120 125

Fig. 15

```
human  MVRPYPLIYFLFLPLGACFPLLDRREPTDAHGGLGAGERWADLAHGPRPH   50
rat    MRCLCSWLC-LLLPLSACFPLLDRRCPTD-IGDIGARNSWVQLTSGHTPR   48

human  SVWGSSRWLRASQPQALLVIARGLQTSGREHAGCRFRFGRQDEGSEATGF  100
rat    SV-------QSPRPQALLVVAKEQQASRREHTG--FRLGRQDSGSEATGP   89

human  LPAAGEKTSGPLGNLAEELNGYSRKKGGPSFRFGRR                136
rat    LPTDSEKASGPLGTLAEELSSYSRRKGGPSPRFGR                 124
```

Fig. 16

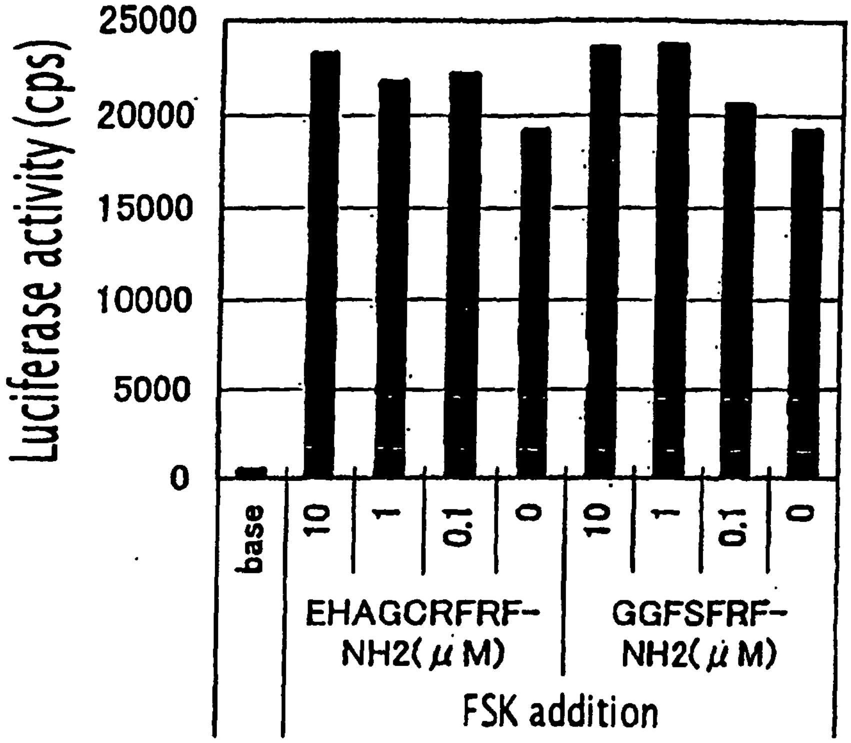

25000
20000
15000
10000
5000
0
Luciferase activity (cps)
base
10
1
0.1
0
10
1
0.1
0
EHAGCRFRF-
NH2(μM)
GGFSFRF-
NH2(μM)
FSK addition

Fig. 17

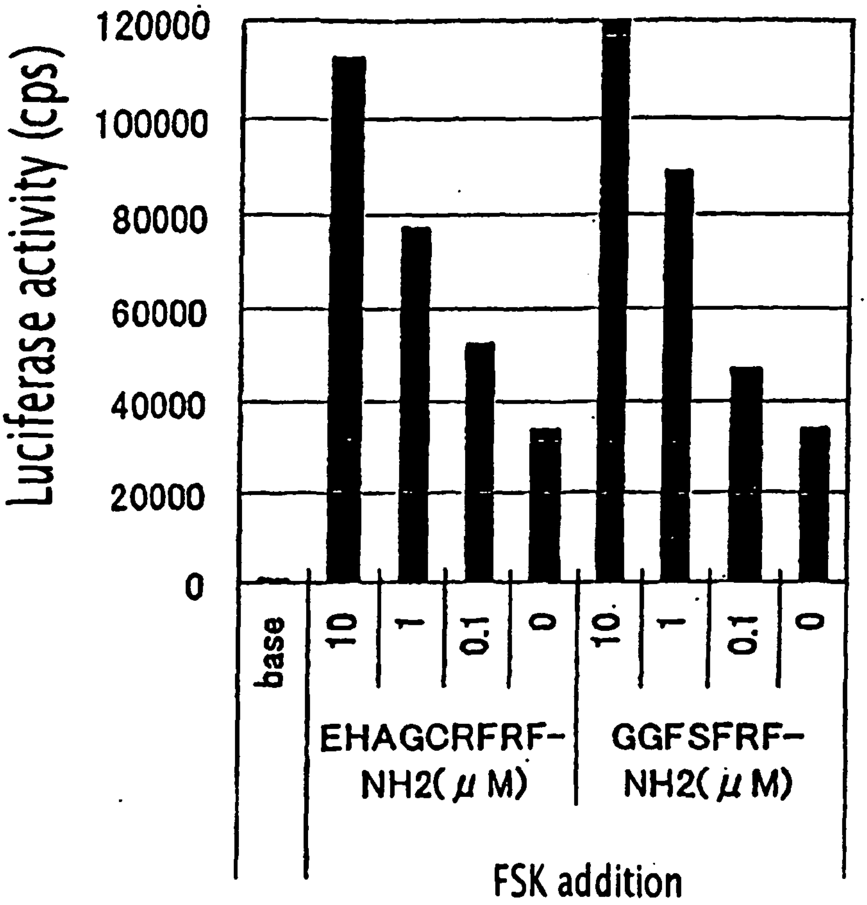

Luciferase activity (cps)
120000
100000
80000
60000
40000
20000
0
base
10
1
0.1
0
10
1
0.1
0
EHAGCRFRF-NH2(μM)
GGFSFRF-NH2(μM)
FSK addition

Fig. 18

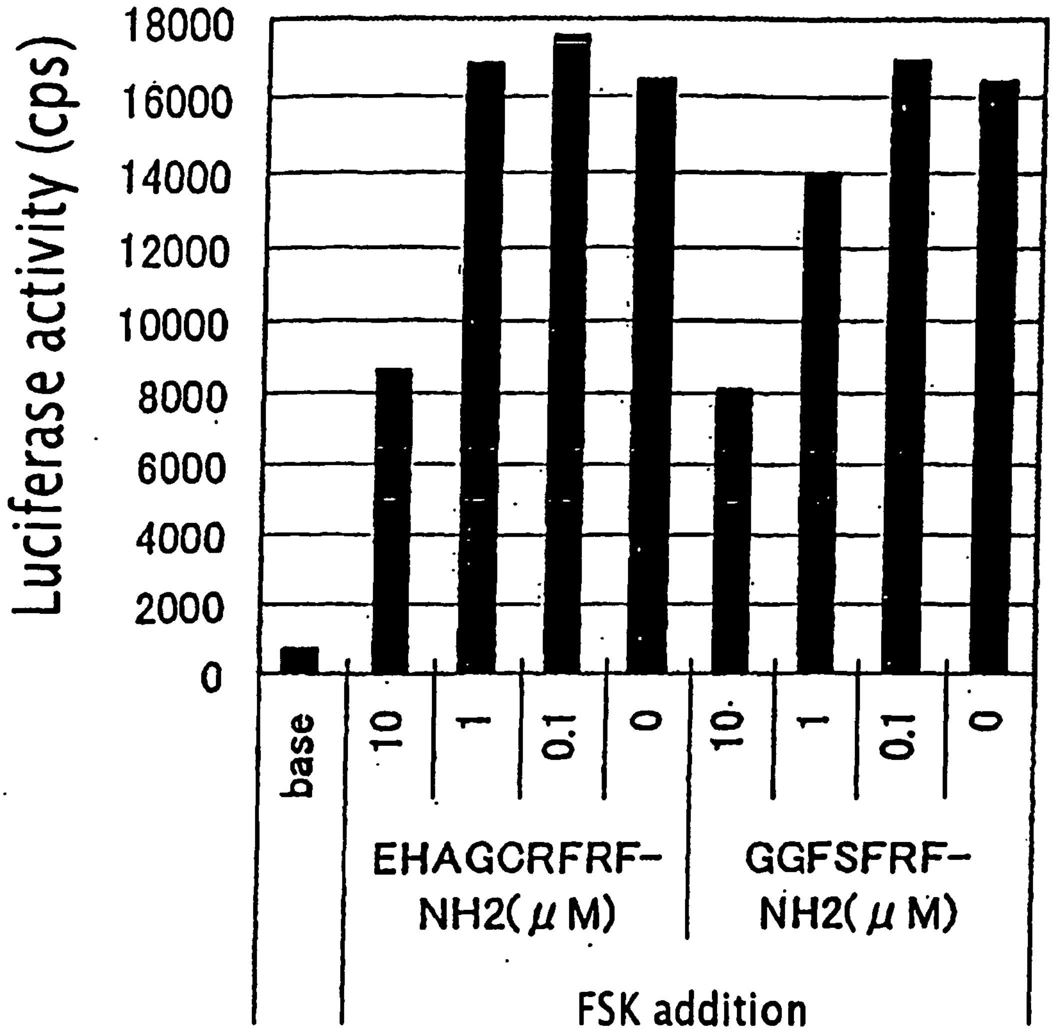
Luciferase activity (cps)
18000
16000
14000
12000
10000
8000
6000
4000
2000
0
base
10
1
0.1
0
10
1
0.1
0
EHAGCRFRF-NH2(μM)
GGFSFRF-NH2(μM)
FSK addition

Fig. 19

```
atgaggggct tccggccttt gctttcccta cttctccctc tgagtgcctg ctttcccctg   60
ctggacagaa ggggacccac agacatcggt gacatcggag ccaggatgaa ctgggcccag  120
ctggctgagg gacatccccc caactcggtt caaaatccac agccacaggc cctgcttgtg  180
gtggccaggg agcagcaggc ctcccacagg gagcacaccg gcttccgtct agggaggcaa  240
gacggtagca gtgaggccgc agggttcctg cccgccgact cggagaaggc cagcggccct  300
ctggggactc tggcagagga gctgagcagc tacagccgga ggaagggagg cttcagcttc  360
cgctttggac ggtga                                                   375
```

Fig. 20

Met Arg Gly Phe Arg Pro Leu Leu Ser Leu Leu Leu Pro Leu Ser Ala
5 10 15
Cys Phe Pro Leu Leu Asp Arg Arg Gly Pro Thr Asp Ile Gly Asp Ile
20 25 30
Gly Ala Arg Met Asn Trp Ala Gln Leu Ala Glu Gly His Pro Pro Asn
35 40 45
Ser Val Gln Asn Pro Gln Pro Gln Ala Leu Leu Val Val Ala Arg Glu
50 55 60
Gln Gln Ala Ser His Arg Glu His Thr Gly Phe Arg Leu Gly Arg Gln
65 70 75 80
Asp Gly Ser Ser Glu Ala Ala Gly Phe Leu Pro Ala Asp Ser Glu Lys
85 90 95
Ala Ser Gly Pro Leu Gly Thr Leu Ala Glu Glu Leu Ser Ser Tyr Ser
100 105 110
Arg Arg Lys Gly Gly Phe Ser Phe Arg Phe Gly Arg End
115 120 125

Fig. 21

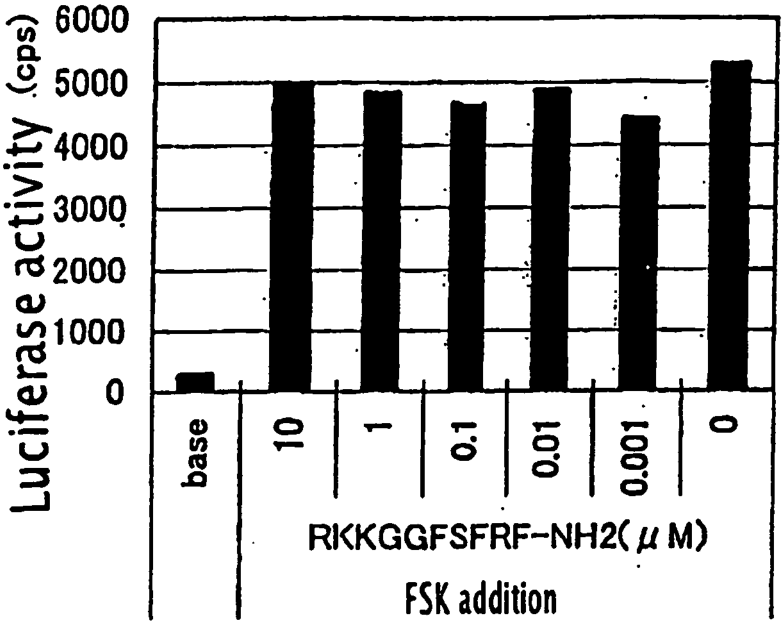
Luciferase activity (cps)
6000
5000
4000
3000
2000
1000
0
base
10
1
0.1
0.01
0.001
0
RKKGGFSFRF-NH2(μM)
FSK addition

Fig. 22

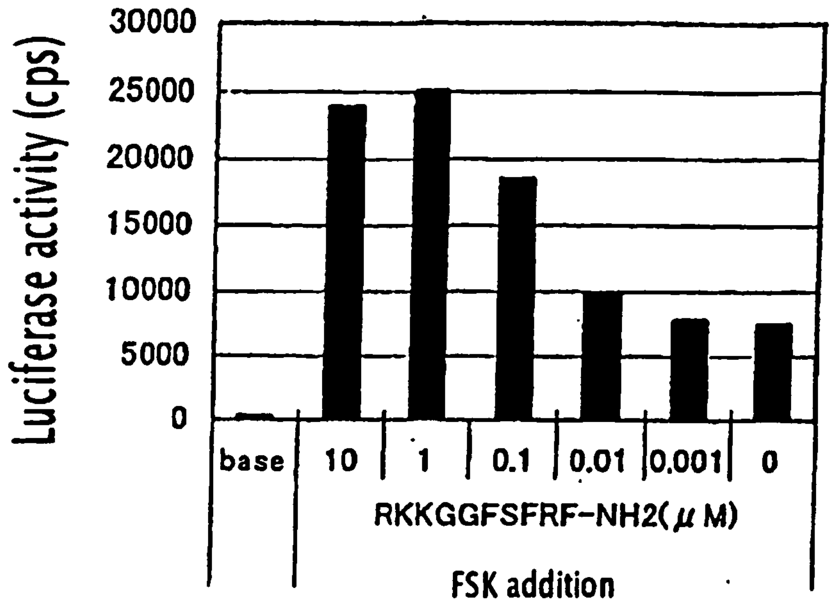
Luciferase activity (cps)
30000
25000
20000
15000
10000
5000
0
base
10
1
0.1
0.01
0.001
0
RKKGGFSFRF-NH2(μM)
FSK addition

Fig. 23

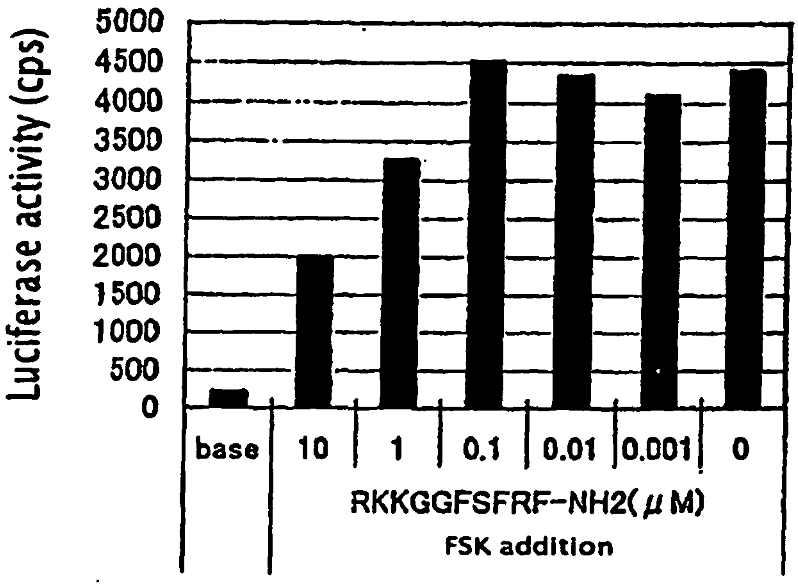
Luciferase activity (cps)
5000
4500
4000
3500
3000
2500
2000
1500
1000
500
0
base
10
1
0.1
0.01
0.001
0
RKKGGFSFRF-NH2(μM)
FSK addition

Fig. 24

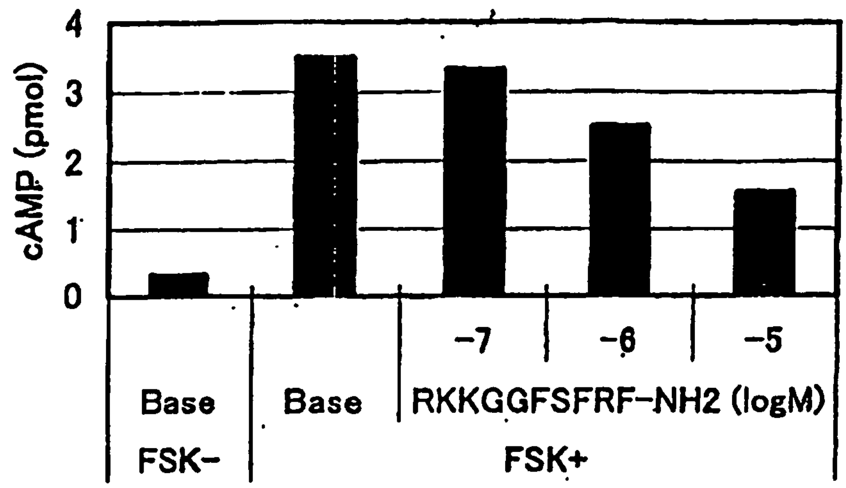

Fig. 25

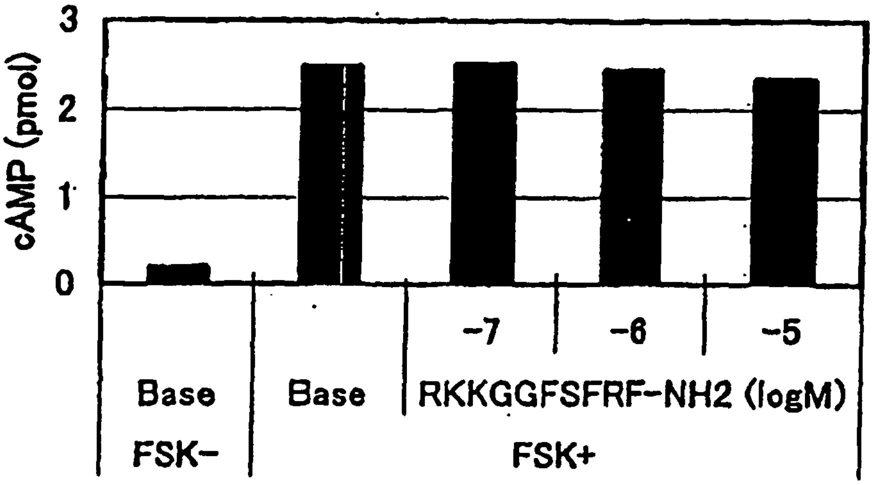

Fig. 26

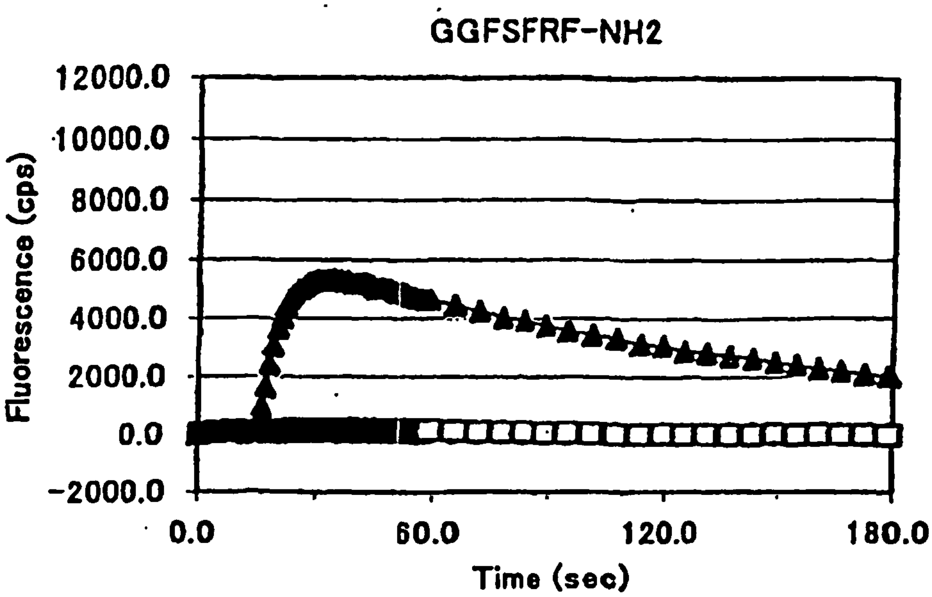
GGFSFRF-NH2
12000.0
10000.0
8000.0
6000.0
4000.0
2000.0
0.0
-2000.0
Fluorescence (cps)
0.0
60.0
120.0
180.0
Time (sec)

Fig. 27

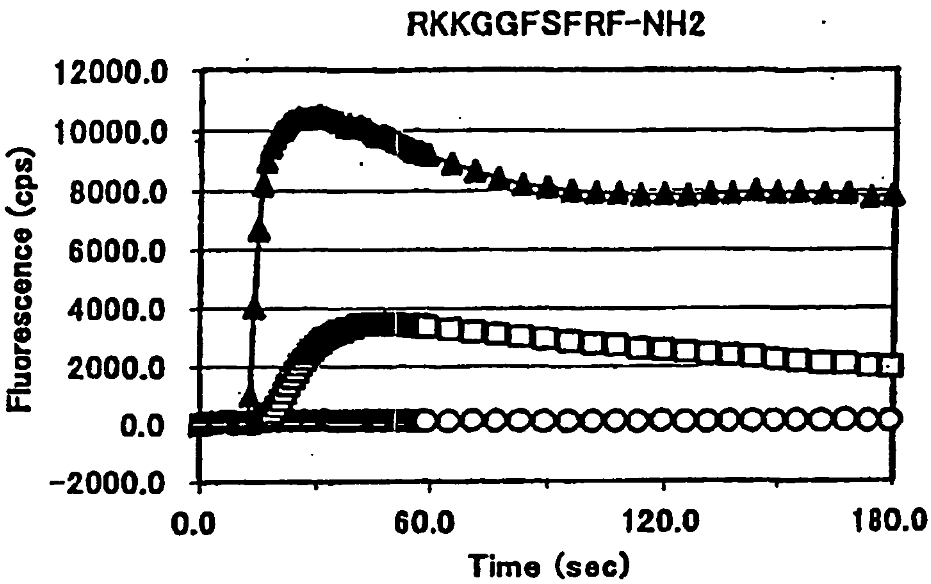
RKKGGFSFRF-NH2
12000.0
10000.0
8000.0
6000.0
4000.0
2000.0
0.0
-2000.0
Fluorescence (cps)
0.0
60.0
120.0
180.0
Time (sec)

Fig. 28

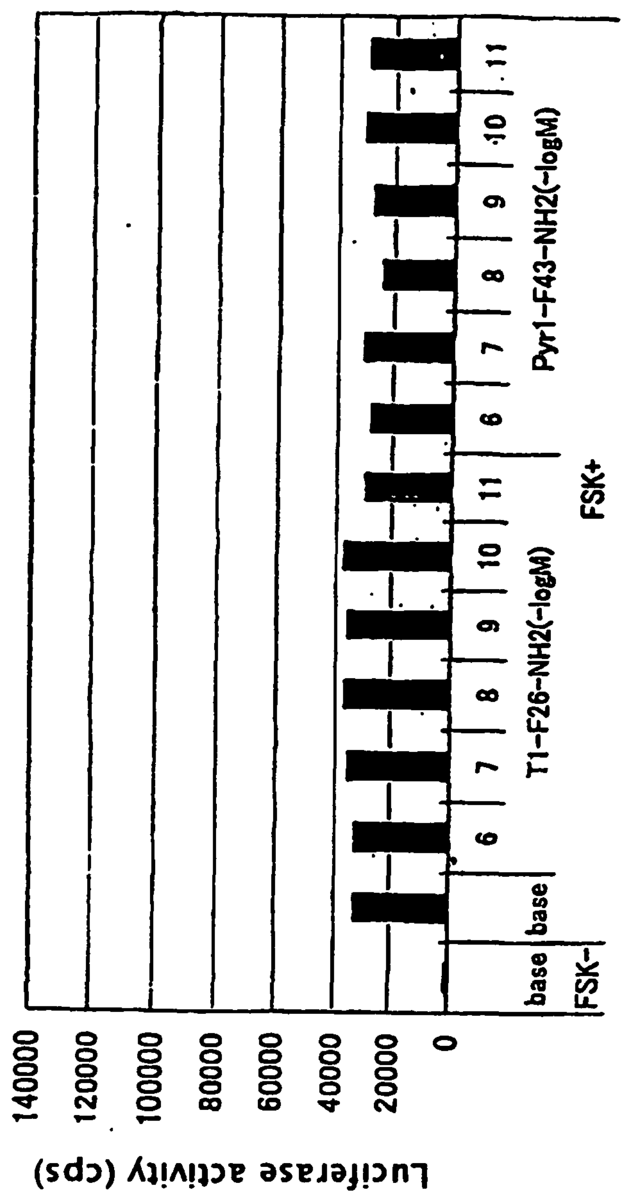
Luciferase activity (cps)
140000
120000
100000
80000
60000
40000
20000
0
base
base
6
7
8
9
10
11
6
7
8
9
10
11
T1-F26-NH2(-logM)
Pyr1-F43-NH2(-logM)
FSK-
FSK+

Fig. 29

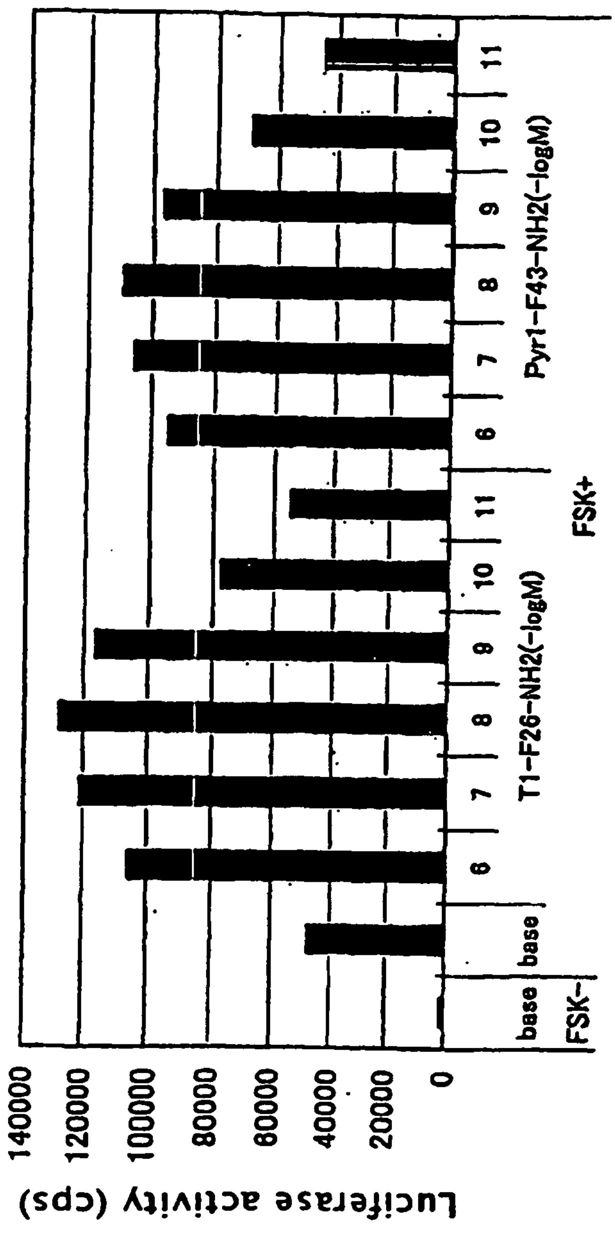
Luciferase activity (cps)
140000
120000
100000
80000
60000
40000
20000
0
base
base
6
7
8
9
10
11
6
7
8
9
10
11
T1-F26-NH2(-logM)
Pyr1-F43-NH2(-logM)
FSK-
FSK+

Fig. 30

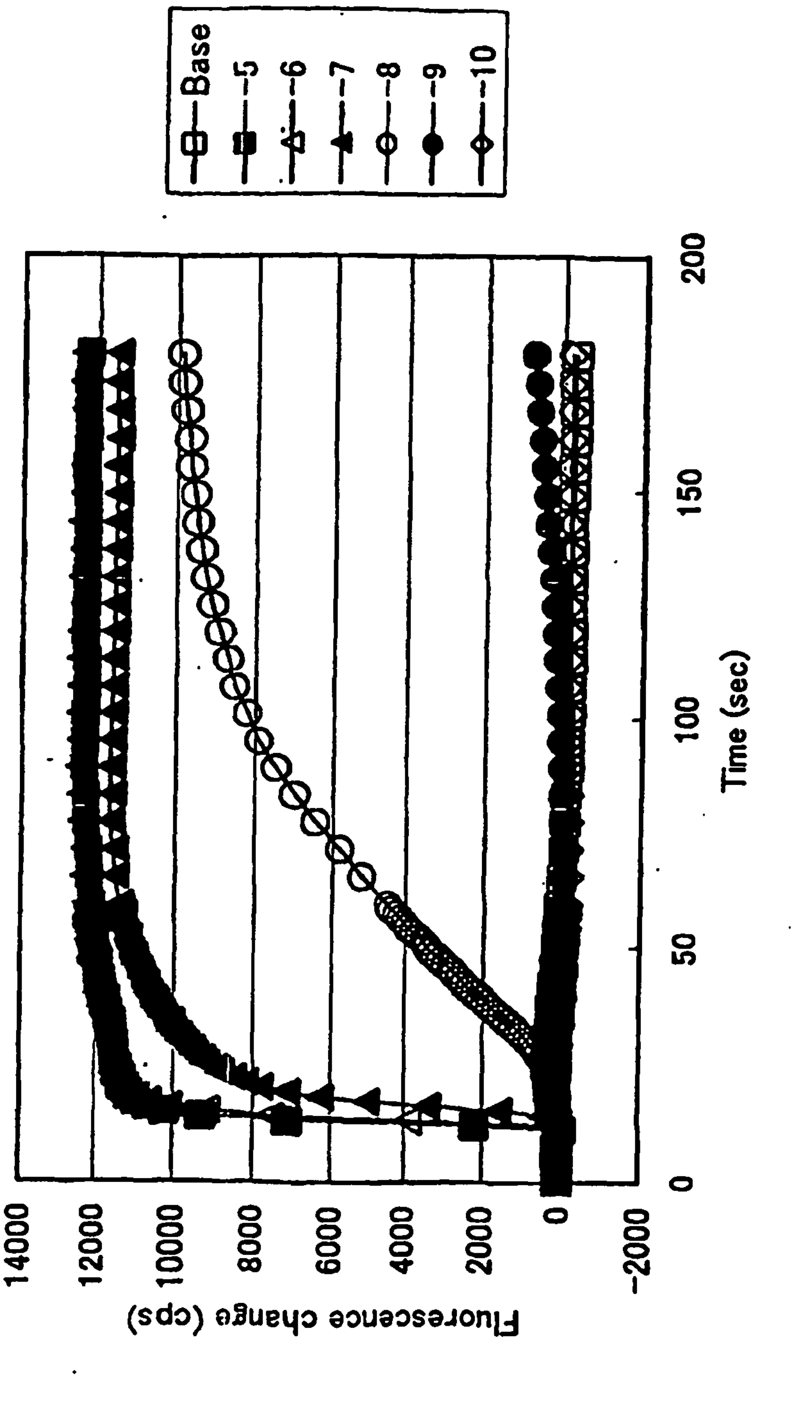
Base
-5
-6
-7
-8
-9
-10
Fluorescence change (cps)
14000
12000
10000
8000
6000
4000
2000
0
-2000
0
50
100
150
200
Time (sec)

Fig. 31

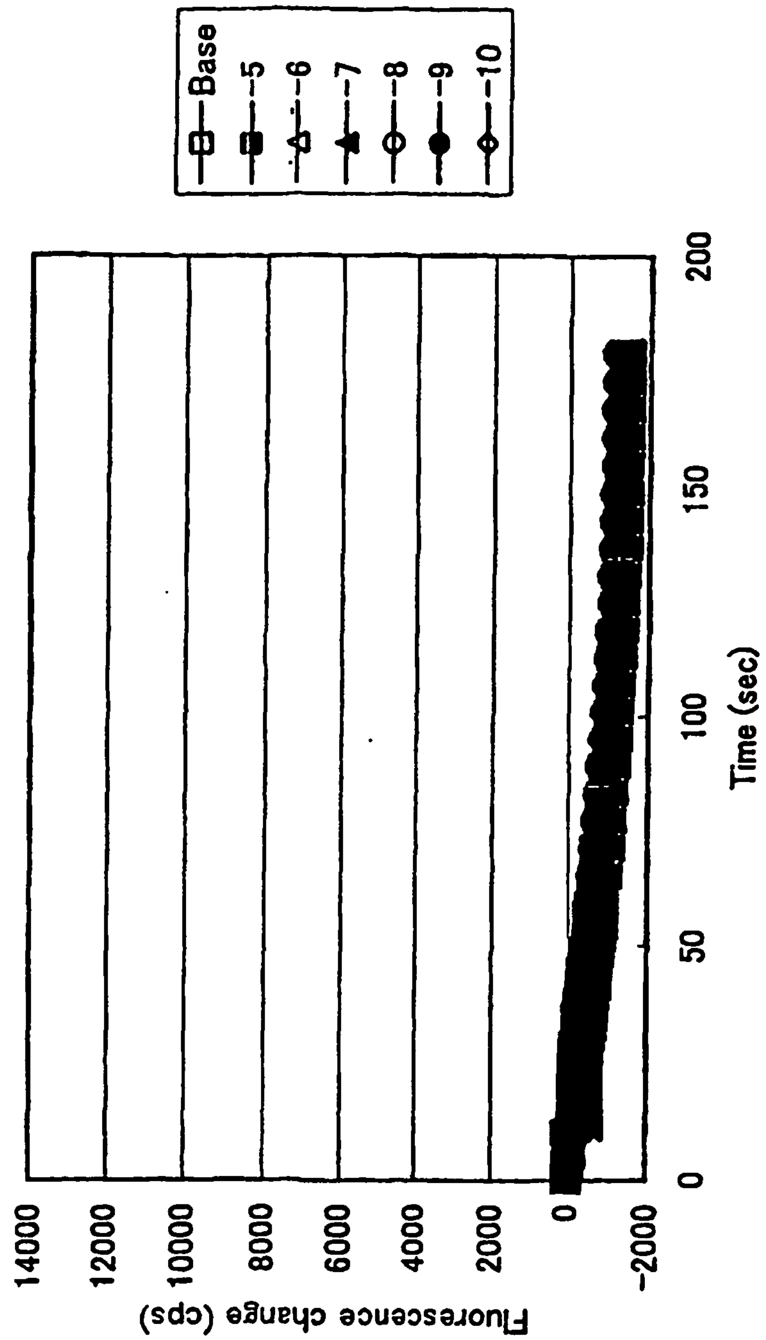

Base
5
6
7
8
9
10
Fluorescence change (cps)
14000
12000
10000
8000
6000
4000
2000
0
−2000
0
50
100
150
200
Time (sec)

Fig. 32

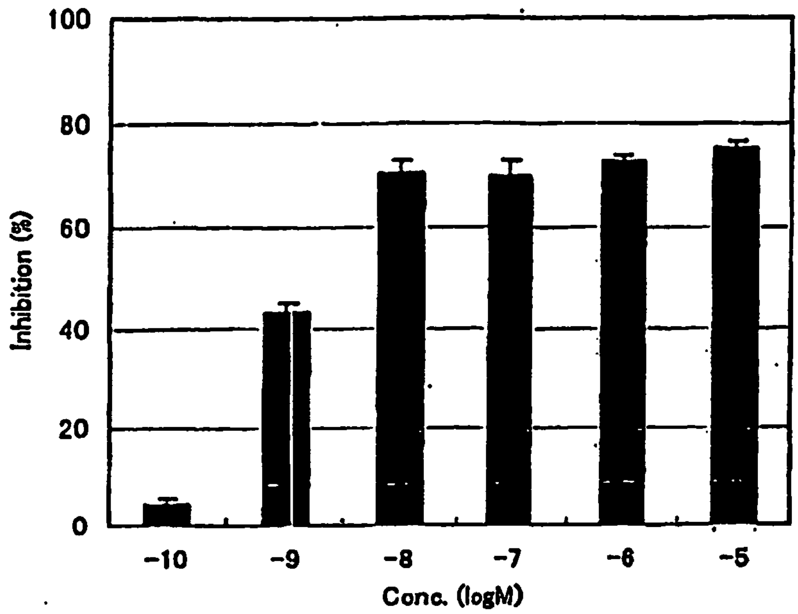

Fig. 33

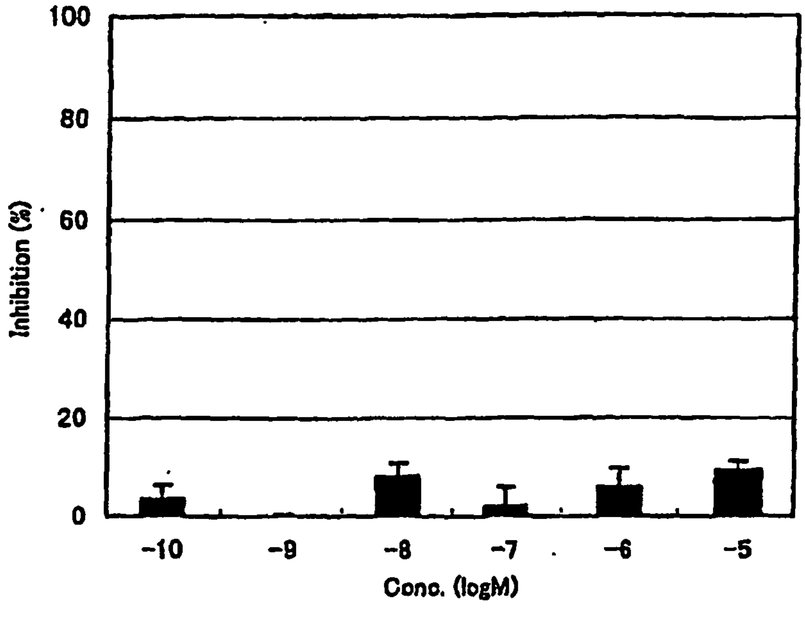

Fig. 34

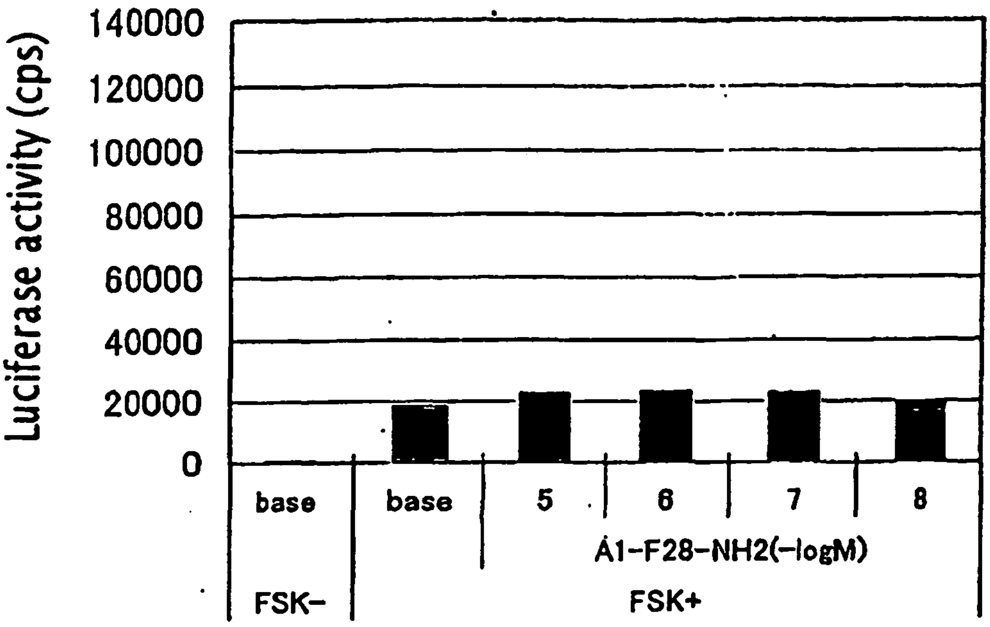
Luciferase activity (cps)
140000
120000
100000
80000
60000
40000
20000
0
base
base
5
6
7
8
A1-F28-NH2(-logM)
FSK-
FSK+

Fig. 35

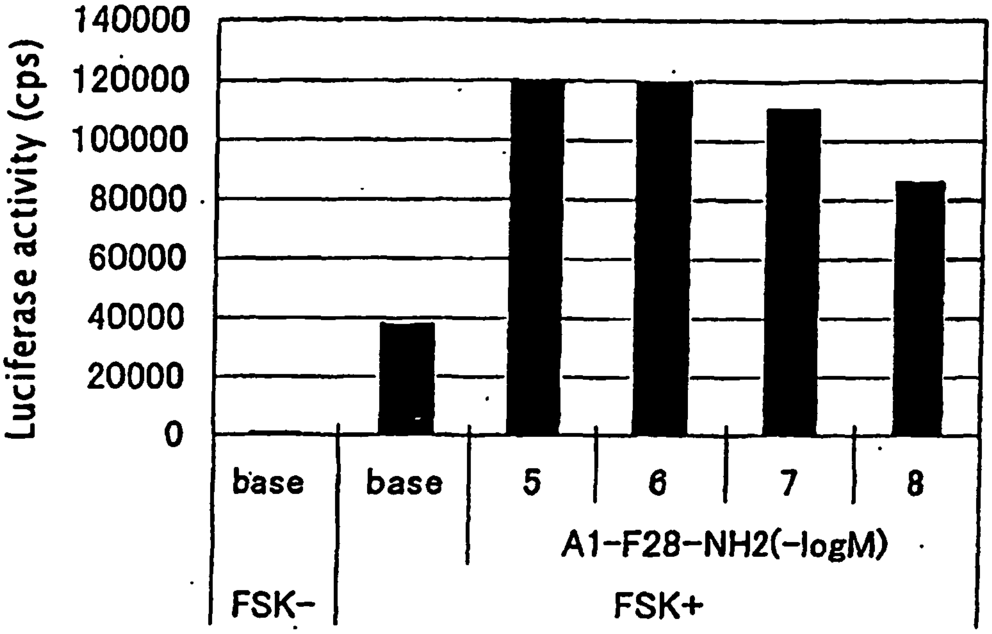
Luciferase activity (cps)
140000
120000
100000
80000
60000
40000
20000
0
base
base
5
6
7
8
A1-F28-NH2(-logM)
FSK-
FSK+

Fig. 36

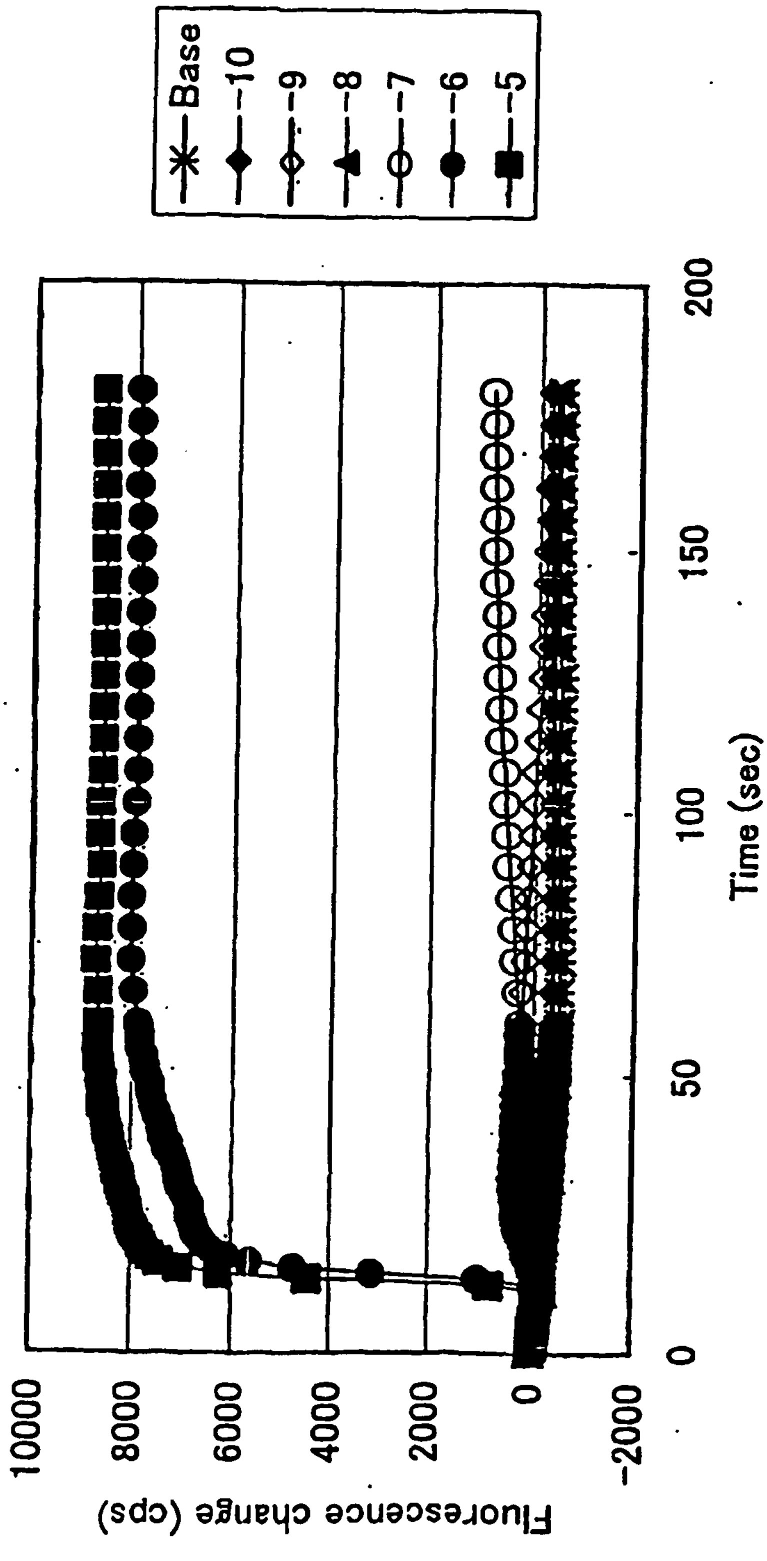

Fluorescence change (cps)
10000
8000
6000
4000
2000
0
-2000
Time (sec)
0
50
100
150
200
Base
-10
-9
-8
-7
-6
-5

Fig. 37

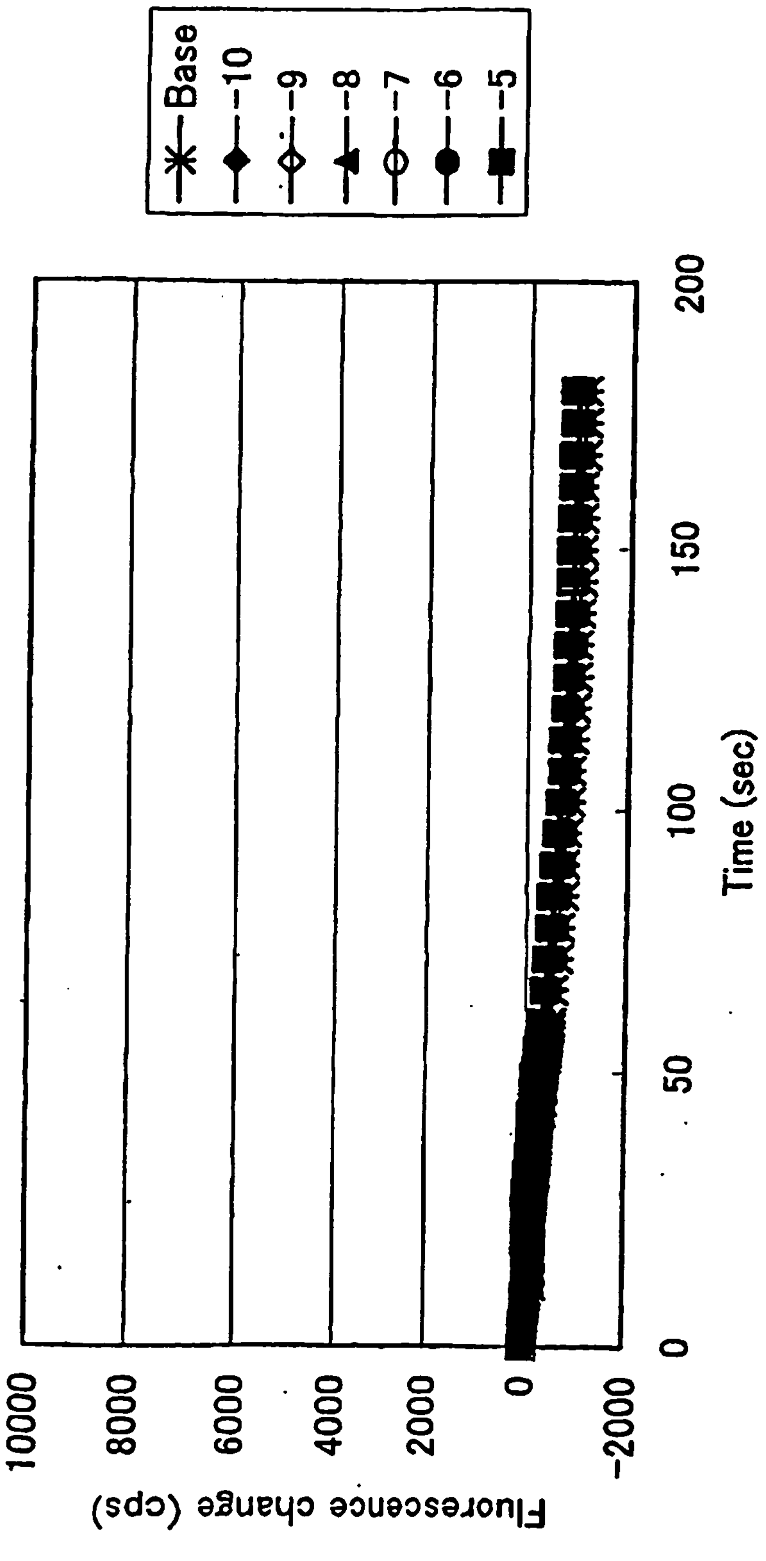
Base
-10
-9
-8
-7
-6
-5
Fluorescence change (cps)
10000
8000
6000
4000
2000
0
-2000
Time (sec)
0
50
100
150
200

Fig. 38

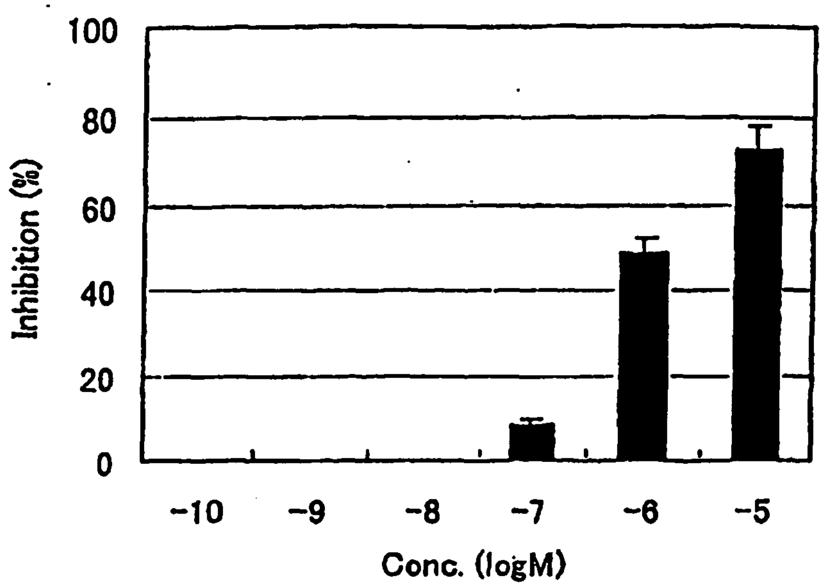

Fig. 39

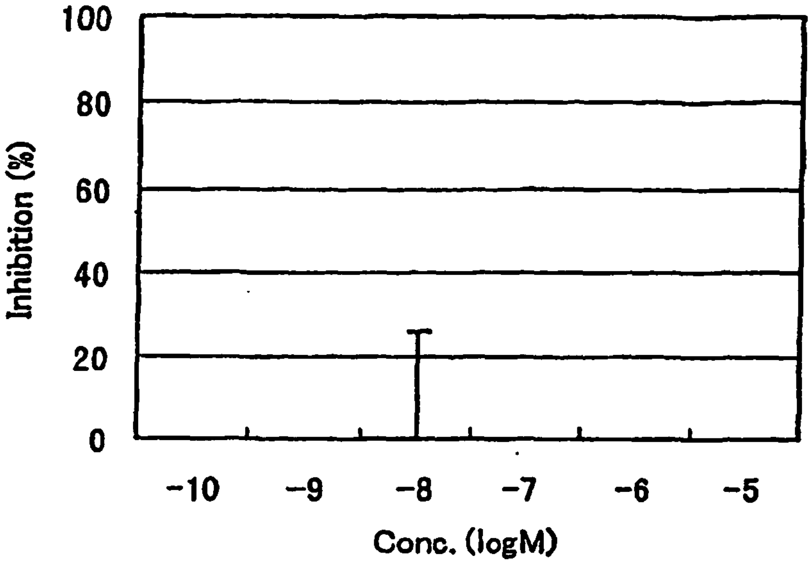

Fig. 40

```
atgcggagcc cttactccct gccctacctc ctgttcctgc ccctgggtgc ctgcttcccg   60
gtgctggaca cagaggagcc tgtggatgcc gtagggggca ccggacgtga aatgagctgg  120
atggacccgg cgaggggacg cccctttccg tggggctccc ctgggtggcc gagagccccg  180
tacccacacg ccctgctcgt cacggccaag gagctgcggg cgtcaggcaa ggcgcgtgct  240
ggcttccagc tgcgtctcgg gaggcaggac gatggcagcg aggccactgg cctcctcctg  300
ggggaggccg agaaggtggg gggcctgttg ggaaccctgg ctgaggagct caatggctac  360
agcaggaaga aggggggctt cagcttccgc ttcggtcggc ggtga                  405
```

# Fig. 41

Met Arg Ser Pro Tyr Ser Leu Pro Tyr Leu Leu Phe Leu Pro Leu Gly
5 10 15
Ala Cys Phe Pro Val Leu Asp Thr Glu Glu Pro Val Asp Ala Val Gly
20 25 30
Gly Thr Gly Arg Glu Met Ser Trp Met Asp Pro Ala Arg Gly Arg Pro
35 40 45
Phe Pro Trp Gly Ser Pro Gly Trp Pro Arg Ala Pro Tyr Pro His Ala
50 55 60
Leu Leu Val Thr Ala Lys Glu Leu Arg Ala Ser Gly Lys Ala Arg Ala
65 70 75 80
Gly Phe Gln Leu Arg Leu Gly Arg Gln Asp Asp Gly Ser Glu Ala Thr
85 90 95
Gly Leu Leu Leu Gly Glu Ala Glu Lys Val Gly Gly Leu Leu Gly Thr
100 105 110
Leu Ala Glu Glu Leu Asn Gly Tyr Ser Arg Lys Lys Gly Gly Phe Ser
115 120 125
Phe Arg Phe Gly Arg Arg End
130 135

Fig. 42

```
atgcaggcgc tcaacatcac cgcggagcag ttctcccggc tgctgagcgc gcacaacctg   60
actcgggagc agttcattca tcgctatggg ctgagaccgc tggtctacac tccggagctg  120
cccgcgcgtg ctaaagtggc ctttgcgctg gcaggagcac tcatttttgc cctggcgctc  180
ttcggcaact ctctggtcat ctatgtggtg acccgcagca aggccatgcg caccgtcacc  240
aacatcttca tctgctctct ggcactcagt gatctgctca ttgccttctt ctgcatcccc  300
gtcacgatgc tccagaacat ctccgacaag tggctgggtg gtgccttcat ctgcaagatg  360
gtacccttttg tccagtccac ggccgtcgtg acagaaatcc tcaccatgac ctgcatcgct  420
gttgagaggc accaaggact tgtccatcct tttaaaatga agtggcagta caccacccga  480
agggccttca cgatcttggg cgtggtctgg ttggcggcca tcatcgtagg atcacccatg  540
tggcacgtgc aacgccttga gattaagtat gacttcctct atgaaaaaga acacatctgc  600
tgcttggaag aatgggccag ccccgtgcac cagagaatct acagcacctt cattctcgtc  660
atcctcttcc tcctgcctct tgtggtaatg ctagtcctct atagcaagat tggctatgaa  720
ctgtggatca agaagagagt gggagacagt tcagcgcttc aaactatcca cgggaaagaa  780
atgtccaaaa tagccaggaa gaagaagcgg gctgtcatta tgatggtgac tgtggtggct  840
ctctttgctg catgctgggc acctttccac gttgttcaca tgatggttga gtacagtaat  900
tttgaaaaag aatatgatga tgtcacaatc aagatggtct ttgctgtcgc gcagacaatt  960
ggctttttca actccatctg taatcccttt gtgtatgcgt ttatgaatga aaacttcaaa 1020
aagaattttc tgtctgctgt ttgttattgc atagtgaaag aatcctcctc cccagcacgg 1080
aagcctggga attctggaat atcaatgatg cagaagagag caaagttatc tcgaccacag 1140
cgtccagtgg aagaaaccaa aggagacaca ttcagtgatg ccagcattga tgtcaaattg 1200
tgcgagcagc cgcgggagaa aagacaactc aagagacagc tagccttctt cagttctgaa 1260
ctttctgaaa actctacttt tggtagtggc catgaactgt aa                   1302
```

# Fig. 43

Met Gln Ala Leu Asn Ile Thr Ala Glu Gln Phe Ser Arg Leu Leu Ser
5 10 15

Ala His Asn Leu Thr Arg Glu Gln Phe Ile His Arg Tyr Gly Leu Arg
20 25 30

Pro Leu Val Tyr Thr Pro Glu Leu Pro Ala Arg Ala Lys Val Ala Phe
35 40 45

Ala Leu Ala Gly Ala Leu Ile Phe Ala Leu Ala Leu Phe Gly Asn Ser
50 55 60

Leu Val Ile Tyr Val Val Thr Arg Ser Lys Ala Met Arg Thr Val Thr
65 70 75 80

Asn Ile Phe Ile Cys Ser Leu Ala Leu Ser Asp Leu Leu Ile Ala Phe
85 90 95

Phe Cys Ile Pro Val Thr Met Leu Gln Asn Ile Ser Asp Lys Trp Leu
100 105 110

Gly Gly Ala Phe Ile Cys Lys Met Val Pro Phe Val Gln Ser Thr Ala
115 120 125

Val Val Thr Glu Ile Leu Thr Met Thr Cys Ile Ala Val Glu Arg His
130 135 140

Gln Gly Leu Val His Pro Phe Lys Met Lys Trp Gln Tyr Thr Thr Arg
145 150 155 160

Arg Ala Phe Thr Ile Leu Gly Val Val Trp Leu Ala Ala Ile Ile Val
165 170 175

Gly Ser Pro Met Trp His Val Gln Arg Leu Glu Ile Lys Tyr Asp Phe
180 185 190

Leu Tyr Glu Lys Glu His Ile Cys Cys Leu Glu Glu Trp Ala Ser Pro
195 200 205

Val His Gln Arg Ile Tyr Ser Thr Phe Ile Leu Val Ile Leu Phe Leu
210 215 220

Leu Pro Leu Val Val Met Leu Val Leu Tyr Ser Lys Ile Gly Tyr Glu
225 230 235 240

Leu Trp Ile Lys Lys Arg Val Gly Asp Ser Ser Ala Leu Gln Thr Ile
245 250 255

His Gly Lys Glu Met Ser Lys Ile Ala Arg Lys Lys Lys Arg Ala Val
260 265 270

Ile Met Met Val Thr Val Val Ala Leu Phe Ala Ala Cys Trp Ala Pro
275 280 285

Phe His Val Val His Met Met Val Glu Tyr Ser Asn Phe Glu Lys Glu
290 295 300

Tyr Asp Asp Val Thr Ile Lys Met Val Phe Ala Val Ala Gln Thr Ile
305 310 315 320

Gly Phe Phe Asn Ser Ile Cys Asn Pro Phe Val Tyr Ala Phe Met Asn
325 330 335

Glu Asn Phe Lys Lys Asn Phe Leu Ser Ala Val Cys Tyr Cys Ile Val
340 345 350

Lys Glu Ser Ser Ser Pro Ala Arg Lys Pro Gly Asn Ser Gly Ile Ser
355 360 365

Met Met Gln Lys Arg Ala Lys Leu Ser Arg Pro Gln Arg Pro Val Glu
370 375 380

Glu Thr Lys Gly Asp Thr Phe Ser Asp Ala Ser Ile Asp Val Lys Leu
385 390 395 400

Cys Glu Gln Pro Arg Glu Lys Arg Gln Leu Lys Arg Gln Leu Ala Phe
405 410 415

Phe Ser Ser Glu Leu Ser Glu Asn Ser Thr Phe Gly Ser Gly His Glu
420 425 430

Leu End

Fig. 44

```
atgcaggcgc tcaacatcac cgcggagcag ttttcccggc tgctgagcgc gcacaacctg    60
actcgggaac agttcattca tcgctatggg ctgcgaccgc tggtctacac tccggagctg   120
cccgcgcgcg ctaaactggc ctttgcgctg gctggagcac tcatttttgc cctggcgctc   180
tttggcaact ctctggtcat ctatgtggtg acccgcagca aggccatgcg caccgtcacc   240
aacatcttca tctgctctct ggcactcagt gatctgctca ttgccttctt ctgcatcccc   300
gtcacgatgc tccagaacat ctccgacaag tggctgggtg gtgccttcat ctgcaagatg   360
gtgcccttcg tccagtccac tgctgttgtg acggaaatcc tcaccatgac ttgcatcgct   420
gttgagaggc accaaggact catccatcct tttaaaatga agtggcagta cactaccgga   480
agggctttca caatcttggg tgtggtctgg ttggcagcca tcatcgtagg atcacccatg   540
tggcacgtac aacgcctcga gattaagtat gacttcctct atgagaaaga acatgtctgc   600
tgtttggaag agtgggccag ccccatgcac cagagaatct acaccacctt catcctcgtc   660
atcctcttcc tcctgccgct tgtggtgatg cttgtcctct acagcaagat tggctatgaa   720
ctgtggatca agaagagagt tggagacagt tcagcacttc agactatcca cgggaaagaa   780
atgtccaaaa tagccaggaa gaagaagcgg gctgtcgtta tgatggtgac agtggtggct   840
ctcttcgctg cgtgctgggc acctttccat gttgttcaca tgatggttga gtacagtaac   900
tttgaaaaag agtatgatga tgtcacaatc aagatggttt ttgctgttgc acaaacaatt   960
ggctttttca actccatctg taatcccttt gtgtatgcat ttatgaatga aaacttcaaa  1020
aagaattttt tgtctgcggt ttgttattgc atagtaagag aaaccttctc cccaggacag  1080
aagcctggaa attctgggat ttcaatgatg caaaagagag caaagttatc acgatcacag  1140
cgtccagtgg cggaagccaa aggagactta ttcagcgatg ccaacgttga tgtcaaattg  1200
tgtgagcagc caggggagaa aaggcaactc aagcgacagc ttgccttctt tagttctgaa  1260
ctttctgaaa actctacttt cggcagtgga catgaactgt aatatcgatg a            1311
```

## Fig. 45

Met Gln Ala Leu Asn Ile Thr Ala Glu Gln Phe Ser Arg Leu Leu Ser
5 10 15

Ala His Asn Leu Thr Arg Glu Gln Phe Ile His Arg Tyr Gly Leu Arg
20 25 30

Pro Leu Val Tyr Thr Pro Glu Leu Pro Ala Arg Ala Lys Leu Ala Phe
35 40 45

Ala Leu Ala Gly Ala Leu Ile Phe Ala Leu Ala Leu Phe Gly Asn Ser
50 55 60

Leu Val Ile Tyr Val Val Thr Arg Ser Lys Ala Met Arg Thr Val Thr
65 70 75 80

Asn Ile Phe Ile Cys Ser Leu Ala Leu Ser Asp Leu Leu Ile Ala Phe
85 90 95

Phe Cys Ile Pro Val Thr Met Leu Gln Asn Ile Ser Asp Lys Trp Leu
100 105 110

Gly Gly Ala Phe Ile Cys Lys Met Val Pro Phe Val Gln Ser Thr Ala
115 120 125

Val Val Thr Glu Ile Leu Thr Met Thr Cys Ile Ala Val Glu Arg His
130 135 140

Gln Gly Leu Ile His Pro Phe Lys Met Lys Trp Gln Tyr Thr Thr Arg
145 150 155 160

Arg Ala Phe Thr Ile Leu Gly Val Val Trp Leu Ala Ala Ile Ile Val
165 170 175

Gly Ser Pro Met Trp His Val Gln Arg Leu Glu Ile Lys Tyr Asp Phe
180 185 190

Leu Tyr Glu Lys Glu His Val Cys Cys Leu Glu Glu Trp Ala Ser Pro
195 200 205

Met His Gln Arg Ile Tyr Thr Thr Phe Ile Leu Val Ile Leu Phe Leu
210 215 220

Leu Pro Leu Val Val Met Leu Val Leu Tyr Ser Lys Ile Gly Tyr Glu
225 230 235 240

Leu Trp Ile Lys Lys Arg Val Gly Asp Ser Ser Ala Leu Gln Thr Ile
245 250 255

His Gly Lys Glu Met Ser Lys Ile Ala Arg Lys Lys Lys Arg Ala Val
260 265 270

Val Met Met Val Thr Val Val Ala Leu Phe Ala Ala Cys Trp Ala Pro
275 280 285

Phe His Val Val His Met Met Val Glu Tyr Ser Asn Phe Glu Lys Glu
290 295 300

Tyr Asp Asp Val Thr Ile Lys Met Val Phe Ala Val Ala Gln Thr Ile
305 310 315 320

Gly Phe Phe Asn Ser Ile Cys Asn Pro Phe Val Tyr Ala Phe Met Asn
325 330 335

Glu Asn Phe Lys Lys Asn Phe Leu Ser Ala Val Cys Tyr Cys Ile Val
340 345 350

Arg Glu Thr Phe Ser Pro Gly Gln Lys Pro Gly Asn Ser Gly Ile Ser
355 360 365

Met Met Gln Lys Arg Ala Lys Leu Ser Arg Ser Gln Arg Pro Val Ala
370 375 380

Glu Ala Lys Gly Asp Leu Phe Ser Asp Ala Asn Val Asp Val Lys Leu
385 390 395 400

Cys Glu Gln Pro Gly Glu Lys Arg Gln Leu Lys Arg Gln Leu Ala Phe
405 410 415

Phe Ser Ser Glu Leu Ser Glu Asn Ser Thr Phe Gly Ser Gly His Glu
420 425 430

Leu End Tyr Arg End
435

# Fig. 46

```
1    MQALNI TPEQFSRLLRDHNLTREQFIALMRLRPLVYTPELPGRAKLALVL  A
1    MQALNI TAEQFSRLLSAHNLTREQFI HRYGLRPLVYTPELPARAKMAFAL  B
1    MQALNI TAEQFSRLLSAHNLTREQFI HRYGLRPLVYTPELPARAKLAFAL  C

51   TGVLI FALALFGNALVFYVVTRSKAMRTVTNI FI CSLALSDLLITFFCI P  A
51   AGALI FALALFGNSLVI YVVTRSKAMRTVTNI FI CSLALSDLLI AFFCI P  B
51   AGALI FALALFGNSLVI YVVTRSKAMRTVTNI FI CSLALSDLLI AFFCI P  C

101  VTMLQNI SDNWLGGAFI CKMVPFVQSTAVVTEI LTMTCI AVERHQGLVHP  A
101  VTMLQNI SDKWLGGAFI CKMVPFVQSTAVVTEI LTMTCI AVERHQGLVHP  B
101  VTMLQNI SDKWLGGAFI CKMVPFVQSTAVVTEI LTMTCI AVERHQGLI HP  C

151  FKMKWQYTNRRAFTMLGVVWLVAVI VGSPMWHVQQLEI KYDFLYEKEHI C  A
151  FKMKWQYTTRRAFTI LGVVWLAAI I VGSPMWHVQRLEI KYDFLYEKEHI C  B
151  FKMKWQYTTRRAFTI LGVVWLAAI I VGSPMWHVQRLEI KYDFLYEKEHVC  C

201  CLEEWTSPVHQKI YTTFI LVI LFLLPLMVMLI LYSKI GYELW KKRVGDG  A
201  CLEEWASPVHQRI YSTFI LVI LFLLPLVVMLVLYSKI GYELW KKRVGDS  B
201  CLEEWASPMHQRI YTTFI LVI LFLLPLVVMLVLYSKI GYELW KKRVGDS  C

251  SVLRTI HGKEMSKI ARKKKRAVI MMVTVVALFAVCWAPFHVVHMMI EYSN  A
251  SALQTI HGKEMSKI ARKKKRAVI MMVTVVALFAACWAPFHVVHMMVEYSN  B
251  SALQTI HGKEMSKI ARKKKRAVVMMVTVVALFAACWAPFHVVHMMVEYSN  C

301  FEKEYDDVTI KMI FAI VQI I GFSNSI CNPI VYAFMNENFKKNVLSAVCYC  A
301  FEKEYDDVTI KMVFAVAQTI GFFNSI CNPFVYAFMNENFKKNFLSAVCYC  B
301  FEKEYDDVTI KMVFAVAQTI GFFNSI CNPFVYAFMNENFKKNFLSAVCYC  C

351  I VNKTFSPAQRHGNSGI TMMRKKAKFSLRENPVEETKQEAFSDGNI EVKL  A
351  I VKESSSPARKPGNSGI SMMQKRAKLSRPQRPVEETKGDTFSDASI DVKL  B
351  I VRETFSPGQKPGNSGI SMMQKRAKLSRSQRPVAEAKGDLFSDANVDVKL  C

401  CEQTEEKKKLKRHLALFRSELAENSPLDSGH                       A
401  CEQPREKRQLKRQLAFFSSELSENSTFGSGHEL.                    B
401  CEQPGEKRQLKRQLAFFSSELSENSTFGSGHEL.                    C
```

FIG. 47

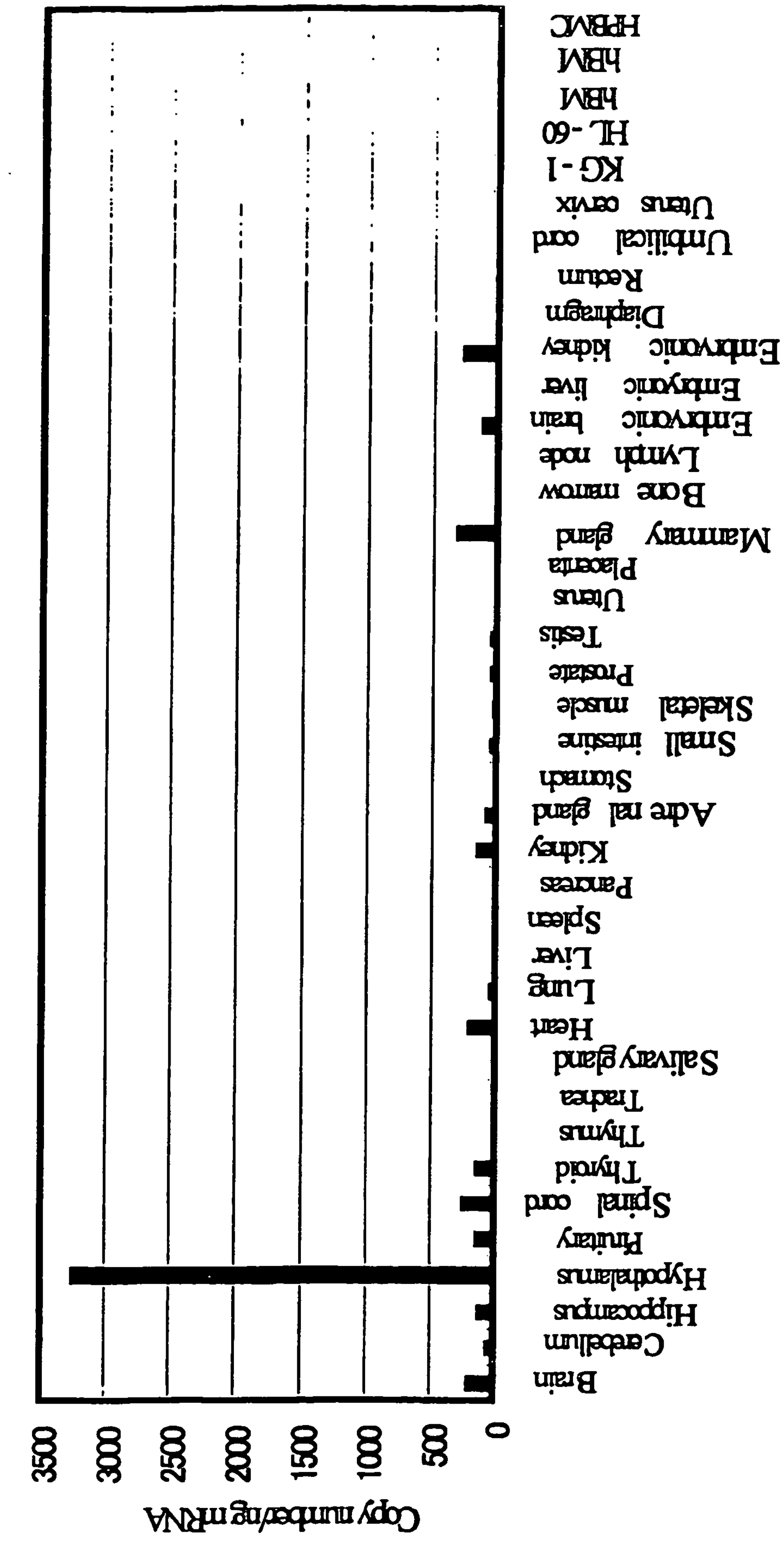

Copy number/ng mRNA
0
500
1000
1500
2000
2500
3000
3500
Brain
Cerebellum
Hippocampus
Hypothalamus
Pituitary
Spinal cord
Thyroid
Thymus
Trachea
Salivary gland
Heart
Lung
Liver
Spleen
Pancreas
Kidney
Adre nal gland
Stomach
Small intestine
Skeletal muscle
Prostate
Testis
Uterus
Placenta
Mammary gland
Bone marrow
Lymph node
Embryonic brain
Embryonic liver
Embryonic kidney
Diaphragm
Rectum
Umbilical cord
Uterus cervix
KG-1
HL-60
hBM
hBM
HPBMC

FIG. 48

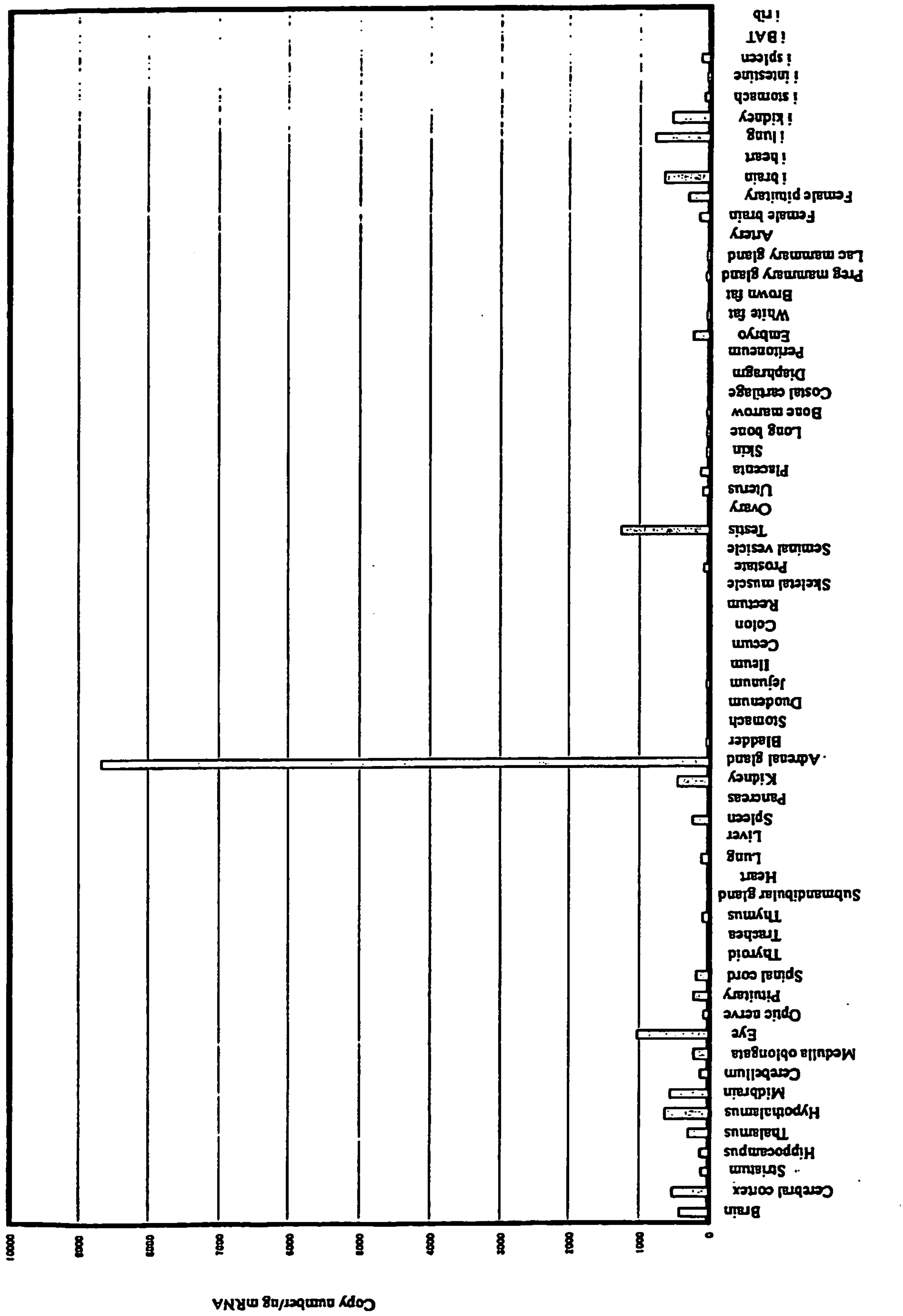
Brain
Cerebral cortex
Striatum
Hippocampus
Thalamus
Hypothalamus
Midbrain
Cerebellum
Medulla oblongata
Eye
Optic nerve
Pituitary
Spinal cord
Thyroid
Trachea
Thymus
Submandibular gland
Heart
Lung
Liver
Spleen
Pancreas
Kidney
Adrenal gland
Bladder
Stomach
Duodenum
Jejunum
Ileum
Cecum
Colon
Rectum
Skeletal muscle
Prostate
Seminal vesicle
Testis
Ovary
Uterus
Placenta
Skin
Long bone
Bone marrow
Costal cartilage
Diaphragm
Peritoneum
Embryo
White fat
Brown fat
Preg mammary gland
Lac mammary gland
Artery
Female brain
Female pituitary
i brain
i heart
i lung
i kidney
i stomach
i intestine
i spleen
i BAT
i rib
0
Copy number/ng mRNA

FIG. 49

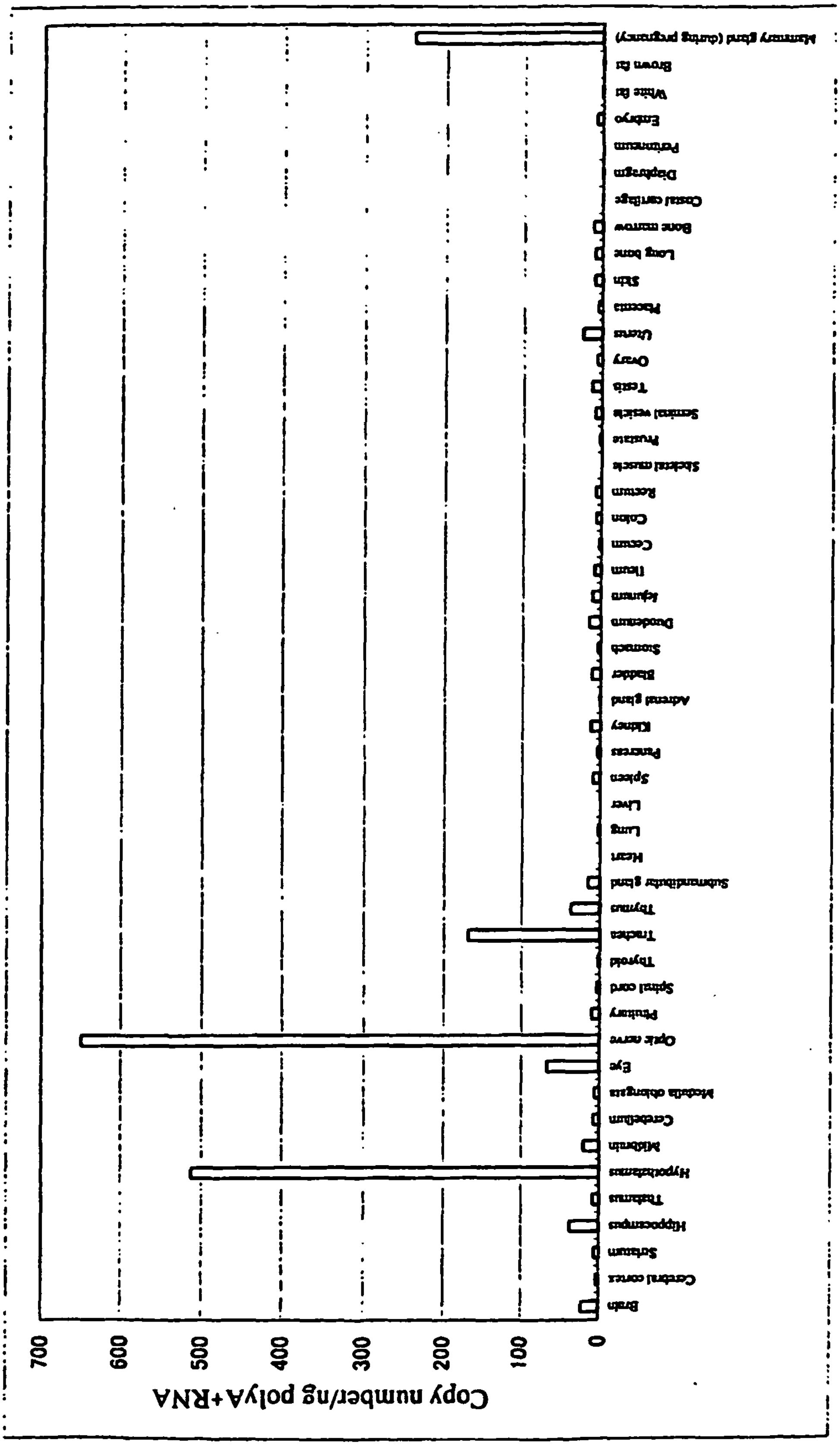
Copy number/ng polyA+RNA
0
100
200
300
400
500
600
700
Brain
Cerebral cortex
Striatum
Hippocampus
Thalamus
Hypothalamus
Midbrain
Cerebellum
Medulla oblongata
Eye
Optic nerve
Pituitary
Spinal cord
Thyroid
Trachea
Thymus
Submandibular gland
Heart
Lung
Liver
Spleen
Pancreas
Kidney
Adrenal gland
Bladder
Stomach
Duodenum
Jejunum
Ileum
Cecum
Colon
Rectum
Skeletal muscle
Prostate
Seminal vesicle
Testis
Ovary
Uterus
Placenta
Skin
Long bone
Bone marrow
Costal cartilage
Diaphragm
Peritoneum
Embryo
White fat
Brown fat
Mammary gland (during pregnancy)

FIG 50

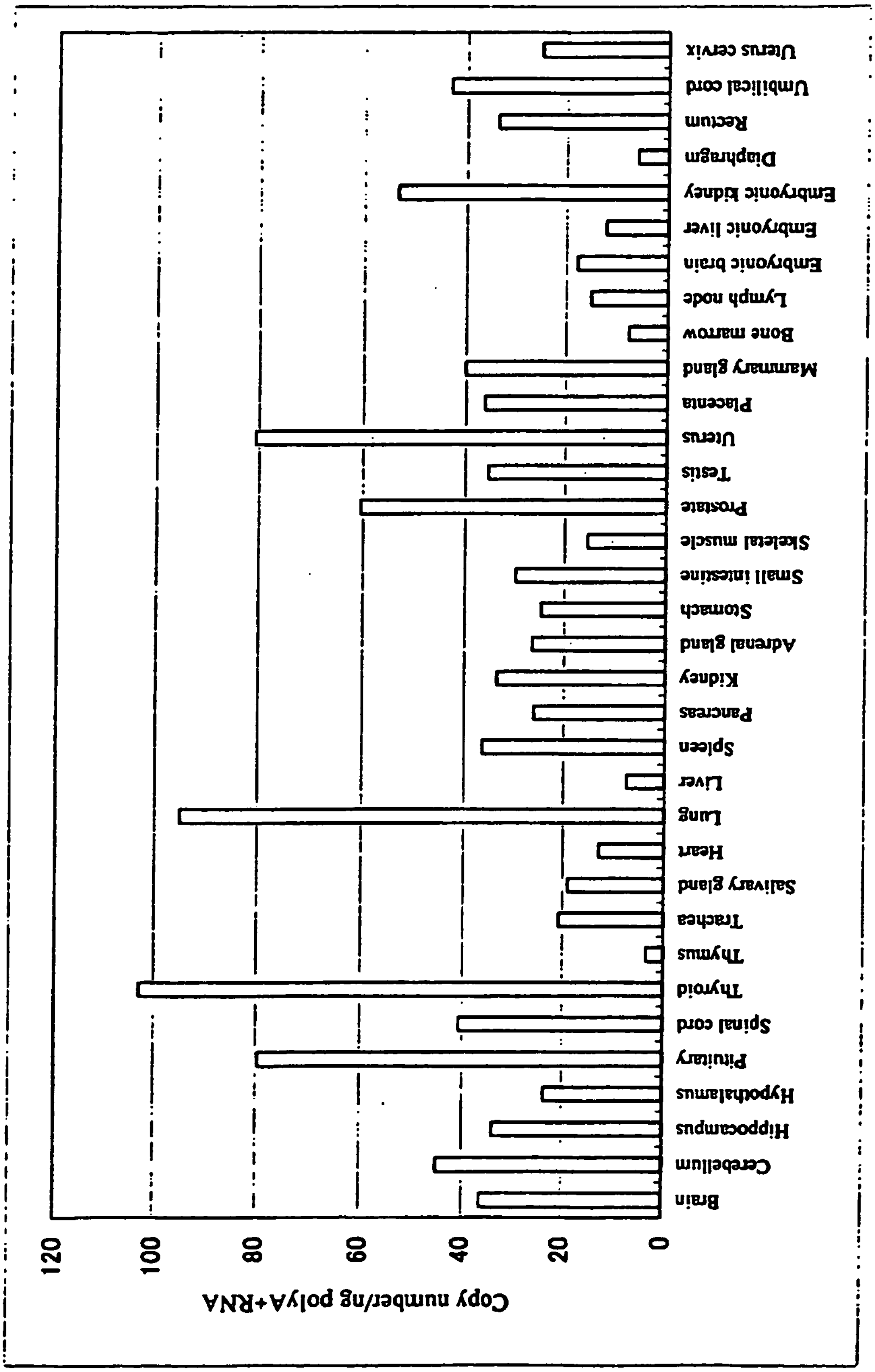
Copy number/ng polyA+RNA
0
20
40
60
80
100
120
Brain
Cerebellum
Hippocampus
Hypothalamus
Pituitary
Spinal cord
Thyroid
Thymus
Trachea
Salivary gland
Heart
Lung
Liver
Spleen
Pancreas
Kidney
Adrenal gland
Stomach
Small intestine
Skeletal muscle
Prostate
Testis
Uterus
Placenta
Mammary gland
Bone marrow
Lymph node
Embryonic brain
Embryonic liver
Embryonic kidney
Diaphragm
Rectum
Umbilical cord
Uterus cervix

# FIG. 51

| Abbreviations | Name | AQ27 | Secretory protein G |
|---|---|---|---|
| Pir | Piriform cortex | +++ | |
| CxA | Cortex-amygdara transition zone | +++ | |
| VP | Ventral pallidum | ++ | |
| BSTMA | Bed nucleus of the stria terminalis, medial division, anterior part | + | |
| BSTLP | Bed nucleus of the stria terminalis, lateral division, posterior part | + | |
| BSTLV | Bed nucleus of the stria terminalis, lateral division, ventral part | + | |
| LPO | Lateral preoptic area | ++ | |
| LVPO | Lateral-ventral periolivary nucleus | + | |
| ADP | Anterodorsal preoptic nucleus | + | |
| VMPO | Ventromedial hypothalamic nucleus, posterior part | + | |
| MPOM | Medial preoptic nucleus, medial part | + | |
| AHA | Anterior hypothalamic area, anterior part | + | |
| AHC | Anterior hypothalamic area, central part | + | |
| AHP | Anterior hypothalamic area, posterior part | + | |
| MCPO | Magnocellular preoptic nucleus | ++ | |
| PaAP | Paraventricular hypothalamus, anterior part | + | |
| PaDC | Paraventricular nucleus, dorsal cap | ++ | |
| PVA | Paraventricular thalamic nucleus, anterior part | + | |
| PVP | Paraventricular thalamic nucleus, posterior part | + | |
| VMHC | Ventromedial hypothalamic nucleus, central part | + | |
| VMHDM | Ventromedial hypothalamic nucleus, dorsomedial part | + | |
| VMHVL | Ventromedial hypothalamic nucleus, ventrolateral part | ++ | |
| ZI | Zona incerta | ++ | |
| RCh | Retrochiasmatic or anterobasal region | + | +++ |
| ArcD | Arcuate hypothalamic nucleus, dorsal part | + | ++ |
| ArcM | Arcuate hypothalamic nucleus, medial part | + | |
| ArcL | Arcuate hypothalamic nucleus, lateral part | + | + |
| ArcMP | Arcuate hypothalamic nucleus, medioposteror part | ++ | |
| PH | Posterior hypothalamic area | ++ | |
| LH | Lateral hypothalamic area | + | |
| MPT | Medial pretectal nucleus | + | |
| IGL | Intergeniculate leaf | +++ | |
| M1 | Primary motor cortex | + | |
| M2 | Secondary motor cortex | + | |
| Cg1 | Cingulate cortex area 1 | + | |
| Cg2 | Cingulate cortex area 2 | + | |
| MeAD | Medial amygdaloid nucleus | + | |
| MZMG | Marginal zone median geniculate | ++ | |
| DMPAG | Dorsomedial periaqueductal gray | ++ | |
| LPAG | Lateral periaqueductal gray | + | |
| VLPAG | Ventrolateral periaqueductal gray | + | |
| BIC | Nucleus of brachium inferior colliculus | ++ | |
| DRD | Dorsal raphe nucleus, dorsal part | +++ | |
| DRV | Dorsal raphe nucleus, ventrolateral part | +++ | |
| PPtg | Pedunculopontine tegmental nucleus | + | |
| DRC | Dorsal raphe nucleus, caudal part | +++ | |
| LDTg | Laterodorsal tegmental nucleus | ++ | |
| PPy | Parapyramidal nucleus | + | |
| LC | Locus coeruleus | +++ | |
| GGA | Central gray, alpha part | +++ | |
| CGB | Central gray, beta part | +++ | |
| Sol | Nucleus tractus solitarius | + | |

FIG. 52

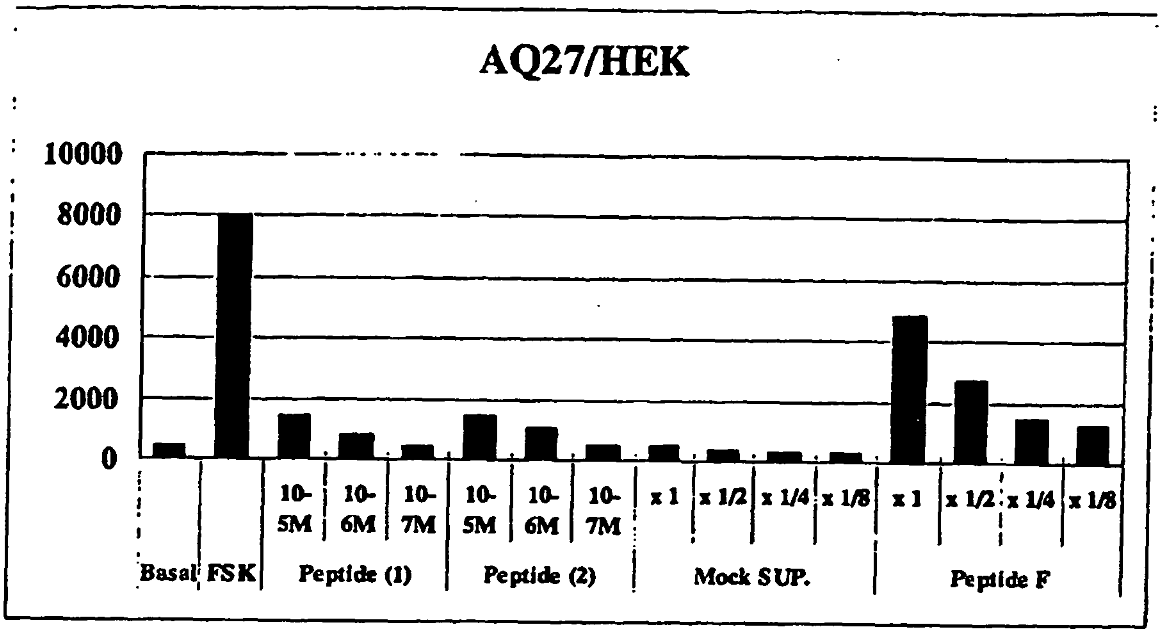
AQ27/HEK
10000
8000
6000
4000
2000
0
10-5M
10-6M
10-7M
10-5M
10-6M
10-7M
x 1
x 1/2
x 1/4
x 1/8
x 1
x 1/2
x 1/4
x 1/8
Basal
FSK
Peptide (1)
Peptide (2)
Mock SUP.
Peptide F

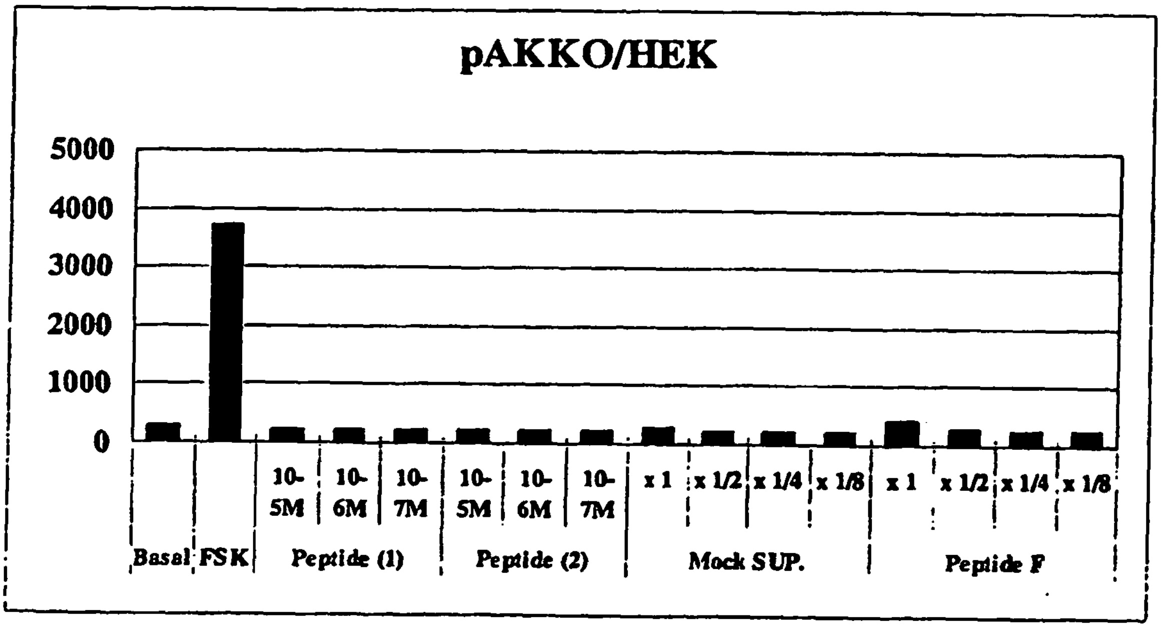
pAKKO/HEK
5000
4000
3000
2000
1000
0
10-5M
10-6M
10-7M
10-5M
10-6M
10-7M
x 1
x 1/2
x 1/4
x 1/8
x 1
x 1/2
x 1/4
x 1/8
Basal
FSK
Peptide (1)
Peptide (2)
Mock SUP.
Peptide F

Fig. 53

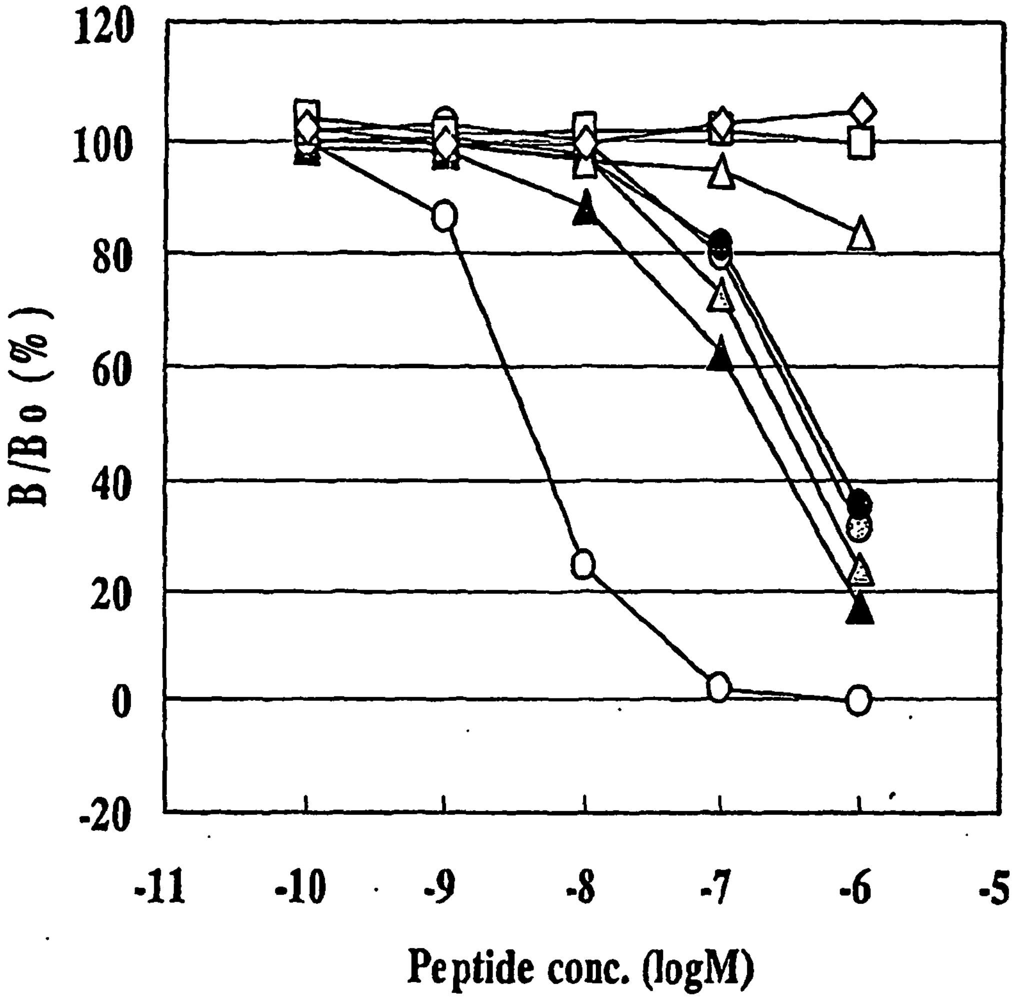

Fig. 54

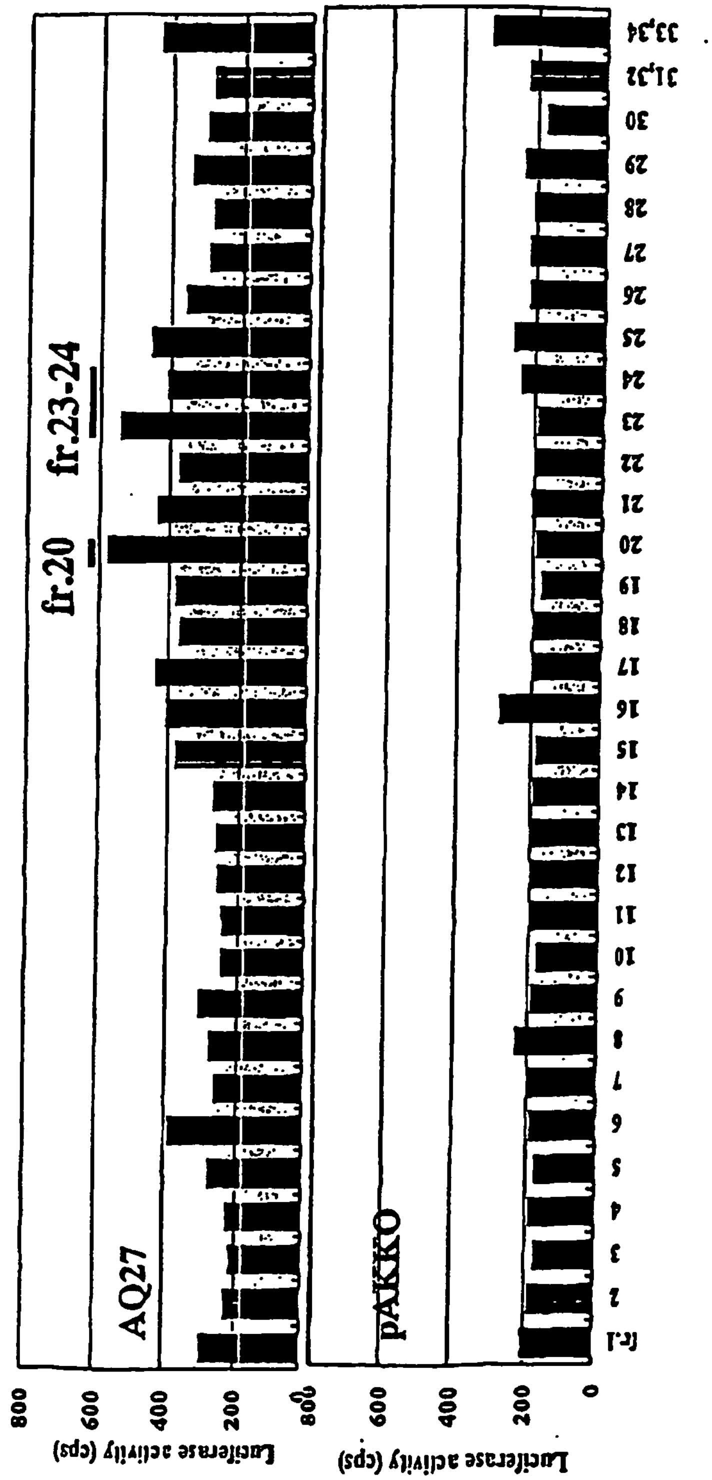
fr.20
fr.23-24
AQ27
pAKKO
Luciferase activity (cps)
Luciferase activity (cps)

Fig. 55

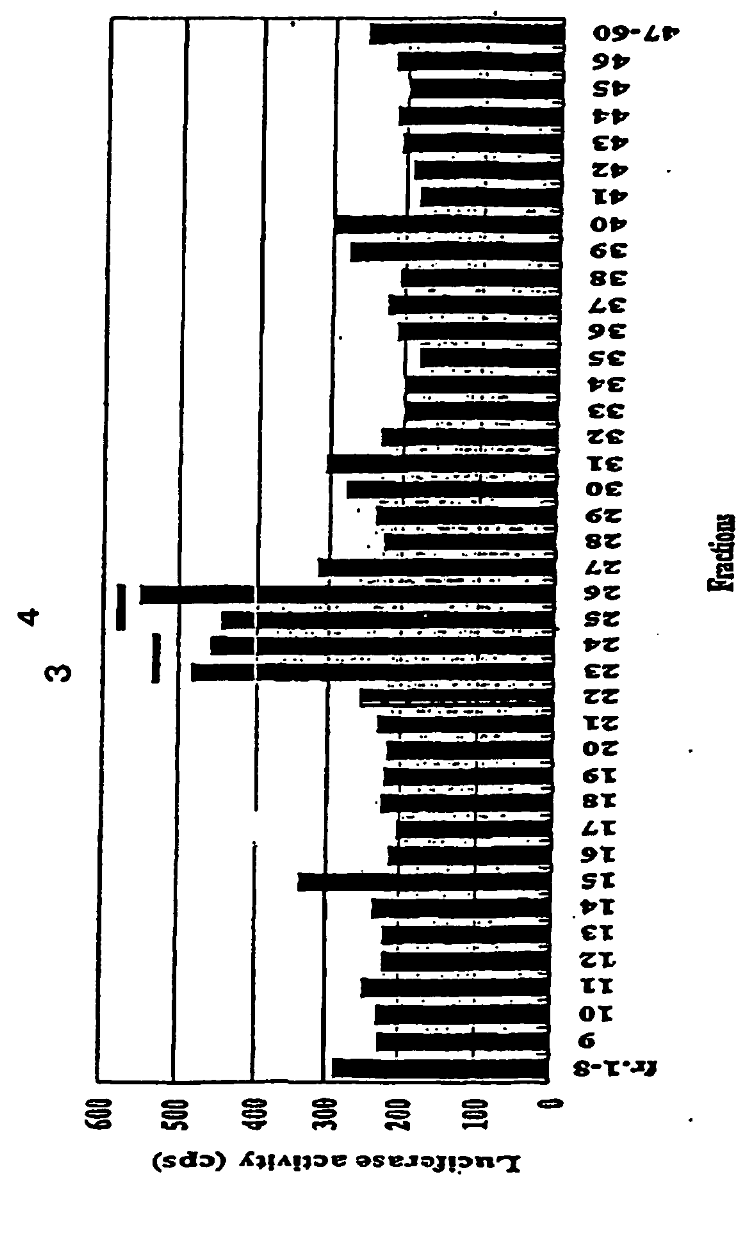
Luciferase activity (cps)
0
100
200
300
400
500
600
fr.1-8
9
10
11
12
13
14
15
16
17
18
19
20
21
22
23
24
25
26
27
28
29
30
31
32
33
34
35
36
37
38
39
40
41
42
43
44
45
46
47-60
Fractions
3
4

Fig. 56

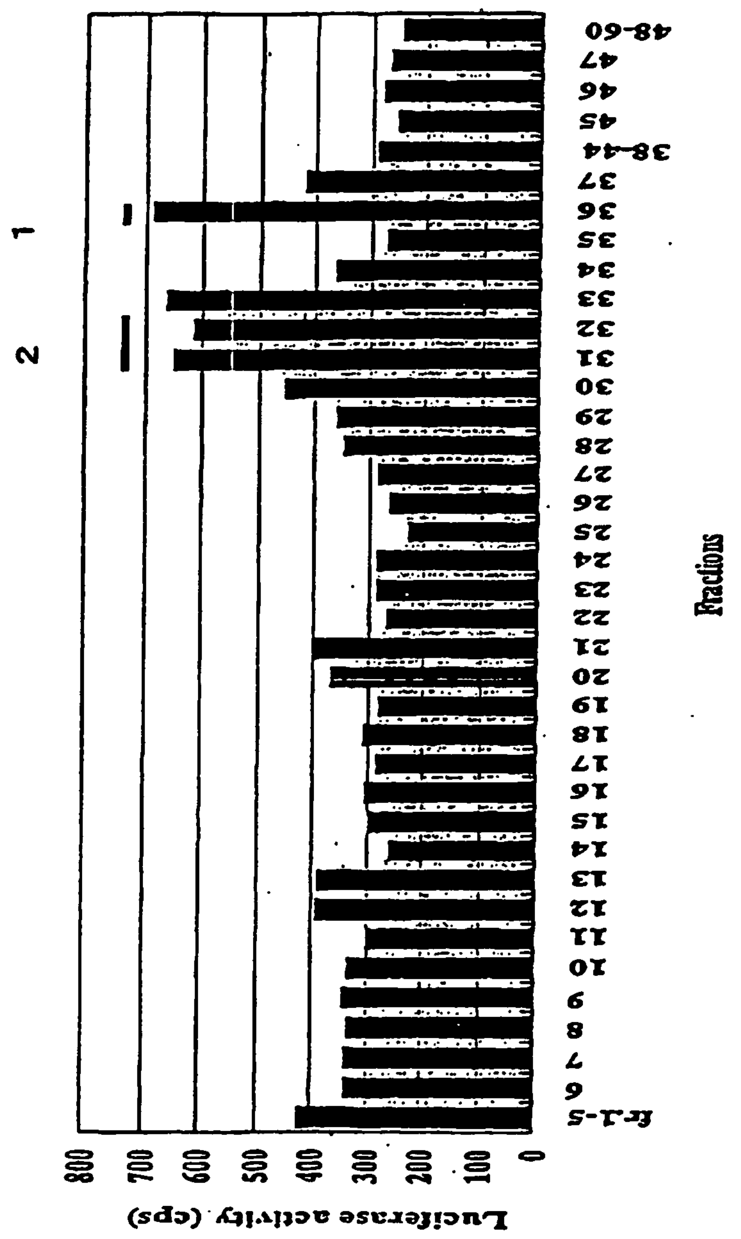
1
2
Luciferase activity (cps)
0
100
200
300
400
500
600
700
800
Fractions
fr-1-5
6
7
8
9
10
11
12
13
14
15
16
17
18
19
20
21
22
23
24
25
26
27
28
29
30
31
32
33
34
35
36
37
38-44
45
46
47
48-60

FIG. 57

| Peak number | Molecular weight Determined value/theoretical value | Amino acid sequence |
|---|---|---|
| 1: | 4505.7/ 4505.0 | Pyr-DEGSEATGFLPAAGEKTSGPLGNLAEELNGYSRKKGGFSFRF-$NH_2$ ( 43 amino acids ) |
| 2: | 4678.3/ 4678.2 | RQDEGSEATGFLPAAGEKTSGPLGNLAEELNGYSRKKGGFSFRF-$NH_2$ ( 44 amino acids ) |
| | 4277.9/ 4278.8 | EGSEATGFLPAAGEKTSGPLGNLAEELNGYSRKKGGFSFRF-$NH_2$ ( 41 amino acids ) |
| 3: | 3217.3/ 3218.6 | AGEKTSGPLGNLAEELNGYSRKKGGFSFRF-$NH_2$ ( 30 amino acids ) |
| 4: | 2731.9/ 2732.1 | SGPLGNLAEELNGYSRKKGGFSFRF-$NH_2$ ( 25 amino acids ) |

Fig. 58

| Name | Sequence | $EC_{50}$ (nM) |
|---|---|---|
| Tyr-AQ27L-43 | YQDEGSEATGFLPAAGEKTSGPLGNLAEELNGYSRKKGGFSFRF$_{NH2}$ | 3.2 |
| AQ27L-43 | pQDEGSEATGFLPAAGEKTSGPLGNLAEELNGYSRKKGGFSFRF$_{NH2}$ | 2.7 |
| AQ27L-26 | TSGPLGNLAEELNGYSRKKGGFSFRF$_{NH2}$ | 3.9 |
| AQ27L-26-OH | TSGPLGNLAEELNGYSRKKGGFSFRF$_{OH}$ | >1000 |
| AQ27L-23 | PLGNLAEELNGYSRKKGGFSFRF$_{NH2}$ | 8.9 |
| AQ27L-19 | LAEELNGYSRKKGGFSFRF$_{NH2}$ | 440 |
| AQ27L-15 | LNGYSRKKGGFSFRF$_{NH2}$ | 480 |
| AQ27L-N23 | pQDEGSEATGFLPAAGEKTSGPLGNLAEE | Not detected |
| AQ27L-15+AQ27L-N23 | pQDEGSEATGFLPAAGEKTSGPLGNLAEE + LNGYSRKKGGFSFRF$_{NH2}$ | 460 |
| AQ27L-10 | RKKGGFSFRF$_{NH2}$ | >1000 |
| AQ27L-8 | KGGFSFRF$_{NH2}$ | >1000 |
| AQ27L-7 | GGFSFRF$_{NH2}$ | >1000 |

Fig. 59

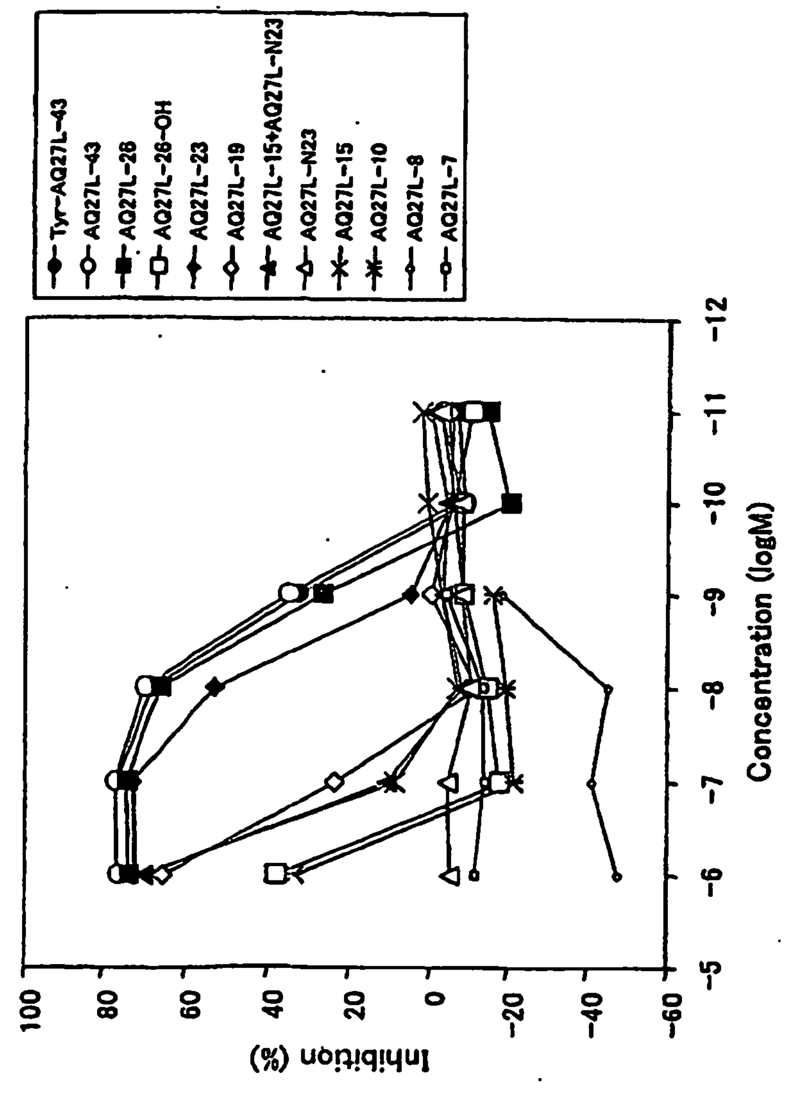
Tyr-AQ27L-43
AQ27L-43
AQ27L-26
AQ27L-26-OH
AQ27L-23
AQ27L-19
AQ27L-15+AQ27L-N23
AQ27L-N23
AQ27L-15
AQ27L-10
AQ27L-8
AQ27L-7
Inhibition (%)
100
80
60
40
20
0
-20
-40
-60
Concentration (logM)
-5
-6
-7
-8
-9
-10
-11
-12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO O116313 A
- WO 0029441 A
- WO 0166134 A
- WO 0116316 A
- WO 0116313 A


### Non-patent literature cited in the description


- **M. BODANSZKY ; M.A: ONDETTI.** Peptide Synthesis. Interscience Publishers, 1966
- **SCHROEDER ; LUEBKE.** The Peptide. Academic Press, 1965
- **NOBUO IZUMIYA et al.** Peptide Gosei-no-Kiso to Jikken. Mamzen Co, 1975
- Seikagaku Jikken Koza. **HARUAKI YAJIMA ; SHUNPEI SAKAKIBARA.** Tanpakushitsu no Kagaku. 1977, vol. IV, 205
- Zoku Iyakuhin no Kaihatsu. Peptide Synthesis. Hirokawa Shoten, vol. 14
- Shin PCR to Sonooyo. *Jikken Igaku,* 1997, vol. 15 (7
- **J. SAMBROOK et al.** Molecular Cloning. Cold Spring Harbor Lab. Press, 1989
- **J. KAWAKAMI et al.** *Pharm Tech Japan,* 1992, vol. 8, 247
- *PHARM TECH JAPAN,* 1992, vol. 8, 395
- Antisense Research and Applications. CRC Press, 1993
- *Proc. Natl: Acad. Sci. U.S.A.,* 1968, vol. 60, 160
- *Nucleic Acids Research,* 1981, vol. 9, 309
- *Journal of Molecular Biology,* 1978, vol. 120, 517
- *Journal of Molecular Biology,* 1969, vol. 41, 459
- *Genetics,* 1954, vol. 39, 440
- *Gene,* 1983, vol. 24, 255
- *Journal of Biochemistry,* 1984, vol. 95, 87
- **VAUGHN, J. L. et al.** *In Vivo,* 1977, vol. 13, 213-217
- **MAEDA et al.** *Nature,* 1985, vol. 315, 592
- *Proc. Natl. Acad. Sci. U.S.A.,* 1972, vol. 69, 2110
- *Gene,* 1982, vol. 17, 107
- *Molecular & General Genetics,* 1979, vol. 168, 111
- *Methods in Enzymology,* 1991, vol. 194, 182-187
- *Proc. Natl. Acad. Sci. U.S.A.,* 1978, vol. 75, 1929
- *Bio/Technology,* 1988, vol. 6, 47-55
- Saibo Kogaku. Shin Saibo Kogaku Jikken Protocol. Shujunsha, 1995, 263-267
- *Virology,* 1973, vol. 52, 456
- **MILLER.** Journal of Experiments in Molecular Genetics. Cold Spring Harbor Laboratory, 1972, 431-433
- **BOSTIAN, K. L. et al.** *Proc. Natl. Acad. Sci. U.S.A.,* 1980, vol. 77, 4505
- **BITTER, G. A. et al.** *Proc. Natl. Acad. Sci. U.S.A.,* 1984, vol. 81, 5330
- **GRACE, T. C. C.** *Nature,* 1962, vol. 195, 788
- *Science,* 1952, vol. 122, 501
- *Virology,* 1959, vol. 8, 396
- *The Journal of the American Medical Association,* 1967, vol. 199, 519
- *Proceeding of the Society for the Biological Medicine,* 1950, vol. 73, 1
- **KOEHLER ; MILSTEIN.** *Nature,* 1975, vol. 256, 495
- *Neuroendocrinology,* 1995, vol. 62, 198-206
- *Neuroscience Letters,* 1996, vol. 203, 164-170
- *Molecular Biotechnology,* 1999, vol. 13, 29-43
- Shin Seikagaku Jikken Koza. *Kakusan,* 84-103
- Radioimmunoassay. Kodansha, 1974
- Radioimmunoassay; Second Series. Kodansha, 1979
- Enzyme Immunoassay. Igaku Shoin, 1978
- Enzyme Immunoassay. Igaku Shoin, 1982
- Enzyme Immunoassay. Igaku Shoin, 1987
- Methods in Enzymology. vol. 70
- METHODS IN ENZYMOLOGY. vol. 73
- METHODS IN ENZYMOLOGY. vol. 74
- METHODS IN ENZYMOLOGY. vol. 84
- METHODS IN ENZYMOLOGY. vol. 92
- METHODS IN ENZYMOLOGY. Academic Press, vol. 121
- *Genomics,* 1989, vol. 5, 874-879

- *Proceedings of the National Academy of Sciences of the United States of America,* 1989, vol. 86, 2766-2770
- *Nature,* 2001, vol. 411, 494
- *TRENDS in Molecular Medicine,* 2001, vol. 7, 221
- **M. J. EVANS ; M. H. KAUFMAN.** *Nature,* 1981, vol. 292, 154
- **G. R. MARTIN.** *Proc. Natl. Acad. Sci. U.S.A.,* 1981, vol. 78, 7634
- **T. C. DOETSCHMAN et al.** *Journal of Embryology Experimental Morphology,* 1985, vol. 87, 27
- Enzynie Immunoassay. Igaku Shoin, 1982
- enzyme Immunoassay. Igaku Shoin, 1987
- Immuochemical Techniques (Part A. Methods in Enzymology. vol. 70
- Immunochemical Techniques (Part B. METHODS IN ENZYMOLOGY. vol. 73
- Immunochemical Techniques (Part C. METHODS IN ENZYMOLOGY. vol. 74
- Immunochemical Techniques (Part D: Selected Immunoassays. METHODS IN ENZYMOLOGY. vol. 84
- Immunochemical Techniques (Part E: Monoclonal Antibodies and General Immunoassay Methods. METHODS IN ENZYMOLOGY. vol. 92
- Immunochemical Techniques (Part I: Hybridoma Technology and Monoclonal Antibodies. METHODS IN ENZYMOLOGY. Academic Press, vol. 121
- **HINUMA, S. et al.** *Biochem. Biophys. Acta,* 1994, vol. 1219, 251-259